# EUROPEAN PATENT SPECIFICATION

(11) **EP 2 886 149 B1**
(45) Date of publication and mention of the grant of the patent: **10.10.2018**
(21) Application number: 13879599.2
(22) Date of filing: 16.08.2013
(51) Int. Cl.: A61M 21/02

(54) **VIBRATION PROCESSING DEVICE**
SCHWINGUNGSVERARBEITUNGSVORRICHTUNG
DISPOSITIF DE TRAITEMENT DE VIBRATION

(30) Priority: 16.08.2012 JP 2012180558
(43) Date of publication of application: 24.06.2015
(73) Proprietor: Action Research Co., Ltd., Tokyo 164-0003 (JP)
(72) Inventor: OOHASHI, Tsutomu, Tokyo 164-0003 (JP); KAWAI, Norie, Tokyo 164-0003 (JP); NISHINA, Emi, Tokyo 164-0003 (JP); HONDA, Manabu, Tokyo 164-0003 (JP); MAEKAWA, Tadao, Tokyo 164-0003 (JP); YAGI, Reiko, Tokyo 164-0003 (JP); UENO, Osamu, Tokyo 164-0003 (JP); FUKUSHIMA, Ariko, Tokyo 164-0003 (JP)
(74) Representative: Eisenführ Speiser
(86) International application number: PCT/JP2013/072031
(87) International publication number: WO 2014/027695

(56) References cited:
- EP-A1- 1 679 093
- JP-A- 2004 272 066
- JP-A- 2010 081 208
- JP-A- 2011 115 598
- US-A1- 2011 152 729

## Description

### TECHNICAL FIELD

The present invention relates to a vibration processing apparatus, capable of increasing or decreasing the activity of a brain (hereinafter also referred to as brain activity) of the fundamental brain including the brain stem, thalamus and hypothalamus, which are the regions that bear the fundamental functions of the brain of a living body and the inside and outside of the fundamental brain network of intra-cerebral projection based on the fundamental brain (they are hereinafter collectively referred to as a fundamental brain network system) by applying or removing vibrations containing predetermined frequency components to or from the living body.

### BACKGROUND ART

The present inventor and the others discovered that a hypersonic sound, which was a sound having a very unsteady structure abundantly containing super-high frequency components exceeding the audible frequency upper limit, induced a brain activity increasing effect of increasing the regional cerebral blood flow rate of specific brain regions of a human being listening to the sound, i.e., the fundamental brain including the brain stem, thalamus and hypothalamus and the fundamental brain network (fundamental brain network system) of intra-cerebral projection based on the fundamental brain and boosting the brain wave α wave power of an index of the fundamental brain activation, i.e., a hypersonic effect (See, for example, the Patent Documents 1 to 6 and the Non-Patent Documents 1 to 4).

Moreover, it was also discovered that the super-high frequency components applied to a human being in this phenomenon was received not from the airway auditory sense system but from the body surface to produce the effect (See the Non-Patent Document 7).

On the other hand, the sampling frequency of a general compact disc (CD) is 44.1 kHz in the digital audio field, and the reproducible frequency is limited up to 22.05 kHz. Moreover, the sampling frequency of digital broadcasting is 48 kHz, and the reproducible frequency is limited up to 24 kHz.

However, development of wideband audio of which the reproduction band is expanded to the super-high frequency region has been promoted by using optical discs as media with the discovery of the hypersonic effect by the present inventors as a momentum. The Super Audio CD (reproducible up to about 50 kHz) of which the sampling frequency was set to 2.8 MHz by the DSD system was put to practical use in the year 1999, and the DVD-Audio of which the sampling frequency was set to 96 kHz (reproducible up to 48 kHz) by the PCM system and also enabled the sampling frequency of 192 kHz (reproducible up to 96 kHz) as an option subsequently appeared in the same year. The Blu-ray (registered trademark) Disc, which was media for video use, appeared in the year 2002 with the 192-kHz sampling standard provided for the audio channel. These package media have not been popularized compared to CD for the reason that they need large initial investment and the other reasons. On the other hand, in the distribution field of music contents by the Internet with minor initial investment, distribution of the so-called high-resolution audio is getting on track, and various standards such as the sampling frequencies of 88.2 kHz, 96 kHz, 176.4 kHz and 192 kHz by the PCM system, and the sampling frequencies of 2.8 MHz and 5.6 MHz proposed by the DSD system appear, causing a chaotic situation with the opinion of the necessary and sufficient frequency undecided.

On the other hand, it has been discovered that a decrease in the brain activity is sometimes induced by applying a vibration by electroencephalogram measurement and cerebral blood flow measurement in the researches of the hypersonic effect conducted by the present inventor and the others (See the Non-Patent Document 1 etc.) Therefore, deliberate investigations are necessary as to whether super-high frequency components exceeding the audible frequency upper limit invariably have the brain activity increasing effect.

Moreover, in recent years, it has been discovered that super-high frequency components constituted of sine wave like signals having peaks at particular frequencies generated from artificial objects such as electronic equipment induce negative effect such as unpleasant sensations and escape behaviors of the human being or animal to which the components are applied, and they are used as young expellant apparatus, rat repulse apparatus and the like (See, for example, the Non-Patent Documents 5 and 6). It is considered that they impair the pleasant sensations and induce escape behaviors by suppressing the activity of the reward system neural circuit of the brain belonging to specific portions of the brain, and in particular, the fundamental brain network system.

The suppression of the activity of the fundamental brain network system as described above induces various mental and behavioral diseases, and induces failures of the homeostatic maintenance function and the biophylaxis function leading to the crises of lifestyle-related diseases. Therefore, the relationship between the super-high frequency components of aerial vibrations and the brain activity includes not only subjective impressions of sound in the audio field but also problems directly connected to the human health and medical treatment, more and more increasing the importance in applying the super-high frequency components to various fields of the society.

It is noted that the aforementioned brain activity collectively gives a generic name to an active state in which a huge amount of nerve cells existing in the brain portions perform activities including information propagation through generation of activity potential and emission of neurotransmitter and the like. By utilizing the property that the activity of the nerve cells and the cerebral blood flow of the portion are proportional to each other, the present inventor and the others observed the activities of the brain portions by measuring the blood flow (regional cerebral blood flow) of the brain portions by using a PET (positron emission tomography). In the present specification, a decrease in the regional cerebral blood flow is defined as a decrease in the activity of the portion, and an increase in the regional cerebral blood flow is defined as an increase in the activity of the portion. Further, the present inventor and the others have discovered that the cerebral blood flow of the fundamental brain constituted of the brain stem, thalamus and hypothalamus and the fundamental brain network of intra-cerebral projection based on the fundamental brain (i.e., the fundamental brain network system) is significantly related to the electroencephalogram data of specific band components derived from specific portions on the head skin (See the Non-Patent Documents 1 and 8) and have defined an index (deep index of brain activity) for estimating the activity of the fundamental brain network system from the electroencephalogram data by utilizing this (the Patent Documents 7 and 9). In the present specification, it is defined that a decrease in the activity of the fundamental brain network system is induced when the deep index of brain activity decreases, and an increase in the activity of the fundamental brain network system is induced when the deep index of brain activity increases.

The brain activity performs important works in maintaining the homeostasis of the whole body and maintaining the mental and physical health concurrently with governing various perceptions, ideas, emotions and actions of an animal. Since various portions of the brain have peculiar functions in a sharing manner, it is extremely important to appropriately increase or decrease the activities of various portions of the brain in appropriately controlling the human ideas, emotions and actions and treating and preventing various mental and physical decreases.

For example, the human fundamental brain network system includes important nerve systems such as the monoamine nerve system and the opioid nerve system having close relations to controlling the human emotions and actions. Therefore, it has been known that a malfunction in the activity of the fundamental brain network system induces various mental and behavioral diseases. Further, the fundamental brain is the highest center of the autonomic nerve system and the internal secretion system, and controls the immune system through them to bear a function to maintain the homeostasis of the whole body and the biophylaxis function through them. Therefore, the malfunction in the activity of the fundamental brain network system has close relations to the crises of lifestyle-related diseases that are rapidly increasing in the modern society by causing failures in the homeostasis maintenance function and the biophylaxis function. It is extremely important to appropriately increase or decrease the activity of the fundamental brain network system in treating and preventing the mental and physical diseases and conducting researches and development of them.

### PRIOR ART DOCUMENS

### PATENT DOCUMENTS

Patent Document 1: Japanese patent laid-open publication No. JP H09-313610 A;
Patent Document 2: Japanese patent No. JP 3933565B;
Patent Document 3: Japanese patent No. JP 4009660B;
Patent Document 4: Japanese patent No. JP 4009661B
Patent Document 5: Japanese patent laid-open publication No. JP 2005-111261 A;
Patent Document 6: Japanese patent laid-open publication No. JP 2002-015522 A;
Patent Document 7: International application publication No. WO2010/089911A;
Patent Document 8: JP 2003-223174 A; and
Patent Document 9: Japanese patent laid-open publication No. JP 4663034B.

### NON-PATENT DOCUMENTS

Non-Patent Document 2: Oohashi, T. et al., "Inaudible high-frequency sounds affect brain activity: hypersonic effect", Journal of Neurophysiology, Vol. 83, pp.3548-3558, June 2000;
Non-Patent Document 3: Oohashi T. et al., "High-Frequency Sound above the Audible Range Affects Brain Electric Activity and Sound Perception", Audio Engineering Society Preprint, 3207, October 1991;
Non-Patent Document 4: Tsutomu Ohashi, "Sound and civilization", Iwanami Shoten, pp.53-113, October 2003;
Non-Patent Document 5: Emi Nishina, "Progress of researches on development mechanism of hypersonic effect", Journal of Acoustical Society of Japan, Vol. 65, pp.40-45, January 2009;
Non-Patent Document 6: Kaoru Ashihara, "Actual condition survey of super-high frequency sound existing in the environment", Journal of Acoustical Society of Japan, Vol. 65, pp.23-28, January 2009;
Non-Patent Document 7: Tomomi Yamada, "super-high frequency sounds generated from dental instruments", Journal of Acoustical Society of Japan, Vol. 65, pp.52-57, January 2009;
Non-Patent Document 8: Oohashi T. et al., "The role of biological system other than auditory air-conduction in the emergence of the hypersonic effect", Brain Research, Vo1.1073-1074, pp.339-347, February 2006; and
Non-Patent Document 9: Sadato N. et al., "Neural networks for generation and suppression of alpha rhythm: a PET study", Neuro Report, No. 9, pp.893-897, March 1998. US 2011/0152729 discloses a vibration processing apparatus comprising vibration attenuator apparatuses and vibration presenting means.

This time, as described in detail later, the present inventor and the others have newly discovered that the effects exerted on the brain activity when super-high frequency components exceeding 16 kHz, or the upper limit of the frequency of aerial vibration perceivable as a sound by human beings are applied to living bodies are mutually reversed across the border of about 32 to 40 kHz. That is, it has been clarified that the brain activity increases when the super-high frequency components of 32 to 40 kHz and higher are applied together with audible range components of equal to or lower than 16 kHz in contrast to the fact that the brain activity decreases when the super-high frequency components ranging from 16 kHz to 32-40 kHz are applied together with the audible range components of equal to or lower than 16 kHz. In addition, it has also been clarified that the degree continuously changes depending on the frequency of the applied super-high frequency.

On the basis of such a knowledge newly discovered this time, a decrease in the brain activity and accompanying harmful phenomena occurring when the super-high frequency ranging from 16 kHz to 32-40 kHz, or the upper limit of the frequency of the vibration components perceivable as sounds by the human auditory system is presented, i.e., diversified harmful effects on both the mental and physical aspects, such as a decrease in the regional cerebral blood flow of the fundamental brain network system, attenuation of the brain wave α-wave, a decrease in the immune activity, an increase in the stress hormones, deterioration of sensitivity to audiovisual inputs, evasive actions to sounds, and a decline in the cognitive function, has been named a hypersonic negative effect. Then, the hypersonic effect, which is a profitable effect produced by the super-high frequency components exceeding the audible range upper limit and has been clarified so far, is determined to be called a hypersonic positive effect when necessary, and distinguished from the hypersonic negative effect.

In addition, the present inventor and others, have discovered that the super-high frequency components causing the hypersonic positive effect are mainly the components of the band of equal to or higher than 40 kHz, and the effect is remarkable in particular in a band of 80 kHz to 96 kHz, and possibly appears when the components of equal to or higher than 112 kHz are applied.

In order to record and reproduce the band of 80 kHz to 96 kHz in particular effective for developing such a hypersonic positive effect, a sampling frequency of equal to or higher than 192 kHz is required according to the standard of the PCM system. Further, according to the DSD system of which the sampling frequency is 2.8 MHz, there is the existing problem that the theoretically unavoidable quantization noises significantly contaminate the in particular effective band of equal to or higher than 50 kHz in developing the hypersonic positive effect.

This discovery by the present inventor and others has clarified that in particular the conventional digital audio standard includes extremely serious problems from the viewpoint of increasing and decreasing the brain activity due to vibration information, and not only the CD, SACD, DVD Audio and MP3 but also the current so-called high-resolution audio, which can record and reproduce the super-high frequency components exceeding the audible range upper limit, need a basal review.

First of all, according to the standards of CD (reproducible up to 22.05 kHz) currently internationally widespread, digital broadcasting (reproducible up to 24 kHz) and the like, the super-high frequency components of about 32 to 40 kHz and higher that induce the hypersonic positive effect cannot be recorded and reproduced. Therefore, opportunities to enjoy various utility effects of an increase in the beauty and pleasure of audio-visual information, enhancement of healing and pleasant sensation, improvement of acknowledgment function, improvement of mental and physical functions, included in the hypersonic positive effect, are abandoned.

Further serious problems, caused by the containment of part of the super-high frequency components of equal to or higher than 16 kHz and equal to or lower than 32 kHz decreasing the brain activity, are unignorable risks that the attenuation of the beauty and pleasure of audio-visual information, attenuation in healing and pleasant sensation, degradation of the acknowledgment function, degradation of mental and physical functions, and degradation of the protective power to the lifestyle-related diseases, mental illness and the like, which are included in the hypersonic positive effect developed by them, are invited.

Furthermore, these currently dominant digital audio standards of, for example, the broadcasting standard provide no other realistic choices for the users who are in a state substantially compelled to use them. A possibility that these standards currently popularized in the human society have relations to the mental and physical disorders named the modern disease related to a decrease in the brain activity propagating in the modern society cannot be denied.

The problem caused by such a hypersonic negative effect is not only unsolved by many digital audio standards currently used as high-resolution audio capable of recording and reproducing super-high frequency components exceeding the audible range upper limit but also possibly causes a more serious problem depending on the standard.

That is because the standard of the sampling frequency of equal to or lower than 96 kHz of the PCM system among the standards currently generally popularized as high-resolution audio can record and reproduce only the frequency components of equal to or lower than 48 kHz. Therefore, even when these standards are used, the band components of equal to or higher than 48 kHz capable of remarkably generating the hypersonic positive effect can neither be recorded nor reproduced.

On the other hand, according to the aforementioned standard of high-resolution audio, it is possible to more predominantly record and reproduce the components ranging from 16 kHz up to about 32 to 40 kHz that generate the hypersonic negative effect by comparison to the conventional digital audio standards of lower sampling frequencies of 44.1 kHz and 48 kHz, consequently allowing a more remarkable negative effect to be induced. The high-resolution audio developed for improving the sound quality cannot achieve its purpose and also decreases the activity of the fundamental brain network system, and this requires supposition of the possibility of inducing development of various negative effects.

### DISCLOSURE OF INVENTION

Therefore, the first object of the present invention is to provide a vibration processing apparatus including a vibration presenting apparatus, which can improve the mental and physical functions and exalt the reactions of beauty and pleasure by preventing the mental and physical diseases by attenuating the hypersonic negative effect induced by a decrease in the brain activity unavoidably developed by the digital audio standards that are currently generally popularized and inducing hypersonic positive effect by increasing the brain activity.

On the other hand, the techniques of, for example, craniotomy procedures, chemical substances, electromagnetic stimulations, and conditioning reflections, which have been used as techniques for conventionally artificially increasing or decreasing the brain activity, have respective large limitations. For example, the technique of destroying brain tissues by a craniotomy procedure and artificially decreasing their functions, which gives very large invasions to animals, therefore disadvantageously causes various malfunctions in the whole body besides the brain activity served as the target. Moreover, an increase or a decrease in the brain activity by a chemical substance induces complicated interactions with various chemical substances existing in the body, and this leads to causing side effects and resistance properties. Further, the method of increasing or decreasing the activity by applying a load to a specific brain region by applying an intense electrical stimulation or highly frequent magnetic stimulations to the specific brain region highly possibly causes irreversible changes in the nerve system. Operant conditioning to repeat listening to a sound while giving an intense stress of electroshock or the like and changing the brain activity by subsequently listening to the sound requires a long term, and the response of the living body irreversibly changes and is almost unable to be restored to the original normal response.

Difficulties and restrictions observed in various techniques that have conventionally been used for increasing or decreasing the brain activity become further serious problems in social applications and clinical studies intended for human beings. In particular, it is keenly demanded to make a pathological model of the modern decreases and a remedial model therefor by artificially performing activity regulations of the fundamental brain network system that is closely related to the crisis of modern diseases.

Conventionally, it is not only systematically impossible but also difficult from humane and ethical viewpoints to artificially make a pathological model intended for human beings by the methods of the craniotomy procedures, administration of chemical substances, intense electromagnetic stimulations, the conditioning to give excessive stresses and so on as those conducted with laboratory animals. Above all, since various life support functions are concentrated on the fundamental brain, applying a direct treatment or an indirect treatment currently known to it is accompanied by serious risks.

Therefore, since no technology to appropriately increases or decreases the human brain activity has been developed under the present situation, clinical studies using an effective pathological model or a remedial model are practically impossible. In order to safely efficiently make a "pathological model" or "remedial model" using animals and human beings to develop treatment methods and treatment medicines for various diseases, it is necessary to establish a technique for freely increasing or decreasing the brain activity by a method that is noninvasive, reversible and free of side effects.

Moreover, by generating pleasant and unpleasant sensations in human beings and animals other than human beings, the emotion system nerve circuit included in the fundamental brain network system has the effect of inducing actions of the human beings and animals getting closer to specific stimulations and objectives or conversely evasive actions getting away from specific stimulations and objectives. Although it becomes possible to safely guide human beings and animals by information so that they can select appropriate actions depending on the situations if a technology to increase or decrease the brain activity of the fundamental brain network system without using medicines, there is no such technology currently observed.

Further, it has been known that a decrease in the activity of the fundamental brain decreases the pleasant sensation and aesthetic sensitivity through the decrease in the activity of the emotion system nerve circuit, and induces decreases in various life support functions concentrated on the fundamental brain, consequently inducing serious diseases. Therefore, if the activity of the fundamental brain network system can be artificially increased by a method that is noninvasive, reversible and free of side effects for a human being in a state in which the activity of the fundamental brain network system decreases, it becomes possible to prevent various modern diseases and to provide effective means for increasing the pleasant sensation and aesthetic sensitivity, exhaling the reactions of pleasure, beauty and emotion to the artistic productions including musics, and enhancing the expressive effects. However, it is unrealistic to artificially increase or decrease the activity of the fundamental brain network system by using the conventional, irreversible techniques including the chemical substances and craniotomy procedures since they have the problems described above. Therefore, it is a present situation that no effective measures can be taken for the decrease in the activity of the fundamental brain network system.

The second object of the present invention is to solve the aforementioned problems and provide a vibration processing apparatus capable of decreasing or increasing the brain activity by a noninvasive, reversible method free of side effects without using, for example, craniotomy procedures, chemical substances, electromagnetic stimulations, conditioning reflections and so on, which have been used as conventional techniques to artificially increase or decrease the brain activity.

### MEANS FOR SOLVING THE PROBLEMS

The present inventor and others have discovered a principle that makes it possible to variously increase or decrease the activity of the inside and outside of the fundamental brain by applying or removing super-high frequency vibrations that have various frequency bands and intensities with various temporal organizations while applying vibrations including the frequency band perceived as a sound from the auditory system. Hereinafter, means for solving the problems will be described below.

According to the present invention, there is provided a vibration processing apparatus as defined in claim 1.

The above-mentioned vibration processing apparatus includes vibration presenting means configured to apply the vibration to the living body.

Also described is a vibration processing apparatus including generating means configured to eliminate, limit or attenuate a vibration or an oscillation signal that includes a second band including vibration components exhibiting an effect of lowering brain activity, from vibration components from among bands beyond a first band including vibration components perceived as sound by an auditory system of a living body.

The above-mentioned vibration processing apparatus further includes vibration presenting means configured to applying a vibration obtained by eliminating, limiting or attenuating components of the second band, to the living body.

Also described is a vibration processing apparatus including generating means configured to generate a vibration or an oscillation signal, that includes a first band including vibration components perceived as sound by an auditory system of a living body, and a third band including vibration components exhibiting an effect of increasing brain activity from among bands beyond the first band.

The above-mentioned vibration processing apparatus further includes vibration presenting means configured to apply the vibration of the third band to the living body together with the vibration of the first band, and inhibit or limit application of vibration of a second band to the living body, the second band including vibration components exhibiting an effect of lowering the brain activity from among bands beyond the first band.

Also described is a vibration processing apparatus including generating means configured to generate a vibration or an oscillation signal, that includes a first band including vibration components perceived as sound by an auditory system of a living body, a second band including vibration components exhibiting an effect of lowering brain activity from among bands beyond the first band, and the third band including vibration components increasing the brain activity from among bands beyond the first band.

The above-mentioned vibration processing apparatus further includes vibration presenting means configured to apply the vibration of the second band and the vibration of the third band to the living body, simultaneously, together with the vibration of the first band.

In addition, the above-mentioned vibration processing apparatus further includes vibration presenting means configured to apply at least one of the vibration of the second band and the vibration of the third band to the living body together with the vibration of the first band, to present the vibrations for setting degree of decrease or degree of increase of brain activity level of the living body to a predetermined value.

Further, the above-mentioned vibration processing apparatus further includes vibration presenting means configured to change an intensity of at least one of the vibration of the second band applied to the living body and the vibration of the third band applied to the living body, to present the vibrations for setting degree of decrease or degree of increase of brain activity level of the living body to a predetermined value.

Still further, the above-mentioned vibration processing apparatus further includes vibration presenting means configured to apply the vibration of the second band to the living body together with the vibration of the first band, and thereafter, apply the vibration of the third band to the living body together with the vibration of the first band.

Still further, the above-mentioned vibration processing apparatus further includes vibration presenting means configured to apply the vibration of the third band to the living body together with the vibration of the first band, and thereafter, apply the vibration of the second band to the living body together with the vibration of the first band.

In addition, the above-mentioned vibration processing apparatus further includes a signal processing apparatus configured to band-convert or band-expand a signal representing the vibration of the second band into a signal representing the vibration of the third band.

Further, the above-mentioned vibration processing apparatus further includes an encoder apparatus, and a decoder apparatus. The encoder apparatus is configured to band-convert or band-compress a signal representing the vibration of the third band into a signal representing the vibration of the second band, and thereafter, add a signal representing the vibration of the first band to a processed signal to output a signal of addition result as a transmission signal. The decoder apparatus is configured to receive the transmission signal,
(a) band-convert a signal having the second band into a signal having the third band from among the transmission signal when the transmission signal is band-converted, and
(b) band-expand a signal having the second band into a signal having the third band from among the transmission signal when the transmission signal is band-compressed, and thereafter,
add the signal having the first band from among the transmission signal to a processed signal to output a signal of addition result.

Still further, the above-mentioned vibration processing apparatus further includes means configured to detect a vibration level of at least one of the vibration of the second band and the vibration of the third band.

Still further, in the above-mentioned vibration processing apparatus, the vibration presenting means presents the vibration for setting a degree of decrease or a degree of increase of brain activity level of the living body to a predetermined value, based on a control signal from an external apparatus.

In the above-mentioned vibration processing apparatus, the control signal from the external apparatus is a control signal of a physiological index representing the degree of brain activity measured from the living body, and the vibration presenting means sets the brain activity of the living body within a predetermined range.

In addition, in the above-mentioned vibration processing apparatus, the control signal from the external apparatus is a control signal representing a number of living bodies in a predetermined area, and the vibration presenting means presents the vibration for setting a degree of decrease or a degree of increase of brain activity level of the living body to a predetermined value so as to adjust a number of living bodies in the area within a predetermined range.

Further, in the above-mentioned vibration processing apparatus, the control signal from the external apparatus is a control signal representing a number of living bodies in a plurality of predetermined areas, and the vibration presenting means presents the vibration for setting a degree of decrease or a degree of increase of brain activity level of the living body to such a predetermined value that respective numbers of living bodies in the areas have a predetermined ratio of them.

In the above-mentioned vibration processing apparatus, the first band is at least one part of bands equal to or lower than 20 kHz.

In addition, in the above-mentioned vibration processing apparatus, the second band is a band that includes at least a band of 16 kHz to 32 kHz, and is at least one part of bands of 16 kHz to 32 kHz, 40 kHz or 48 kHz.

Further, in the above-mentioned vibration processing apparatus, the third band is a band equal to or higher than the second band, and the second band includes at least a band of 16 kHz to 32 kHz, and is at least one part of bands of 16 kHz to 32 kHz, 40 kHz or 48 kHz.

Also described is a vibration presenting space apparatus including one of following: means configured to project the vibrations, that are presented by the vibration processing apparatus provided in the vibration presenting space apparatus configured to form a vibration presenting space, into the vibration presenting space apparatus; means configured to add or interfere the vibrations to each other in the vibration presenting space apparatus; and means configured to resonate an object configuring the vibration presenting space apparatus with the vibrations. This leads to a negative effect of lowering the brain activity of the living body within the vibration presenting space.

Also described is a vibration presenting space apparatus including one of following: means configured to project the vibrations, that are presented by the vibration processing apparatus provided in the vibration presenting space apparatus configured to form a vibration presenting space, into the vibration presenting space apparatus; means configured to add or interfere the vibrations to each other in the vibration presenting space apparatus; and means configured to resonate an object configuring the vibration presenting space apparatus with the vibrations. This leads to a positive effect of increasing the brain activity of the living body within the vibration presenting space.

Also described is a vibration presenting space apparatus including one of following: means configured to project the vibrations, that are presented by the vibration processing apparatus provided in the vibration presenting space apparatus configured to form a vibration presenting space, into the vibration presenting space apparatus; means configured to add or interfere the vibrations to each other in the vibration presenting space apparatus; and means configured to resonate an object configuring the vibration presenting space apparatus with the vibrations. This leads to increase or decrease of the brain activity of the living body within the vibration presenting space.

Also described is a vibration signal including a vibration signal of a second band including vibration components exhibiting an effect of lowering brain activity, from among bands beyond a first band including vibration components perceived as sound by an auditory system of a living body.

Also described is a vibration signal including a vibration signal of a third band including vibration components exhibiting an effect of increasing brain activity, from among bands beyond a first band including vibration components perceived as sound by an auditory system of a living body.

Also described is a vibration signal including a vibration signal, that includes a second band including vibration components exhibiting an effect of lowering brain activity, from among bands beyond a first band including vibration components perceived as sound by an auditory system of a living body; and a third band including vibration components exhibiting an effect of increasing the brain activity, from among bands beyond the first band.

Also described is a vibrating body having a vibration state vibrating at a super-high frequency band including a vibration signal of a second band including vibration components exhibiting an effect of lowering brain activity, from among bands beyond a first band including vibration components perceived as sound by an auditory system of a living body.

Also described is a vibrating body having a vibration state vibrating at a super-high frequency band including a vibration signal of a third band including vibration components exhibiting an effect of increasing brain activity, from among bands beyond a first band including vibration components perceived as sound by an auditory system of a living body.

Also described is a vibrating body having a vibration state vibrating at a super-high frequency band including a vibration signal, that includes a second band including vibration components exhibiting an effect of lowering brain activity, from among bands beyond a first band including vibration components perceived as sound by an auditory system of a living body; and a third band including vibration components exhibiting an effect of increasing the brain activity, from among bands beyond the first band.

Also described is a recording medium reproducible by a reproducing apparatus, the recording medium configured to store a vibration signal, that is generated or represented by the vibration processing apparatus, and can be reproduced by the reproducing apparatus.

Also described is a recording medium reproducible by a computer, the recording medium configured to store a vibration signal, that is generated or represented by the vibration processing apparatus, and can be reproduced by the computer.

Also described is a vibration processing method including a step of generating a vibration or an oscillation signal, that includes a first band including vibration components perceived as sound by an auditory system of a living body, and a second band including vibration components exhibiting an effect of lowering brain activity from among bands beyond the first band.

The above-mentioned vibration processing method further includes a step of applying the vibration of the second band to the living body, together with the vibration of the first band, and this leads to lowing the brain activity of the living body.

Also described is a vibration processing method including a step of eliminating, limiting or attenuating components of a second band from a vibration or an oscillation signal that includes a second band including vibration components exhibiting an effect of lowering brain activity, from among bands beyond a first band including vibration components perceived as sound by an auditory system of a living body.

The above-mentioned vibration processing method further includes a step of applying a vibration obtained by eliminating, or attenuating components of the second band, to the living body, and this leads to reducing or suppressing decrease of the brain activity of the living body, or increasing the brain activity of the living body.

Also described is a vibration processing method including a step of generating a vibration or an oscillation signal, that includes a first band including vibration components perceived as sound by an auditory system of a living body, and a third band including vibration components exhibiting an effect of increasing brain activity from among bands beyond the first band.

The above-mentioned vibration processing method further includes a step of applying the vibration of the third band to the living body together with the vibration of the first band, and inhibiting or limiting application of vibration of a second band to the living body, and this leads to increasing the brain activity of the living body.

Also described is a vibration processing method including a step of generating a vibration or an oscillation signal, that includes a first band including vibration components perceived as sound by an auditory system of a living body, a second band including vibration components exhibiting an effect of lowering brain activity from among bands beyond the first band, and the third band including vibration components increasing brain activity from among bands beyond the first band.

The above-mentioned vibration processing method further includes a step of applying the vibration of the second band and the vibration of the third band to the living body, simultaneously, together with the vibration of the first band, and this leads to increasing or lowing the brain activity of the living body.

### EFFECTS OF THE INVENTION

The present inventor and others have discovered such a principle that it becomes possible to variously increase or decrease the activity of the inside and outside of the fundamental brain by applying or removing super-high frequency vibrations that have various frequency bands and intensities with various temporal organizations to or from a living body while applying vibrations including the frequency band perceived as a sound from the auditory system. An effect of producing an operation to increase the brain activity (hereinafter, also referred to as a positive effect) is provided in particular when the vibration having the aforementioned third band is applied to the living body together with the vibration of the aforementioned first band, and an effect of producing an operation to decrease the brain activity (hereinafter, also referred to as a negative effect) is provided in particular when the vibration having the aforementioned second band is applied to the living body together with the vibration of the aforementioned first band.

By removing or attenuating the vibration of the second frequency band that induces the negative effect to decrease the brain activity from various environmental sounds including traffic noises and artificial sounds generated by the electronic equipment, signals recorded on media, voices, music signals, and their reproduction sounds and the like distributed or transmitted by broadcasting or communications by applying the present invention, it becomes possible to conduct technological researches to prevent the modern diseases by removing the negative influences including the causes of the modern diseases and securing safety, and to allow the achievement of various application developments such as increasing the pleasant sensation, aesthetic sensibility and perception ability and improving the mental and physical functions by adding or enhancing the vibration structure of the third frequency band that increases the brain activity and induces the positive effect.

A majority of audio digital media of CD, DVD, Internet distribution (downloading and streaming etc. of music files), which are currently widely used, including the media called the high-quality audio and high-resolution audio capable of reproduction up to a frequency exceeding the audible range components, has a theoretical recordable and reproducible frequency band limited to the first band of the audible range components and part of the second band that has the negative effect. Therefore, exposure to the sounds produced by such audio digital media for a long time has a risk that the brain activity decreases to cause a serious pathological state.

The present invention makes it possible to avoid such a risk potentially owned by those media. In particular, the digital audio standards do not provide practical alternatives for the users and put them in a state in which they are substantially compulsorily used. On the other hand, it is often the case where a long time is necessary for the change of them, and the risks cannot be avoided on the user side. The present invention makes it possible to provide a solution to practically avoid the risks caused by a decrease in the brain activity by removing or attenuating the second band components that have the negative effect while using the digital sound standards that have the above risks.

Moreover, regarding the audio media capable of reproduction up to the third band components that can theoretically produce the positive effect, of, for example, DVD-Audio and Blu-ray (registered trademark) Discs capable of reproduction up to 96 kHz at a sampling frequency of 192 kHz, SACD practically capable of reproduction from 50 kHz up to about 100 kHz at sampling frequencies of 2.8 MHz and 5.6 MHz, and some of high-resolution audio having sampling frequencies of 176.4 kHz, 192 kHz and 384 kHz, and so on, it becomes possible to cancel the negative effect and enhance the positive effect by removing or attenuating the negative second band or concurrently enhancing or newly adding the third band components in order to sufficiently produce the positive effect that are prevented from expressing due to the existence of the second band components that have the negative effect.

Further, a majority of the currently widely popularized signal transmission means of digital broadcasting and the like has a transmittable frequency band almost limited to the first band of the audible range and part of the second band having the negative effect, and cannot transmit the third band components that have the positive effect. Accordingly, it becomes possible to improve the sound quality, improve the sense of beauty of visual information, improve the learning effect and increase the brain activity by attenuating the negative effect on the mental and physical functions while utilizing the signal transmission means currently widely put to practical use by encoding the third band components that have the positive effect into a frequency band that can be transmitted by the transmission means on the signal transmission side and decoding the components to the third band components that have the positive effect on the receiving side of the signal by the present invention. Moreover, it becomes also possible to utilize the negative second band as presentation effects of new idea by adding and emphasizing it.

Further, by generating the frequency components of the third band that can neither be recorded nor reproduced by the aforementioned audio digital media and the digital broadcasting currently widely popularized or artificially enhancing it by the present invention, the brain activity is further increased, making it possible to not only remove the negative influence including the causes of the modern diseases but also induce more health state to produce the effects of increasing the pleasant sensation and the aesthetic sensitivity. Such effects are expected to produce innovative effects to the conventional acoustic digital technology.

Further, by removing the second band components containing hiss noises and switching noises or adding and emphasizing the third band components from or to analog audio sound sources such as analog tapes, LP records, SP records and the like other than sound sources recorded by the digital audio standards, it becomes possible to improve the sound quality for more pleasant beautiful listening of sounds and increase the brain activity while utilizing old sound sources, archive sound sources and the like.

Further, it becomes possible to increase the brain activity by band converting the second band components that have the negative effect into the third band components that have the positive effect by the present invention while utilizing the audio digital media that are currently widely used, making it possible to produce the preventive effects by removing the negative influences including the causes of the modern diseases and to produce the effects of increasing the pleasant sensation and the aesthetic sensitivity.

Furthermore, it becomes possible to attenuate the second band components that have the negative effect included in the artificial and natural voices, musics and environmental sounds existing in the nature by generating and presenting anti-phase vibrations to them on the basis of the present invention, to prevent or attenuate the risks of a decrease in the brain activity and enhance the positive effect owned by the vibration by producing earplugs, ear mufflers, clothes, sound barriers, sound insulating walls, acoustical panels, curtains and so on, which have the effects of interrupting or attenuating or absorbing the second band components that have the negative effect in a frequency band selecting manner.

Moreover, various medical applications and researches and developments related to them can be expected by the present invention. Conventionally, as techniques for artificially increasing or decreasing the brain activity, for example, craniotomy procedures, chemical substances, electromagnetic stimulations, conditioning reflections and so on have been used. By using such techniques, there are widely used techniques for making "clinical state models" such that the activities of various brain regions are artificially decreased by using animals or human beings in order to develop treatment methods and treatment medicines for various mental and physical diseases, and making "remedial models" on the basis of them.

The vibrations applied by the present invention are processed as information inputted to the brain and therefore have no invasiveness. Moreover, the risks of causing side effects can be suppressed extremely low by comparison to medicine administration and the like. Therefore, by using this invention, it becomes possible to arbitrarily control a decrease and an increase in the brain activity promptly, safely and reversibly without requiring craniotomy procedures, medicine administration, electromagnetic stimulations, operant conditioning and the like, which have the limits of hazardous nature, status recovery, difficulties in result interpretation, and so on.

Accordingly, by applying the present invention, a model of the pathological state and its remedial model can be provided safely and effectively. For example, by presenting a vibration of a specific frequency band that induces the effect of decreasing the brain activity to a healthy test human subject, a specific brain activity can be decreased within a few minutes, allowing a pathological state model to be produced. In addition, if a vibration of another frequency band that induces the effect of increasing the brain activity is subsequently presented, the activity can be reversed and turned to an increase within a few minutes. It is also possible to achieve both the pathological state and the state in which the mental and physical conditions are improved in a short time with an identical test human subject.

Furthermore, by making use of this method, even when an experiment having risks to possibly decrease the brain activity of the test human subject is conducted, it becomes possible to finally maintain the brain activity level to a definite level at which no adverse effect is exerted on the mind and body even if the brain activity relatively decreases by presenting a vibration of the frequency band that has the effects of increasing the brain activity preceding it and conducting the experiment in a state in which the base line of the brain activity of the test human subject is increased. Accordingly, there is the advantage that the experiment can be conducted while securing safety without causing actual harms on the aspect of health.

This method is extremely effective since it has an important advantage that it is safe for human beings and it is possible to make a pathological model and its remedial model promptly, reversibly and efficiently also when an animal experiment is conducted.

As described above, according to the present invention, it becomes possible to make a pathological model and its remedial model intended for human beings and animals other than human beings very safely and efficiently, allowing extreme effectiveness to be produced for many clinical studies aimed at developing a new treatment method, a treatment medicine and the like.

Further, as an application thereto, it becomes possible to guide human beings and animals close to desirable places and objects by variously increasing or decreasing the activity of the emotion system of the brain that strongly controls animal actions by pleasantness or unpleasantness or guide human beings and animals so as to select safe actions conforming to situations by conversely inducing evasive actions of animals and human beings getting away from undesirable places, objects, dangers and the like. In addition, since the effect of decreasing the stress is expected by improving the activity of the emotion system, it becomes possible to prevent or treat various mental and physical disorders attributed to stresses.

Moreover, by using the apparatus, method and space for artificially enhancing the vibration of the third frequency band that induces the effect of increasing the brain activity, they can be used for medical applications as they are as safe treatment and prevention means free of side effects without using medicines. In addition, it is possible to develop various applications such as applications as effective means for increasing the expressive effects by exalting the reactions of pleasure, beauty, and emotion of artistic productions including musics, applications as effective means for making living spaces, duty spaces, amusement spaces, public spaces, vehicles and so on comfortable.

### BRIEF DESCRIPTION OF THE DRAWINGS

Figure 1 is a block diagram showing a configuration of a bi-channel system used in the embodiments and implemental examples of the present invention;
Figure 2 is a projection view showing regions in which the regional cerebral blood flow (r-CBF) of the head portion decreases when an electrical signal having frequency components of equal to or lower than 22 kHz is applied as an aerial vibration to a test human subject compared to a background noise condition indicated by hatching by using the bi-channel system of Figure 1, where Figure 2(a) is a sagittal projection view, Figure 2(b) is a coronal projection view and Figure 2(c) is a horizontal plane projection view;
Figure 3 is a projection view showing regions in which the regional cerebral blood flow (r-CBF) of the head portion decreases when an electrical signal having frequency components of equal to or lower than 26 kHz is applied as an aerial vibration to the test human subject compared to the background noise condition indicated by hatching by using the bi-channel system of Figure 1, where Figure 3(a) is a sagittal projection view, Figure 3(b) is a coronal projection view and Figure 3(c) is a horizontal plane projection view;
Figure 4 is a graph showing a blood flow change at the brain stem that is a portion of the fundamental brain generated when an aerial vibration having frequency components of equal to or lower than 22 kHz is applied and when an aerial vibration having frequency components of equal to or lower than 26 kHz is applied by using the bi-channel system of Figure 1 by percentage to the background noise condition;
Figure 5 is a graph showing a deep index of brain activity (DBA-index) measured by brain waves when an aerial vibration having frequency components of equal to or lower than 22 kHz is applied and when an aerial vibration having frequency components of equal to or lower than 26 kHz is applied by using the bi-channel system of Figure 1;
Figure 6 is a graph showing an average value of variation in the deep index of brain activity (DBA-index) when an aerial vibration of a negative band group of 16 kHz to 32 kHz is applied in addition to the aerial vibration of the band of lower than 16 kHz, and an average value of variation in the deep index of brain activity (DBA-index) when an aerial vibration of a positive band group of equal to or higher than 32 kHz is applied in addition to the aerial vibration of the band of lower than 16 kHz by using the bi-channel system of Figure 1;
Figure 7 is a graph showing an average value of variation in the deep index of brain activity (DBA-index) when the aerial vibrations of individual divided bands are applied in addition to the aerial vibration of the band of lower than 16 kHz by using the bi-channel system of Figure 1;
Figure 8(a) show ranges of the first, second and third bands in the schematic graph of the power spectrum of the presented vibrations of the divided bands regarding a vibration signal that has the first, second and third bands, and Figure 8(b) shows an index of brain activity (difference in DBA-index) in the divided bands;
Figure 9 is a graph showing a vibration presentation method (negative effect) of a vibration presenting apparatus according to the first embodiment, where Figure 9(a) is a schematic graph of the power spectrum of the presented vibrations of the divided bands regarding a vibration signal that has the first and second bands, and Figure 9(b) is a graph showing an index of brain activity (difference in DBA-index) in the divided bands;
Figure 10A is a block diagram showing a configuration of a vibration presenting apparatus according to an embodiment 1-1, and a schematic diagram when a signal recorded in a recording medium is presented;
Figure 10B is a block diagram showing a configuration of a vibration presenting apparatus according to a modified embodiment of the embodiment 1-1, and a schematic diagram when a vibration signal is artificially synthesized by a vibration synthesizing apparatus;
Figure 10C is a block diagram showing a configuration of a vibration presenting apparatus according to a modified embodiment of the embodiment 1-1, and a schematic diagram when a vibration generated from a vibration generator apparatus such as a musical instrument is converted to a vibration signal by using a signal transducer apparatus such as a microphone;
Figure 11A is a block diagram showing a configuration of a vibration presenting apparatus according to an embodiment 1-2, and a schematic diagram when a vibration including the first band and a vibration including the second band are presented by using separate amplifier apparatuses and vibration presenting apparatuses;
Figure 11B is a block diagram showing a configuration of a vibration presenting apparatus according to the embodiment 1-2, and a schematic diagram when a vibration including the first band and a vibrations including the second band are presented by using identical amplifier apparatus and vibration presenting apparatus;
Figure 12A is a graph showing a vibration presentation method (suppression or reduction of the negative effect) of a vibration presenting apparatus and a vibration attenuator apparatus according to an embodiment 1-A, where Figure 12A(a) is a schematic graph of the power spectrum of the presented vibrations of the divided bands regarding a vibration signal that has the first and second bands, and Figure 12A(b) is a graph showing an index of brain activity (difference in DBA-index) in the divided bands;
Figure 12B is a block diagram showing a configuration of a vibration presenting apparatus (suppression or reduction of negative effect) according to an embodiment 1A-1;
Figure 12C is a block diagram showing a configuration of a modified embodiment of the embodiment 1A-1 of Figure 12B;
Figure 12D is a block diagram showing a configuration of a vibration presenting apparatus including a vibration attenuator apparatus (suppression or reduction of negative effect) according to an embodiment 1A-2;
Figure 12E is a block diagram showing a configuration of a modified embodiment of the embodiment 1A-2 of Figure 12D;
Figure 13 is a graph showing a vibration presentation method (strong positive effect) of a vibration presenting apparatus according to an embodiment 2, where Figure 13(a) is a schematic graph of the power spectrum of the presented vibrations of the divided bands regarding a vibration signal that has the strong positive effect among the first and third bands, and Figure 13(b) is a graph showing an index of brain activity (difference in DBA-index) in the divided bands;
Figure 14A is a block diagram showing a configuration of a vibration presenting apparatus according to an embodiment 2-1;
Figure 14B is a block diagram showing a configuration of a vibration presenting apparatus according to an embodiment 2-2;
Figure 15A is a block diagram showing a configuration of a vibration presenting apparatus according to an embodiment 2-3, and a schematic diagram when a vibration including the first band and a vibration including the second band are presented by using separate amplifier apparatuses and vibration presenting apparatuses;
Figure 15B is a block diagram showing a configuration of a vibration presenting apparatus according to the embodiment 2-3, and a schematic diagram when a vibration including the first band and a vibration including the second band are presented by using identical amplifier apparatus and vibration presenting apparatus;

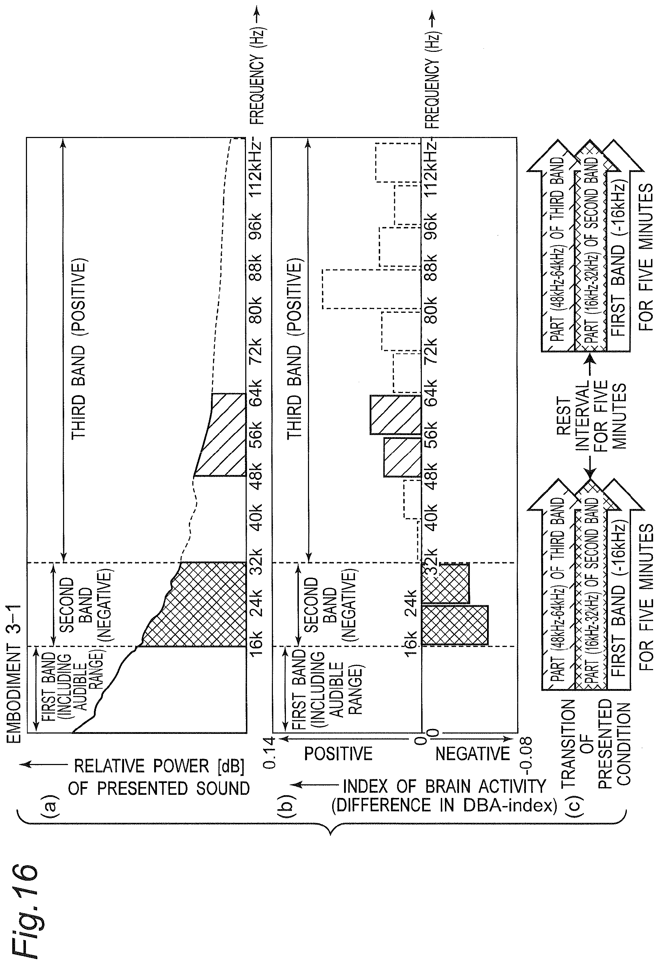

Figure 16 is a graph showing a vibration presentation method (presentation method of a combination of bands to maintain the brain activity substantially constantly) of a vibration presenting apparatus according to an embodiment 3-1, where Figure 16(a) is a schematic graph of the power spectrum of the presented vibrations of the divided bands regarding a vibration signal that has the first and second bands and part of the third band, Figure 16(b) is a graph showing an index of brain activity (difference in DBA-index) in the divided bands, and Figure 16(c) is a graph showing a temporal transition of the presentation condition of the vibration presentation method;

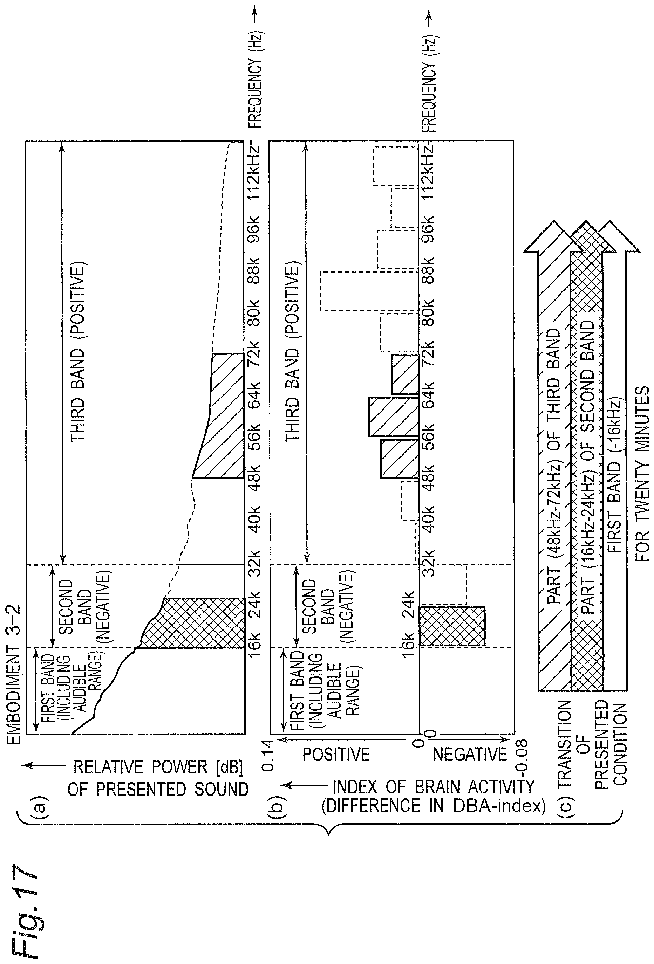

Figure 17 is a graph showing a vibration presentation method (presentation method of a combination of bands to induce a feeble increase in the brain activity) of a vibration presenting apparatus according to an embodiment 3-2, where Figure 17(a) is a schematic graph of the power spectrum of the presented vibrations of the divided bands regarding a vibration signal that has the first and second bands and part of the third band, Figure 17(b) is a graph showing an index of brain activity (difference in DBA-index) in the divided bands, and Figure 17(c) is a graph showing a temporal transition of the presentation condition of the vibration presentation method;
Figure 18 is a block diagram showing a configuration of a vibration presenting apparatus according to an embodiment 3-3;
Figure 19 is a block diagram showing a configuration of a vibration presenting apparatus according to an embodiment 3-4;
Figure 20 is a block diagram showing a configuration of a vibration presenting apparatus according to an embodiment 3-5;
Figure 21 is a block diagram of an apparatus to generate an output signal capable of inducing an increase or a decrease in the brain activity by adding a vibration signal that partially or totally includes the second or third band to an original vibration signal according to an embodiment 3-6;
Figure 22 is a block diagram showing a modified embodiment of the apparatus of Figure 21;
Figure 23 is a perspective view showing an example of a vibration complementing apparatus to add a vibration signal capable of inducing an increase or a decrease in the brain activity to an original vibration that does not induce the fundamental brain activation effect according to the embodiment 3-6;
Figure 24 is a perspective view showing an example of a vibration complementing apparatus to add a vibration signal capable of inducing an increase or a decrease in the brain activity to an original vibration outputted from a portable player or the like according to the embodiment 3-6;
Figure 25 is a perspective view showing an example of a vibration complementing apparatus to add a vibration signal capable of inducing an increase or a decrease in the brain activity to an original vibration signal outputted from broadcasting receiver equipment or the like according to the embodiment 3-6;
Figure 26 is a block diagram showing an example of a vibration complementing apparatus that concurrently uses band extending means of the existing technology with an addition means of a vibration capable of inducing an increases or a decrease in the brain activity according to the embodiment 3-6;
Figure 27 is a block diagram showing an example of a vibration complementing apparatus to generate a vibration signal capable of inducing an increase or a decrease in the brain activity as an output signal by adding a signal obtained by extracting the super-high frequency components of a vibration signal capable of inducing an increase or a decrease in the brain activity to an original vibration signal according to the embodiment 3-6;
Figure 28 is a perspective view showing examples of applying a vibration complementing apparatus capable of inducing an increase or a decrease in the brain activity to TV broadcasting, transmission, communications and reception according to an embodiment 3-7;
Figure 29 is a block diagram showing an example of a vibration complementing apparatus according to a modified embodiment of Figure 28;
Figure 30 is a graph showing a vibration presentation method (presentation method to perform intensity changes) of a vibration presenting apparatus according to an embodiment 4, where Figure 30(a) is a schematic graph of the power spectrum of the presented vibrations of the divided bands regarding a vibration signal that has the first, second and third bands, and Figure 30(b) is a graph showing an index of brain activity (difference in DBA-index) in the divided bands;
Figure 31 is a block diagram showing a configuration of a vibration presenting apparatus according to an embodiment 4-1;
Figure 32 is a block diagram showing a configuration of a vibration presenting apparatus according to an embodiment 4-2;
Figure 33 is a graph showing a vibration presentation method (presentation method using band shift) of a vibration presenting apparatus according to an embodiment 5, where Figure 33(a) is a schematic graph of the transition of the power spectrum of the presented vibrations of the divided bands regarding a vibration signal that has the first and second bands, and Figure 33(b) is a graph showing a change in the index of brain activity (difference in DBA-index) in the divided bands;
Figure 34A is a block diagram showing a configuration of a vibration presenting apparatus according to an embodiment 5-1;
Figure 34B is a block diagram showing a detailed configuration of the band shift circuit 83 of Figure 34A;
Figure 35A is a block diagram showing a configuration of an encoder apparatus 80 used in a vibration presenting apparatus according to an embodiment 5-2;
Figure 35B is a block diagram showing a detailed configuration of the band shift circuit 83A of Figure 35A;
Figure 36A is a block diagram showing a configuration of a decoder apparatus 90 used in the vibration presenting apparatus according to the embodiment 5-2;
Figure 36B is a block diagram showing a detailed configuration of the band shift circuit 93 of Figure 36A;
Figure 37A is a block diagram showing a configuration of a decoder apparatus 90A used in a vibration presenting apparatus according to an embodiment 5-3;

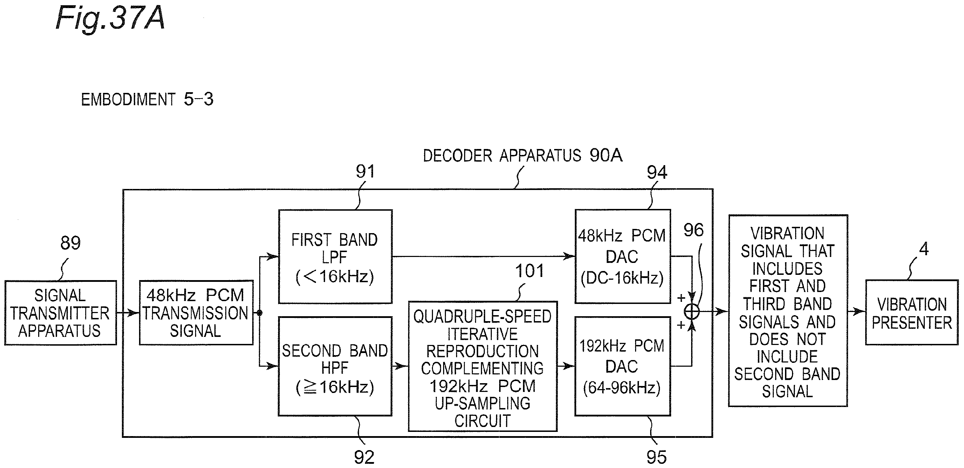

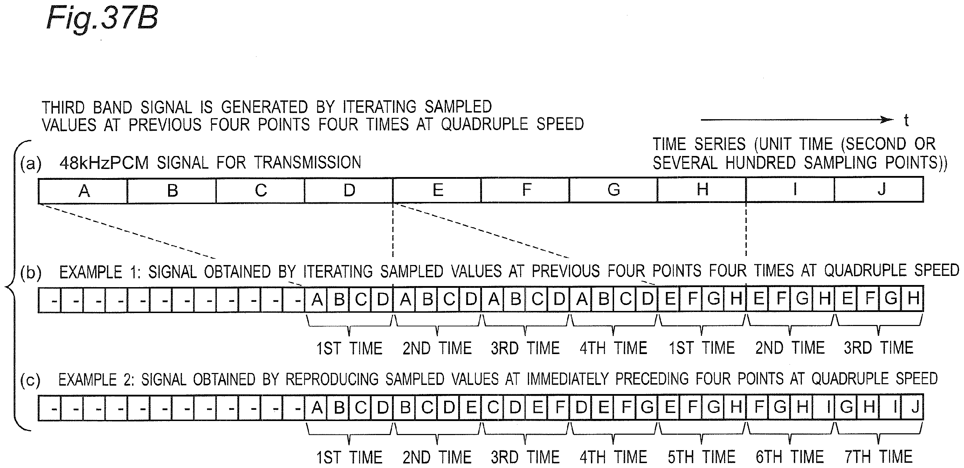

Figure 37B is a graph showing an operation of the up-sampling circuit 101 of Figure 37A, where Figure 37B(a) is a graph showing a time series data of a 48-kHz PCM signal for transmission, Figure 37B (b) is a graph showing a time series data of a signal obtained by iterating sampling of sampled values at previous four points four times at quadruple speed according to an example 1 of the up-sampling method, and (c) is a graph showing a time series data of a signal obtained by reproducing sampled values at immediately preceding four points at quadruple speed according to an example 2 of the up-sampling method;
Figure 38A is a block diagram showing a configuration of an encoder apparatus 80A used in a vibration presenting apparatus according to an embodiment 5-4;
Figure 38B is a block diagram showing a configuration of a decoder apparatus 90B used in the vibration presenting apparatus according to the embodiment 5-4;
Figure 38C is a graph showing compressed storing by 1/4 decimation executed by the encoder apparatus 80A of Figure 38A and a decoding process executed by the decoder apparatuses 90B of Figure 38B, where Figure 38C(a) is the time series data of the signal of original third band components, Figure 38C(b) is the time series data of an encode signal encoded by the encoder apparatus 80A, and Figure 38C(c) is the time series data of a decode signal decoded by the decoder apparatus 90B;

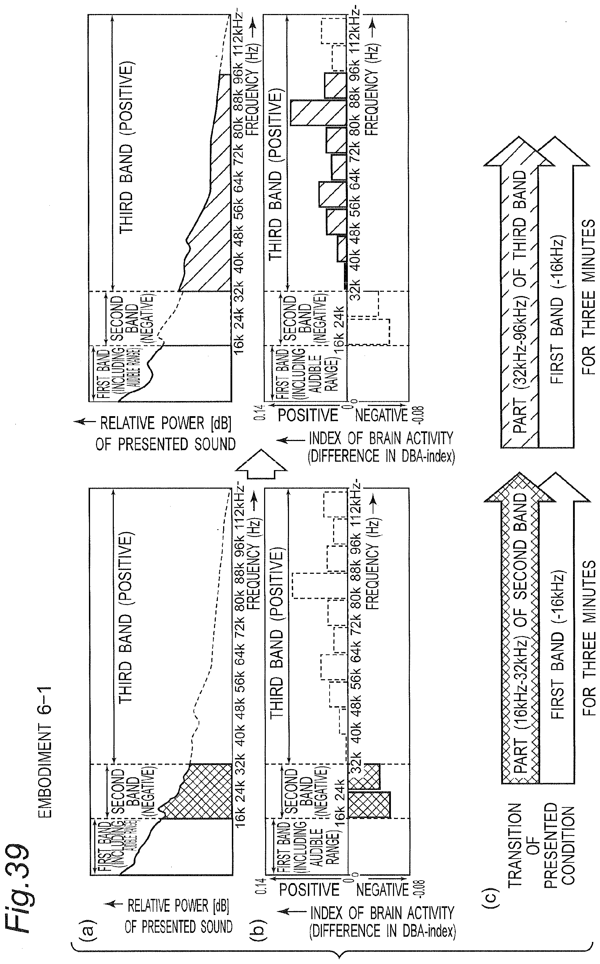

Figure 39 is a graph showing a vibration presentation method (presentation method for inducing an abrupt change from a decrease to an increase in the brain activity) of a vibration presenting apparatus according to an embodiment 6-1, where Figure 39(a) is a schematic graph of changeover of the power spectrum of the presented vibrations of the divided bands regarding a vibration signal having the first, second and third bands, Figure 39(b) is a graph showing an index of brain activity (difference in DBA-index) in the divided bands, and Figure 39(c) is a graph showing a temporal transition of the presentation condition of the vibration presentation method;

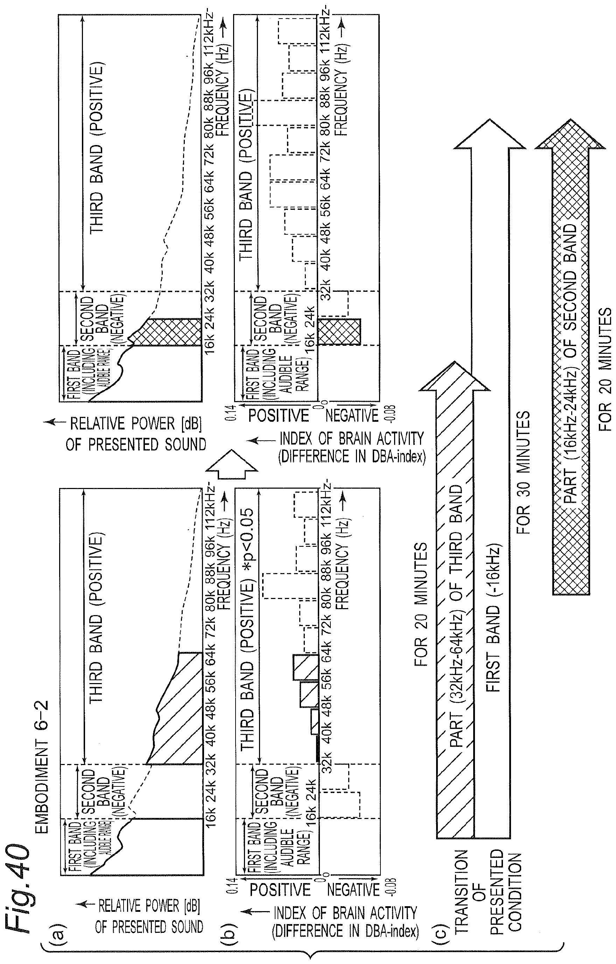

Figure 40 is a graph showing a vibration presentation method (presentation method to induce a gradual change from an increase to a decrease in the brain activity) of a vibration presenting apparatus according to an embodiment 6-2, where Figure 40(a) is a schematic graph of changeover of the power spectrum of the presented vibrations of the divided bands regarding a vibration signal that has the first, second and third bands, Figure 40(b) is a graph showing an index of brain activity (difference in DBA-index) in the divided bands, and Figure 40(c) is a graph showing a temporal transition of the presentation condition of the vibration presentation method;
Figure 41 is a block diagram of a vibration monitor apparatus according to an embodiment 7;
Figure 42 is a schematic graph showing an operation of a super-high frequency monitor apparatus according to an embodiment 7-1 attached to the vibration presenting apparatus;
Figure 43A is a diagram showing a mounting example of an apparatus in which a vibration presenter 411 to present a super-high frequency vibration and a super-high frequency vibration sensor 412 are mechanically bonded to each other in the vibration monitor apparatus according to the embodiment 7-1;
Figure 43B is a longitudinal sectional view along the line A-A' of Figure 43A;
Figure 44 is a graph showing a frequency characteristic when the vibration of an ultra-wideband white noise generated in the vibration presenting apparatus is formed into an electrical signal by a super-high frequency vibration sensor by using an apparatus including the vibration presenting apparatus of the above configuration and the super-high frequency vibration sensor;
Figure 45A shows a modified embodiment of Figure 44, and a plan view showing an example in which the vibration presenting apparatus and the super-high frequency vibration sensor are adjacently arranged;
Figure 45B shows a modified embodiment of Figure 44, and a plan view showing an example in which the vibration presenting apparatus and the super-high frequency vibration sensor are arranged in a complex manner within a single piezoelectric device module;
Figure 45C shows a modified embodiment of Figure 44, and a perspective view showing an example in which two functions of the vibration presenting apparatus and the super-high frequency vibration sensor are implemented by one module by using it sharingly as such a layer that a piezoelectric device of a multilayer structure is utilized as a vibration presenting apparatus and such a layer that it is utilized as a super-high frequency vibration sensor;
Figure 46 is a circuit diagram in a case where the apparatus of Figure 44 is configured as a loudspeaker system with a built-in super-high frequency vibration sensor;
Figure 47 is a circuit diagram of a super-high frequency vibration presentation state display apparatus that can be connected to the apparatus of Figure 46;
Figure 48 is a block diagram showing two modified embodiments of a method for supplying the super-high frequency vibration presentation state display apparatus with an electric power by utilizing a vibration signal, photovoltaic or the like without using an external power source in the vibration monitor apparatus bonded to the vibration presenting apparatus;
Figure 49 is a block diagram of a super-high frequency vibration monitor apparatus according to an embodiment 7-2;
Figure 50 is a Block diagram showing an application example in a case where the vibration presenting apparatus with the built-in super-high frequency vibration monitor apparatus according to the embodiment 7-2 is configured as a loudspeaker system;
Figure 51 is a block diagram showing an application example in a case where the vibration presenting apparatus with the built-in super-high frequency vibration monitor apparatus according to the embodiment 7-2 is configured as a portable telephone;

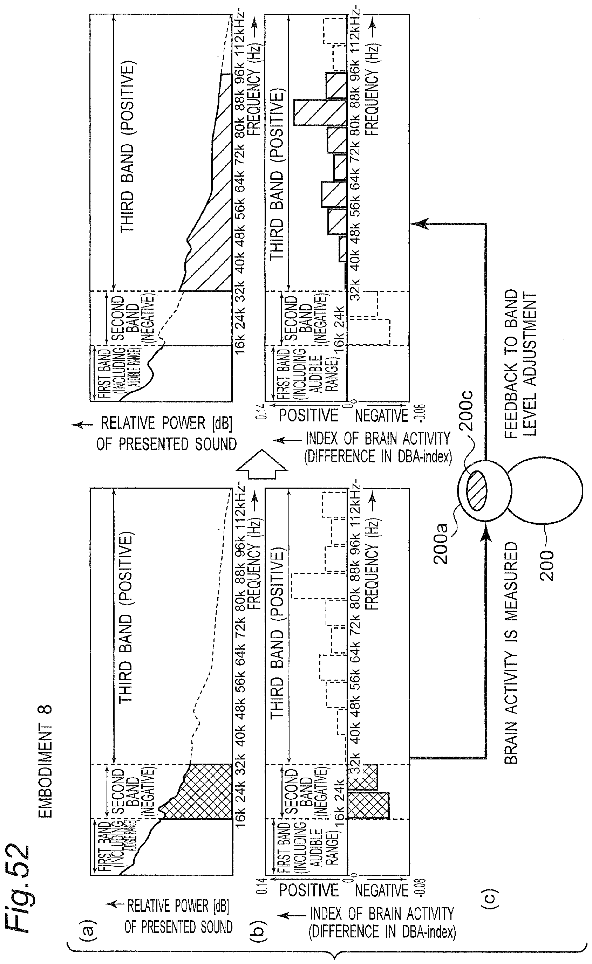

Figure 52 is a graph showing a vibration presentation method (presentation method having a feedback function to adjust the level of each divided band) of a vibration presenting apparatus according to an embodiment 8, where Figure 52(a) is a schematic graph of a change in the power spectrum of the presented vibrations of the divided bands regarding a vibration signal that has the first, second and third bands, Figure 52(b) is a graph showing an index of brain activity (difference in DBA-index) in the divided bands, and Figure 52(c) is a conceptual diagram to measure the brain activity of a living body and feed it back to a band level adjustment;
Figure 53 is a block diagram showing a configuration of a vibration presenting apparatus according to an embodiment 8-1;
Figure 54 is a block diagram showing a configuration of a vibration presenting apparatus according to an embodiment 8-2;
Figure 55 is a flow chart showing an active processing equalizing process for making the index of brain activity executed by the active processing equalizer 204 of Figure 53 or the active processing equalizer 204 of Figure 54 approach a target value;
Figure 56 is a block diagram showing an implemental example of a high-frequency monitoring system having a feedback control mechanism by sound structure information according to an embodiment 8-3;
Figure 57 is a block diagram showing a detailed configuration of the high-frequency monitoring system of Figure 56;
Figure 58 is a flow chart showing a first part of the detailed processing of the high-frequency monitoring system of Figure 56;
Figure 59 is a flow chart showing a second part of the detailed processing of the high-frequency monitoring system of Figure 56;
Figure 60 is a flow chart showing a third part of the detailed processing of the high-frequency monitoring system of Figure 56;
Figure 61 is a block diagram showing an implemental example of a high-frequency monitoring system provided with a feedback control mechanism by deep brain activation information according to an embodiment 8-4;
Figure 62 is a block diagram showing a detailed configuration of the high-frequency monitoring system of Figure 61;
Figure 63 is a perspective view showing an example of a vibration monitoring system that performs adjustment of vibration generation setting by feedback to the vibration generator apparatus by using the judgment result of intensity of each frequency band of vibration according to an embodiment 8-5;
Figure 64 is a block diagram showing an example of a vibration monitoring system that performs adjustment of vibration generation setting by feedback to the vibration generator apparatus by using the judgment result of intensity of each frequency band of vibration according to the embodiment 8-5;
Figure 65 is a block diagram showing a configuration of a vibration presenting apparatus according to an embodiment 8-6;
Figure 66 is a flow chart showing a process to generate feedback indication contents for performing adjustment of a head-count within a definite range executed by the head-count analyzing and vibration control apparatus 230 of Figure 65;
Figure 67 is a block diagram showing a configuration of a vibration presenting apparatus according to an embodiment 8-7;
Figure 68A is a flow chart showing a process to generate feedback indication contents for averaging the head-count executed by the multidimensional head-count analyzing and vibration control apparatus 230B of Figure 67;
Figure 68B is a flow chart showing a control information generating processes (S26, S27, S28) of Figure 68A;
Figure 69 is a perspective view and a sectional view of a pendant type vibration presenting apparatus 830p according to an application example 1;
Figure 70A is a front view of a brooch (accessory) type vibration presenting apparatus 160 according to an application example 2;
Figure 70B is a right side view of the vibration presenting apparatus 160 of Figure 70A;
Figure 70C is a back side view of the vibration presenting apparatus 160 of Figure 70A;
Figure 71 is a perspective view of a configuration example of a super-high frequency vibration generator apparatus according to an application example 3 showing an example in which vibrations are generated by flowing a liquid while making it collide with obstacles;
Figure 72A is an external view of a clothes embedded type vibration presenting apparatus according to an application example 4;
Figure 72B is an internal view of the apparatus of Figure 71A;
Figure 73 is a sectional view and a block diagram of a skin contact type vibration presenting apparatus according to an application example 5;
Figure 74 is a side view showing an example of an apparatus to apply a vibration capable of inducing an increase or a decrease in the brain activity from the body surface via a solid vibration generating mechanism according to an application example 6;
Figure 75 is a perspective view showing an example of a sauna type vibration presenting apparatus according to an application example 7;
Figure 76 is a perspective view showing an example in which the wall itself constituting a space vibrates to generate super-high frequency components according to an application example 8;
Figure 77 is a side view showing an example of a vibrating body characterized by having a vibration state that includes the second and/or the third band induced by vibrating air of the object surrounding human beings in a super-high frequency band according to an application example 9;
Figure 78 is a block diagram showing an example of a vibrating body in a bathtub according to an application example 10;
Figure 79 is a perspective view of a vibration presenting apparatus for generating a vibration capable of inducing an increase or a decrease in the brain activity because of inclusion of the second and/or the third band and containment of super-high frequency components at close ranges of audience in a space such as a theater, a concert hall 1430, an auditorium or the like according to an application example 11;
Figure 80 is a side view showing an application example 12 of a space in which a portable player to reproduce audible range vibration components is combined with a vibration presenting apparatus to apply super-high frequency components that includes the second and/or the third band and capable of decreasing or increasing the brain activity concurrently to a plurality of human beings;
Figure 81 is a side view showing an application example 13 that shows a modified embodiment of the apparatus of Figure 80;
Figure 82 is a perspective view showing a shower type vibration presenting apparatus according to an application example 14;
Figure 83 is a block diagram showing an example of an apparatus to generate a vibration capable of inducing an increase or a decrease in the brain activity by processing a 1-bit quantization noise owned by a high-speed sampling 1-bit quantization system according to an application example 15;
Figure 84 is a side view showing a vibration presenting apparatus in a vehicle according to an application example 16;
Figure 85 is a side view showing an example of a vibration presenting apparatus at the driver's seat or cockpit of public transportation according to an application example 17;
Figure 86 is an external view showing a mounting example of a vibration presenting apparatus 962a on a passenger platform of a vehicle such as a station yard according to an application example 18;
Figure 87 is a block diagram showing an example in which a vibration including a transmitted sound (audible sound) and the third band is preparatorily recorded in mixture with a balance originally determined, and the signal is reproduced by a public-address system that has a faithful response performance according to an application example 19;
Figure 88 is a block diagram showing an example in which a vibration including a transmitted sound (audible sound) and the third band is generated by different public-address systems depending on different sound sources according to an application example 20;
Figure 89 is a block diagram showing an example in which a vibration including a transmitted sound (audible sound) and the third band is synthesized on the scene and generated from one public-address system according to an application example 21;
Figure 90 is a block diagram showing an example in which a background noise (audible sound) is collected by a microphone, the feature of the background noise (audible sound) is measured on the basis of the collected vibration signal, and the vibration including the transmitted sound (audible sound) and the third band are adjusted in conformity to measured data according to an application example 22;
Figure 91 is a perspective view showing an example in which a vibration generator apparatus according to an application example 23 is installed in a station yard;
Figure 92 is a graph and table showing impression evaluations of sounds evaluated by listeners under a high-cut sound condition and a full-range sound condition generated by using the well-known "Blu-ray (registered trademark) Disc version AKIRA sound track" according to an application example 24;
Figure 93 is a perspective view showing an example of an apparatus to induce an increase in the impression of image representation and an improvement in the image quality by making sounds to be put in the sound track a vibration including the first and third bands in an audiovisual complex package media such as a Blu-ray Disc according to an application example 25;
Figure 94 is a graph and table showing impression evaluations of images evaluated by listeners under a high-cut sound condition and a full-range sound condition generated by using the well-known "Blu-ray (registered trademark) Disc version AKIRA sound track" according to an application example 26;
Figure 95 is a perspective view showing an application example 27 in which a vibration presenting apparatus is mounted on the outside of a vehicle such as an automobile;
Figure 96 is a perspective view showing one example of a vibration presenting apparatus 370 of a loudspeaker system according to an application example 28;
Figure 97 is a front view of a headphone type vibration presenting apparatus according to an application example 29;
Figure 98A is a perspective view showing an example of a vibration generating mechanism utilizing a portable telephone 1410 according to an application example 30;
Figure 98B is a perspective view showing a sectional view of an upper portion of the portable telephone 1410 of Figure 98A;
Figure 98C is a partially sectional perspective view of the headset 1415 of the portable telephone 1410 of Figure 98A;
Figure 98D is a partially sectional perspective view of the headset 1415 of the portable telephone 1410 of Figure 98A;
Figure 98E is a partially sectional perspective view of the cable 1416 of the portable telephone 1410 of Figure 98A;
Figure 98F is a perspective view showing a vibration generator apparatus 1419 other than the portable telephone 1410 of Figure 98A;
Figure 99A is a perspective view showing an example of a vibration generating mechanism utilizing a portable music player 1420 such as iPod (registered trademark) according to an application example 31;
Figure 99B is a perspective view showing a vibration generator apparatus 1423 other than the portable music player 1420 of Figure 61A;
Figure 100 is a perspective view showing an appearance of a portable player according to an application example 32;
Figure 101 is a block diagram showing a configuration of the portable player of Figure 100;
Figure 102 is a perspective view showing an example of an electronic musical instrument apparatus that includes a vibration complementing apparatus to add a super-high frequency vibration signal including the second and/or the third band to an original vibration that does not induce the effect of increasing or decreasing the brain activity generated by playing an electronic musical instrument according to an application example 33;
Figure 103 is a perspective view showing a loudspeaker double helical matrix arrangement according to an application example 34;
Figure 104 is a perspective view showing an arrangement in which loudspeaker double helical matrix arrangements are continuously iterated in two directions according to an application example 35;
Figure 105 is a perspective view showing a loudspeaker six dimension continuous matrix arrangement according to an application example 36;
Figure 106 is a perspective view showing an arrangement in which loudspeaker six dimension continuous matrix arrangements are continuously iterated in two directions according to an application example 37;
Figure 107 is a block diagram of a vibration generator apparatus applied to portable equipment or a distribution network to it according to an application example 38;
Figure 108 is a projection view of the experiment results (reference implemental example 1) of the head portion of a test human subject of a PET apparatus when the first band, the second band and the third band are simultaneously applied in combination measured in the embodiment 3, where Figure 108(a) is a sagittal projection view, Figure 108(b) is a coronal projection view, and (c) is a horizontal plane projection view;
Figure 109 is a graph showing a results (reference implemental example 2) of behavioral testing and electroencephalogram experiment measured in the embodiment 4, or the deep index of brain activity (DBA-index) to each band; and
Figure 110 is a graph showing an electroencephalogram experiment results (reference implemental example 3) measured in the embodiment 6, or a time series variation of a head portion topography map of the normalized power of α-EEG of a value of not smaller than a predetermined value.

### BEST MODE FOR CARRYING OUT THE INVENTION

Implemental examples and embodiments of the present invention will be described below with reference to the drawings. In the following implemental examples and embodiments, like components are denoted by like reference numerals.

### FUNDAMENTAL IMPLEMENTAL EXAMPLE

First of all, a decrease or an increase in the brain activity due to vibration information is described below. The present inventor and others found a technique capable of setting a decrease and an increase in the brain activity to various extents depending on differences in the frequency bands of vibrations applied as sounds or inaudible sounds through an experiment. The experiment that serves as the fundament of the present invention is described in detail below.

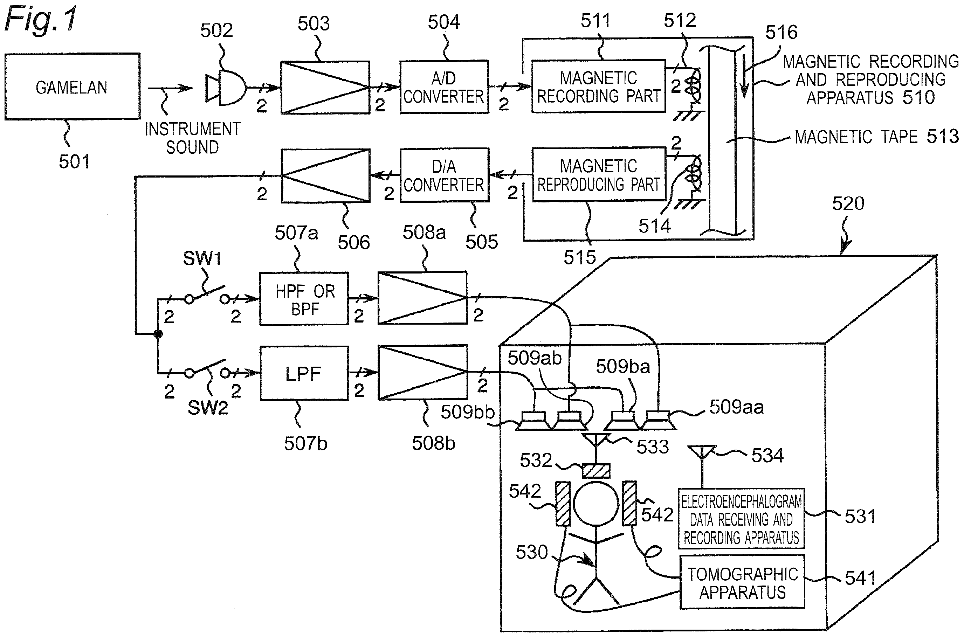

The experimental method is described first. Regarding a sound source signal, the traditional gamelan music of Indonesian Bali Island, which abundantly contained high-frequency components and had a predetermined autocorrelation order structure (See, for example, the Patent Documents 7 and 9) was collected by a ultra-wideband recording system originally developed, and used recorded for 200 seconds. Moreover, regarding a vibration presentation system, the bi-channel system of Figure 1 (See, for example, the Patent Document 2) was used. The vibration presentation system described below includes a vibration generator apparatus, a vibration processing apparatus, a vibration presenting apparatus and so on.

Figure 1 is a block diagram showing a configuration example of the bi-channel system used in the embodiments and implemental examples of the present invention. In the bi-channel system of Figure 1, each stereo sound source signal was separated into audible range components and super-high frequency components at a predetermined crossover frequency by using a high-pass filter (HPF) or a band-pass filter (BPF) 507a and a low-pass filter (LPF) 507b having an attenuation rate of, for example, 80 dB/Oct and a pass-band frequency band ripple of, for example, ±1dB. Both of them were independently amplified and thereafter presented separately or simultaneously via a loudspeaker system having loudspeakers 509aa, 509ab, 509ba and 509bb. In this case, the loudspeakers 509aa and 509ab include, for example, a tweeter and a super-tweeter. Moreover, the high-pass filter (HPF) or band-pass filter (BPF) 507a and the low-pass filter (LPF) may each be a variable frequency filter capable of setting an upper limit and a lower limit at various cutoff frequencies.

Referring to Figure 1, an instrument sound obtained by playing a gamelan 501 that is a percussion instrument made of bronze in the Indonesian Bali Island is collected by a microphone 502. The microphone 502 converts the inputted instrument sound into an analog electrical signal, and the converted analog electrical signal is outputted via a preamplifier 503 to an A/D converter 504. The A/D converter 504 analog-to-digital converts the inputted analog electrical signal into a digital signal at a predetermined sampling frequency and outputs the resulting signal to a magnetic recording part 511.

A magnetic recording and reproducing apparatus 510 is a so-called digital signal recorder that has a magnetic recording part 511, a magnetic recording head 512, a magnetic reproducing head 514 and a magnetic reproducing part 515, and records a digital signal on a magnetic tape 513 or reproduces and outputs the digital signal recorded on the magnetic tape 513. In this case, the magnetic recording and reproducing apparatus 510 is, for example, a DAT of the conventional technology devised by Dr. Yoshio Yamasaki, having a uniform frequency characteristic within a frequency range up to, for example, 200 kHz. The magnetic recording part 511 modulates a carrier wave signal by a predetermined digital modulation system in accordance with the digital signal inputted from the A/D converter 504, and records the modulated signal on the magnetic tape 513 running in a predetermined direction 516 indicated by arrow by using the magnetic recording head 512. On the other hand, the magnetic reproducing part 515 reproduces the modulated signal recorded on the magnetic tape 513 by using the magnetic reproducing head 514, and takes out a digital signal by demodulating the reproduced modulated signal by a digital demodulation system reverse to the digital modulation system.

The reproduced digital signal data is digital-to-analog converted into the original analog signal by a D/A converter 505 and thereafter outputted via a reproduction amplifier 506. An output analog signal from the reproduction amplifier 506 is inputted to the loudspeakers 509aa and 509ab via a switch SW1, a high-pass filter or band-pass filter 507a having a predetermined cutoff frequency and a power amplifier 508a, and inputted to the loudspeakers 509ba and 509bb via a switch SW2, the low-pass filter 507b having a predetermined cutoff frequency and a power amplifier 508b.

The loudspeakers 509aa, 509ab, 509ba and 509bb are placed in a room 520 that is an acoustically insulated anechoic room or conforming to it, and each of the loudspeakers 509aa, 509ab, 509ba and 509bb converts the inputted signal into an aerial vibration and applies the vibration to a human being 530 of the test human subject.

In the fundamental implemental example, an electrical signal, which had frequency components of equal to or lower than 22 kHz obtained by filtering the sound source signal by using the low-pass filter (LPF) 507b having a cutoff frequency of 22 kHz by using the bi-channel system of Figure 1, was applied as an aerial vibration to the test human subject, and the regional cerebral blood flow (hereinafter, referred to as r-CBF) of the test human subject was measured. Moreover, r-CBF in a case where the aerial vibrations were not applied and only the background noise existed was measured as a control condition (background noise condition).

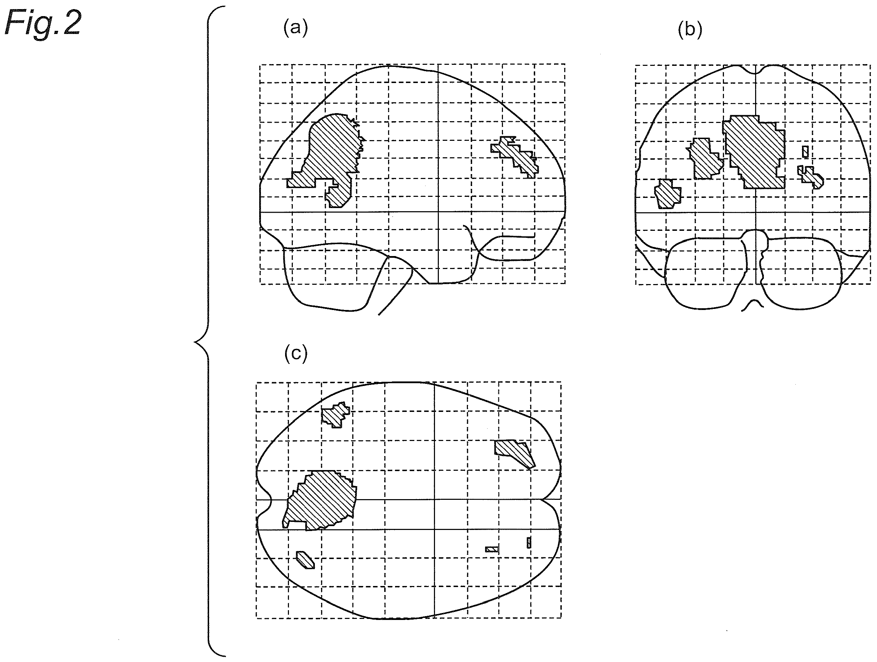

Figure 2 is a projection view showing regions in which the regional cerebral blood flow (r-CBF) of the head portion decreases when an electrical signal having frequency components of equal to or lower than 22 kHz is applied as an aerial vibration to a test human subject compared to a background noise condition indicated by hatching by using the bi-channel system of Figure 1, where Figure 2(a) is a sagittal projection view, Figure 2(b) is a coronal projection view and Figure 2(c) is a horizontal plane projection view. That is, Figure 2 shows regions in which the r-CBF decreases when the aforementioned aerial vibration is applied by comparison to the background noise condition. A decrease in the r-CBF is observed in a wide region at the front portion of the parietal lobe. Moreover, a decrease in the r-CBF is observed also in the external side portion of the left prefrontal region. In Figure 1, the switch SW1 is turned off, and the switch SW2 is turned on.

Next, the r-CBF of the test human subject was measured in a case where the same sound source was applied to the test human subject by converting an electrical signal obtained by filtering by using the low-pass filter (LPF) 507b of which the cutoff frequency was set at 26 kHz into an aerial vibration.

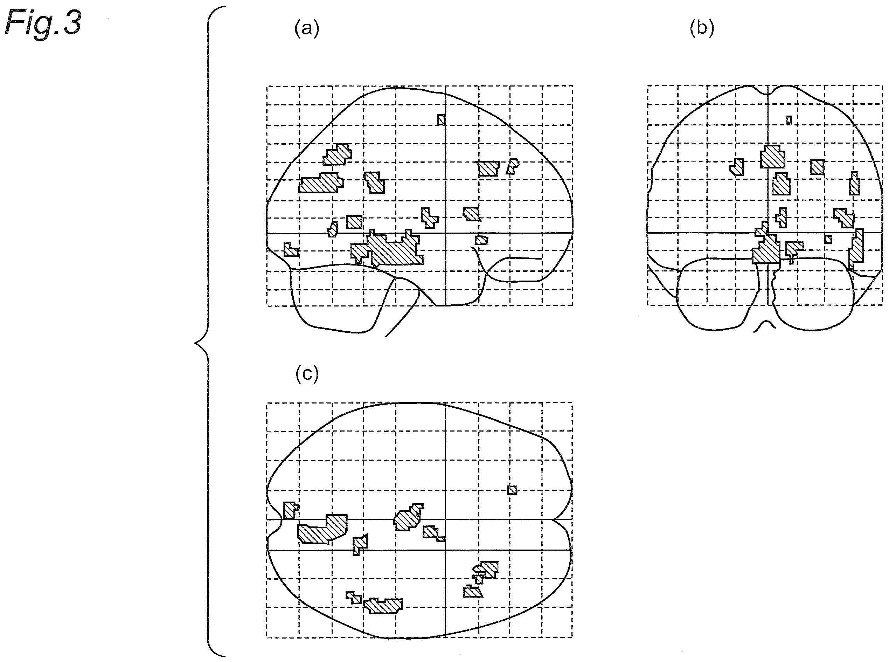

Figure 3 is a projection view showing regions in which the regional cerebral blood flow (r-CBF) of the head portion decreases when an electrical signal having frequency components of equal to or lower than 26 kHz is applied as an aerial vibration to the test human subject compared to the background noise condition indicated by hatching by using the bi-channel system of Figure 1, where Figure 3(a) is a sagittal projection view, Figure 3(b) is a coronal projection view and (c) is a horizontal plane projection view. That is, Figure 3 shows portions in which the r-CBF decreases when the aforementioned aerial vibration is applied by comparison to the background noise condition. A decrease in the blood flow was observed in the region of a very limited range of the parietal lobe when the aerial vibration having the frequency components of equal to or lower than 26 kHz was applied by comparison to the background noise condition, and a remarkable decrease in the blood flow was observed in the fundamental brain constituted of the brain stem, the thalamus, the hypothalamus and so on where no decrease in the blood flow was observed when the aerial vibration of the components of equal to or lower than 22 kHz was applied at the same time. In Figure 1, the switch SW1 is turned off, and the switch SW2 is turned on.

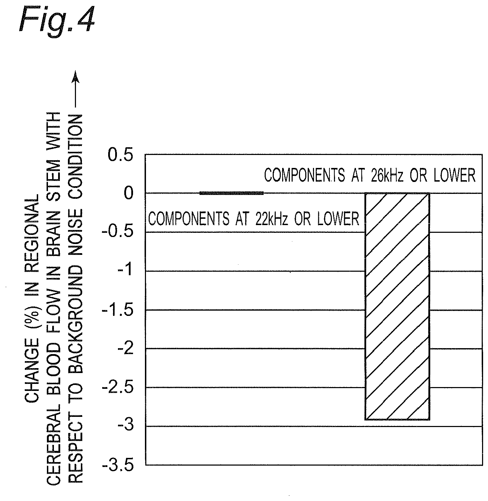

Figure 4 is a graph showing a blood flow change at the brain stem that is a portion of the fundamental brain generated when an aerial vibration having frequency components of equal to or lower than 22 kHz is applied and when an aerial vibration having frequency components of equal to or lower than 26 kHz is applied by using the bi-channel system of Figure 1 by percentage to the background noise condition. As apparent from Figure 4, it was indicated that the activity of the brain stem decreased by about 3.0% when the components of equal to or lower than 26 kHz were applied in contrast to the fact that the activity of the brain stem scarcely increased or decreased (+0.02%) when only the components of equal to or lower than 22 kHz were applied by comparison to the background noise condition. Further, it was examined how the activity of the fundamental brain including the brain stem changed in the case where the cutoff frequency of the low-pass filter (LPF) 507b was 22 kHz and in the case where it was 26 kHz by using a deep index of brain activity (DBA-index) obtained from electroencephalogram data that generally reflected the activity of the fundamental brain by comparison to the background noise condition.

A telemetry electroencephalogram measurement system to minimize the constraint level to the test human subject was used for electroencephalogram measurement, and the measurement was conducted by using earlobe connection as reference electrodes from twelve electrodes (electrode names: Fp1, Fp2, F7, Fz, F8, C3, C4, T5, T6, Pz, O1, O2) on the scalp based on the International 10-20 method. The obtained electroencephalogram data was recorded through AD conversion at a sampling frequency of 256 Hz. After removing an interval including artifact, a power was obtained at a frequency resolution of 0.5 Hz by FFT analysis for the analytical interval of two seconds with an overlap of every one second for each electrode, and the square root of the power of the band components of 10.0 Hz to 12.5 Hz was calculated as an equivalent potential of the α2-band brain wave. Data obtained from the central parietoocipital seven electrodes (electrode names: C3, C4, T5, T6, Pz, O1, O2) were averaged to be obtained as a "deep index of brain activity (DBA-index)". This index indicates that it is significantly correlated to the activity of the whole neural network of the fundamental brain that is the region to bear the fundamental functions of the brain including the brain stem, the thalamus and the hypothalamus considered to be the neural base of the hypersonic effect by the prior art researches (See the Patent Documents 7 and 9).

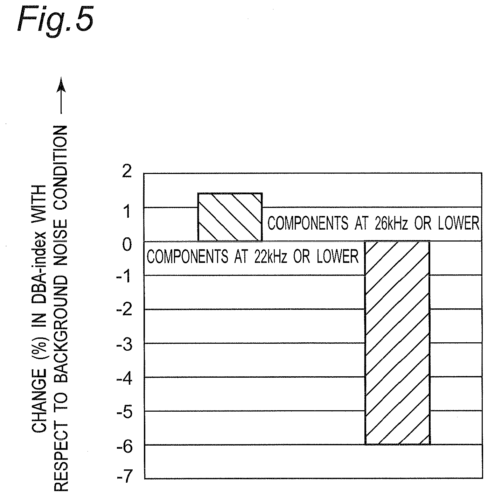

Figure 5 is a graph showing a deep index of brain activity (DBA-index) measured by brain waves when an aerial vibration having frequency components of equal to or lower than 22 kHz is applied and when an aerial vibration having frequency components of equal to or lower than 26 kHz is applied by using the bi-channel system of Figure 1. Referring to Figure 5, it was indicated that the deep index (DBA-index) of brain activity of the brain wave exhibited a decrease of 6.0% when the components of equal to or lower than 26 kHz were applied by comparison to the background noise condition in contrast to the fact that the deep index of brain activity of the brain wave exhibited a slight increase (+1.5%) when only the components of equal to or lower than 22 kHz were applied by comparison to the background noise condition. These experiment results indicate that nerve activities at various levels can be set to various regions in the brain depending on the different frequency components applied to a human being. In particular, in this experiment, the human sensations of beauty and pleasure are controlled and the activity of the fundamental brain that maintains the homeostasis of a living body remarkably decreased by increasing the cutoff frequency of the low-pass filter (LPF) 507b from 22 kHz to 26 kHz so that the band components of 22 kHz to 26 kHz are more intensely applied.

The above indicated that the activities of the different regions of the brain were changed by applying aerial vibrations of different frequency structures.

Next, paying attention to the fundamental brain that bears the fundamental functions of the brain such as behavioral control and adaptive control among the various brain regions, a decrease or an increase in the activity level of the fundamental brain by vibration information that have various frequency bands is described below.

The fundamental brain is the center of the reward system neural circuit, and an increase in the activity generates the sensations of beauty, pleasure and emotion, sharpens the senses and raises the arousal level, allowing the human error to be suppressed. Conversely, it is indicated that a decrease in the activity of the fundamental brain becomes the bottom cause of various maladies such as negative symptoms of depression, suicide, eating disorder, wrist cutting, schizophrenic disorder and the like, attention deficit hyperactivity disorder, autism, and Alzheimer-type dementia.

Further, the fundamental brain bears the highest center of the autonomic nerve system, the internal secretion system and the immune system, and plays the role of maintaining the homeostasis of the whole body serving as the highest control tower to govern the physical fitness. Therefore, if the activity of the fundamental brain rises, the immunity of NK-cell activity and the like rises, and stress hormones of adrenaline and cortisol reduce. These whole body effects can produce therapeutic effects for various metabolic syndromes including hypertension, diabetes, and hyperlipemia.

Therefore, it is expected to prevent and treat these maladies by increasing the activity of the fundamental brain, and it becomes possible to produce an illness model and a clinical condition model of these maladies by decreasing the activity of the fundamental brain.

Accordingly, the present inventor and others performed elaborate investigation as to what sort of increase or decrease effect the specific band components of a vibration exhibited to the activity of the fundamental brain first paying attention to the activity level of the fundamental brain including the brain stem on the basis of the principle of decreasing or increasing the activity of various regions in the brain to various extents by a variety of vibration information. The outline of the experiments is shown.

In the experiments, the traditional gamelan music of Indonesian Bali Island, which abundantly contained high-frequency components and had a predetermined autocorrelation order structure (See, for example, the Patent Documents 7 and 9) was collected by a ultra-wideband recording system originally developed, and recorded for 200 seconds to be used as a sound source signal. Moreover, the bi-channel system of Figure 1 was used as a presentation system. The sound source signal was separated into audible range components (LFC) band-limited to frequency components of equal to or lower than 16 kHz served as a cutoff frequency by the low-pass filter (LPF) 507b and high-frequency components (HFC) obtained through band-pass filtering or high-pass filtering by setting various cutoff frequencies at the upper limit and the lower limit by the high-pass filter (HPF) or band-pass filter (BPF) 507b of a frequency variable filter. After independently amplifying the respective signals, the vibration of the audible range components (LFC) was presented to the test human subject by the loudspeakers 509ba and 509bb of the loudspeaker system, and the vibration of the high-frequency components (HFC) was presented to the test human subject by the loudspeakers 509aa and 509ab of the loudspeaker system. The above-mentioned loudspeaker system was installed in a position located two meters apart from the test human subject.

The frequency bands of the signal of the high-frequency components (HFC) were set to totally ten kinds of 16 kHz to 24 kHz, 24 kHz to 32 kHz, 32 kHz to 40 kHz, 40 kHz to 48 kHz, 48 kHz to 56 kHz, 56 kHz to 64 kHz, 64 kHz to 72 kHz, 72 kHz to 80 kHz, 80 kHz to 88 kHz, and 88 kHz to 96 kHz every 8 kHz from 16 kHz to 96 kHz. Further, bands of 96 kHz to 112 kHz of a 16-kHz width and all bands of equal to or higher than 112 kHz were also set.

In this case, a subexperiment was constituted for each of the frequency bands of the high-frequency components (HFC). In the subexperiment, two experiment conditions were set, and differences in the brain activity between them were compared. That is, only the vibration of the audible range components (LFC) of equal to or lower than 16 kHz were presented in a condition 1 (control condition), and the vibration of the high-frequency components (HFC) having a specified bandwidth were presented simultaneously with presenting the vibration of the audible range components (LFC) of equal to or lower than 16 kHz in a condition 2. The order of presenting these two conditions was assumed to be the following two kinds.
(A) Presentation order 1: Presentation was performed in the order of condition 1, condition 2, condition 2 and condition 1, and after a few minutes of rest, presentation was performed in the order of condition 2, condition 1, condition 1 and condition 2.
(B) Presentation order 2: Presentation was performed in the order of condition 2, condition 1, condition 1 and condition 2, and after a few minutes of rest, presentation was performed in the order of condition 1, condition 2, condition 2 and condition 1.

That is, the presentation was performed totally four times under each of both the two conditions. Sorting of the presentation orders of two kinds was counterbalanced between test human subjects. In order to avoid the discomfort of the test human subjects, the temperature and humidity were maintained within definite ranges, and special attentions were paid to the circumferential environment. The test human subjects were instructed to open their eyes naturally during measurements.

Then, electroencephalogram measurement was performed during the presentation. For the electroencephalogram measurement, a telemetry electroencephalogram measurement system to minimize the constraint level to the test human subject was used, and the measurement was performed by using earlobe connection as reference electrodes from twelve electrodes (electrode names: Fp1, Fp2, F7, Fz, F8, C3, C4, T5, T6, Pz, O1, O2) on the scalp based on the International 10-20 method. The obtained electroencephalogram data was recorded through AD conversion at a sampling frequency of 256 Hz. After removing an interval including the artifact, a power was obtained at a frequency resolution of 0.5 Hz by FFT analysis for the analytical interval of two seconds with an overlap of every one second for each electrode, and the square root of the power of the band components of 10.0 Hz to 12.5 Hz was calculated as an equivalent potential of the α2-band brain wave. Data obtained from the central parietoocipital seven electrodes (electrode names: C3, C4, T5, T6, Pz, O1, O2) were averaged to be obtained as the "deep index of brain activity (DBA-index)". This index indicates that it is significantly correlated to the activity of the whole neural network of the fundamental brain that is the region to bear the fundamental functions of the brain including the brain stem, the thalamus and the hypothalamus considered to be the neural base of the hypersonic effect by the prior art researches (See the Patent Documents 7 and 9).

Since the transitional electroencephalogram data exhibit a clear delay to the sound presentation, an average value of the deep index of brain activity (DBA-index) for the latter half of 100 seconds of each condition was obtained. These values were standardized with respect to the value averaged through all the conditions every test human subject in order to remove the variations among the test human subjects. On the above basis, a difference between the average value of totally four times of the condition 2 and totally four times of the condition 1 (control condition) was obtained, and subjected to a statistical calibration. Healthy 22 Japanese males and females participated as the test human subjects.

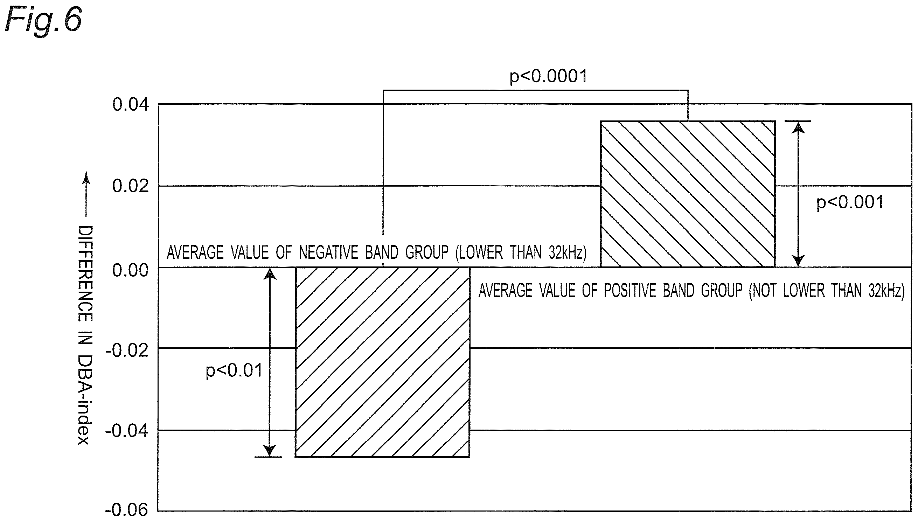

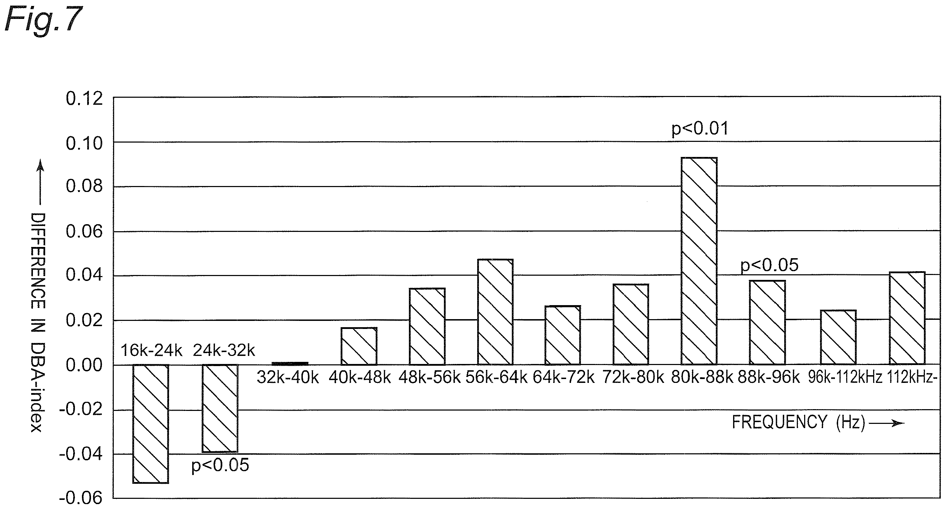

Next, experiment results are described with reference to Figures 6 and 7. Figure 6 is a graph showing a difference between an average value of the deep index of brain activity (DBA-index) when the aerial vibration of the high-frequency components (HFC) is applied in addition to the aerial vibration of the band of lower than 16 kHz (condition 2) and an average value of the deep index of brain activity (DBA-index) when only the aerial vibration of the band of lower than 16 kHz is applied (condition 1) by using the bi-channel system of Figure 1. According to the listening conditions of the present experiment, a classification is made into a negative band group of 16 kHz to 32 kHz and a positive band group of equal to or higher than 32 kHz depending on the high-frequency components (HFC) additionally applied under the condition 2, and both are compared. Figure 7 is a graph showing a difference between an average value of the deep index of brain activity (DBA-index) when the aerial vibrations of the divided bands are applied in addition to the aerial vibration of the band of lower than 16 kHz (condition 2) and an average value of the deep index of brain activity (DBA-index) when only the aerial vibration of the band of lower than 16 kHz is applied (condition 1). This value, which becomes an index indicating how much the activity of the neural network of the fundamental brain increases or decreases by applying the high-frequency components (HFC) of each divided band, is therefore hereinafter referred to as a variation index (difference of DBA-index in the figure) of the deep brain activity.

The deep index of brain activity (DBA-index) obtained by the aforementioned method was examined, and there was discovered, on the listening conditions of the present experiment, a tendency that the deep index of brain activity (DBA-index) increased in the condition 2, i.e., when the audible range components (LFC) + high-frequency components (HFC) were simultaneously presented by comparison to the condition 1, i.e., when only the audible range components (LFC) were presented according to a subexperiment (32 kHz to 40 kHz, 40 kHz to 48 kHz, 48 kHz to 56 kHz, 56 kHz to 64 kHz, 64 kHz to 72 kHz, 72 kHz to 80 kHz, 80 kHz to 88 kHz, 88 kHz to 96 kHz, 96 kHz to 112 kHz, 112 kHz and higher) with the band components of equal to or higher than 32 kHz served as HFC. In other words, it was confirmed that the activity of the fundamental brain network system was raised by adding the high-frequency components (HFC) to the audible range components (LFC). This is a tendency matched with a conventional knowledge (See the Non-Patent Documents 1 and 7).

On the other hand, on the listening conditions of the present experiment, there was discovered a tendency that the deep index of brain activity (DBA-index) decreased in the condition 2, i.e., when the audible range components (LFC) + high-frequency components (HFC) were simultaneously presented by comparison to the condition 1, i.e., when the audible range components (LFC) were singly presented according to a sub-experiment (16 kHz to 24 kHz, 24 kHz to 32 kHz) with the band components of equal to or lower than 32 kHz served as HFC. In other words, it was discovered that the activity of the fundamental brain network system was lowered by adding the high-frequency components (HFC) to the audible range components (LFC).

Accordingly, the sub-experiment was classified into two groups. On the listening conditions of the present experiment, the band of equal to or higher than 16 kHz and lower than 32 kHz was served as a negative band group, and the band of equal to or higher than 32 kHz was served as a positive band group. Then, bivariate t-test was performed for the negative band group and the positive band group with the variation index (difference in DBA-index) of the deep brain activity served as a variable, and it was clarified that the variation index (difference in DBA-index) of the deep brain activity of the positive band group was statistically significantly larger than that of the negative band group with a high significance that significance level p < 0.0001 as apparent from Figure 6.

Next, the mono-variate t-test was performed with the variation index (difference in DBA-index) of the deep brain activity of each band group served as an index in order to confirm whether the activity of the fundamental brain really increased or decreased in the experiment of each band group, and it was indicated that the activity of the fundamental brain increased with a high significance that significance level p < 0.001 regarding the positive band group (band in which the high-frequency components (HFC) are equal to or higher than 32 kHz on the listening conditions of the present experiment). On the other hand, it was indicated that the activity of the fundamental brain decreased statistically significantly at a significance level p < 0.01 regarding the negative band group (band in which the high-frequency components (HFC) are equal to or higher than 16 kHz and lower than 32 kHz on the listening conditions of the present experiment) as apparent from Figure 6.

Further, the mono-variate t test was performed every band more finely divided from the high-frequency components (HFC), and it was indicated that the deep index of brain activity (DBA-index) significantly decreased at significance level p < 0.05 when the vibration of the band of 24 kHz to 32 kHz was presented in addition to the band of lower than 16 kHz. Moreover, it was indicated that the deep index of brain activity (DBA-index) significantly increased at significance level p < 0.01 when the vibration of the band of 80 kHz to 88 kHz was presented in addition to the band of lower than 16 kHz. Moreover, it was indicated that the deep index of brain activity (DBA-index) significantly increased at significance level p < 0.05 when the vibration of the band of 88 kHz to 96 kHz was presented in addition to the band of lower than 16 kHz.

It is considered that these bands can efficiently induce a negative effect and a positive effect with a small amount of information of a limited bandwidth.

The first, second and third bands were defined as follows on the basis of the aforementioned experiment results.
(A) The first band is the band that includes the audible range, i.e., the band of 20 Hz to 16 kHz.
(B) The second band is the band that has a tendency to induce the effect of decreasing the activity of the fundamental brain (negative effect = hypersonic negative effect) when presented with the first band, i.e., the band of 16 kHz to 32 kHz on the listening conditions of the present experiment.
(C) The third band is the band that has a tendency to induce the effect of increasing the activity of fundamental brain (positive effect = hypersonic positive effect) when presented with the first band. For example, on the listening conditions of the present experiment, it is the band of equal to or higher than 32 kHz up to a predetermined highest frequency. In this case, the highest frequency is the maximum frequency processable by a signal processing circuit, and, for example, the frequencies of 96 kHz, 112 kHz, 128 kHz, 140 kHz, 152 kHz, 168 kHz, 184 kHz, and 192 kHz.

The values of the lower limit frequency and the upper limit frequency of each band indicated above are not universally constant but allowed to vary depending on other various vibration presentation conditions or listening conditions including the power of the vibration signal, the degree of amplification of the amplifier circuit of the vibration presenting apparatus, the number of the vibration presenting apparatuses, a distance from the vibration presenting apparatus to the living body, and so on.

Further, it has been known that the audible range upper limits of animals other than human beings depend on the species, and therefore, it is considered that the lower and upper limit frequencies of the first band including the audible range, the lower limit frequency and the upper limit frequency of the second band that has the negative effect, and the lower limit frequency and the upper limit frequency of the third band that has the positive effect can assume different values every animal species different from the frequencies identified by the listening conditions of the present experiment intended for human beings.

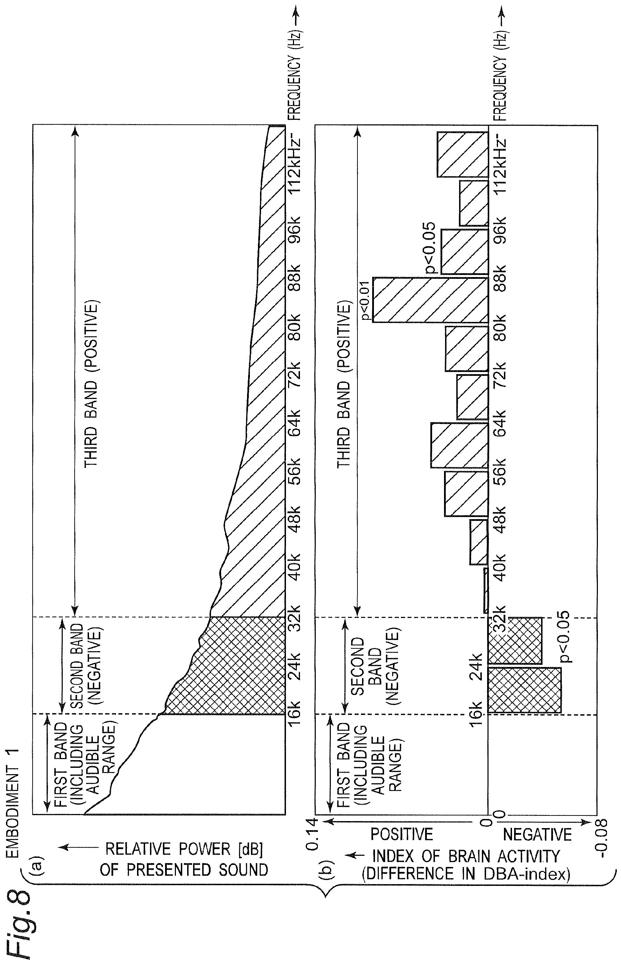

If the above results are schematically collectively illustrated, they can be indicated in a manner similar to that of Figure 8. Figure 8(a) is a graph showing a ranges of the first, second and third bands in the schematic graph of the power spectrum of the presented vibrations of the divided bands regarding the vibration signal that has the first, second and third bands, and Figure 8(b) is a graph showing an index of brain activity (difference in DBA-index) in the divided bands. In this case, Figure 8(b) illustrates the direction and the magnitude of the effects that the bands set by the experiment exert on the fundamental brain activity. They respectively show a magnitude of the effect of decreasing the fundamental brain activity in the second band (negative effect) and the effect of increasing it in the third band (positive effect) by being presented with the first band. It is noted that the embodiments or implemental examples indicated below are described on concrete examples of the activity of the fundamental brain on the basis of the aforementioned experiment, and its principle is also applicable to the activities of other brain regions. It is noted that the frequencies corresponding to the boundaries of the bands of Figure 8 are shown as one example on the listening conditions of the present experiment as described above, and it is considered that they assume different values depending on the vibration presentation conditions or the vibration listening conditions or the species of the animals served as the subject.

### EMBODIMENT 1: NEGATIVE EFFECT

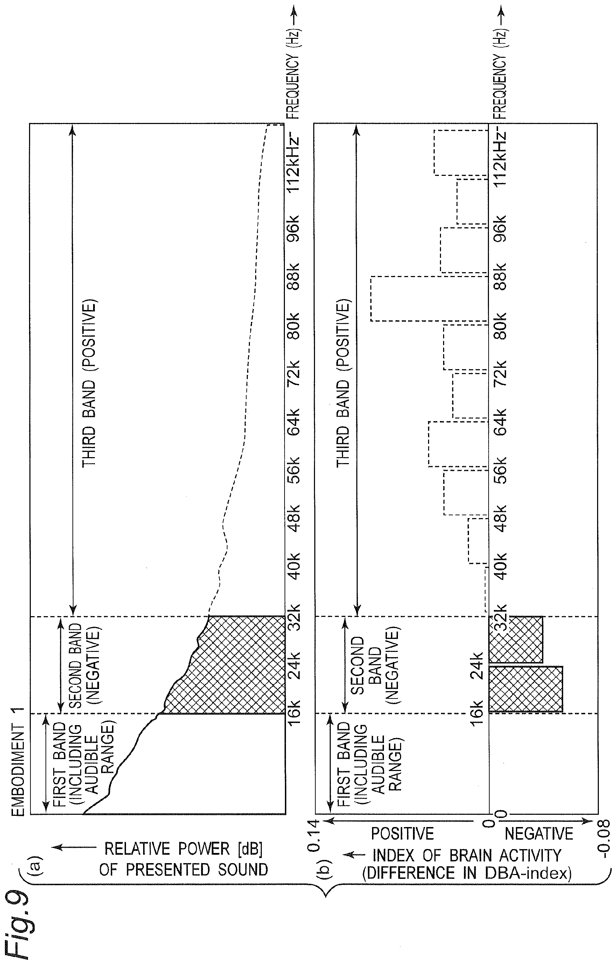

Figure 9 is a graph showing a vibration presentation method (negative effect) of a vibration presenting apparatus according to the first embodiment, where Figure 9(a) is a schematic graph of the power spectrum of the presented vibrations of the divided bands regarding a vibration signal that has the first and second bands, and Figure 9(b) is a graph showing an index of brain activity (difference in DBA-index) in the divided bands. The vibration presenting apparatus, method and space according to the first embodiment are characterized in that the "negative effect" of decreasing the brain activity by presenting the vibration of the second band with the first band is induced as shown in Figure 9. Hereinafter, an example of the apparatus, method and space to induce the negative effect of decreasing the brain activity by presenting the vibration of the second band simultaneously with the vibration of the first band is shown. In all the embodiments including the present embodiment, the frequencies corresponding to the boundaries of the bands are indicated as one example on the listening conditions of the aforementioned experiment, and it is considered that they assume different values depending on the vibration presentation conditions or the vibration listening conditions or the species of the animals served as the subject. Moreover, through all the embodiments, the structural features of the vibration or the vibration signal do not matter. That is, they may be a stationary wave of, for example, a sine wave, a sawtooth wave or a white noise, or a non-stationary wave having a predetermined autocorrelation order (See the Patent Document 9) or other vibration and vibration signals or a complex of them. Moreover, in all the embodiments, the medium in presenting a vibration and applying it to a living body may be any of a gas, a liquid and a solid or a complex of them.

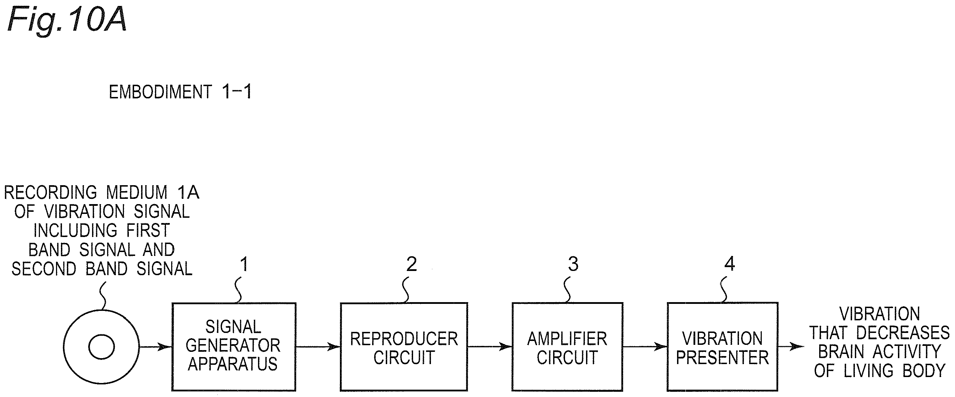

Figure 10A is a block diagram showing a configuration of a vibration presenting apparatus according to an embodiment 1-1. Referring to Figure 10A, the vibration presenting apparatus according to the embodiment 1-1 is configured to include a signal generator apparatus 1 including the drive of a recording medium 1A of a vibration signal, a reproducer circuit 2, an amplifier circuit 3, and a vibration presenter 4. Referring to Figure 10A, the recording medium 1A, in which a vibration signal including both the first band and the second band is preparatorily recorded originally in a vibration signal source of, for example, the audio signal (frequency: 20 Hz to 22.05 kHz) of CD and the audio signal (frequency: 20 Hz to 24 kHz) of the audio track of DVD, is inserted into the drive of the signal generator apparatus 1 to read the vibration signal from the vibration generator apparatus 1. The signal is reproduced by the reproducer circuit 2, amplified by the amplifier circuit 3, and thereafter converted into a vibration by the vibration presenter 4 to be presented to animals including human beings, and this leads to inducing the negative effect of decreasing the brain activity. It is noted that the signal generator apparatus 1 is allowed to have a built-in memory to preparatorily record a vibration signal that includes both the first band and the second band in the vibration signal source and to generate the vibration signal. Moreover, it is acceptable to wiredly or wirelessly receive broadcasted and communicated signals or use a signal inputted from external equipment in place of the recording medium of the vibration signal, the signal generator apparatus 1 and the reproducer circuit 2. Further, in each of the embodiments, the signal may either be digital or analog, and the recording medium is a medium that can be read by a computer or a medium that can be read by a drive including a computer or a signal generator apparatus including it, or a medium reproducible by the reproducer circuit.

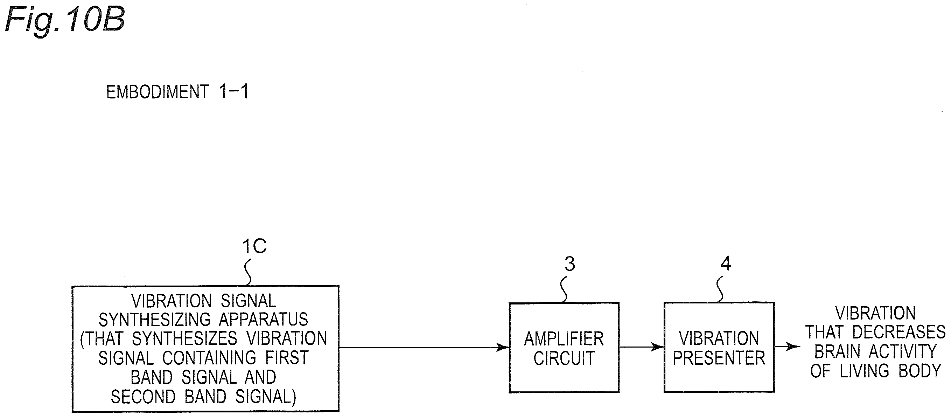

Figure 10B shows a modified embodiment of Figure 10A, which is an apparatus that induces the negative effect by artificially synthesizing a vibration signal that includes both the first band and the second band by, for example, a vibration signal synthesizing apparatus 1C of, for example, a synthesizer in place of the recording medium of the vibration signal, the signal generator apparatus 1 and the reproducer circuit 2, amplifying the signal by the amplifier circuit 3, thereafter converting it into a vibration by the vibration presenter 4 and presenting it to animals including human beings. It is noted that the vibration signal synthesizing apparatus may be of a type that uses an external input from a sampling sound source or the like.

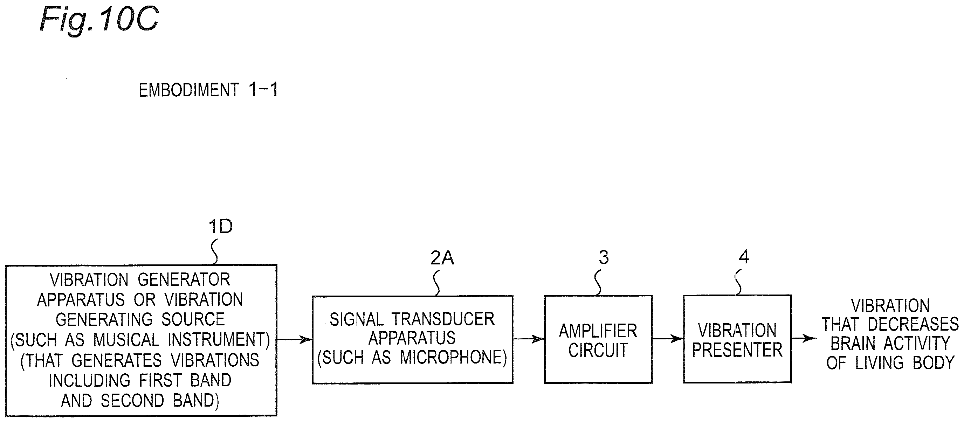

Figure 10C is also a modified embodiment of Figure 10A, which induces a negative effect by forming a signal of a vibration that includes both the first band and the second band generated by a vibration generator apparatus or a vibration generating source 1D such as a musical instrument played on the scene by using a signal transducer apparatus 2A such as a microphone or a pickup, amplifying the signal by the amplifier circuit 3, thereafter converting it into a vibration by the vibration presenter 4 and presenting it to animals including human beings. For the vibration generated by the vibration generator apparatus or the vibration generating source 1D, there may be used a significant or non-significant sound (hereinafter, referred to as a voice) generated by living matters and nonliving matters, a music, an environmental sound or the like. Further, the generated vibration may be applied to the living body via a gas, a liquid or a solid (hereinafter, referred to as a vibrating body) as it is without being converted into a signal.

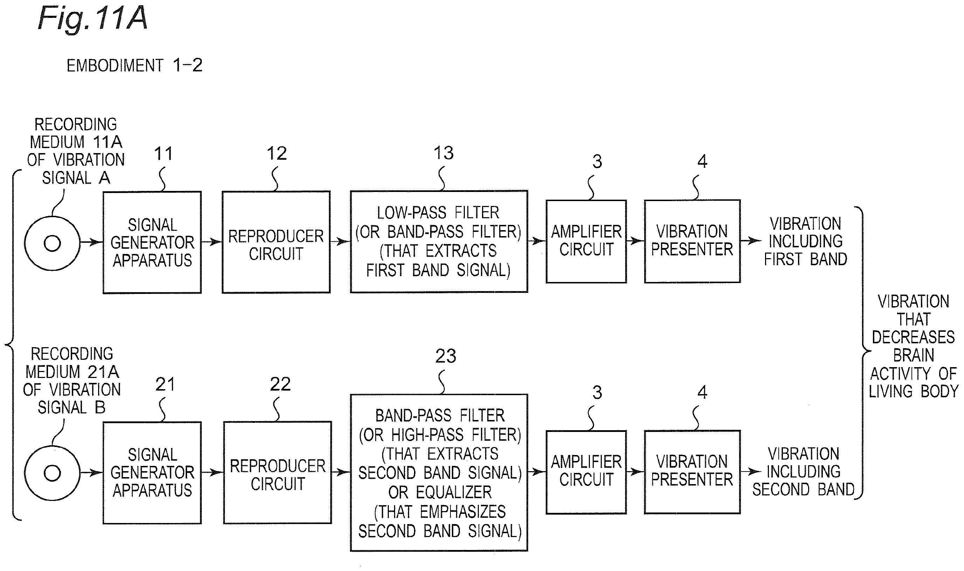

Figure 11A is a block diagram showing a configuration of a vibration presenting apparatus according to an embodiment 1-2. Referring to Figure 11A, the vibration presenting apparatus A according to the embodiment 1-2 is configured to include a signal generator apparatus 11 including a drive to generate the vibration signal preparatorily recorded in the recording medium 11A of a vibration signal A, a reproducer circuit 12, a low-pass filter 13, an amplifier circuit 3, and a vibration presenter 4. The vibration presenting apparatus B is configured to include a signal generator apparatus 21 including a drive to generate the vibration signal preparatorily recorded in the recording medium 21A of a vibration signal B, a reproducer circuit 22, a band-pass filter or equalizer 23, an amplifier circuit 3, and a vibration presenter 4. Referring to Figure 11A, each of the recording media 11A and 21A has experienced a recording by preparatorily recording the vibration signals of both the first band and the second band or extracting it from an identical or separate vibration signal sources. The vibration signal is read from the recording medium 11A of the vibration signal A by the signal generator apparatus 11, and reproduced by the reproducer circuit 12. For example, only the first band signal of equal to or lower than 16 kHz is extracted by the low-pass filter 13, amplified by the amplifier circuit 3, thereafter converted into a vibration by the vibration presenter 4 and applied to animals including human beings. A vibration signal is read from the recording medium 21A of the vibration signal B by the signal generator apparatus 21, and reproduced by the reproducer circuit 22. For example, only the second band signal of 16 kHz to 32 kHz is extracted by the band-pass filter 23, amplified by the amplifier circuit 3, thereafter converted into a vibration by the vibration presenter 4 and applied to animals including human beings. As described above, in the present embodiment, the brain activity of the living body can be decreased by presenting the vibration including the first band and the vibration including the second band. In this case, it is acceptable to use a vibration signal synthesizing apparatus 1C shown in Figure 10B or a combination of a musical instrument or a vibration generator apparatus or vibration generating source 1D shown in Figure 10C to generate a voice, an environmental sound or the like and a signal transducer apparatus 2A such as a microphone or a pickup or wiredly or wirelessly receive a signal broadcasted and communicated such as the audio signals of digital broadcasting and high-resolution distribution or use a signal inputted from external equipment in place of the recording medium of the vibration signal, the signal generator apparatus and the reproducer circuit. Further, the generated vibration may be applied as it is to a living body via a vibrating body without being converted into a signal.

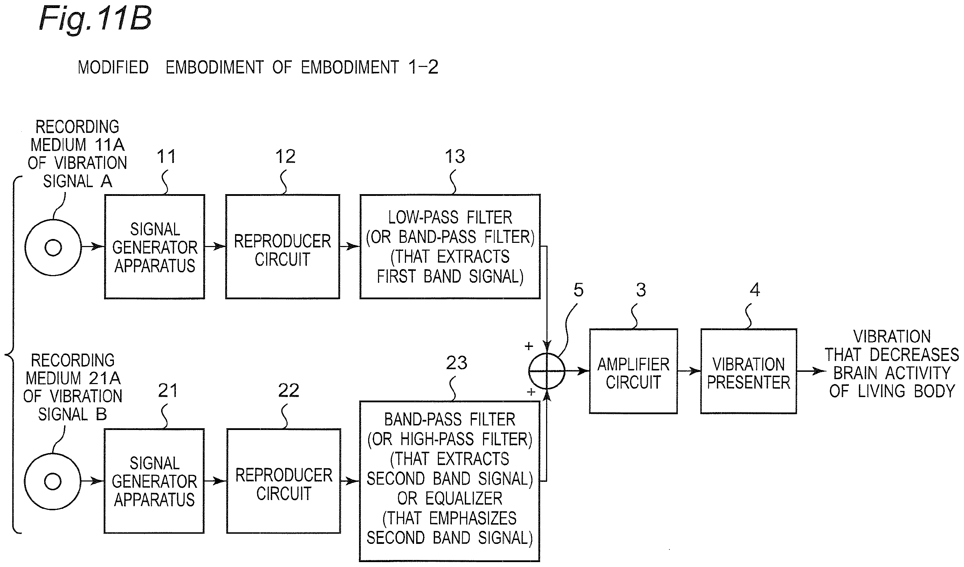

Figure 11B shows a modified embodiment of the embodiment 1-2 of Figure 11A. A first band signal extracted from the vibration signal A and a second band signal extracted from the vibration signal B are outputted to an adder 5. The two signals inputted to the adder are added together, amplified by the amplifier circuit 3, thereafter converted to a vibration by the vibration presenter 4 and presented to the animals including human beings. By this operation, the negative effect of decreasing the brain activity is induced. The vibration generator apparatuses 11 and 21 are each allowed to have a built-in memory to preparatorily store a predetermined vibration signal in the vibration signal source and to generate the vibration signal. Moreover, it is acceptable to use a vibration signal synthesizing apparatus or a combination of a musical instrument or a vibration generator apparatus or vibration generating source 1D shown in Figure 10C to generate a voice, an environmental sound or the like and a signal transducer apparatus such as a microphone or a pickup, wiredly or wirelessly receive broadcasted and communicated signals such as the audio signals of digital broadcasting and high-resolution distribution or use a signal inputted from external equipment in place of the recording medium of the vibration signal, the signal generator apparatus and the reproducer circuit. Further, the generated vibration may be applied as it is to a living body via a vibrating body without being converted into a signal.

In the apparatuses of Figures 11A and 11B, the filter to extract the components of the first band may be a band-pass filter instead of the low-pass filter 13. If the band originally included in the vibration signal A is only the first band, then the filter may be eliminated. Moreover, when the filter 23 to extract the components of the second band may be a high-pass filter instead of the band-pass filter 23 when the band originally included in the vibration signal B is, for example, lower than 32 kHz, or the filter may be eliminated when the band originally included in the vibration signal B is only the second band. Otherwise, it is acceptable to emphasize the second band signal by an equalizer or attenuate the band signals other than the second band.

Next, the operational effects and applications of the vibration presenting apparatus, method and space of the first embodiment are described below. By presenting a vibration from the apparatus to living bodies including human beings and animals, it becomes possible to promptly, noninvasively and reversibly induce a decrease in the brain activity, and to safely and effectively produce a pathologic state model by comparison to the means to use chemicals. A "pathological model generator apparatus" utilizing this effect is considered. Moreover, when the brain activity of the test human subject is temporarily increased by experiment in each experiment using the test human subject, it becomes possible to efficiently perform the next experiment by rapidly cooling it down and putting it back to the initial state by using the apparatus of the present embodiment. A "test human subject cooling down apparatus" utilizing this effect is considered. Further, by using the apparatus of the present embodiment, it becomes possible to strongly control the actions of the animals including human beings by pleasant or unpleasant sensation, decrease the activity of the emotion system included in the fundamental brain, and induce escapement behaviors. By utilizing this effect, applications to "silent honker equipped for automobiles" and "animal expulsion apparatus" are considered. Moreover, by utilizing this effect, an application for cooldown use by decreasing the activity of the fundamental brain in duty spaces, living spaces, amusement spaces, public spaces, vehicles and so on.

### EMBODIMENT 1A: SUPPRESSION OR REDUCTION OF NEGATIVE EFFECT

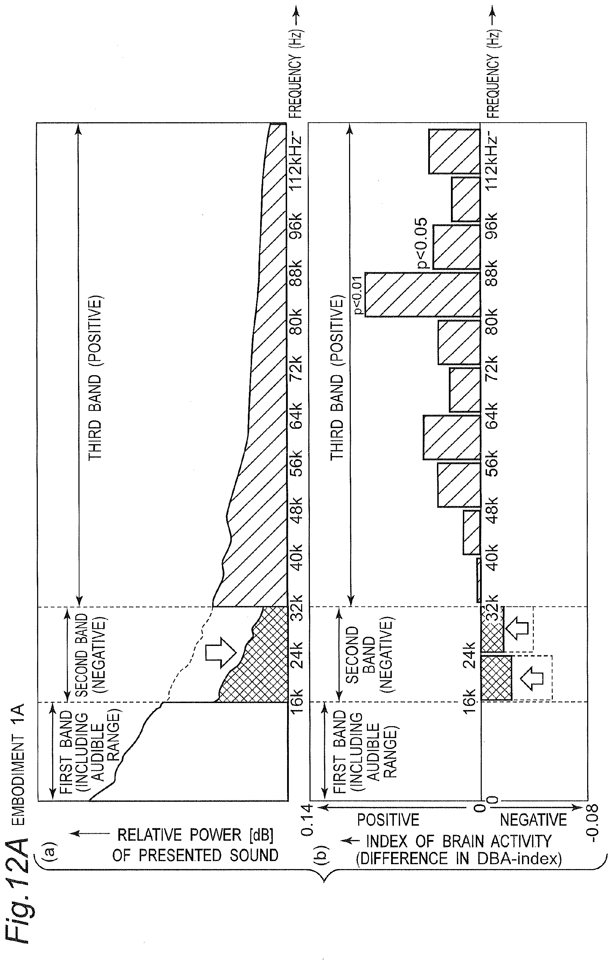

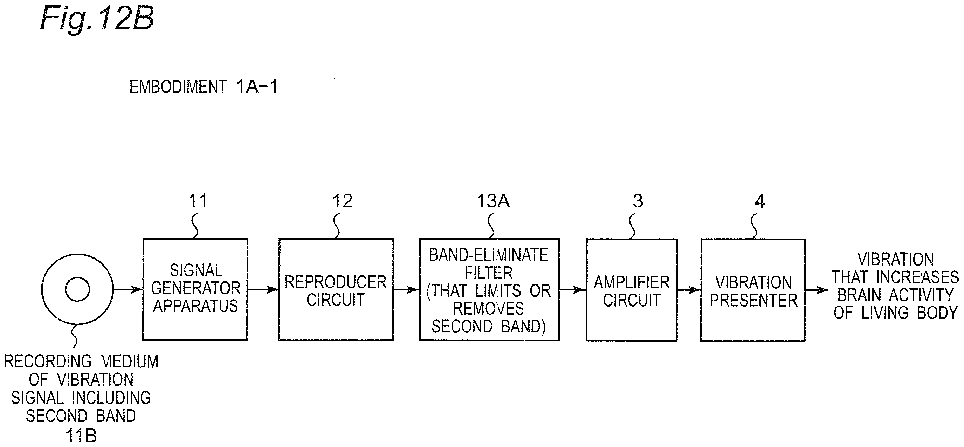

Figure 12A is a graph showing a vibration processing method (suppression or reduction of the negative effect) of a vibration processing apparatus including a vibration presenting apparatus and a vibration attenuator apparatus according to an embodiment 1-A, where Figure 12(a) is a schematic graph of the power spectrum of the presented vibrations of the divided bands regarding a vibration signal that has first and second bands, and Figure 12(b) is a graph showing an index of brain activity (difference in DBA-index) of the divided bands. Referring to Figure 12A, the vibration presenting apparatus, method and space according to the embodiment 1-A are characterized in that the negative effect of decreasing the brain activity is suppressed or reduced by removing or attenuating the vibration or vibration signal of the second band or both of them.

Figure 12B is a block diagram showing a configuration of a vibration presenting apparatus (suppression or reduction of the negative effect) according to an embodiment 1A-1. Referring to Figure 12B, the vibration presenting apparatus according to the embodiment 1A-1 is configured to include a signal generator apparatus 11 including the drive of a recording medium 11B of a vibration signal including the second band, a reproducer circuit 12, a band-eliminate filter (or equalizer) 13A, an amplifier circuit 3, and a vibration presenter 4. Referring to Figure 12B, the signal is read from the recording medium 11B of the vibration signal including the second band in the vibration source by the signal generator apparatus 11 and reproduced by the reproducer circuit 12. By removing or attenuating only the vibration components of the second band by the band-eliminate filter (or equalizer) 13A, thereafter amplifying the resulting signal by the amplifier circuit 3, converting it into a vibration by the vibration presenter 4 and presenting it to a living body such as a human being, the effect of suppressing or alleviating a decrease in the brain activity or conversely enhancing it is induced. It is noted that the signal generator apparatus 11 is allowed to have a built-in memory to preparatorily store the vibration signal of the aforementioned band of the vibration signal source and to generate the vibration signal. Moreover, it is acceptable to use a vibration signal synthesizing apparatus or a combination of a vibration generator apparatus or vibration generating source to generate a voice, a music, an environmental sound or the like and a signal transducer apparatus such as a microphone or a pickup, wiredly or wirelessly receive broadcasted and communicated signals such as the audio signals of digital broadcasting and high-resolution distribution or use a signal inputted from external equipment in place of the recording medium 11B of the vibration signal, the signal generator apparatus 11 and the reproducer circuit 12.

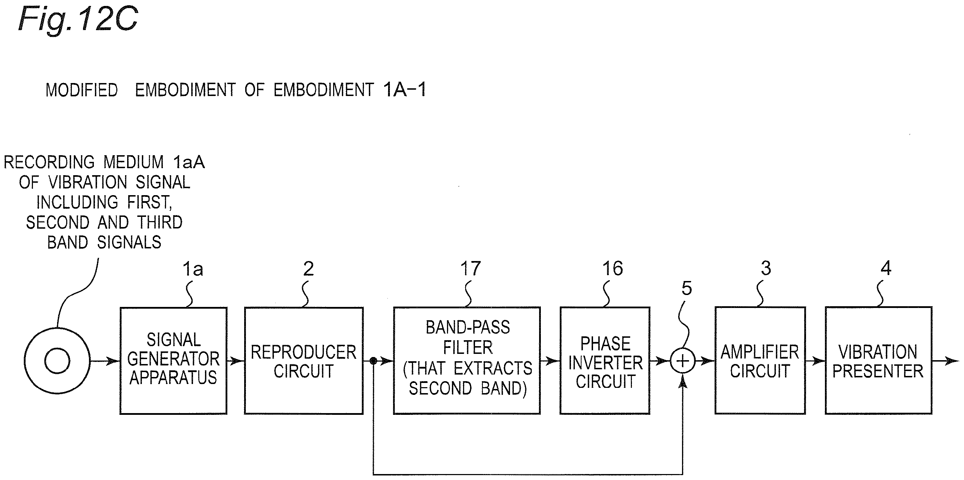

Figure 12C is a block diagram showing a configuration of a modified embodiment of the embodiment 1A-1 of Figure 12B. The vibration presenting apparatus of Figure 12C replaces the band-eliminate filter 13A with a band-pass filter 17, a phase inverter circuit 16 and an adder 5 by comparison to the vibration presenting apparatus of Figure 12B. In this case, the effect of suppressing and alleviating the decrease in the brain activity is induced by counterbalancing and attenuating the second band components included in the original vibration signal by extracting a vibration signal having the second frequency band components that induce the negative effect contained in the vibration signal that contains the first, second and third band signals by the band-pass filter 17, thereafter inverting the phase by the phase inverter circuit 16 and adding the resulting signal to the original vibration signal.

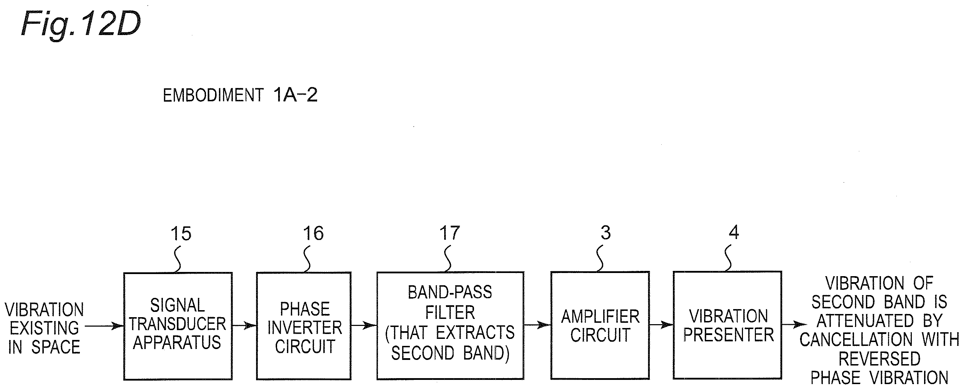

Figure 12D is a block diagram showing a configuration of a vibration presenting apparatus including a vibration attenuator apparatus (suppression or reduction of the negative effect) according to an embodiment 1A-2. Referring to Figure 12D, the vibration presenting apparatus according to the embodiment 1A-2 is configured to include a signal transducer apparatus 15 that detects a vibration existing in the space by a signal detector such as a microphone and converts it into a vibration signal, a phase inverter circuit 16, a band-pass filter (or equalizer) 17, an amplifier circuit 3, and a vibration presenter 4. The effect of suppressing or alleviating a decrease in the brain activity is induced by counterbalancing and attenuating in real time the second band components existing in the space by further applying a vibration having the anti-phase components of the second frequency band components that induce the negative effect included in the aerial vibration existing in the space to the identical space. In this case, the aerial vibration existing in the space may be either a vibration generated by some vibration generator apparatus like an audio system or a musical instrument or a vibration generated by some vibration generating source existing in the space like an environmental sound. Moreover, the order of the phase inverter circuit 16 and the band-pass filter 17 may be reversed in Figure 12D.

Moreover, it is acceptable to provide the vibration signal inputted to the band-pass filter (or equalizer) 17 by a vibration signal read from a recording medium including the package media of, for example, CD, DVD, Blu-ray (registered trademark) and a solid memory, wiredly or wirelessly received signals broadcasted and communicated such as the audio signals of digital broadcasting and high-resolution distribution or a signal inputted from external equipment besides the signal obtained by converting the vibration existing in the space into a vibration signal by the signal transducer apparatus 15 such as a microphone and a pickup.

Although the vibration of the second band is counterbalanced and attenuated by the anti-phase vibration regarding the vibration existing in the space of Figure 12D, the present invention is not limited to this, and it is acceptable to counterbalance and attenuate the vibration existing in the space with the anti-phase vibration by using the vibration signal of the second band in the vibration presenting apparatus in the following modified embodiment.

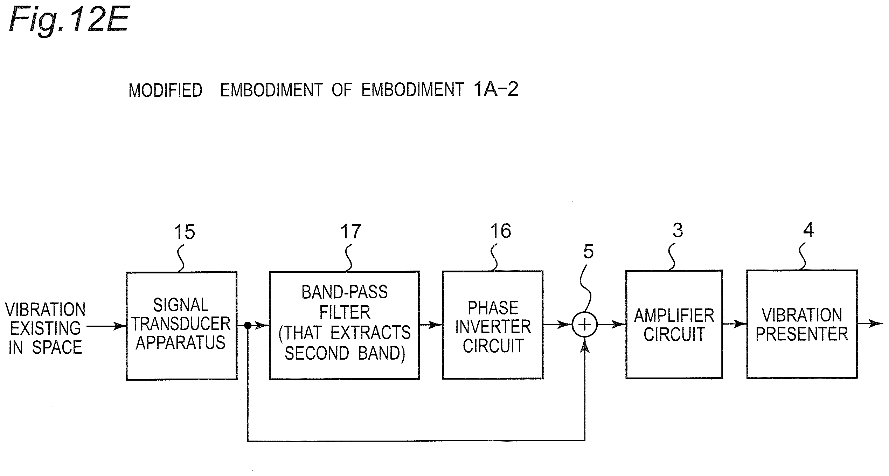

Figure 12E is a block diagram showing a configuration of a modified embodiment of the embodiment 1A-2 of Figure 12D. Referring to Figure 12E, the vibration presenting apparatus is configured to include a signal transducer apparatus 15 that detects a vibration existing in the space by a signal detector such as a microphone and converts it into a vibration signal, a band-pass filter 17 that extracts the second band, a phase inverter circuit 16, an adder 5, an amplifier circuit 3, and a vibration presenter 4. Referring to Figure 12E, the effects of suppressing or alleviating the decrease in the brain activity is induced by generating a vibration signal obtained by counterbalancing and attenuating in real time the second band components existing in the space and presenting it as a vibration in the space by extracting the vibration signal of the second frequency band components that induce the negative effect included in the aerial vibration existing in the space by the band-pass filter 17, thereafter inverting the phase by the phase inverter circuit 16 and adding the resulting signal to the vibration signal of the original aerial vibration.

Vibration processing apparatuses or vibration presenting apparatuses including various vibration attenuator apparatuses to remove or attenuate the band that has the negative effect are considered by subjecting the aerial vibration that propagates in the air to acoustic processing besides the above embodiment 1A. They can be classified roughly into the following two types.
(A) An absorber apparatus: an apparatus that removes, limits or attenuates the vibration components of the second band having the negative effect by utilizing the fact that the vibration of a specific band is selectively absorbed when the aerial vibration passes through a matter.
(B) A reflector apparatus: an apparatus that removes, limits or attenuates the vibration components of the second band having the negative effect by utilizing the fact that the vibration of a specific band is selectively reflected when the aerial vibration collides with a matter and reflected on it.

The operational effects and applications of the vibration presenting apparatus, method and space of the present embodiment are described below. The majority of the sound environments including traffic noises and artificial sounds generated by electronic equipment propagating in the modern society, signals recorded in audio digital media such as CDs and DVDs that are currently widely used, voices, music signals and their reproduced sounds distributed or transmitted by broadcasting or communications and so on have their recordable and reproducible frequency bands limited to the aforementioned first band of audible range components and part of the second band that has the negative effect. Moreover, in the cases of high-resolution distribution capable of including the third band in terms of the standard and Blu-ray (registered trademark) Discs that adopt the ultra-wideband standards of the Dolby True HD system and the like, there are not a few cases where the vibration signal of the third band that substantially has the positive effect is scarcely included in the contents, i.e., cases where the vibration signal components are limited to the second band that has the negative effect. It is more serious that, even in the case of contents that contain the vibration signal components of the third band having the positive effect, they include the second band having the negative effect, and the vibration of the second band is possibly reproduced. Therefore, if exposed to the sound reproduced by such audio digital media for a long time, the brain activity decreases to possibly have the risk of causing serious morbid states. Accordingly, it becomes possible to suppress or alleviate the negative effect and avoid the decrease in the brain activity by presenting the vibration with the second band having the negative effect removed or attenuated by using the apparatus, method and space of the present embodiment. By avoiding the risk of the brain activity decrease owned by the conventional audio digital media with this arrangement, it becomes possible to provide electronic equipment, audio equipment, communication equipment, broadcasting equipment and the like, which improve the pleasant sensation and prevent various diseases attributed to the decrease in the brain activity.

### EMBODIMENT 2: STRONG POSITIVE EFFECT

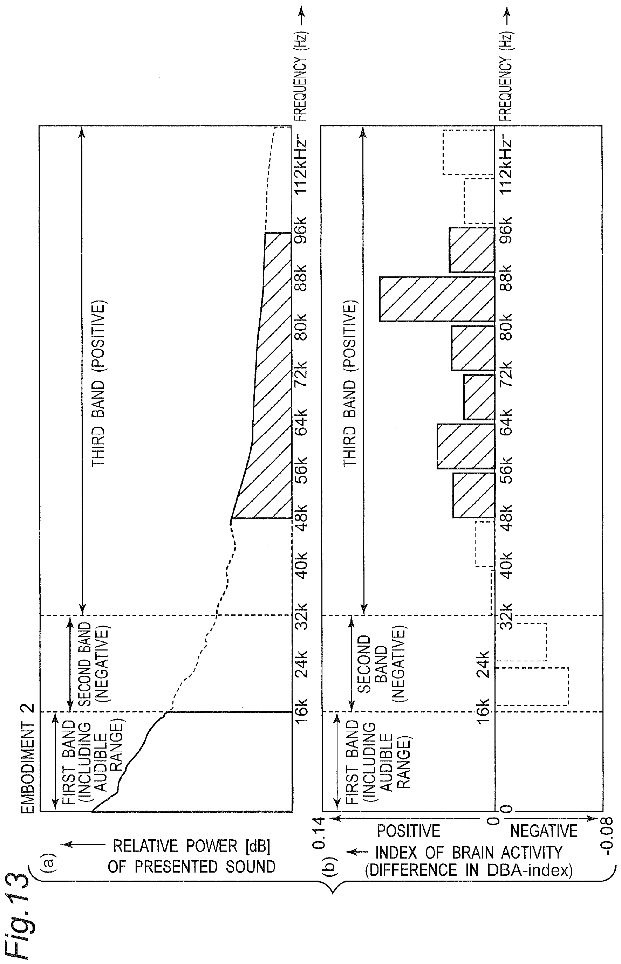

Figure 13 is a graph showing a vibration presentation method (strong positive effect) of a vibration presenting apparatus according to an embodiment 2, where Figure 13(a) is a schematic graph of the power spectrum of the presented vibrations of the divided bands regarding a vibration signal that has the first and third bands, and Figure 13(b) is a graph showing an index of brain activity (difference in DBA-index) of the divided bands. Referring to Figure 13, the vibration presenting apparatus, method and space according to the embodiment 2 are characterized in that the "strong positive effect" to strongly enhance the brain activity is induced by not presenting the vibration of the second band having the negative effect (i.e., presentation of the vibration is inhibited or limited) and presenting the third band having the positive effect or its part. An example of the apparatus, method and space to induce the positive effect of strongly enhancing the brain activity by presenting a vibration that includes the first band and the third band and does not include the second band is shown below. When presentation of the vibration is limited, it is possible to decrease or attenuate the level of each predetermined band by using, for example, an equalizer that has a plurality of band-pass filters and adjusts the level of it. Moreover, frequencies corresponding to the boundaries of the bands are shown as one example on the listening conditions of the present experiment, and it is considered that they assume different values depending on the vibration presentation conditions or the vibration listening conditions or the species of the animals served as the subject.

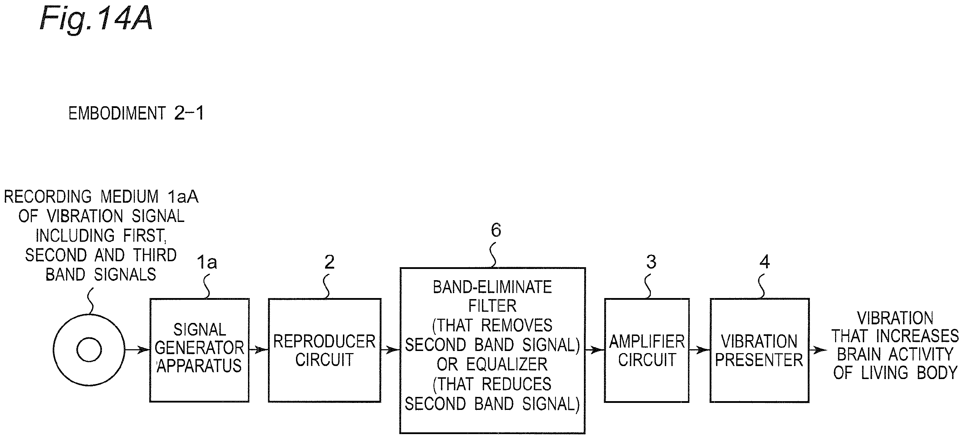

Figure 14A is a block diagram showing a configuration of a vibration presenting apparatus according to an embodiment 2-1. Referring to Figure 14A, the vibration presenting apparatus according to the embodiment 2-1 is configured to include a signal generator apparatus 1a including the drive of a recording medium 1aA of a vibration signal, a reproducer circuit 2, a band-eliminate filter or equalizer 6, an amplifier circuit 3, and a vibration presenter 4. Referring to Figure 14A, a signal of, for example, the sound signal (containing frequencies up to 96 kHz in the case of recording by the Dolby True HD system) of the audio track of a Blu-ray (registered trademark) Disc is read from the recording medium 1aA of the vibration signal that include all of the first band, the second band and the third band originally in the vibration source by the signal generator apparatus 1a and reproduced by the reproducer circuit 2. Only the vibration components of the second band are removed or limited by the band-eliminate filter (or equalizer) 6 or the vibration components of the third band are enhanced in the signal, thereafter amplified by the amplifier circuit 3, converted into a vibration by the vibration presenter 4 and presented to the living body of a human being or the like, and this leads to inducing the positive effect of strongly enhancing the brain activity. It is noted that the signal generator apparatus 1a is allowed to have a built-in memory to preparatorily store the vibration signal of the band as a vibration signal source and to generate the vibration signal. Moreover, it is acceptable to use a vibration signal synthesizing apparatus or a combination of a vibration generator apparatus or vibration generating source to generate a voice, a music, an environmental sound or the like and a signal transducer apparatus such as a microphone or a pickup, wiredly or wirelessly receive broadcasted and communicated signals such as the audio signals of digital broadcasting and high-resolution distribution or use a signal inputted from external equipment in place of the recording medium of the vibration signal, the signal generator apparatus and the reproducer circuit. Further, the vibration generated by the vibration generator apparatus may be applied as it is to a living body without being converted into a signal.

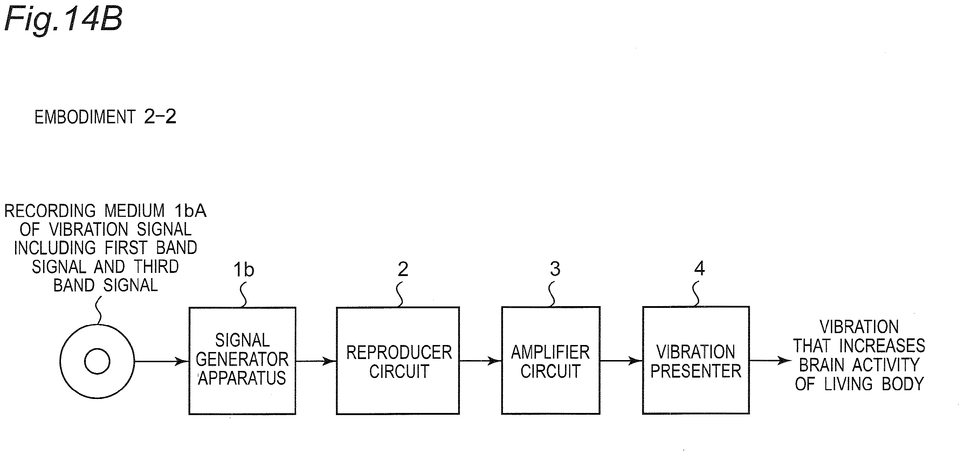

Figure 14B is a block diagram showing a configuration of a vibration presenting apparatus according to an embodiment 2-2. Referring to Figure 14B, the apparatus is configured to include a signal generator apparatus 1b including the drive of a recording medium IbA of a vibration signal, a reproducer circuit 2, an amplifier circuit 3, and a vibration presenter 4. The apparatus of Figure 14B is characterized in that the recording medium IbA of the vibration signal including the first band and the third band and the signal generator apparatus 1b are provided in place of the recording medium 1aA1 of the vibration signal including the first band and the second band and the signal generator apparatus 1a by comparison to the apparatus of Figure 10A. The signal generator apparatus is allowed to have a built-in memory to preparatorily store the vibration signal of the above-mentioned band of the vibration signal source and to generate the vibration signal. Moreover, it is acceptable to use a vibration signal synthesizing apparatus to synthesize a vibration signal including the first band and the third band or a combination of a vibration generator apparatus or vibration generating source that generates a vibration including the first band and the third band as shown in Figure 10C and generates a voice, a music, an environmental sound or the like and a signal transducer apparatus such as a microphone or a pickup, wiredly or wirelessly receive broadcasted and communicated signals such as the audio signals of digital broadcasting and high-resolution distribution or use a signal inputted from external equipment in place of the recording medium of the vibration signal, the signal generator apparatus and the reproducer circuit. Further, the vibration generated by the vibration generator apparatus may be applied as it is to a living body without being converted into a signal.

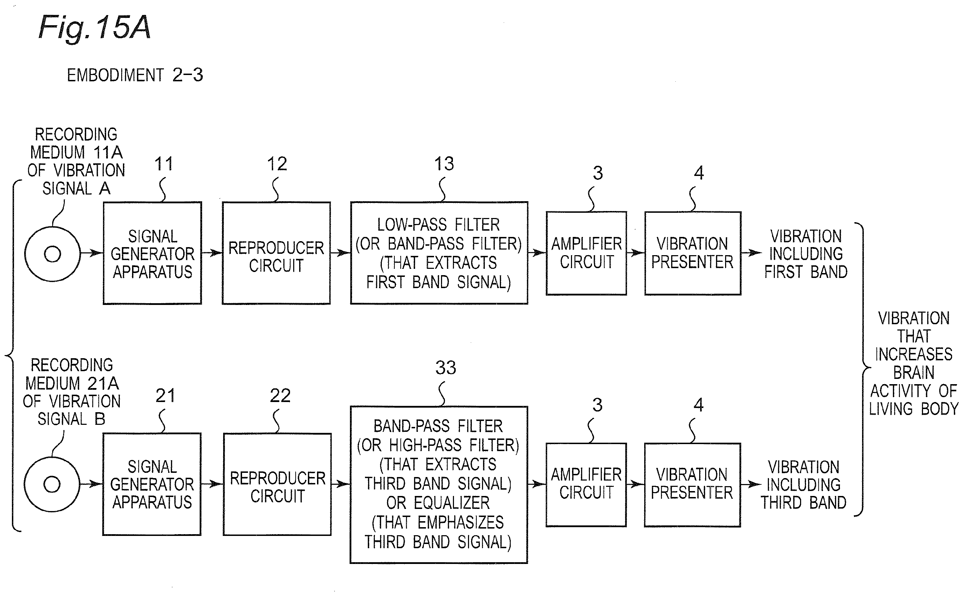

Figure 15A is a block diagram showing a configuration of a vibration presenting apparatus according to an embodiment 2-3. Referring to Figure 15A, the vibration presenting apparatus A is configured to include a signal generator apparatus 11 including the drive of a recording medium 11A of a vibration signal A, a reproducer circuit 12, a low-pass filter 13, an amplifier circuit 3, and a vibration presenter 4. The vibration presenting apparatus B is configured to include a signal generator apparatus 21 including the drive of a recording medium 21A of a vibration signal B, a reproducer circuit 22, a band-pass filter or equalizer 33, an amplifier circuit 3, and a vibration presenter 4. The apparatus of Figure 15A is characterized in that the band-pass filter or equalizer 33 that passes only the third band components is provided in place of the band-pass filter 23 that passes only the second band components by comparison to the apparatus of Figure 11A. In the present embodiment, the brain activity of a living body can be increased by presenting a vibration including the first band and a vibration including the third band. The signal generator apparatus is allowed to have a built-in memory to preparatorily store the vibration signal of the band and to generate the vibration signal. Moreover, it is acceptable to use a vibration signal synthesizing apparatus or a combination of a vibration generator apparatus or vibration generating source as shown in Figure 10C that generates a voice, a music, an environmental sound or the like and a signal transducer apparatus such as a microphone or a pickup, wiredly or wirelessly receive broadcasted and communicated signals such as the audio signals of digital broadcasting and high-resolution distribution or use a signal inputted from external equipment in place of the recording medium of the vibration signal, the signal generator apparatus and the reproducer circuit. Further, the vibration generated by the vibration generator apparatus may be applied as it is to a living body without being converted into a signal.

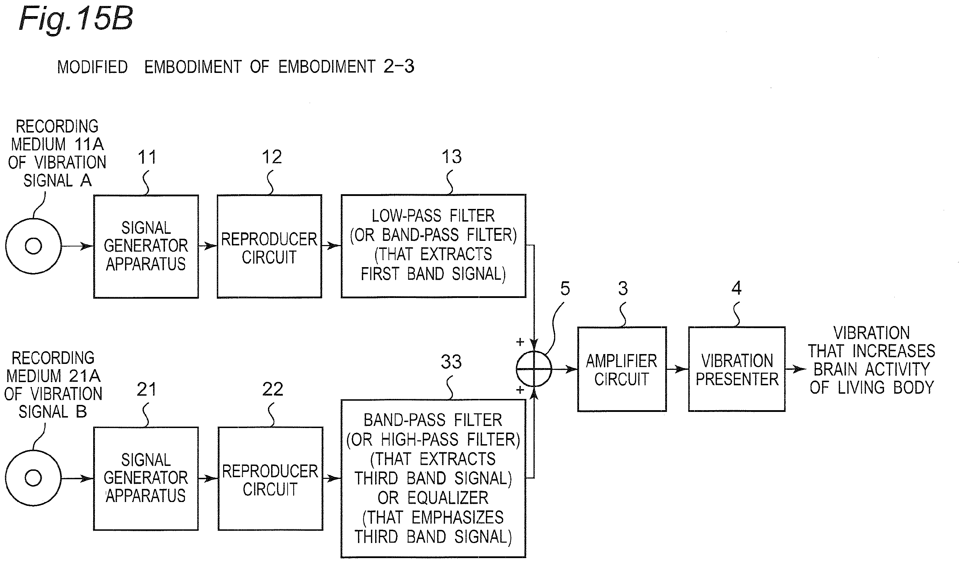

Figure 15B shows a modified embodiment of the embodiment 2-3 of Figure 15A. Referring to Figure 15B, the signal generator apparatuses 11 and 21 generate vibration signals of both the first band and the third band, respectively, or extract and store them from identical or separate vibration signal sources. By reading these vibration signals, reproducing and filtering the signals, adding them together, converting them into a vibration and presenting it, the positive effect of strongly enhancing the brain activity is induced. For example, the components of, for example, the first band of equal to or lower than 16 kHz are extracted from the vibration signal A by the low-pass filter 13, and, the components of, for example, the third band of 48 kHz to 96 kHz are extracted from the vibration signal B by the band-pass filter or equalizer 33. Then, both are added together, amplified, thereafter converted into a vibration and presented. It is noted that the signal generator apparatuses are each allowed to have a built-in memory to preparatorily store the vibration signals of the bands of the vibration signal source and to generate the vibration signal. Moreover, it is acceptable to use a vibration signal synthesizing apparatus or a combination of a vibration generator apparatus or vibration generating source that generates a voice, a music, an environmental sound or the like and a signal transducer apparatus such as a microphone or a pickup, wiredly or wirelessly receive broadcasted and communicated signals such as the audio signals of digital broadcasting and high-resolution distribution or use a signal inputted from external equipment in place of the recording medium of the vibration signal, the signal generator apparatus and the reproducer circuit. Further, the vibration generated by the vibration generator apparatus may be applied as it is to a living body without being converted into a signal.

In the present embodiment, the filter to extract the components of the first band may not be the low-pass filter 13 but a band-pass filter, and the filter may be eliminated if the band originally included in the vibration signal A is only the first band. Moreover, the filter to extract the components of the third band may not be the band-pass filter 33 but a high-pass filter, and the filter may be eliminated if the band originally included in the vibration signal B is only the third band. Otherwise, it is acceptable to emphasize the third band signal by an equalizer or attenuate the band signals other than the third band.

Next, the operational effects and applications of the apparatus, method and space according to the embodiment 2 are described below. By using this apparatus, it becomes possible to strongly increase the brain activity. With this arrangement, in a case where some negative factor to decrease the brain activity exist, there is the effect of removing it and securing the safety. Moreover, there is the effect of increasing the pleasant sensation and aesthetic sensitivity. Applications to improve the pleasant sensations in living spaces, duty spaces, amusement spaces, public spaces, vehicles and so on by utilizing this effect are considered. Moreover, an application to means for increasing the expressive effects of artistic productions in museums, concert halls and so on is considered. Moreover, there is the effect of alleviating unpleasant sensation, irritative feeling, anger and the like. By using this apparatus for troubles such as quarrels and violence in public spaces such as stations, which pose serious problems in recent years, by utilizing this effect, an application to means for avoiding the troubles by turning the brain activity decrease state to the direction of increase is considered.

### EMBODIMENT 3: COMBINATION OF BANDS

The vibration processing apparatus, method and space according to an embodiment 3 are characterized in that the degree of a decrease or an increase in the brain activity level is arbitrarily controlled including canceling the negative effect to induce a decrease in the brain activity by presenting the vibration of the band that has the "positive effect" to induce an increase in the brain activity in combination with the vibration of the second band that has the "negative effect" to induce a decrease in the brain activity.

Figure 16 is a graph showing a vibration presentation method (presentation method of a combination of bands to maintain the brain activity substantially constantly) of a vibration presenting apparatus according to an embodiment 3-1, where Figure 16(a) is a schematic graph of the power spectrum of the presented vibrations of the divided bands regarding a vibration signal that has the first and second bands and the third band, Figure 16(b) is a graph showing an index of brain activity (difference in DBA-index) in the divided bands, and Figure 16(c) is a graph showing a temporal transition of the presentation condition of the vibration presentation method. The vibration processing apparatus, method and space according to the embodiment 3-1 is characterized in that the negative effect is cancelled and the brain activity is maintained almost substantially constantly by presenting the vibration including the third band in combination with the vibration including the first band and the second band.

Referring to Figure 16, vibrations of the first band (e.g., 20 Hz to 16 kHz), the second band (e.g., 16 kHz to 32 kHz) and part of the third band (e.g., 48 kHz to 64 kHz) are intermittently presented for, for example, five minutes every five minutes. This method is able to cancel the negative effect of the second band and to maintain the brain activity at a substantially constant level by presenting a vibration including the third band having the positive effect simultaneously in combination with the vibration of the second band that has the negative effect hardly removable for some reasons. It is noted that the frequencies corresponding to the boundaries of the bands in Figure 16 are shown as one example on the listening conditions of the present experiment, and it is considered that they assume different values depending on the vibration presentation conditions or the vibration listening conditions or the species of the animals served as the subject.

Figure 17 is a graph showing a vibration presentation method (presentation method of a combination of bands to induce a feeble increase in the brain activity) of a vibration presenting apparatus according to an embodiment 3-2, where Figure 17(a) is a schematic graph of the power spectrum of the presented vibrations of the divided bands regarding a vibration signal that has the first and second bands and the third band, Figure 17(b) is a graph showing an index of brain activity (difference in DBA-index) in the divided bands, and Figure 17(c) is a graph showing a temporal transition of the presentation condition of the vibration presentation method. The vibration processing apparatus, method and space according to the embodiment 3-2 are characterized in that a faint brain activity increase is induced.

Referring to Figure 17, the vibration of part of the third band (e.g., 48 kHz to 72 kHz) is presented in addition to the vibrations of the first band (e.g., 20 Hz to 16 kHz) and the second band (e.g., 16 kHz to 24 kHz) continuously for, for example, 20 minutes. This method is able to cancel the negative effect of the second band hardly removable for some reasons and further induce a faint brain activity increase since the vibration of the second band having a weak negative effect and the third band having a slightly stronger positive effect are simultaneously presented. It is noted that the frequencies corresponding to the boundaries of the bands of Figure 17 are shown as one example on the listening conditions of the present experiment, and it is considered that they assume different values differing depending on the vibration presentation conditions or the vibration listening conditions or the species of the animals served as the subject.

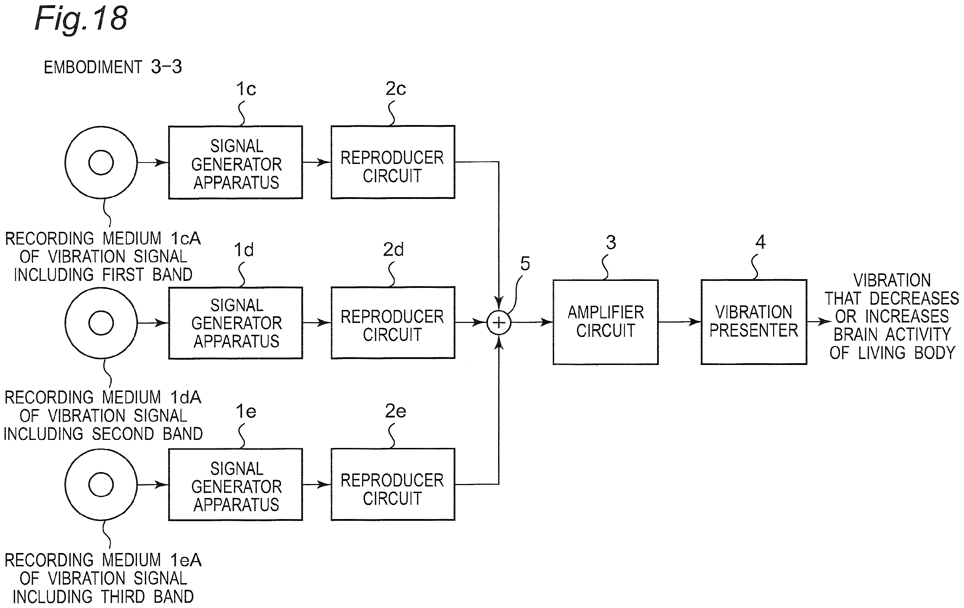

Figure 18 is a block diagram showing a configuration of a vibration presenting apparatus according to an embodiment 3-3. Referring to Figure 18, the vibration presenting apparatus is configured to include signal generator apparatuses 1c, 1d and 1e including the drives of recording media IcA, IdA and 1eA of three vibration signals that include the first band, the second band and the third band, respectively, reproducer circuits 2c, 2d and 2e, an adder (or a mixing circuit capable of adjusting the addition level) 5, an amplifier circuit 3, and a vibration presenter 4. In this case, the signal generator apparatuses 1c, 1d and 1e are each allowed to have a built-in memory to preparatorily store the vibration signals of the bands and to generate the vibration signals. Moreover, it is acceptable to use a vibration signal synthesizing apparatus or a combination of a vibration generator apparatus or vibration generating source that generates a voice, a music, an environmental sound or the like and a signal transducer apparatus such as a microphone or a pickup, wiredly or wirelessly receive broadcasted and communicated signals such as the audio signals of digital broadcasting and high-resolution distribution or use a signal inputted from external equipment in place of the recording media IcA, IdA and 1eA of the vibration signals, the signal generator apparatuses 1c, 1d and 1e and the reproducer circuits 2c, 2d and 2e. Further, the vibration generated by the vibration generator apparatus may be applied as it is to a living body without being converted into a signal.

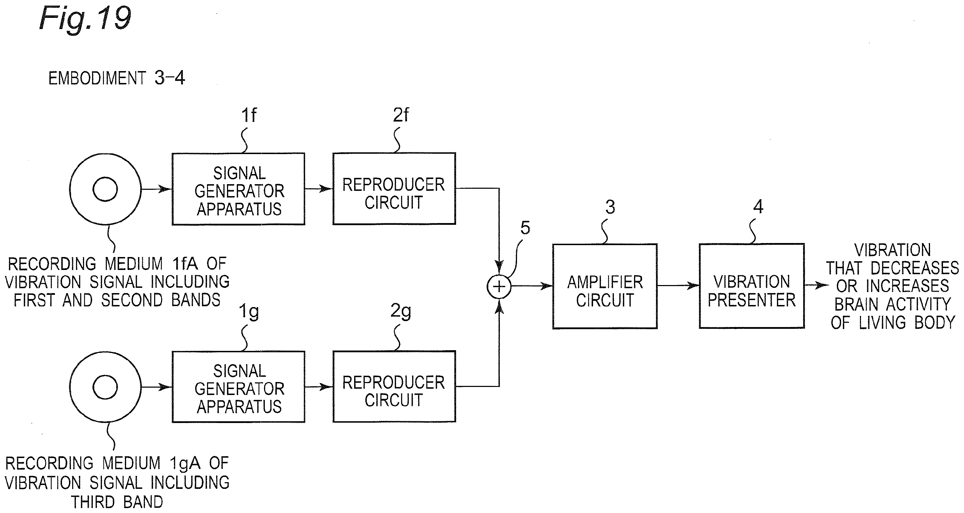

Figure 19 is a block diagram showing a configuration of a vibration presenting apparatus according to an embodiment 3-4. Referring to Figure 19, the vibration presenting apparatus is configured to include a signal generator circuit If including the drive of a recording medium 1fA of a vibration signal including the first band and the second band, a signal generator apparatus 1g including the drive of a recording medium 1gA of a vibration signal of the third band, reproducer circuits 2f and 2g, an adder (or mixing circuit capable of adjusting the addition level) 5, an amplifier circuit 3, and a vibration presenter 4. In this case, the signal generator apparatuses 1f and 1g are each allowed to have a built-in memory to preparatorily store the vibration signals of the bands of the vibration signal sources and to generate the vibration signals. Moreover, it is acceptable to use a vibration signal synthesizing apparatus or a combination of a vibration generator apparatus or vibration generating source that generates a voice, a music, an environmental sound or the like and a signal transducer apparatus such as a microphone or a pickup, wiredly or wirelessly receive broadcasted and communicated signals such as the audio signals of digital broadcasting and high-resolution distribution or use a signal inputted from external equipment in place of the recording media 1fA and 1gA of the vibration signals, the signal generator apparatuses 1f and 1g and the reproducer circuits 2f and 2g. Further, the vibration generated by the vibration generator apparatus may be applied as it is to a living body without being converted into a signal.

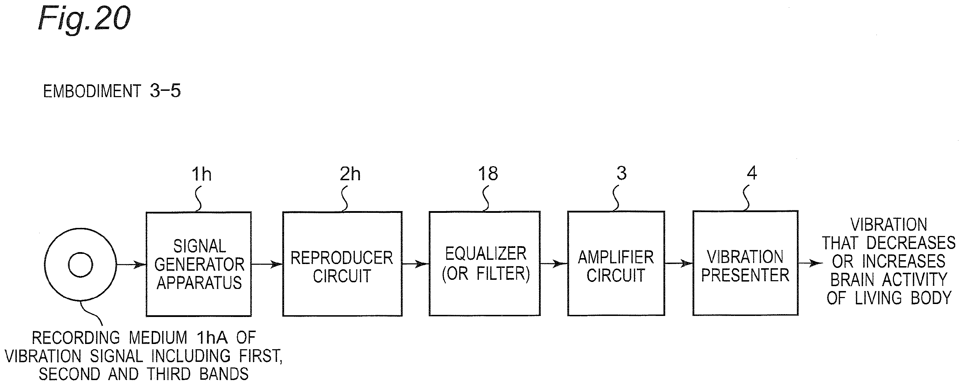

Figure 20 is a block diagram showing a configuration of a vibration presenting apparatus according to an embodiment 3-5. Referring to Figure 20, the vibration presenting apparatus is configured to include a signal generator apparatus 1h including the drive of a recording medium 1hA of a vibration signal including each of the first band, the second band and the third band, a reproducer circuit 2h, an equalizer (or filter) 18 to change the balance of band components, an amplifier circuit 3, and a vibration presenter 4. The balance of the intensities of the first, second and third bands can be changed by the equalizer (or filter) 18. It is acceptable to use one in which the vibrations of the first, second and third bands are preparatorily recorded at a predetermined balance as the recording medium of the vibration signal without using the equalizer (or filter) 18. The signal generator apparatus 1h is allowed to have a built-in memory to preparatorily store the vibration signals of the bands of the vibration signal source and to generate the vibration signals. Moreover, it is acceptable to use a vibration signal synthesizing apparatus or a combination of a vibration generator apparatus or vibration generating source that generates a voice, a music, an environmental sound or the like and a signal transducer apparatus such as a microphone or a pickup, wiredly or wirelessly receive broadcasted and communicated signals such as the audio signals of digital broadcasting and high-resolution distribution or use a signal inputted from external equipment in place of the recording medium 1hA of the vibration signal, the signal generator apparatus 1h and the reproducer circuit 2h. Further, the vibration generated by the vibration generator apparatus may be applied as it is to a living body without being converted into a signal.

Although such a mode that the first, second, and third bands are presented from an identical vibration presenting apparatus has been shown regarding each of the aforementioned embodiments, they may be separately presented. Moreover, the first, second, and third band components may each be a sound existing in the space. That is, in a case where, for example, the first band and the second band are predominant regarding the sound existing as an environmental sound in a space, there may be the effect of alleviating the decrease in the brain activity or inducing an increase in the brain activity by presenting a vibration including the third band from another vibration generator apparatus and presenting it in accordance with the acoustic situation in the space.

The operational effects and applications of the vibration presenting apparatus, method and space of the present embodiment are described below. As described hereinabove, the majority of the sound environments including traffic noises and artificial sounds generated by electronic equipment propagating in the modern society, signals recorded in audio digital media such as CDs and DVDs that are currently widely used, voices, music signals and their reproduced sounds distributed or transmitted by broadcasting or communications and so on have their recordable and reproducible frequency bands limited to the aforementioned first band of audible range components and part of the second band that has the negative effect. Moreover, in the cases of high-resolution distribution capable of including the third band in terms of the standard and Blu-ray (registered trademark) Discs that adopt the ultra-wideband standards of the Dolby True HD system and the like, there are not a few cases where the vibration signal of the third band that substantially has the positive effect is scarcely included in the contents, i.e., cases where the vibration signal components are limited to the second band that has the negative effect. It is more serious that, even in the case of contents that contain the vibration signal components of the third band having the positive effect, they include the second band having the negative effect, and the vibration of the second band is possibly reproduced. Therefore, if exposed to the sound reproduced by such audio digital media for a long time, the brain activity decreases to possibly have the risk of causing serious morbid states. Furthermore, it is often the case where the second band can be hardly removed. Accordingly, by using the apparatus, method and space of the present embodiment, it becomes possible to cancel the negative effect and avoid the brain activity decrease by presenting the vibration of the third band having the positive effect in combination with it. Furthermore, the brain activity can be increased by further emphasizing the vibration of the third band. By thus avoiding the risk of the brain activity decrease owned by the conventional audio digital media, it becomes possible to provide electronic equipment, audio equipment, communication equipment, broadcasting equipment and the like to improve the pleasant sensation and prevent various diseases attributed to the decrease in the brain activity.

Next, embodiments related to an apparatus for complementation with a vibration that can induce an increase or a decrease in the brain activity for an original vibration signal that does not induce the desirable brain activity state are described below.

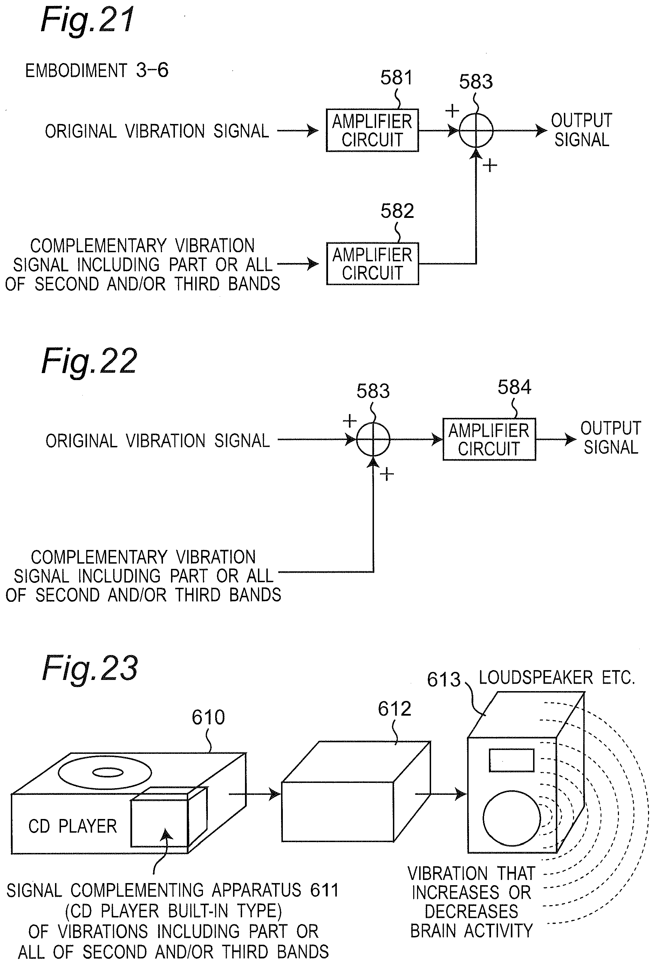

Figure 21 is a block diagram of an apparatus that generates an output signal capable of inducing the fundamental brain activation effect by adding a vibration signal capable of inducing an increase or a decrease in the brain activity to an original vibration signal that does not induce the desirable brain activity state according to an embodiment 3-6. It is noted that each vibration signal is generated by, for example, a vibration signal storage apparatus and its reproducer circuit. Referring to Figure 21, each vibration signal is amplified by amplifiers 581 and 582, and thereafter added together by an adder 583. Even if, for example, a vibration signal that does not contain the vibration signal of the third band like a piano sound and does not induce the fundamental brain activation effect is inputted as an original signal by this apparatus, a vibration signal (hypersonic sound signal) capable of inducing the fundamental brain activation effect can be outputted by complementation with a vibration signal including the vibration signal of the third band. By this operation, there can be obtained the effects of inducing the activation of the fundamental brain and the fundamental brain network (fundamental brain network system) including the reward system neural circuit of the brain responsible for the reactions of pleasantness, beauty and emotion of a human being, and the centers of the autonomic nerve system, the internal secretion system and the immune system, which are responsible for the homeostasis and biophylaxis of the whole body, exalting the aesthetic sensitivity and ameliorating and improving the body state.

Figure 22 is a block diagram showing a modified embodiment of the apparatus of Figure 21. Referring to Figure 22, an output signal capable of inducing an increase or a decrease in the brain activity is obtained by adding an original vibration signal that does not induce the desirable brain activity state to a vibration signal capable of inducing an increase or a decrease in the brain activity by an adder 583 and inputting the resulting signal to an identical amplifier circuit 584.

Figure 23 to Figure 25 show concrete applications. Currently, a very great number of digital formats that cannot record the vibration signal of the third band and analog systems only having a bandwidth that cannot record the vibration signal of the third band are used for the transmission and distribution of vibration signals of musics, voices and the like via package media and broadcasting, communications and the like. Moreover, there are not a few cases where the vibration signal of the third band is not sufficiently contained in the actual contents even when the vibration signal of the third band can be contained in terms of the standard. Vibrations obtained by reproducing the vibration signals recorded or transmitted by these systems cannot induce the fundamental brain activation effect. By using the apparatus of the present invention, it becomes possible to apply a vibration capable of inducing the fundamental brain activation effect to human beings by utilizing the contents that do not induce the fundamental brain activation effect and are accumulated, transmitted and distributed by the existing systems widely popularized in the modern society.

Figure 23 is a perspective view showing an example of a vibration processing apparatus including a vibration complementing apparatus to add a vibration signal capable of inducing the fundamental brain activation effect to an original vibration that does not induce the fundamental brain activation effect according to the embodiment 3-6. In other words, it is an example of a vibration complementing apparatus to add a vibration signal capable of inducing the fundamental brain activation effect to the signal of a digital format that can neither record super-high frequency components including the third band nor induce the fundamental brain activation effect such as the signals of music CDs and the like served as an original vibration signal. This vibration complementing apparatus 611 is built in a CD player 610 and has various built-in storage apparatuses such as a solid memory in which a vibration signal including at least the vibration signal of the third band is recorded. This vibration complementing apparatus reads the vibration signal capable of inducing the fundamental brain activation effect from the storage apparatus, adds it to the original vibration signal that does not contain the vibration signal of the third band read from a CD, and thereafter outputs a vibration signal from the CD player 610. The outputted vibration signal is converted to an aerial vibration by a loudspeaker 613 or the like via an amplifier 612. The aerial vibration converted at this time has become a vibration capable of inducing the fundamental brain activation effect. The above-mentioned vibration complementing apparatus, which is shown as an example built in the CD player, may be an external type. Moreover, here is indicated the example in which the complementation is effected by using the preparatorily set vibration signal capable of inducing the fundamental brain activation effect, the complementation can also be effected by using a vibration signal capable of inducing the fundamental brain activation effect that the user has selected from a plurality of candidates. Although the example in which the brain activity is increased is described here, it is also possible to decrease the brain activity by complementation with the vibration signal of the second band.

The original vibration signals served as the subjects of this vibration complementing apparatus include, besides the aforementioned CD, vibration signals recorded by digital formats that cannot record the vibration signal of the third band in storage media such as DVD video, DVD audio, Blu-ray Disc, hard disk and the like, vibration signals used in equipment using formats that cannot record nor reproduce the vibration signal of the third band of, for example, VTR systems, attraction systems of Theme Parks, game machines and game software, vibration signals that are transmitted and distributed via broadcasting and communications using formats that cannot transmit the vibration signal of the third band such as telephones, TV conference systems, wireless apparatuses and the like, further vibration signals that are transduced into an electrical variation by a transducer from vibrations of solid, liquid, gas and the like using an apparatus that can neither transduce nor transmit the vibration signal of the third band. Moreover, even in the case of a vibration signal recorded by a format that can record the vibration signal of the third band in a storage medium as described above or further in the case of vibration signals that are transduced into an electrical variation by a transducer from vibrations of solid, liquid, gas and the like using an apparatus that can transduce and transmit the vibration signal of the third band, the vibrations are served as the subjects of this vibration complementing apparatus when the vibrations do neither sufficiently contain the vibration of the third band nor induce the fundamental brain activation effect.

By using this apparatus, it becomes possible to apply a vibration capable of inducing the fundamental brain activation effect to human beings by utilizing the existing huge amount of contents in which the vibration signals of the digital formats that can neither record the vibration signal of the third band nor induce the fundamental brain activation effect are recorded. Moreover, it becomes possible to form a vibration capable of inducing the fundamental brain activation effect and apply it to human beings by utilizing the contents constituted of vibration signals that do not induce the fundamental brain activation effect because they do not sufficiently contain the vibration signal of the third band while utilizing the formats of high-resolution audio and the like capable of recording the vibration signal of the third band that are expected to be continuously produced in future.

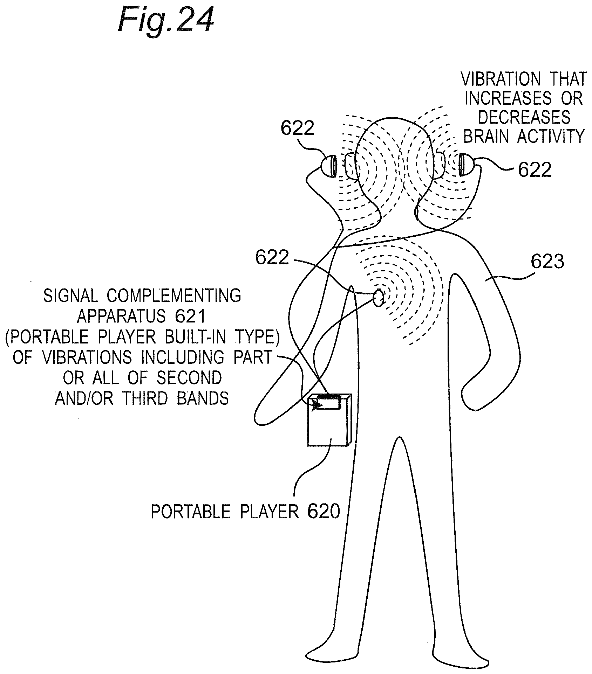

Figure 24 is a perspective view showing an example of a vibration complementing apparatus to add a vibration signal capable of inducing the fundamental brain activation effect to an original vibration that does not induce the fundamental brain activation effect outputted from a portable player or the like according to the embodiment 3-6. Referring to Figure 24, there is shown an example of a vibration complementing apparatus 621 in which a vibration signal that contain at least the vibration signal of the third band and is able to induce the fundamental brain activation effect is added to the signal of a digital format that can neither record the vibration signal of the third band nor induce the fundamental brain activation effect such as the signal of a portable player 620 served as an original vibration signal. This vibration complementing apparatus 621 is mounted in a portable player 620 and has a built-in storage apparatus such as a solid memory in which a vibration signal containing at least the vibration signal of the third band is recorded. This vibration complementing apparatus 621 has a function to read a vibration signal capable of inducing the fundamental brain activation effect from the storage apparatus and add it to a vibration signal that does not contain the vibration signal of the third band read from the solid memory or the like of the portable player 620 and thereafter output a vibration signal from the portable player. The added signal is applied to a human being 623 by an apparatus 622 of a headphone, earphone or the like or an apparatus 622 to apply the vibration to the body surface, or the like. The vibration applied at this time is the vibration capable of inducing the fundamental brain activation effect. Although the example built in the portable player is shown, the aforementioned vibration complementing apparatus 620 may be an external type or provided by an input of the vibration signal of the third band from external equipment or being received via broadcasting and communications. Although the example in which the brain activity is increased is described here, it is also possible to decrease the brain activity by complementation with the vibration signal of the second band.

The original vibration signals served as the subjects of this vibration complementing apparatus 620 include, besides the signals of musics and the like recorded by digital formats that cannot record the vibration signal of the third band in various recording media such as a solid memory, signals of the formats that cannot record the vibration signal of the third band transmitted and distributed by digital broadcasting and communications such as the current one-segment broadcasting, and signals that do not sufficiently contain the vibration signal of the third band in the contents though a format that can record and reproduce the vibration signal of the third band is used.

By using the apparatus of the present embodiment, it becomes possible to apply the vibration capable of inducing the fundamental brain activation effect to human beings by utilizing the contents of musics and the like of digital formats that can neither record the vibration signal of the third band nor induce the fundamental brain activation effect used in the existing portable player or the like.

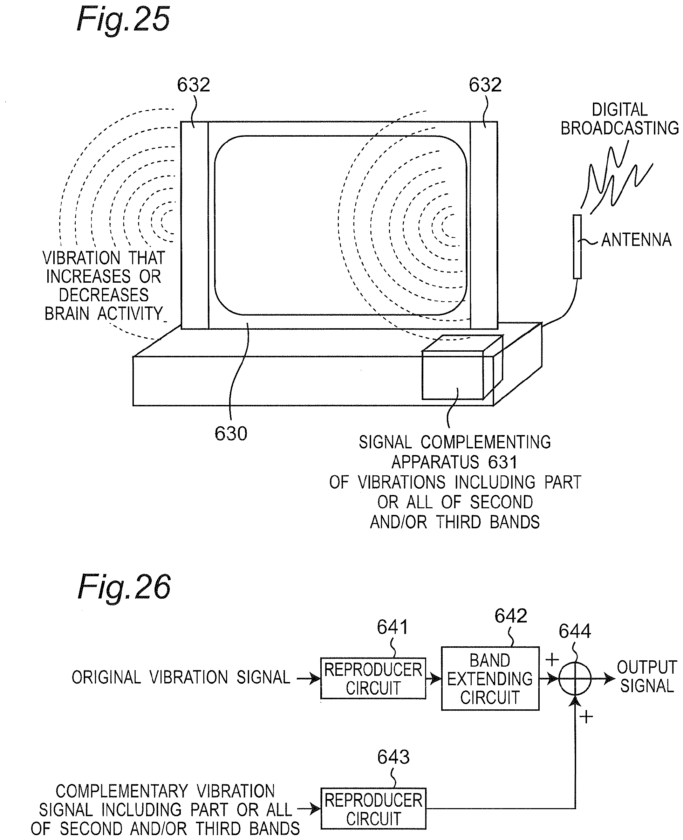

Figure 25 is a perspective view showing an example of a vibration complementing apparatus to add a vibration signal capable of inducing the fundamental brain activation effect to an original vibration signal that does not induce the fundamental brain activation effect outputted from broadcasting receiver equipment or the like according to the embodiment 3-6. Referring to Figure 25, there is shown an example of a vibration complementing apparatus to add a vibration signal including the vibration signal of the third band capable of inducing the fundamental brain activation effect to a vibration signal of a digital format that does neither contain the vibration signal of the third band nor induce the fundamental brain activation effect served as an original vibration signal. This vibration complementing apparatus 631 is mounted in broadcasting receiver equipment such as a television receiver 630 and has a built-in storage apparatus such as a solid memory in which a vibration signal including the vibration signal of the third band is recorded. This vibration complementing apparatus 631 has a function to read a vibration signal capable of inducing the fundamental brain activation effect from the storage apparatus, add the signal to the vibration signal that does not contain the received vibration signal of the third band, and thereafter output a vibration signal. The added signal is converted into an aerial vibration by a loudspeaker 632 or the like attached to the broadcasting receiver equipment. The aerial vibration converted at this time has become a vibration capable of inducing the fundamental brain activation effect. The above-mentioned vibration complementing apparatus 631, which is shown as a built-in example, may be an external type or provided by an input of a vibration signal of the third band for complementation from external equipment or received via broadcasting and communications. Moreover, the stored signal can be automatically complemented, or the user can select and complement his or her favorite vibration signal. Although the example in which the brain activity is increased is described here, it is also possible to decrease the brain activity by complementation with the vibration signal of the second band.

The original vibration signals served as the subjects of this vibration complementing apparatus include, signals of the digital formats and analog formats that cannot transmit the vibration signal of the third band transmitted or distributed by the current terrestrial digital broadcasting, BS digital broadcasting, analog TV broadcasting, AM radio broadcasting, FM radio broadcasting, communications of the Internet and the like, telephone lines, wireless communications, intercoms, interphones and the like, and signals that do not sufficiently contain the vibration signal of the third band in the contents though the format that can record and reproduce the vibration signal of the third band is used.

By using the apparatus of the present embodiment, it becomes possible to apply the vibration capable of inducing the fundamental brain activation effect by utilizing the vibration signal transmitted by the existing broadcasting or the like.

Next, implemental examples corresponding to the vibration complementing apparatus combined with existing band extending are described below.

In recent years, a variety of band extending methods are proposed as one technique for complementing a vibration signal from which the super-high frequency components exceeding the audible range are missing with the super-high frequency components. However, there are not a few examples in which the extended band is limited to the second band, and the vibration signal of the third band is not sufficiently contained.

Regarding this problem, in the present embodiment, not only the safety of the band-extended vibration signal is improved but also activation of the fundamental brain network is induced by complementation with the vibration signal of the third band, and this leads to obtaining the effects of exalting the aesthetic sensitivity and ameliorating and improving the physical state.

Figure 26 is a block diagram showing an example of a vibration complementing apparatus that concurrently uses the prior art band extending means and addition means of a vibration capable of inducing the fundamental brain activation effect according to the embodiment 3-6. Referring to Figure 26, the apparatus is configured to include a reproducer circuit 641 and a band extending circuit (also generally referred to as a band expanding circuit) 642 of an original vibration signal that does neither have the super-high frequency components nor induce the fundamental brain activation effect, a reproducer circuit 643 for a vibration signal capable of inducing the fundamental brain activation effect to the signal, and an adder 644 to add together these vibration signals.

Here is shown an example of an apparatus that generates an output signal capable of inducing the fundamental brain activation effect by using the existing band extending circuit 642 (See, for example, the Patent Documents 6 and 7) for the original vibration signal that does neither have the super-high frequency components nor induce the fundamental brain activation effect by adding a vibration signal including the vibration signal of the third band to a signal that does not sufficiently contain the vibration signal of the third band though it has been extended to the band of equal to or higher than 20 kHz that is the upper limit of the human audible frequency by the adder 644. With this arrangement, there can be obtained the effects of inducing the activation of the fundamental brain network (fundamental brain network system) including the reward system responsible for the reactions of pleasantness, beauty and emotion of a human being, and the centers of the autonomic nerve system, the internal secretion system and the immune system, which are responsible for the homeostasis and biophylaxis of the whole body, exalting the aesthetic sensitivity and ameliorating and improving the body state.

Next, an implemental example of a vibration complementing apparatus in which a high-pass filter is incorporated is described below.

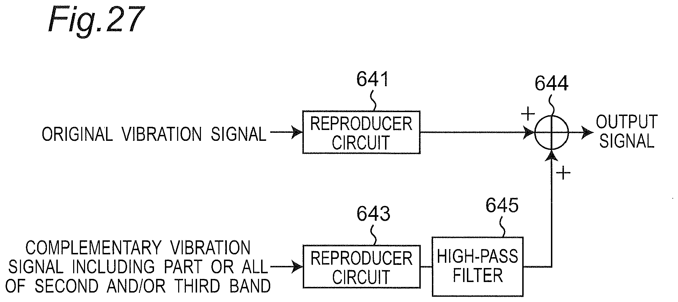

Figure 27 is a block diagram showing an example of a vibration complementing apparatus to generate a vibration signal capable of inducing the fundamental brain activation effect by adding a signal obtained by extracting the super-high frequency components of a vibration signal capable of inducing the fundamental brain activation effect to an original vibration signal as an output signal according to the embodiment 3-6. Referring to Figure 27, by adding a signal obtained by extracting only the vibration signal of the third band through filtering by a high-pass filter 645 to the original vibration signal that does neither have the vibration signal of the third band nor induce the fundamental brain activation effect by the adder 644, an output signal that complements the components satisfying the aforementioned properties and are able to consequently induce the fundamental brain activation effect is generated. Since the audible range components are not included in the vibration signal of the third band added to it, there occurs no such situation that, when the original signal is, for example, a music, both of them interfere each other causing difficulties in receiving the original vibration as a music. That is, it becomes possible to induce the fundamental brain activation effect without disturbing the reception of the audible range components of the original vibration. It is noted that the high-pass filter 645 may be a band-pass filter. Moreover, the existing band extending circuit of Figure 26 may be concurrently used for the original vibration signal. Although the example in which the brain activity is increased is described here, it is also possible to decrease the brain activity by complementation with the vibration signal of the second band.

Next, an application example of an embodiment 3-7 is shown.

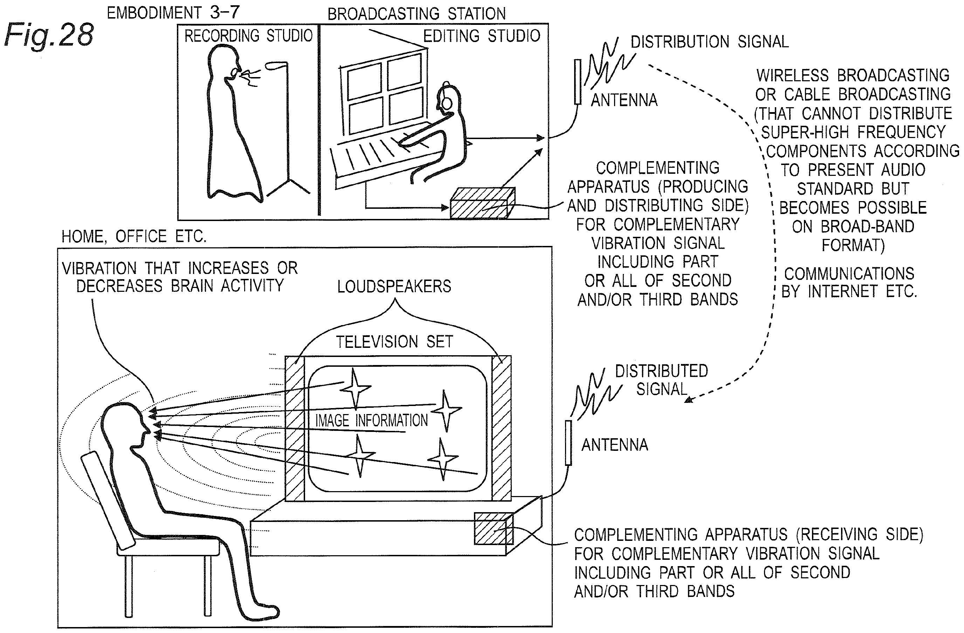

Figure 28 is an example of an apparatus capable of exalting the aesthetic sensitivity of a viewer and improving the television image quality for reception more pleasantly, beautifully and movingly by applying to the viewer a vibration capable of inducing the fundamental brain activation effect from a loudspeaker that reproduces the television sounds by transmitting a vibration signal capable of inducing the fundamental brain activation effect in television broadcasting.

When the sound source to be broadcasted is itself a vibration that contains the vibration signal of the third band and is able to induce the fundamental brain activation effect, the vibration of the third band can neither be contained nor transmitted according to the audio standard of the current television broadcasting, whereas it becomes possible to transmit the television signal that is made to have this effect by actualizing the wideband of the audio standard. Moreover, it is acceptable to perform the transmission by using Internet communications of a high speed and a large capacity or the like such as high-resolution distribution.

Moreover, when the sound source to be broadcasted is a vibration that cannot induce the fundamental brain activation effect, by performing transmission after complementation with a vibration signal capable of inducing the fundamental brain activation effect by various complementing apparatuses and complementary methods described in the embodiment 3-6 in editing at the broadcasting station, it is also possible to activate the reward system neural circuit, enhance the aesthetic sensitivity of the recipient, exalt the pleasantness, beauty and emotion and achieve an improvement in the received image quality. Even in the case, transmission becomes possible by achieving the wideband of the audio standard though the super-high frequency components can neither be contained nor transmitted according to the audio standard of the current television broadcasting. Moreover, it is acceptable to perform transmission by using high-speed large-capacity Internet communications or the like such as high-resolution distribution.

Further, when the vibration signal transmitted to broadcasting peripheral equipment in the current digital television is a vibration signal that does neither contain the vibration signal of the third band nor induce the fundamental brain activation effect, it may be reproduced by complementation with a vibration capable of inducing the fundamental brain activation effect in the peripheral equipment by the various apparatuses and methods described in the embodiment 3-6. By the operation, it is also possible to activate the reward system neural circuit, enhance the aesthetic sensitivity of the recipient, achieve an improvement in the received image quality, and exalt the pleasantness, beauty and emotion. Although the example in which the brain activity is increased by complementation with the vibration of the third band is described here, it is also possible to decrease the brain activity by complementation with the vibration signal of the second band. For example, it can be utilized for producing to temporally increase or decrease the brain activity according to, for example, the progress of the contents.

This apparatus is intended for video and audio contents that are transmitted and distributed by the current terrestrial digital broadcasting (including one-segment broadcasting), BS digital broadcasting, analog TV broadcasting, Internet communications and the like.

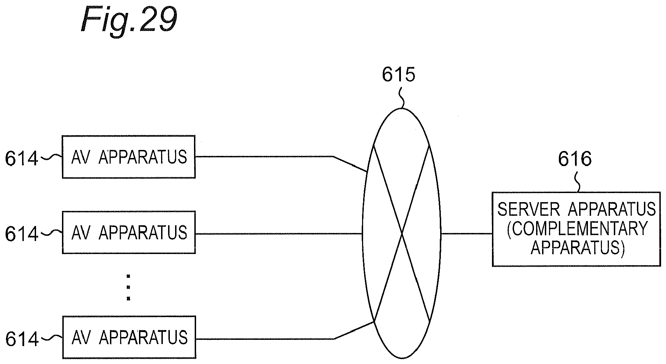

Figure 29 is a block diagram showing an example of a vibration complementing apparatus according to a modified embodiment of Figure 28. Referring to Figure 29, a plurality of AV apparatuses (video and/or audio reproducing, recording, and display apparatus) 614 are connected to a network 615 of the Internet or the like, and a server apparatus 616 is connected to the network 615. Although the complementing apparatus is provided for the AV apparatus 614 at the terminal side in the implemental example of Figure 28, the complementing apparatus may be provided for the network equipment of the server apparatus 616 or the like on the network 615 as shown in Figure 29.

The other applications are shown. Shown below are more affirmative applications, which are intended not to solve the problem that the qualities of different sensory information are in an antinomy state with a trade-off relation due to a technological constraint but to induce the effects of the reward system that has a function to collectively govern the generation of pleasantness, beauty and emotion through the fact that a vibration applied to a living body activates the fundamental brain in complex sensation information generating means that works on a plurality of sensory systems. For example, by constituting the vibration of a music to which the audience listens as a vibration capable of inducing the fundamental brain activation effect by sufficiently containing the vibration of the third band in a dance performance in a theater, it is possible to exalt the aesthetic sensitivity of the audience and make the audience feel the dance more beautiful and pleasant. This example can be applied also to other live performances, art galleries, museums, picture galleries, jewelry shops, boutiques, cosmetic departments and so on.

As applications to other senses, by constituting the music to which the guest listens as a vibration capable of inducing the fundamental brain activation effect in, for example, a music restaurant, the guest's gustatory sensitivity is improved to feel the dish to be more delicious. This example can be applied also to coffee shops, dining rooms, bars and so on.

Moreover, by constituting the music to which the guest listens as a hypersonic sound capable of inducing the fundamental brain activation effect regarding bath, massage and musics in a music spa or the like, the guest's somatic sensitivity is improved to feel the bathing and massage more pleasurably.

Further, by constituting the musics to which the passengers or crews listen as vibrations capable of inducing the fundamental brain activation effect in the vehicles of railways, automobiles, airplanes, ocean vessels, rockets and so on, the favorability ratings of the somatic sensitivities of the passengers or crews are improved to feel comfortable ride quality.

Moreover, by constituting musics to which the guests listen as a vibration capable of inducing the fundamental brain activation effect regarding the fragrance and music in music aroma therapy or the like, the sensitivity of the guest's sense of smell is improved to feel more pleasant fragrance and induce a high healing effect.

As described above, by applying the vibrations of the first and third bands to a human being while applying predetermined information to the human being regarding at least one of visual sensation, gustatory sensation, somatic sensation and sense of smell except the auditory sensation, the fundamental brain and the fundamental brain network (fundamental brain network system) including the reward system neural circuit that is the brain function region to integrally collectively govern the reactions of all sorts of pleasantness, beauty and emotion in the human being are activated, and this leads to allowing the aesthetic sensitivity to the sensational inputs other than the auditory sensation to be enhanced and allowing the expressive effects of the sensational information other than the auditory sensation to be improved.

In the aforementioned embodiments 3-1 to 3-7, by changing the mixture or the combination of these apparatuses, methods and spaces used for human beings and animals, the brain activity can be increased or decreased to various extents (adjustment of allowance becomes possible). When this is applied to a pathological state model or its remedial model, it becomes possible to produce pathological models and remedial models in various states. A "pathological model or remedial model generator apparatus" to which this is applied is considered. Moreover, an application as a safe curative medicine that scarcely causes side effects and induces an appropriate effect according to the extent of the clinical condition can be made by utilizing the fact that the brain activity can be controlled to various extents from an intense increase to a slight increase by changing the mixture of the bands. It becomes possible to perform fine adjustment similar to the so-called "allowance control" of a medicine.

Moreover, an analgesic effect, a sleep-inducing effect, an anxiolytic effect and the like are considered as concrete applications. Further, even when an experiment that has a concern about giving a negative effect to the test human subject due to a decrease in the brain activity by applying this method, by presenting the vibration of the frequency band that induces the positive effect in advance and conducting the experiment in a state in which the base line of the brain activity of the test human subject is raised, it becomes possible to maintain the brain activity level to a definite level at which no adverse effects are exerted on the mind and body even if a relatively negative effect is given. Accordingly, there is the advantage that the experiment can be conducted while securing the safety without inducing actual harms in the aspect of health. An application example as a "test human subject's safety apparatus" utilizing this effect is considered. Moreover, applications that induce appropriate effects according to the situations by increasing or decreasing the brain activity to various extents in living spaces, duty spaces, amusement spaces, public spaces, vehicles and so on by utilizing this effect are considered.

### EMBODIMENT 4: NEGATIVE AND POSITIVE INTENSITY CHANGES

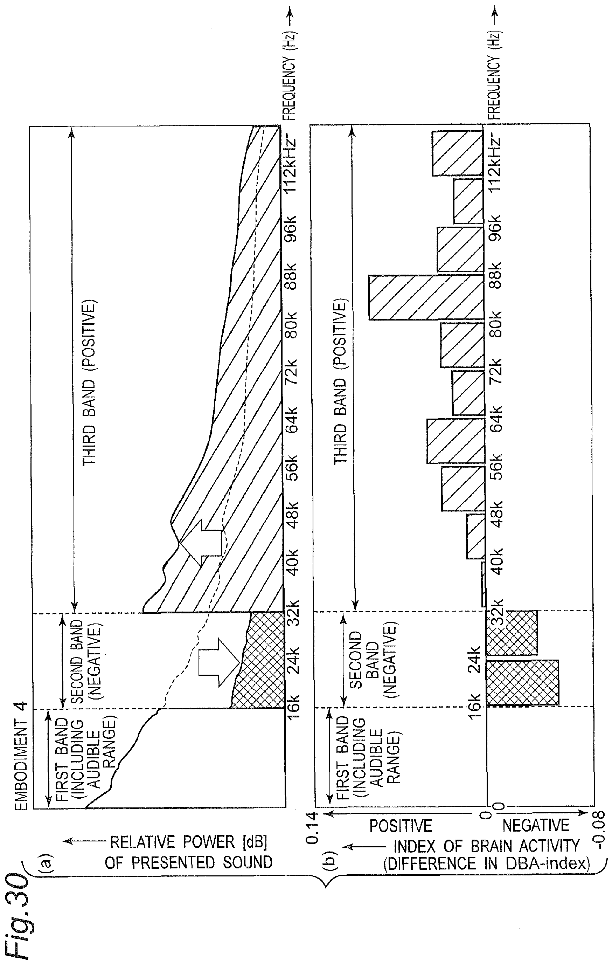

Figure 30 is a graph showing a vibration presentation method (presentation method to perform intensity changes) of a vibration presenting apparatus according to an embodiment 4, where Figure 30(a) is a schematic graph of the power spectrum of the presented vibrations of the divided bands regarding a vibration signal that has the first, second and third bands, Figure 30(b) is a graph showing an index of brain activity (difference in DBA-index) in the divided bands. Referring to Figure 30, the vibration processing apparatus, method and space of the embodiment 4 are characterized in that the degree of an increase or a decrease in the brain activity is arbitrarily controlled by attenuating or enhancing the intensity of the vibration of the band having the "negative effect" or enhancing or attenuating the intensity of the vibration of the band having the "positive effect" or combinations of them. Among others, means for preventing a decrease in the brain activity by removing or attenuating the band having the negative effect is included as one that has a particularly high effectiveness of application. It is noted that the frequencies corresponding to the boundaries of the bands are shown as one example on the listening conditions of the present experiment of Figure 30, and it is considered that they assume different values depending on the vibration presentation conditions or the vibration listening conditions or the species of the animals served as the subject.

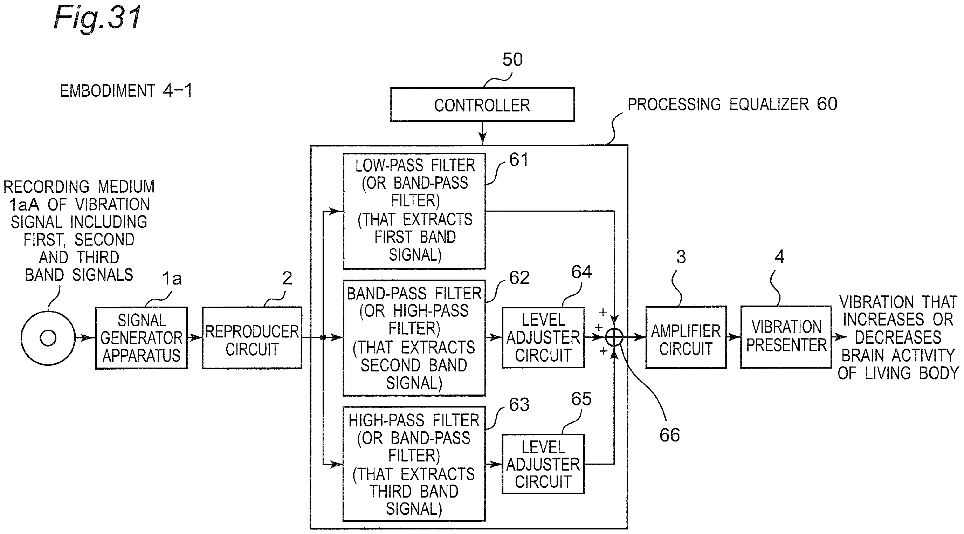

Figure 31 is a block diagram showing a configuration of a vibration presenting apparatus according to an embodiment 4-1. Referring to Figure 31, the vibration presenting apparatus according to the embodiment 4-1 is configured to include a signal generator apparatus 1a including the drive of a recording medium 1aA of a vibration signal, a reproducer circuit 2, a processing equalizer 60 controlled by a controller 50, an amplifier circuit 3, and a vibration presenter 4. In this case, the processing equalizer 60 is configured to include a low-pass filter 61, a band-pass filter 62, a high-pass filter 63, a level adjuster circuit 64, and an adder 66. In the present embodiment, an example of a vibration presenting apparatus system that removes or attenuates the band having the negative effect and enhances the band having the positive effect by signal processing is shown. It is noted that the signal generator apparatus is allowed to have a built-in memory to preparatorily store the vibration signal of the aforementioned band of the vibration signal source and generate the vibration signal. Moreover, it is acceptable to use a vibration signal synthesizing apparatus or a combination of a vibration generator apparatus or vibration generating source that generates a voice, a music, an environmental sound or the like and a signal transducer apparatus such as a microphone or a pickup, wiredly or wirelessly receive broadcasted and communicated signals such as the audio signals of digital broadcasting and high-resolution distribution or use a signal inputted from external equipment in place of the recording medium of the vibration signal, the signal generator apparatus and the reproducer circuit. Further, it is acceptable to remove or attenuate a specific band by acoustic absorbent or the like or enhance a specific band by a resonance apparatus or the like without converting the vibration outputted from the vibration generator apparatus into a signal.

Referring to Figure 31, the controller 50 controls the degree of attenuation or amplification of each level adjuster circuit 64. The components of the second band (e.g., 16 kHz to 32 kHz) having the "negative effect" are extracted by the band-pass filter 62 from the vibration signal including the first, second and third bands read from the recording medium 1aA of the vibration signal by the signal generator apparatus 1a to attenuate the components by the level adjuster circuit 64, and the components of the third band (e.g., equal to or higher than 32 kHz) having the "positive effect" are extracted by the high-pass filter 63 and amplified by the level adjuster circuit 64. A signal obtained by adding together them and the first band components extracted by the low-pass filter 61 in the adder 66 is amplified by the amplifier circuit 3, thereafter converted into a vibration and presented by the vibration presenter 4.

Moreover, in the present embodiment, the filter to extract the components of the first band may not be the low-pass filter 61 but a band-pass filter, and the filter to extract the components of the second band may not be the band-pass filter 62 but a high-pass filter. Moreover, the filter to extract the components of the third band may not be the high-pass filter 63 but a band-pass filter.

As a modified embodiment of the system of the present embodiment, there may be an apparatus that removes or attenuates only the second band from the vibration signal that includes only the first and second bands. Moreover, as another modified embodiment, there may be an apparatus that enhances only the third band regarding the vibration signal that includes only the first and third bands. Further, a filter apparatus such as a band-eliminate filter to attenuate, for example, the second band (e.g., 16 kHz to 32 kHz) may be used in place of the combinational circuit of the band-pass filter 62 and the level adjuster circuit 64. It is also possible to enhance the band having the negative effect and remove or attenuate the band having the positive effect by performing the level control in the reverse direction by using the present embodiment.

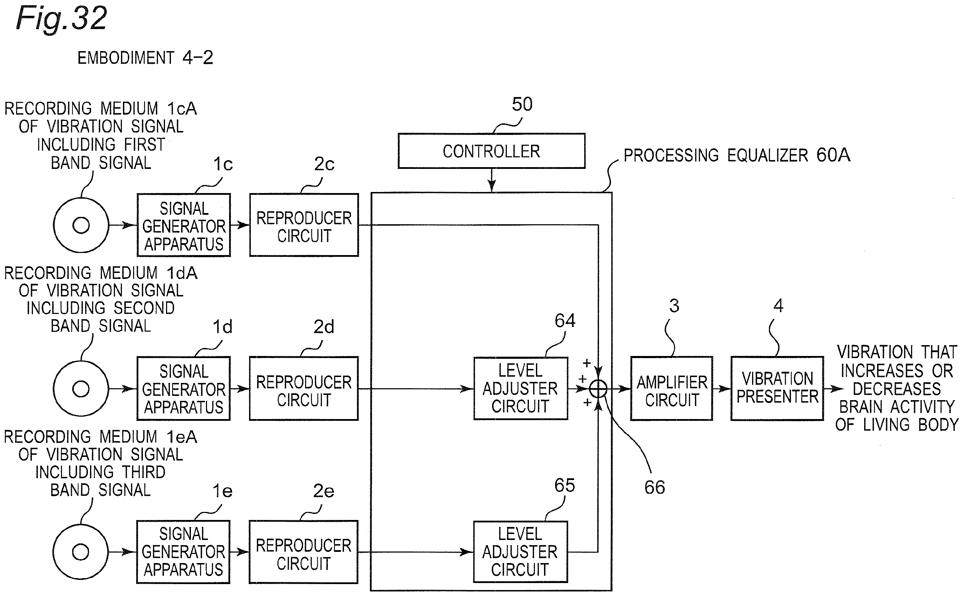

Figure 32 is a block diagram showing a configuration of a vibration presenting apparatus according to an embodiment 4-2. The apparatus of Figure 32 is provided in the case where sound sources exist in each of the first, second and third bands as a modified embodiment of Figure 31. Referring to Figure 32, the vibration presenting apparatus according to the embodiment 4-2 is configured to include signal generator apparatuses 1c, 1d and 1c including the drives of recording media IcA, IdA and 1eA of a vibration signal, reproducer circuits 2c, 2d and 2e, a processing equalizer 60A controlled by a controller 50, an amplifier circuit 3, and a vibration presenter 4. In this case, the processing equalizer 60A is configured to include a level adjuster circuit 64 and an adder 66. It is noted that the controller 50 controls the degree of attenuation or amplification of the level adjuster circuit 64.

Referring to Figure 32, the vibration signal of the first band read from the recording medium 1cA of the vibration signal by the signal generator circuit 1c is reproduced by the reproducer apparatus 1c and inputted to the adder 66. Moreover, the vibration signal of the second band (e.g., 16 kHz to 32 kHz) having the "negative effect" read from the recording medium IdA of a vibration signal by the signal generator apparatus 1d is reproduced by the reproducer circuit 1d, interrupted, attenuated or enhanced by the level adjuster circuit 64 and inputted to the adder 66. Moreover, the components of the third band (e.g., equal to or higher than 32 kHz) having the "positive effect" read from the recording medium 1e of a vibration signal by the signal generator apparatus 1e is reproduced by the reproducer circuit 1e, interrupted, attenuated or enhanced by the level adjuster circuit 64 and inputted to the adder 66. The adder 66 adds together the inputted three signals, and the signal of the addition result is amplified is amplified by the amplifier circuit 3, thereafter converted into a vibration and presented by the vibration presenter 4. By this operation, it becomes possible to arbitrarily control the degree of an increase or a decrease in the brain activity by adjusting the level of the vibration signal of the second band or the third band and adding it to the vibration signal that does not include the second band or the third band. Although the example in which the vibration signals of the bands are added together by the adder 66 is shown in the processing equalizer 60A of Figure 32, it is acceptable to provide an independent amplifier and a vibration presenting apparatus for each of the bands without via the adder 66.

Various apparatuses to remove or attenuate the band having the negative effect and enhance the band having the positive effect by performing acoustic processing of the aerial vibration that propagates in the air are considered besides the above embodiments. They can be classified roughly into the following three types by the principle.
(A) An absorber apparatus: An apparatus to attenuate the vibration components of the second band having the negative effect taking advantage of the fact that the vibration of a specific band is selectively absorbed when the aerial vibration penetrates a matter.
(B) A reflecting apparatus: An apparatus to remove the vibration components of the second band having the negative effect and enhance the vibration components of the third band having the positive effect taking advantage of the fact that the vibration of a specific band is selectively reflected when the aerial vibration collides with and reflected on a matter.
(C) A resonance apparatus: An apparatus to enhance the vibration components of the third band having the positive effect taking advantage of the fact that the vibration of a specific band selectively resonates when the aerial vibration resonates on the indoor walls and the like.

Next, the operational effects and applications of the apparatus, method and space related to the present embodiment are described below.

It is considered that the means for preventing a decrease in the brain activity by removing or attenuating the second band having the negative effect and enhancing the brain activity by enhancing the third band having the positive effect in particular provide extremely important, effective effects and applications in the present embodiment.

The acoustic environments including traffic noises and artificial sounds generated by electronic equipment spreading in the modern society, signals recorded in media such as CDs, vocal or music signals and the their reproduced sounds that are distributed or transmitted by broadcasting or communications and so on often include the band that induces the negative effect at a high rate, and they possibly decrease the brain activity of modern people and become causes of the modern diseases. By removing or attenuating the vibrations of the negative band from them, it is possible to obtain the effects of removing the negative influence and securing safety. Further, there are the effects of increasing the pleasant sensation and aesthetic sensitivity together with the means for enhancing or adding the third band having the positive effect and enhancing the fundamental brain activity. With this arrangement, a variety of application developments such as applications as effective means for exalting the reactions of the pleasure, beauty and emotion to the artistic productions including musics, and live performances, exhibitions, screening in halls and event spaces and increasing the expressive effects, and applications as effective means for making living spaces, duty spaces, amusement spaces, public spaces, vehicles and so on pleasant.

Further, various application examples including electronic equipment are considered as follows.
(a) Applications to AV systems: players, effectors, mixers, amplifiers, loudspeakers, components and the like with a built-in negative band attenuator apparatus, and a positive band enhancement or attachment apparatus. Video decks, tuners, displays, TVs and the like with a built-in negative band attenuator apparatus, and a positive band enhancement or attachment apparatus. Computers with a built-in negative band attenuator apparatus, and a positive band enhancement or attachment apparatus.
(b) Applications to a portable equipment and portable electronic telephone: portable media players, portable telephones, smart phones, portable information equipment and the like with a built-in negative band attenuator apparatus, and a positive band enhancement or attachment apparatus. An earphone built-in type and cable embedded type negative band attenuator apparatus, and a positive band enhancement or attachment apparatus.
(c) As examples of applications for negative effect attenuation or positive effect enhancement or attachment apparatuses besides them, there are considered clothes, hats, accessories, cosmetics, bedclothes, curtains, baths, water closets, room wall surfaces, ceiling surfaces, floor surfaces, pillars, fixtures and furnitures, carved ornaments, interior accessories, exterior accessories, vehicle interior decorations, vehicle bodies and tires, building exterior or interior packaging, earplugs, hearing aids and so on.

### EMBODIMENT 5: BAND SHIFT

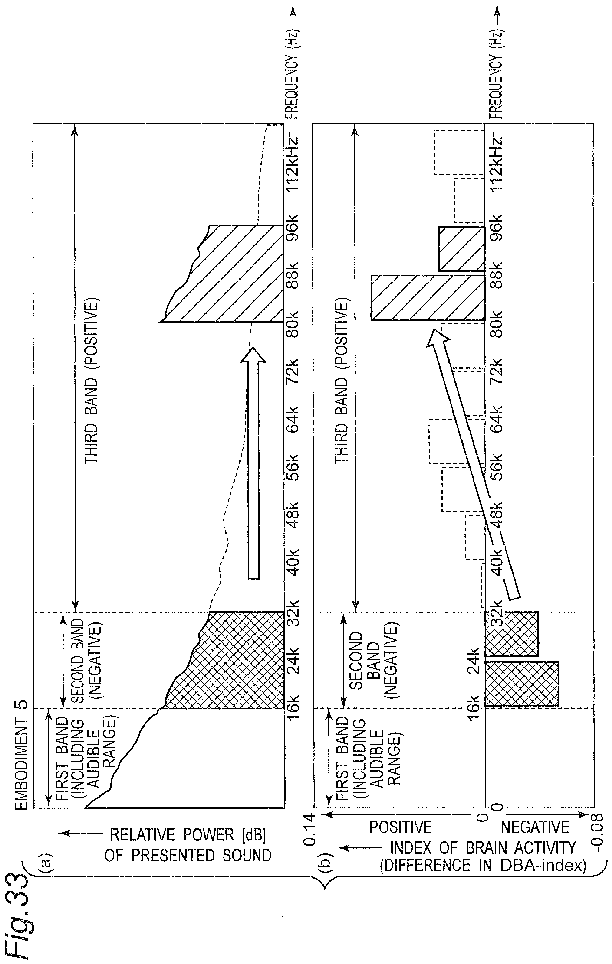

Figure 33 is a graph showing a vibration presentation method (presentation method using band shift) of a vibration presenting apparatus according to an embodiment 5, where Figure 33(a) is a schematic graph of the power spectrum of the presented vibrations of the divided bands regarding a vibration signal that has the first, second and third bands, and Figure 33(b) is a graph showing an index of brain activity (difference in DBA-index) of a change in the fundamental brain activity in the divided bands. Referring to Figure 33, the vibration presenting apparatus, method and space according to the embodiment 5 are characterized in that an increase in the brain activity is effectively induced by converting the vibration signal of the band having the "negative effect" into the band having the "positive effect".

The present embodiment can be implemented by using every sorts of band complementary systems including the existing band complementary system (e.g., the fluency theory by Professor Toraichi Kazuo, the sample value control theory by Professor Yutaka Yamamoto, the harmonator system by Nakagawa Shin of FIDELIX Co., Ltd., systems using the characteristics of a mere slow roll-off filter), and concrete examples are described below.

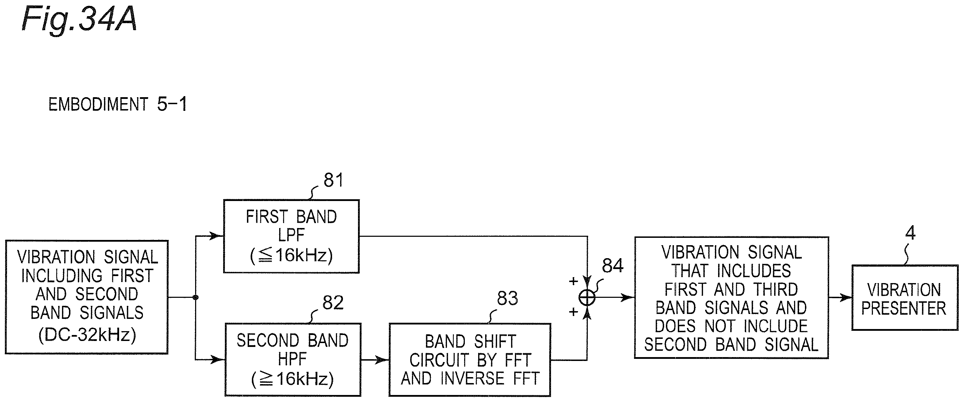

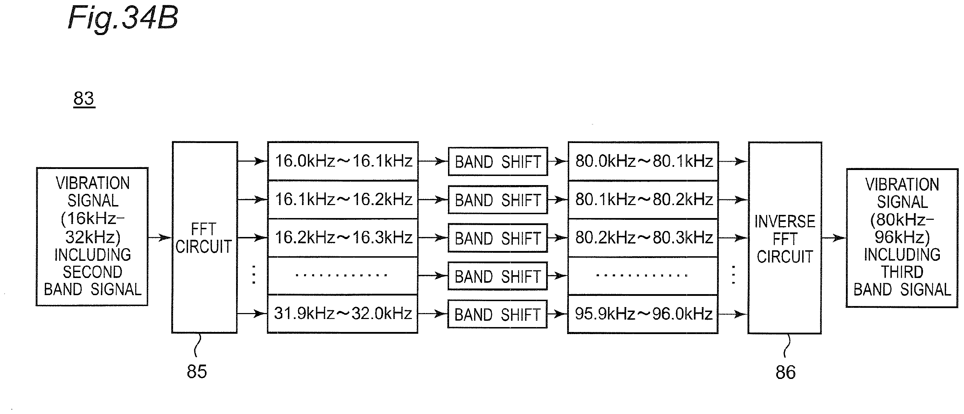

Figure 34A is a block diagram showing a configuration of a vibration presenting apparatus according to an embodiment 5-1, and Figure 34B is a block diagram showing a detailed configuration of the band shift circuit 83 of Figure 34A. The vibration presenting apparatus according to the embodiment 5-1 is characterized in that the vibration signal of the second band (e.g., 16 kHz to 32 kHz) is shifted to the vibration signal of the third band (e.g., equal to or higher than 32 kHz) by combining the FFT (Fast Fourier Transform) calculation with the inverse FFT calculation. That is, there is the feature that a spectrum structure to effectively induce an increase in the brain activity such that the components of the first band and the components of the third band producing a positive effect exist and the components of the second band producing a negative effect do not exist is inevitably taken. This has a feature largely different from the existing band complementary systems (e.g., the fluency theory by Professor Toraichi Kazuo, the sample value control theory by Professor Yutaka Yamamoto, the harmonator system by Nakagawa Shin of FIDELIX Co., Ltd., systems using the characteristics of a mere slow roll-off filter), which are intended to restore or complement the continuity of the spectral structure and waveform of the high-frequency band that has been lost at a low sampling rate. Further, regarding the mounting of these existing systems, it is often the case where complementation of the PCM signals of 44.1 kHz to 48 kHz of CDs, DVDs and the like is effected twofold of the recordable and reproducible band, i.e., up to the band of 44.1 kHz to 48 kHz for extension. The thus-obtained band components mainly include the first band and the second band and scarcely include the third band, and this has the adverse effects that the reproduction of the continuity of the spectral structure and waveform are insufficient as well as the risk of inducing a decrease in the brain activity is extremely large. Referring to Figure 34A, the vibration presenting apparatus is configured to include a low-pass filter (LPF) 81 to extract the components of the first band, a high-pass filter (HPF) 82 to extract the components of the second band, a band shift circuit 83 by FFT and inverse FFT, an adders 84, and a vibration presenter 4.

Many of the signals recorded in the recording media such CDs and DVDs or the transmission signals distributed or transmitted by broadcasting or communications have their bands limited for various restrictions including technological constraints and often include only the first band and part of the second band. This system uses such a vibration signal that includes the first band and part of the second band as a vibration source.

Referring to Figure 34A, the first band signal is extracted from the signal containing the first and second band signals by the low-pass filter 81 and inputted to the adder 84. The second band signal is extracted from the signal containing the first and second band signals by the high-pass filter 82, and the second band signal is band-shifted to a third band signal by the band shift circuit 83 and thereafter inputted to the adder 84. The adder 84 adds together the inputted two signals and outputs the resulting signal to the vibration presenter 4.

Referring to Figure 34B, the band shift circuit 83 is configured to include an FFT circuit 85 and an inverse FFT circuit 86. The FFT circuit 85 obtains a power spectrum that keeps phase information by performing the FFT calculation of the vibration signal including the second band. By moving the data of, for example, the band of 16 kHz to 32 kHz (divided, for example, every 100 Hz) to, for example, the band of 80 kHz to 96 kHz (divided, for example, every 100 Hz) in the power spectrum data and subsequently performing the inverse FFT calculation by the inverse FFT circuit 86, the vibration signal including the third band is obtained. Subsequently, a vibration signal including the first band is obtained by the adder 84 of Figure 34A and presented. In a case where the input signal has a sampling frequency of equal to or higher than PCM 96 kHz and contains the first, second and third band components, it is acceptable to parallel shift the entire second and third band components of equal to or higher than 15 kHz to, for example, the band components of equal to or higher than 32 kHz or shift, for example, only the second band components of 16 kHz to 32 kHz to a band (equal to or higher than 48 kHz in the case of PCM 96 kHz) higher than the recorded band without shifting the recorded third band components.

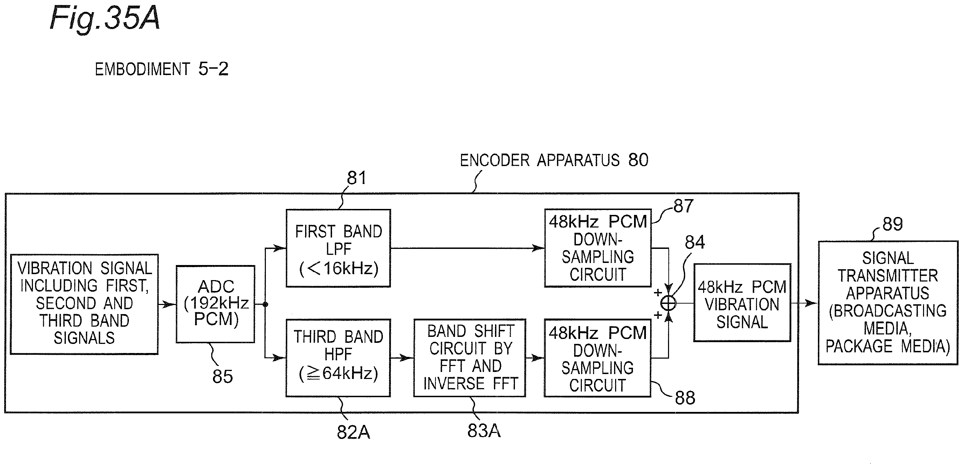

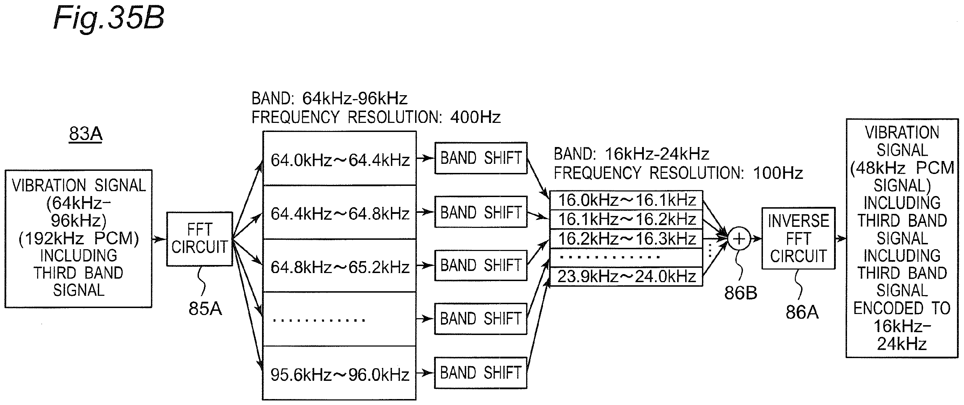

Figure 35A is a block diagram showing a configuration of an encoder apparatus 80 used in a vibration presenting apparatus according to an embodiment 5-2, and Figure 35B is a block diagram showing a detailed configuration of the band shift circuit 83A of Figure 35A. The apparatuses of Figures35A and 35B are modified embodiments of Figures 34A and 35B, showing means, when distributing or transmitting a vibration source including the third band by broadcasting or communications, for discarding the original signal components of the second band having the effects of decreasing the brain activity in a situation where the distribution or transmission can be achieved only within the first and second bands due to technological constraints, and distributing or transmitting the signal including the third band through conversion into the vacant second band, and performing restoration and reproduction to the third band again at the terminal of the receiving person. Even if the original vibration source is a wide-band vibration signal also including the third band (or even if the band extending complementation is effected to synthesize the vibration signal of the third band that has not existed originally), it is sometimes a case where the listener cannot receive the third band because, for example, the digital broadcasting sound or the like is band-limited in the stages of recording, editing and distributing or transmitting. This is a distribution or transmission system that is an extremely effective in such a case and has a feature that the vibration signal that the receiving person enjoys in this case inevitably takes a spectrum structure to effectively induce an increase in the brain activity, in which the first band components and the third band components producing the positive effect exist and the second band components producing the negative effect does not exist.

Referring to Figure 35A, the encoder apparatus 80 is configured to include an A/D converter (ADC) 85, a low-pass filter (LPF) 81 to extract the first band signal, a down-sampling circuit 87, a high-pass filter (HPF) 82A to extract the third band signal, a band shift circuit 83A by FFT and inverse FFT, a down-sampling circuit 88, an adder 84, and a signal transmitter apparatus 89. Referring to Figure 35B, the band shift circuit 83A is configured to include an FFT circuit 85A, an adder 86B, and an inverse FFT circuit 86A. The encoder apparatus 80 includes a band converter circuit to convert the signal of the third band into the second band by combining the FFT calculation with the inverse FFT calculation. In this case, the signal of the second band generated through conversion can be restored into the signal of the third band on the receiver side after transmission by using a decoder apparatus or the like described in detail later.

Referring to Figure 35A, in the encoder apparatus 80 on the distribution side such as a broadcasting station, an original signal (or a band-extended signal) including the vibrations of all the first, second, and third bands is first encoded to a PCM signal at a sampling frequency of 192 kHz by the A/D converter 85. After only the first band signal is extracted from the PCM signal by the low-pass filter 81, it is down-sampled to a 48-kHz PCM signal by the down-sampling circuit 87 and outputted to the adder 84. On the other hand, after only the third band signal is extracted from the PCM signal by the high-pass filter 82A, it is band-converted into the second band signal by the band shift circuit 83A, down-sampled to a 48-kHz PCM signal by the down-sampling circuit 88, and outputted to the adder 84. The adder 84 adds together the inputted two PCM signals to obtain a 48-kHz PCM vibration signal, and it is signal transmitted via a signal transmitter apparatus 89 of the broadcasting media, package media or the like.

In the band shift circuit 83A of Figure 35B, the entire band of the vibration signal containing the third band signal is subjected to, for example, FFT calculation of a BIN width of 100 Hz by the FFT circuit 85A to obtain power spectrum data including phase information. The power spectrum data of the second band (e.g., 16 kHz to 32 kHz) is discarded from the data, and data of four BIN widths (i.e., 400-Hz width) are averaged regarding, for example, the power spectrum data of 64 kHz to 96 kHz (32-kHz bandwidth) of the third band. By assuming it to be corresponding to the BIN width of 100 Hz at 16 to 24 kHz (8-kHz bandwidth), it becomes possible to settle the data of 64 to 96 kHz (32-kHz bandwidth, BIN width of 400 Hz) into an 8-kHz bandwidth of 16 to 24 kHz. After the thus-moved components of 16 to 24 kHz (8-kHz band) are added together by the adder 86B, the components of the addition result are subjected to inverse FFT calculation by the inverse FFT circuit 86A. By this operation, a power spectrum of equal to or lower than 24 kHz is obtained in addition to the spectrum data of the first band of lower than 16 kHz. Then, by subjecting the components of equal to or lower than 24 kHz to inverse FFT at a sampling frequency of 48 kHz in the power spectrum data obtained in this case, a PCM data signal of the sampling frequency of 48 kHz is obtained. This PCM data signal is outputted to the signal transmitter apparatus 89 and is able to be transmitted and variously processed within the audio standard of the conventional digital television broadcasting or the like. The thus-converted PCM data signal is distributed or transmitted, and delivered to the listeners.

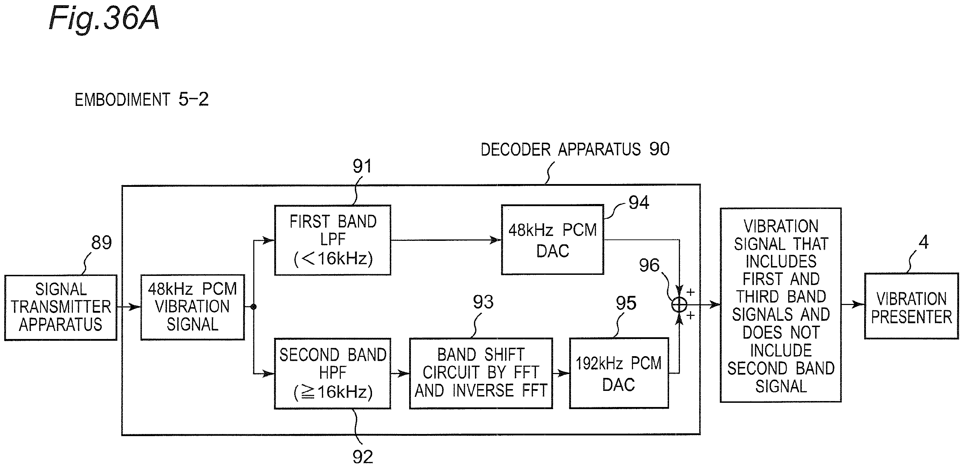

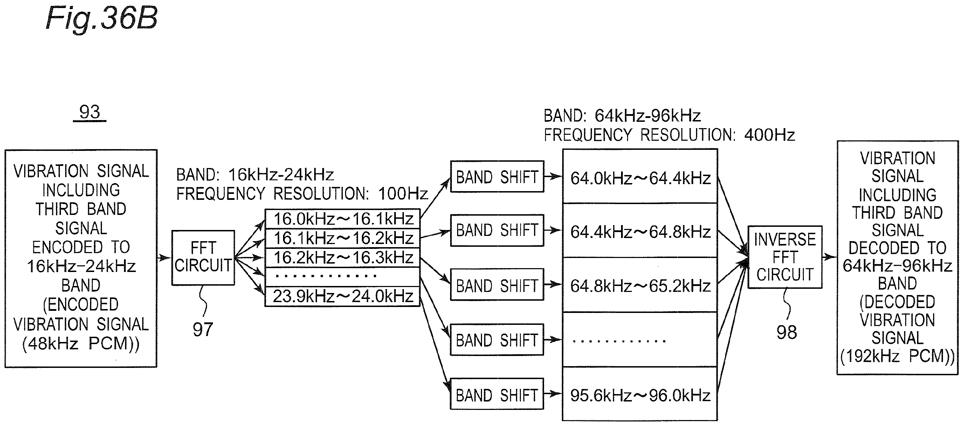

Figure 36A is a block diagram showing a configuration of a decoder apparatus 90 used in a vibration presenting apparatus according to the embodiment 5-2, and Figure 36B is a block diagram showing a detailed configuration of the band shift circuit 93 of Figure 36A. Referring to Figure 36A, the decoder apparatus 90 is configured to include a low-pass filter (LPF) 91 to extract the first band signal, a D/A converter 94 to perform DA conversion from the 48-kHz PCM signal to an analog signal, a high-pass filter (HPF) 92 to extract the second band signal, a band shift circuit 93 by FFT and inverse FFT, a D/A converter 95 to perform DA conversion from the 192-kHz PCM signal to an analog signal, an adder 96, and a vibration presenter 4. Referring to Figure 36B, the band shift circuit 93 is configured to include an FFT circuit 97 and an inverse FFT circuit 98. In contrast to this, regarding the 48-kHz PCM data signal delivered to the listener, the signal of the second band contained in the 48-kHz PCM data signal can be shifted to the third band by executing a process reverse to the process of the encoder apparatus 80 of Figures 36A and 36B. Although the distribution and transmission of the vibration signal have been described in the embodiment, it can also be applied to a case where recording is performed in media of narrow bandwidth or the like.

Figure 37A is a block diagram showing a configuration of a decoder apparatus 90A used in a vibration presenting apparatus according to an embodiment 5-3. Moreover, Figure 37B is a graph showing an operation of the up-sampling circuit 101 of Figure 37A. Figure 37B(a) is a graph showing a time series data of the 48-kHz PCM signal for transmission, Figure 37B(b) is a graph showing a time series data of a signal obtained by iterating four times at quadruple speed the sampled values of previous four points according to an example 1 of the up-sampling method, and Figure 37B(c) is a graph showing a time series data of a signal obtained by reproducing at quadruple speed the sampled values of the immediately preceding four points according to an example 2 of the up-sampling method. The decoder apparatus 90A used in the vibration presenting apparatus according to the embodiment 5-3 is characterized in that the vibration signal of the third band is generated by complementing a temporal dropout by iterating the quadruple-speed reproduction of data in a definite time of the vibration signal of the second band.

Referring to Figure 37A, the decoder apparatus 90A is characterized in that an up-sampling circuit 101 to perform up-sampling to a 192-kHz PCM signal through quadruple-speed iterative reproduction complementation in place of the band shift circuit 93 by FFT and inverse FFT by comparison to the decoder apparatus 90 of Figure 36A.

In the decoder apparatus 90A of Figure 37A, a 48-kHz sampling PCM data signal is first separated into a signal of the first band of lower than 16 kHz and a signal of the second band of 16 kHz to 24 kHz by the low-pass filter 91 and the high-pass filter 92, respectively. By iteratively reproducing at quadruple speed the signal of the second band, a 192-kHz sampling PCM data signal of the band of 64 kHz to 96 kHz can be obtained. This is, in a word, the third band. By mixing (adding together) a signal obtained through DA conversion by the D/A converter 95 with a signal obtained through DA conversion of the initially separated first band by the D/A converter 94 in the adder 96, a vibration signal that contains the first and third band signals and does not contain the second band signal can be obtained, and presented by the vibration presenter 4.

Two examples of the algorithm of the iterative reproduction at quadruple speed of the above are shown in Figure 37B. The received 48-kHz sampling PCM signal string is shown in Figure 37B(a). Referring to Figure 37B(b), the first algorithm iteratively outputs at quadruple speed four times the signal string configured to include the sampled values of four points received in the previous, subsequently forms a signal string from the four points received during the outputting, and forms an output by iterating four times this operation at quadruple speed in the same manner. This procedure is repeated until the received signal ends. Referring to Figure 37B(c), the second algorithm outputs at quadruple speed a signal string configured to include the immediately received sampled values of four points, subsequently discards the value at the top of the signal string, adds a new sampled value received during it to the extreme tail of the signal string, and outputs the signal string at quadruple speed. This procedure is repeated until the received signal ends.

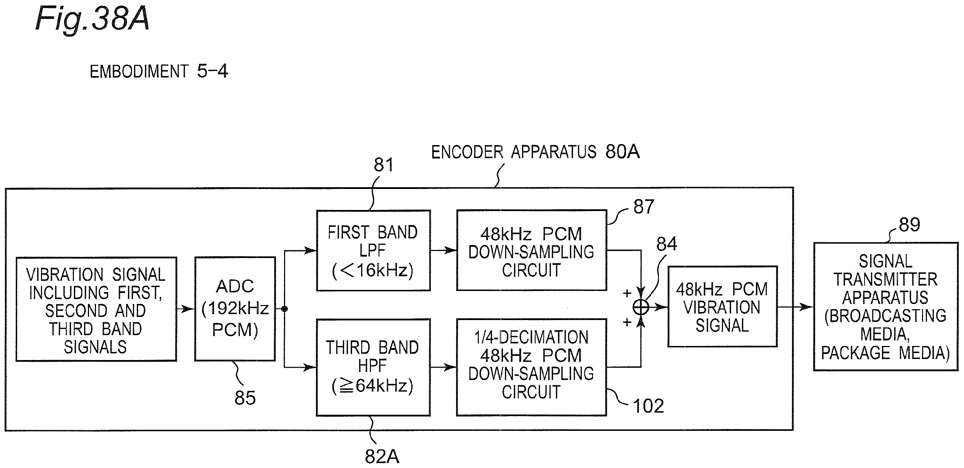

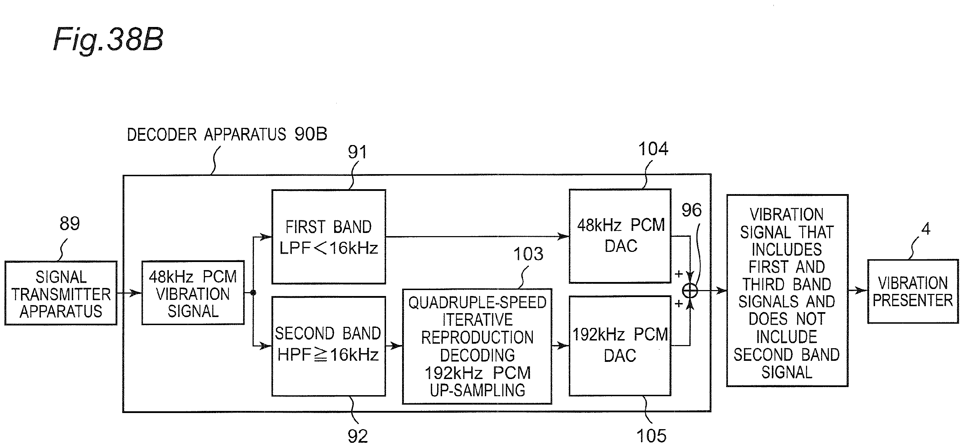

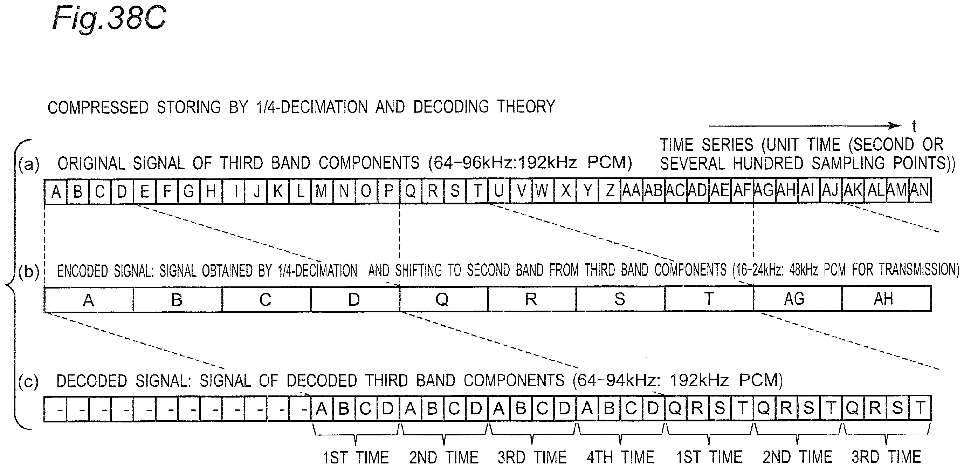

Figure 38A is a block diagram showing a configuration of an encoder apparatus 80A used in a vibration presenting apparatus according to an embodiment 5-4, and Figure 38B is a block diagram showing a configuration of a decoder apparatus 90B used in the vibration presenting apparatus of the embodiment 5-4. Figure 38C is a graph showing a compressed storing by 1/4 decimation executed by the encoder apparatus 80A of Figure 38A, and a decoding process therefor executed by the decoder apparatus 90B of Figure 38B. Figure 38C(a) is the time series data of the signal of the original third band components, Figure 38(b) is the time series data of the encode signal encoded by the encoder apparatus 80A, and Figure 38(c) is the time series data of the decode signal decoded by the decoder apparatus 90B. The present embodiment is characterized in that, as a modified embodiment of Figures 37A and 37B, in a situation in which it can be distributed or transmitted within only the first and second bands due to technological constraints when a vibration source including the third band is distributed or transmitted by broadcasting or communications, the signal including the third band is distributed or transmitted by being converted into the second band, and the signal is reproduced by being restored into the third band at the terminal apparatus of the receiving person.

Referring to Figure 38A, the encoder apparatus 80A is configured to include an A/D converter 85, a low-pass filter 81 to extract the first band, a down-sampling circuit 87 to perform down-sampling to a 48-kHz PCM signal, a high-pass filter 82A to extract the third band, a down-sampling circuit 102 to perform down-sampling to the 48-kHz PCM signal by 1/4 decimation, an adder 84, and a signal transmitter apparatus 89. The encoder apparatus 80A includes a band converter circuit to convert the signal of the third band to the second band by down sampling. In this case, it is possible to restore the signal of the second band that has been band-converted and generated into the signal of the third band on the receiver side after transmission by using means described hereinbefore or hereinafter or the like.

Referring to Figure 38A, an original signal (or band-extended signal) including the vibration of a wide band is first encoded to PCM by 192-kHz sampling by the A/D converter 85 in the encoder apparatus 80A on the distribution side such as a broadcasting station, and the first band of lower than 16 kHz and the third band of equal to or higher than 64 kHz are extracted therefrom by the low-pass filter 81 and the high-pass filter 82A, respectively. The vibration signal of 16 kHz to 64 kHz is not used in the subsequent process. A signal obtained by down-sampling the obtained vibration signal of the first band at a sampling frequency of 48 kHz by the down-sampling circuit 87 and a signal obtained by down-sampling the vibration signal of the third band at a sampling frequency of 48 kHz by 1/4 decimation by the down-sampling circuit 102 are added together by the adder 84, obtaining an output of 48-kHz sampling PCM vibration signal. This can be transmitted by the signal transmitter apparatus 89 or used for recording in broadcasting media and package media.

Referring to Figure 38B, the decoder apparatus 90B is configured to include a low-pass filter 91 to extract the first band, a D/A converter 104 to perform DA conversion from the 48-kHz PCM signal to an analog signal, a high-pass filter 92 to extract the second band, an up-sampling circuit 103 to perform up-sampling to a 192-kHz PCM signal by quadruple-speed iterative reproduction encoding, a D/A converter 105 to perform DA conversion from the 192-kHz PCM signal to an analog signal, and an adder 96. That is, the decoder apparatus 90B includes a band converter circuit to generate a pseudo-restored vibration signal that contains the first and third band signals and does not contain the vibration signal of the second band by reproducing at high speed the 48-kHz PCM data signal delivered to the listener.

In the decoder apparatus 90B of Figure 38B, the 48-kHz sampling PCM data signal is separated into the signal of the first band of lower than 16 kHz and the signal of the second band of 16 kHz to 24 kHz by the low-pass filter 91 and the high-pass filter 92, respectively. By iteratively reproducing at quadruple speed the signal of the second band by the up-sampling circuit 103, a 192-kHz PCM data signal of the band of 64 kHz to 96 kHz can be obtained. This is, in a word, the third band. By mixing (adding together) a signal obtained through DA conversion by the D/A converter 105 and a signal obtained through DA conversion of the initially separated first band, a vibration signal that contains the first and third band signals and does not contain the second band signal can be obtained, and presented by the vibration presenter 4.

Figure 38C shows an algorithm of the portion where the third band components are decimated into 1/4 thereof through the encoding of the encode apparatus 80A of Figure 38A, and the algorithm of the portion where the quadruple-speed reproduction is repeated through the decoding of the decoder apparatus 90B of Figure 38B. Figure 38C(a) shows a signal string of the original third band components. Referring to Figure 38C(b), this signal string becomes an encoded signal that is decimated into 1/4 thereof and shifted to the second band by the encoder apparatus 80A shown in Figure 38A. The signal string configured to include the sampled values at received four points are iteratively outputted four times at quadruple speed by the decoder apparatus 90B shown in Figure 38B, and subsequently a signal string is formed from the sampled values at the four points received during outputting, and this is outputted likewise iteratively four times at quadruple speed. This procedure is repeated until the received signal ends. The original third band components are pseudo-reproduced as above. Although the distribution and transmission of the vibration signal have been described in the embodiment, it can be applied to a case where recording is performed on a medium (signal recording medium) of a narrow bandwidth.

For the processing in the up-sampling circuit 103, it is acceptable to use the algorithm shown in Figure 38C(c) or another method.

According to the present embodiment, a vibration that includes the first band and the third band having the "positive effect" is to be reproduced at the terminal equipment of the listener.

Next, the operational effects and applications of the apparatus, method and space of the present embodiment are described below.

In the present embodiment, many of the signals recorded in recording media (signal recording media) or distributed or transmitted by broadcasting or communications are band-limited due to various technological constraints such as the sampling frequency standards and restrictions on the transmission rate, and therefore, the second band having the negative effect is inevitably contained relatively plentifully, and the third band having the positive effect does not exist. In order to band-extend this and transmit the third band inclusive, the standards, hardware and the like need to be reviewed, and the society is totally required to bear huge costs and obligations. However, if the examples described here are applied, it becomes possible to shift only the band without increasing the amount of information and extremely efficiently achieve a reversal from the "negative effect" to the "positive effect". There is needed no drastic change in the hardware standards since the amount of information is not increased, and the current media, broadcasting or communication lines can be used almost as they are, allowing the cost to be also suppressed. Moreover, this apparatus can be applied to a variety of sound reproduction apparatuses such as audio apparatuses, television sets, radio receivers, personal computers, portable telephones, portable music players, BGM transmission systems in public spaces and the like. With this arrangement, a variety of application developments such as applications as effective means for making living spaces, duty spaces, amusement spaces, public spaces, vehicles and so on comfortable are possible.

### EMBODIMENT 6: CHANGEOVER PRESENTATION

Figure 39 is a graph showing a vibration processing method (presentation method to induce an abrupt change from a decrease to an increase in the brain activity) of a vibration processing apparatus according to an embodiment 6-1. Figure 39(a) is a schematic graph of the power spectrums of the presented vibrations of the divided bands regarding a vibration signal that has the first, second and third bands, Figure 39(b) is a graph showing an index of brain activity (difference in DBA-index) in the divided bands, and Figure 39(c) is a graph showing a temporal transition of the presentation condition of the vibration presentation method. As shown in Figure 39, the vibration presenting apparatus, method and space of the present embodiment are characterized in that the brain activity is arbitrarily temporally changed by presenting the band having the "negative effect" and the band having the "positive effect" in a temporal changeover manner.

Referring to Figure 39, an example of the presentation method for inducing the abrupt change from a decrease to an increase in the brain activity is shown. Referring to Figure 39, first of all, the vibrations of the first band (e.g., equal to or lower than 16 kHz) + second band (e.g., 16 kHz to 32 kHz) are presented for three minutes. Subsequently, the vibrations of the first band (e.g., equal to or lower than 16 kHz) + third band (e.g., 32 kHz to 96 kHz) are presented for three minutes without interval. By the presentation method as above, it becomes possible to control to first decrease the brain activity and to subsequently abruptly increase the brain activity.

Figure 40 is a graph showing a vibration presentation method (presentation method for inducing a gradual change from an increase to a decrease in the brain activity) of a vibration presenting apparatus according to an embodiment 6-2. Figure 40(a) is a schematic graph of the power spectrums of the presented vibrations of the divided bands regarding a vibration signal that has the first, second and third bands, Figure 40(b) is a graph showing an index of brain activity (difference in DBA-index) in the divided bands, and Figure 40(c) is a graph showing a temporal transition of the presentation condition of the vibration presentation method.

Referring to Figure 40, an example of the presentation method for inducing a gradual change from an increase to a decrease in the brain activity is shown. First of all, the vibrations of the first band (e.g., equal to or lower than 16 kHz) + part of third band (e.g., 32 kHz to 64 kHz) are presented. After a lapse of ten minutes partway, the vibration of part of the second band (e.g., 16 kHz to 24 kHz) starts being presented, and the presentation of part of the third band is stopped after a lapse of further ten minutes. Then, the first band + part of the second band are presented for further ten minutes. As described above, it becomes possible to extremely gradually induce a change in the brain activity by not drastically performing the changeover of the bandwidth but overlapping the respective presentation times.

Next, the operational effects and applications of the apparatus, method and space of the present embodiment are described below.

According to the present embodiment, it is possible to decrease the brain activity within a few minutes by presenting the vibration of the specific frequency band that induces the negative effect to a healthy test human subject and make it a pathological state model. Furthermore, if the vibration of another frequency band that induces the positive effect is subsequently presented, the activity can be reversed to an increase within a few minutes. It is also possible to achieve a pathological state and a state in which the mental and physical conditions are improved in a short time with an identical test human subject, and this therefore makes it possible to produce a safe and effective pathological state model and a remedial model therefor. Moreover, it is possible to induce an increase or a decrease in the brain activity in a changeover manner within a few minutes in living spaces, duty spaces, amusement spaces, public spaces, vehicles and so on by utilizing this effect, and an application to induce an appropriate effect according to the situation is considered.

Moreover, an application to increase the expressive effects in artistic products and amusement spaces of halls, theater spaces and the like to perform them by utilizing this effect is considered. That is, in artistic products having story developments such as movies and theatrical performances, it is considered to exalt the effects of beauty, pleasure and emotion by slightly decreasing the brain activity of the viewers by presenting the band having the "negative effect" at a safe level in, for example, a scene where suffering and sadness are desired to be emphasized, and increasing the brain activity of the viewers by strongly presenting the band having the "positive effect" in, for example, a scene where pleasure, beauty and emotion are desired to be emphasized.

As described above, sounds whose bands are changed in accordance with the progress of time can be preparatorily recorded on a sound track in the case of a movie. Even in a case where a music is provided by live performance in a musical or the like, for example, the operator of the equalizer of a PA system can operate while viewing a score by preparatorily recording the temporal progress of band adjustment in the score.

### EMBODIMENT 7

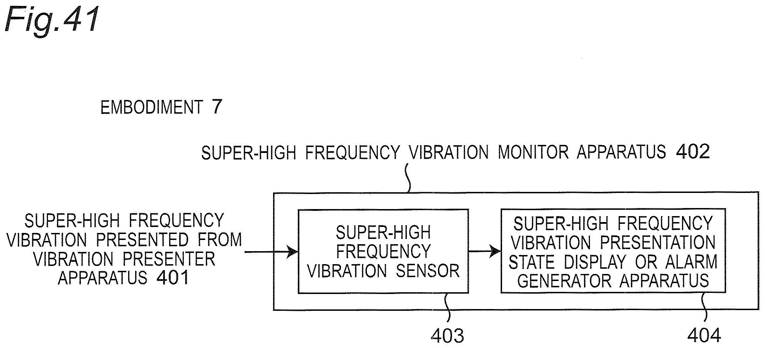

Figure 41 is a block diagram of a vibration monitor apparatus 402 according to an embodiment 7. This vibration monitor apparatus 402 converts a super-high frequency vibration presented from the vibration presenter 401 into an electrical signal by a super-high frequency vibration sensor 403, and displays the presentation state of the super-high frequency vibration by a super-high frequency vibration presentation state display apparatus 404 through conversion based on the signal into sensory stimulations including the visual sensation, auditory sensation, tactile sensation and the like that the listener or the like can recognize. Otherwise, when the super-high frequency vibration deviates from a preset range, an alarm is issued. An ultra-wideband capacitor microphone, a piezoelectric device, a MEMS device or a noncontact vibration detector apparatus or the like utilizing optical reflection and interference can be used for the super-high frequency vibration sensor 403. In particular, the piezoelectric device and the MEMS device, which have the features that they are inexpensive and endure rough handling, are therefore also expected to redeem the defects of the ultra-wideband capacitor microphone that is expensive and has inferiority in durability and weather resistance.

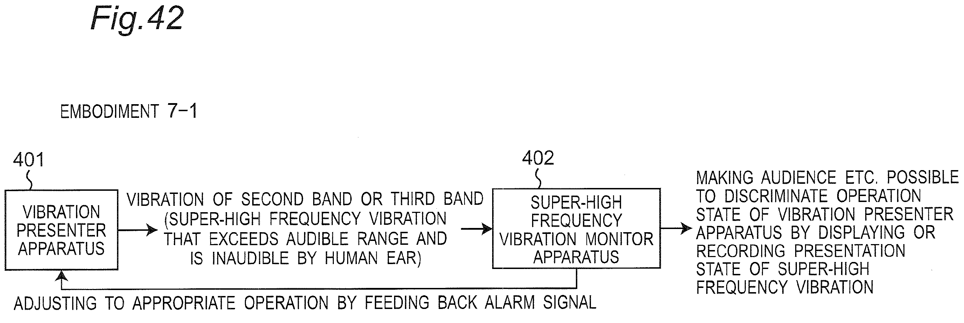

Figure 42 is a schematic graph showing an operation of a super-high frequency monitor apparatus 402 attached to the vibration presenter 401 according to an embodiment 7-1. In the vibration presenter 401, the greater part of the vibration of the second band and the vibration of the third band are super-high frequency vibrations that cannot be perceived by human beings. Therefore, it is difficult for the person to whom the vibrations are applied, the system administrator or such a person to discriminate whether or not the vibrations of the second and third bands are presented by the set presentation method and applied to the living body, and further whether or not the vibration presenter 401 is normally operating. For the above reasons, there is the risk that the brain activity increases or decreases differently from the presetting or set to a level different from the preset level due to, for example, the failure of the vibration presenter 401. Accordingly, the super-high frequency monitor apparatus 402 according to the embodiment 7-1 makes it possible to monitor the presentation state of the super-high frequency vibration presented from the vibration presenter 401 by converting the super-high frequency vibration presented from the vibration presenter 401 into a vibration signal, converting the intensity, variation state and the like of the vibration components of the second band or the third band in the obtained vibration signal into sensory signals including the visual sensation, auditory sensation, tactile sensation and vibration sensation, which can be perceived by human beings, and displaying them or having a function to record the information into a recording medium. Moreover, it is characterized in having a function to discriminate whether or not the actual operating state of the vibration presenter 401 is achieving the preset vibration state and informing the user or the system administrator of a case where the set vibration state and the actual vibration state are different from each other by an alarm signal and a function to send a feedback control signal to the vibration presenter 401. With this arrangement, for example, in a case where it is detected that the vibration of the second band is presented with an intensity higher than the range of the set level or it is detected that the vibration of the third band is feebler than the level of the set range, a reaction to avoid the negative effect can be caused.

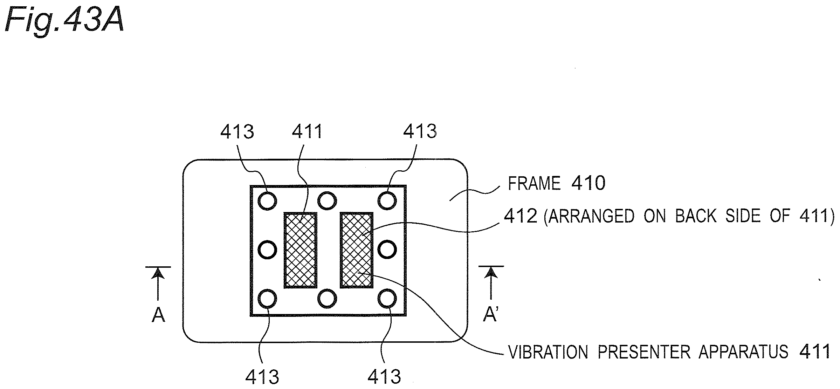

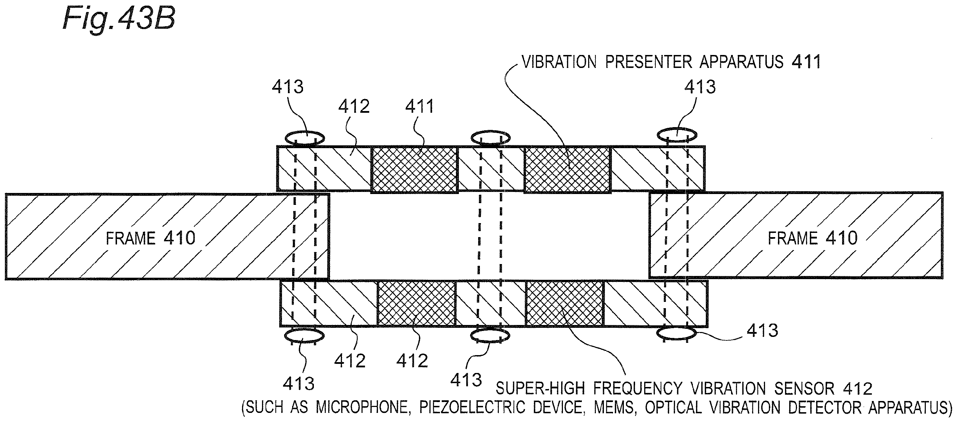

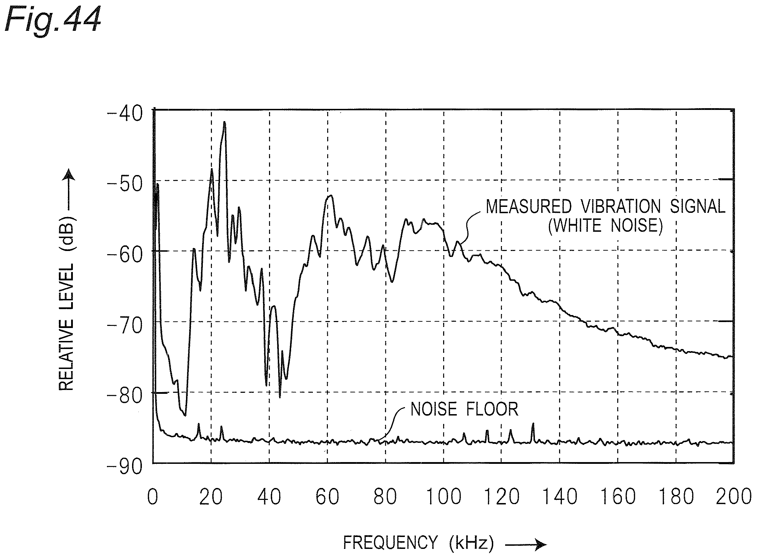

Figure 43A is a diagram showing a mounting example of an apparatus in which a vibration presenter 411 to present a super-high frequency vibration and a super-high frequency vibration sensor 412 are arranged mutually adjacently in the vibration presenting apparatus of the embodiment 7-1, and Figure 43B is a longitudinal sectional view along the line A-A' of Figure 43A. One super-high frequency piezoelectric device is used as the vibration presenter 411 to generate a super-high frequency aerial vibration, and another one is used as the super-high frequency vibration sensor 412 to form the super-high frequency vibration generated by the vibration presenting apparatus into an electrical signal. By adjacently arranging these two devices, the vibration presented by the vibration presenter 411 is transferred to the super-high frequency vibration sensor 412. Figure 44 shows a frequency characteristic when the vibration of an ultra-wideband white noise generated by the vibration presenter 411 is formed into an electrical signal by the super-high frequency vibration sensor 412 by using the apparatus including the vibration presenter 411 and the super-high frequency vibration sensor 412 of the aforementioned configurations. This apparatus including the vibration presenter 411 and the super-high frequency vibration sensor 412 has a frequency characteristic that reaches 200 kHz and detects signals regarding the second band and the third band at an S/N ratio of roughly 20 to 30 dB though it has a large dip at and around 40 kHz, indicating the effectiveness of this embodiment.

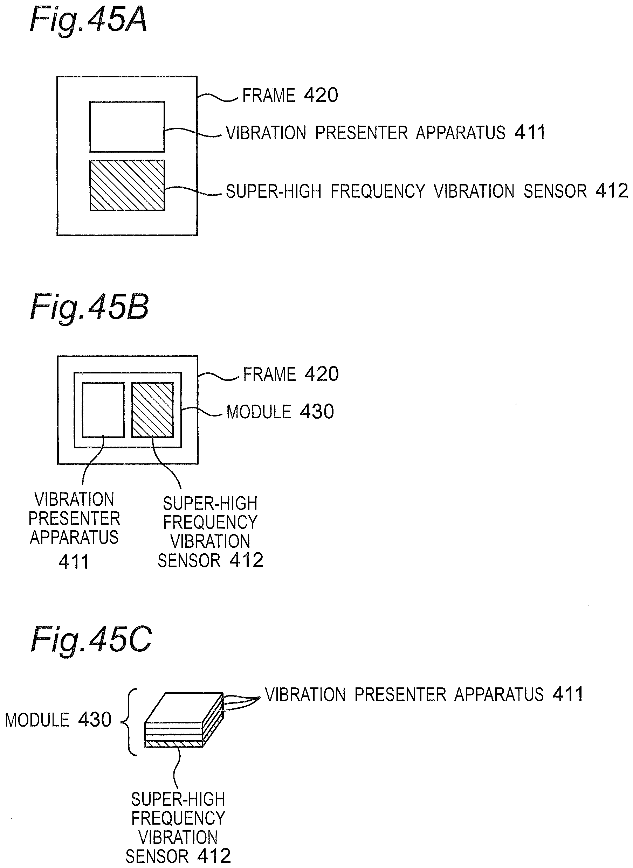

Figures 45A, 45B and 45C are modified embodiments of Figures 43A and 43B. Figure 45A shows an example in which the vibration presenter 411 and the super-high frequency vibration sensor 412 are adjacently arranged, Figure 45B shows an example in which the vibration presenter 411 and the super-high frequency vibration sensor 412 are compositely arranged in a single piezoelectric device module 430, and Figure 45C shows an example in which the two functions of the vibration presenter 411 and the super-high frequency vibration sensor 4122 are achieved by one module 430 by sharingly using the piezoelectric device of a multilayer structure as a layer to be used as the vibration presenter 411 and a layer to be used as the super-high frequency vibration sensor 412.

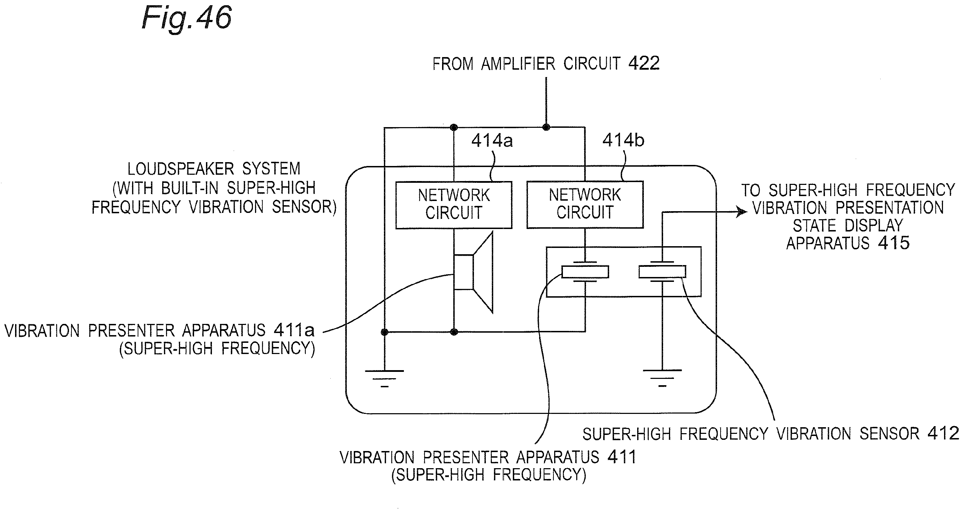

Figure 46 is a circuit diagram in a case where the apparatus of Figures 43A and 43B is constituted as a loudspeaker system with a built-in super-high frequency vibration sensor. It is constituted by mechanically bonding the super-high frequency vibration sensor 412 to the built-in vibration presenter 411 of the loudspeaker system. A signal vibration obtained from the super-high frequency vibration sensor is sent to a super-high frequency vibration presentation state display apparatus. Referring to Figure 46, 414a and 414b are the network circuits to perform band dividing, and 411a is a super-high frequency vibration presenting apparatus.

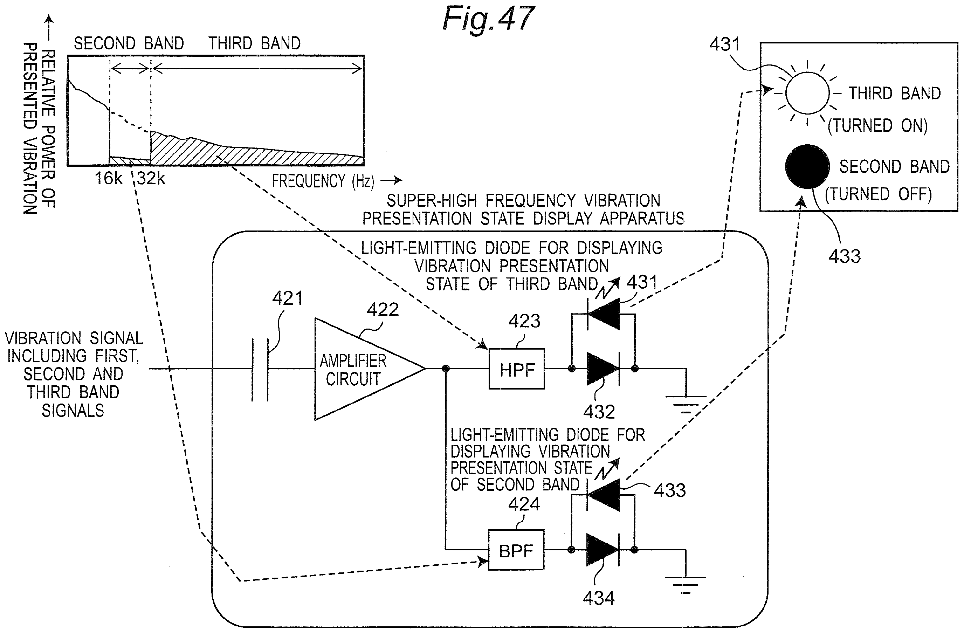

Figure 47 is a circuit diagram of a super-high frequency vibration presentation state display apparatus that can be connected to the apparatus of Figure 46. A super-high frequency vibration signal inputted from the super-high frequency vibration sensor of Figure 46 has its dc component interrupted by a capacitor 421 and then amplified by an amplifier 422. The vibration signal of the third band is extracted by a high-pass filter (HPF) 423, and a vibration presentation state display light emitting diode 431 of the third band is turned on with its brightness changed in accordance with the intensity. Likewise, the vibration signal of the second band is extracted by a band-pass filter (BPF) 424, and a vibration presentation state display light emitting diode 433 of the second band is turned on with its brightness changed in accordance with the intensity. With this arrangement, it becomes possible to visually confirm a state in which the vibrations of the second band and the third band that are not heard as sounds are presented as aerial vibrations, and to judge whether or not a proper presentation state is provided. Moreover, although the example in which the brightness is simply continuously changed in accordance with the intensity is described here, it is acceptable to change the brightness in steps in accordance with the intensity of the vibration to distinguish the vibration presentation state more clearly or to display the intensity in the form of a level meter that changes the number of lighting LEDs arranged in a bar graph. Otherwise, it is acceptable to take display means such that lighting is effected when the vibration intensity of each band exceeds a predesignated threshold value and extinction is effected when it falls under the value, or conversely lighting is effected when the vibration intensity falls under the value and extinction is effected when it exceeds the value. Otherwise, it is acceptable to take display means integrated with an integrator circuit with a predetermined time constant in order to avoid a frequent change in the display contents reflecting a change in a short time occurring in the vibration presentation state. Further, it is acceptable to use, for example, an analog type level meter such that the angle of the needle changes in accordance with the vibration intensity or to digitally display a digitized value of the vibration intensity instead of using the LED. Moreover, although the example in which the vibration state is displayed as visual information is described here, it is acceptable to display it auditorily as an alarm of a voice, a mechanical sound or the like, to display it tactically as a vibration or to display it via another sensation signal.

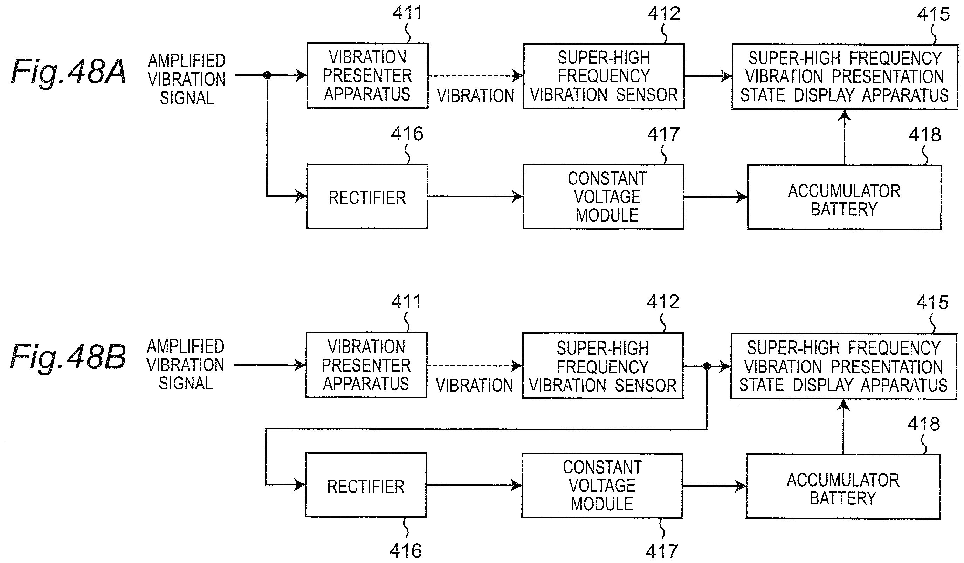

Figure 48 is a block diagram showing two modified embodiments (a) and (b) of a method for supplying the super-high frequency vibration presentation state display apparatus with an electric power by utilizing a vibration signal, photovoltaic or the like without using an external power source in the vibration monitor apparatus bonded to the vibration presenting apparatus. Referring to Figure 48(a), an amplified vibration signal inputted to the vibration presenting apparatus is diverged, and this is put through a rectifier 416 to form only a dc component, and it is further put through a constant voltage module 417 to be formed into a constant voltage and stored into an accumulator battery 418. The super-high frequency vibration presentation state display apparatus 415 is driven by using the power stored in this place. This obviates the need for separately laying cables for performing power supply to the vibration monitor apparatus and the need for performing battery replacement in the case of battery driving, allowing the cost of the installation configuration and the cost and frequency of maintenance to be reduced. As power supply means, a solar photovoltaic system or the like may be used. Moreover, the power obtained in this case may be used for applications other than driving the super-high frequency vibration presentation state display apparatus 415. Further, as shown in Figure 48(b), there may be such a configuration that a power source is obtained from an output signal of the super-high frequency vibration sensor 412.

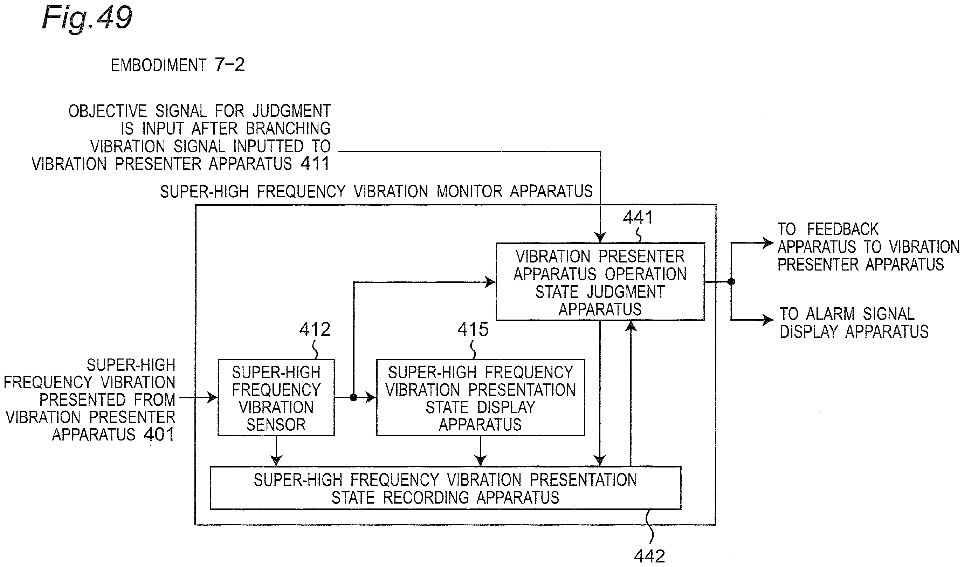

Figure 49 is a block diagram of a super-high frequency vibration monitor apparatus according to an embodiment 7-2. This vibration monitor apparatus is characterized in that a vibration presenting apparatus operation state judgment apparatus 441 and a super-high frequency vibration presentation state recording apparatus 442 are provided in addition to the super-high frequency vibration sensor 412 and the super-high frequency vibration presentation state display apparatus 415. A vibration signal formed into an electrical signal by the super-high frequency vibration sensor 412 is inputted to and processed in the super-high frequency vibration presentation state display apparatus 415 such as the embodiment 7-1, and inputted to the vibration presenting apparatus operation state judgment apparatus 441. The vibration signal inputted to the vibration presenter 411 of the vibration presenter 401 diverges to be separately inputted to the vibration presenting apparatus operation state judgment apparatus 441. The vibration presenting apparatus operation state judgment apparatus 441 evaluates the vibration signal inputted from the super-high frequency vibration sensor 412 using the original vibration signal inputted from the vibration presenter 401 as a reference, and judges whether or not the operation state of the vibration presenter 401 is appropriate, i.e., whether or not the vibrations of the second band and the third band or part of them are presented at the set level. Then, the result is outputted to a feedback apparatus to vibration presenting apparatus, an alarm signal output apparatus and the like of external apparatuses. The super-high frequency vibration presentation state recording apparatus 442 records the processing result of each apparatus in the super-high frequency vibration monitor apparatus. The vibration presenting apparatus operation state judgment apparatus 441 can also evaluate a long-term state with reference to a history recorded in the super-high frequency vibration presentation state recording apparatus 442.

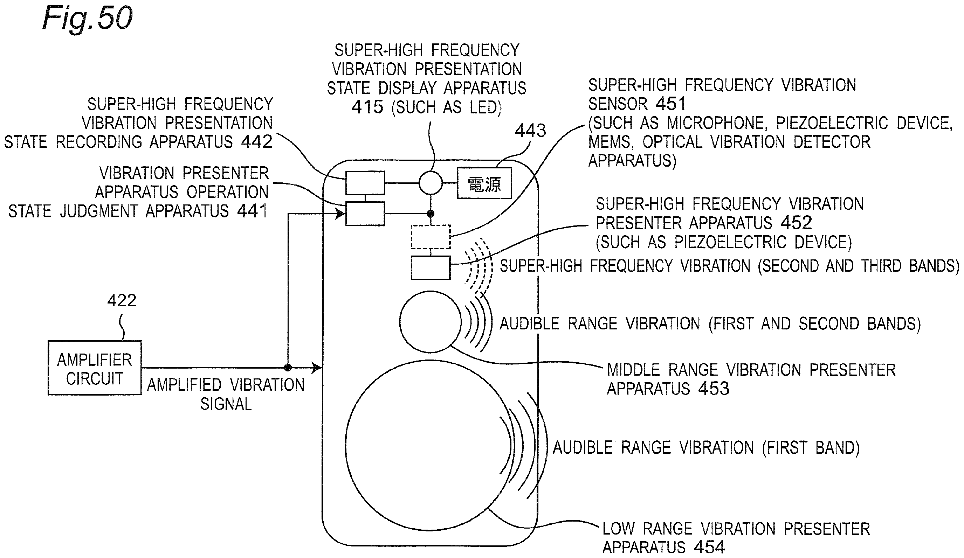

Figure 50 is a block diagram showing an application example in a case where the vibration presenting apparatus with the built-in super-high frequency vibration monitor apparatus according to the embodiment 7-2 is constituted as a loudspeaker system. It may be used for electronic equipment that generates audio signals such as television receivers and computers, electronic equipment that generates noises and driving sounds such as air conditioners and refrigerators, and public address broadcasting electronic facilities and the like to transmit voices and BGMs besides the loudspeaker system. It is acceptable to incorporate the super-high frequency vibration monitor apparatus into the main body of the loudspeaker system or to transmit the vibration signal obtained by the ultrasonic vibration sensor to the outside of the loudspeaker system by a wired, wireless and infrared ray method and the like and process the signal in another apparatus. Among the components of the vibration presenting apparatus, part or all of them other than the vibration presenting apparatus may be built in. There are shown a power source 443, a super-high frequency vibration sensor 451, a super-high frequency vibration presenting apparatus 452, a middle range vibration presenting apparatus 453, and a low range vibration presenting apparatus 454.

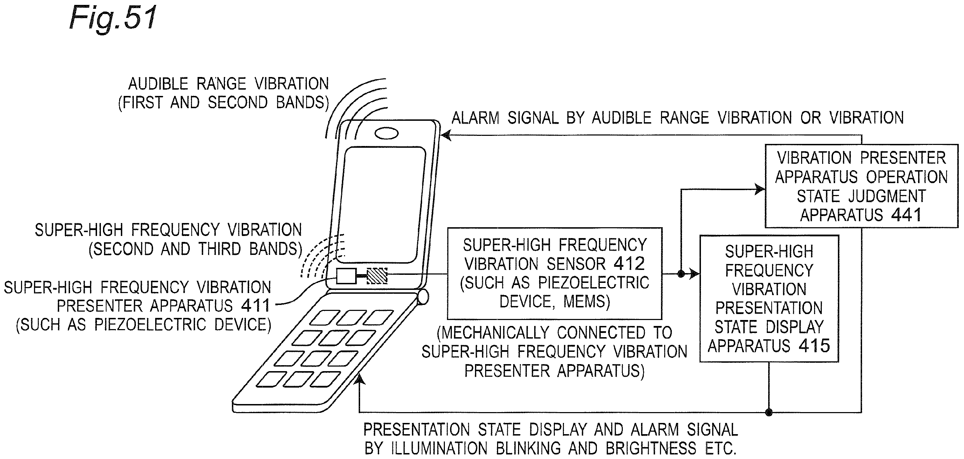

Figure 51 is a block diagram showing an application example in a case where the vibration presenter 401 with the built-in super-high frequency vibration monitor apparatus according to the embodiment 7-2 is constituted as a portable telephone. It may be built in portable electronic equipment such as smart phones, portable terminals, portable players, portable radio receivers, portable television sets and game machines, headphones and cables attached to them or small things and clothes such as accessories, hats, and glasses besides the telephone. In the case of such compact equipment, it is also possible to additionally incorporate means for outputting a display and an alarm signal by vibrations, voices or mechanical sounds into the equipment since it does not catch the listener' eye even if a visual display is performed when it is put in a pocket or the like. It is acceptable to incorporate the super-high frequency vibration monitor apparatus into the main body of the portable electronic equipment including portable telephones or to transmit the vibration signal obtained by the ultrasonic vibration sensor to the outside of the portable telephone by a wired, wireless and infrared ray method and the like and process the signal in another apparatus. The vibration signal may be obtained from the outside by means of data distribution or the like.

Next, the operational effects and applications of the present embodiment are described below.

According to the present embodiment, it becomes possible to monitor whether the vibration components of the second band or the third band containing the components that exceed the audible range and are inaudible to human beings though they are indispensable for the vibration presenting apparatus are actually presented at the preset level. Moreover, by providing a built-in super-high frequency vibration sensor for the vibration presenting apparatus or a built-in sensor in, for example, an accessory, a furniture, a remote controller, a portable telephone or the like near the human being to whom the vibration is presented for the monitoring, it becomes possible to monitor the vibration state more accurately and reliably. Furthermore, it becomes also possible to improve the independency of the system and make it maintenance free by supplying electric power using the vibration signal itself. The vibration state and the alarm signal obtained by this super-high frequency vibration monitor apparatus can be utilized for grasping the operating state of the vibration presenting apparatus in real time and finding troubles by displaying them visually with a light emitting diode or the like for the listener or the system administrator or by transmission with a vibration, a voice or the like, and also used for operating state feedback of the vibration presenting apparatus.

### EMBODIMENT 8

Figure 52 is a graph showing a brain activity setting method (presentation method having a feedback function to adjust the level for each divided band) of a vibration processing apparatus according to an embodiment 8. Figure 52(a) is a schematic graph of the power spectrum of the presented vibrations of the divided bands regarding a vibration signal that has the first, second and third bands, and Figure 52(b) is a graph showing an index of brain activity (difference in DBA-index) in the divided bands. Referring to Figure 52, the vibration presenting apparatus, method and space according to the present embodiment are characterized in having a feedback mechanism that monitors the brain activity or another index of the fundamental brain 200c of a listener 200, and adjusts the level of each frequency band of the vibrations presented to the listener 200 on the basis of the information. Two embodiments classified by the index to be a clue of feedback are shown below. That is, they are (A) a system based on the index of brain activity or another physiological index, and (B) a system based on a quantitative measurement index (action data or the like).

First of all, (A) the feedback system based on the index of brain activity or another physiological index is described below.

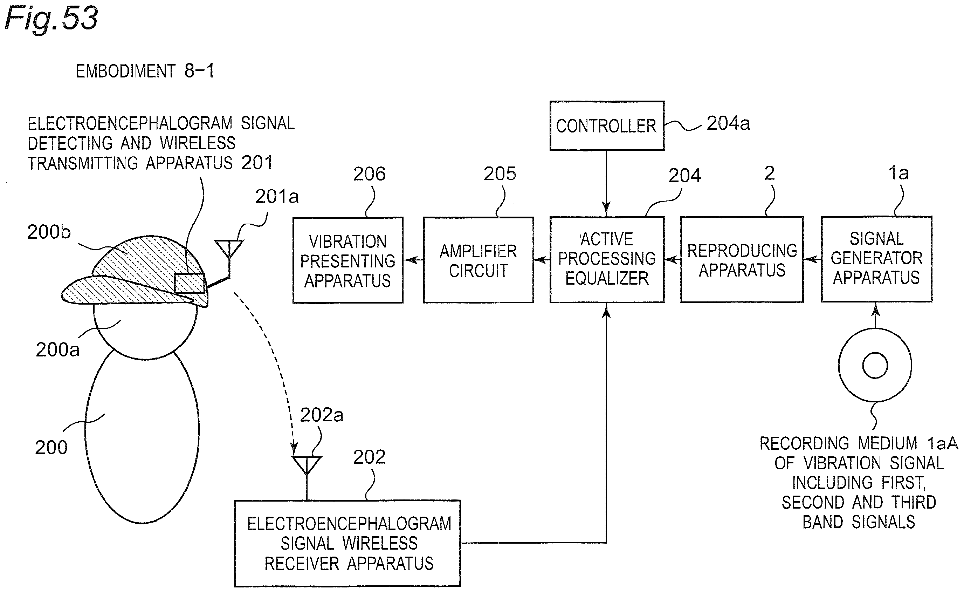

Figure 53 is a block diagram showing a configuration of a vibration presenting apparatus according to an embodiment 8-1. Referring to Figure 53, the vibration presenting apparatus, method and space according to the embodiment 8-1 are characterized by having a feedback system that uses the deep index of brain activity (DBA-index) based on electroencephalogram measurement as an index. Referring to Figure 53, an electroencephalogram signal wireless transmitter apparatus 201 is housed in a hat 200b at the head 200a of a vibration listener 200, and the brain wave of the vibration listener 200 is measured, and a wireless signal including the electroencephalogram signal is wirelessly transmitted to an electroencephalogram signal wireless receiver apparatus 202 via an antenna 201a by, for example, wireless communication means. The electroencephalogram signal wireless receiver apparatus 202 wirelessly receives the wireless signal including the electroencephalogram signal by using the antenna 202a, demodulates the electroencephalogram signal, and outputs the resulting signal to an active processing equalizer 204. The active processing equalizer 204 calculates in real time the deep index of brain activity (DBA-index) of the listener from the measured electroencephalogram signal. After being set by using, for example, an input part (not shown), the level of each band of the vibration to be next presented from the level of each band that is currently being presented is calculated by the vibration signal (containing at least the first, second, and third band signals) that is read from the recording medium 1aA of the vibration signal by the signal generator apparatus 1a and thereafter reproduced by the reproducer circuit 2 on the basis of the condition setting of an index of brain activity target value, an enhancement or reduction width and the like inputted from the controller 204a, performing an equalizing process for the vibration signal inputted from the reproducer circuit 2. The vibration signal after the equalizing process is amplified by an amplifier circuit 205 and thereafter presented to the listener 200 by a vibration presenting apparatus 206. It is noted that the present apparatus may have means for displaying the electroencephalogram signal and the brain activity state or have a function capable of directly controlling the feedback contents by the operator via the input part of the controller 204a.

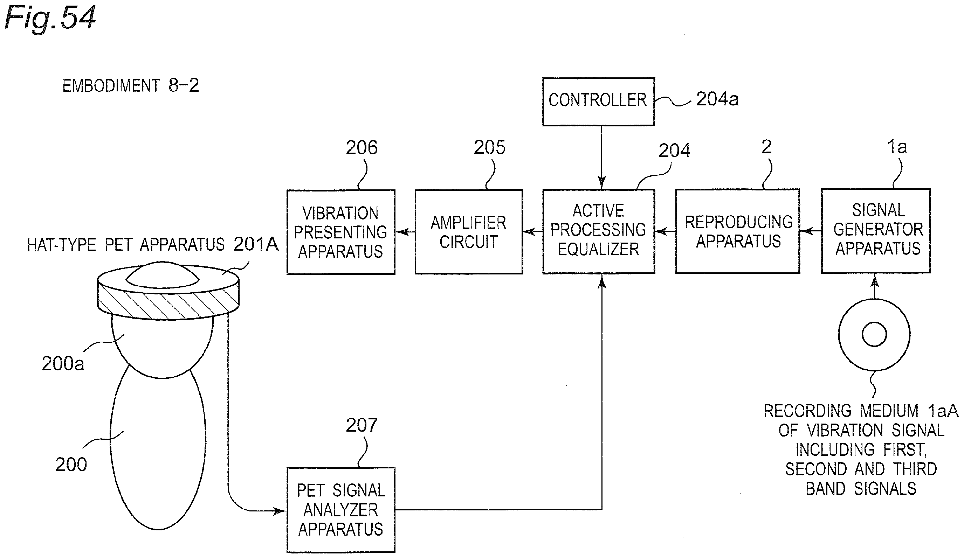

Figure 54 is a block diagram showing a configuration of a vibration presenting apparatus according to an embodiment 8-2. The vibration presenting apparatus, method and space according to the embodiment 8-2 are characterized by having a system that comprehensively judges the brain activity state for a time of, for example, not shorter than several minutes using the regional cerebral blood flow based on PET measurement as an index and feeds it back. Referring to Figure 54, a PET signal analyzer apparatus 207 analyzes the regional cerebral blood flow of each region of the listener's brain by the PET signal analyzer apparatus 207 from a PET signal obtained by a hat type PET (positron emission type tomography) apparatus 201A placed on the head 200a of the listener 200 and outputs a control signal that indicates it to the active processing equalizer 204. The active processing equalizer 204 calculates the level of each band of the vibration to be next presented from the regional cerebral blood flow of the fundamental brain and the level of each band of the vibration that is currently being presented on the basis of setting inputted from the controller 204a, and finds the equalizer quantity with respect to the original signal. After being read from the recording medium 1aA of the vibration signal by the signal generator apparatus 1a, the vibration signal (containing at least the first, second and third band signals) reproduced by the reproducer circuit 2 is subjected to the equalizing process. In this case, a concrete example of the active processing equalizer process is similar to the processing example according to the aforementioned diagram 53. The vibration signal after the equalizing process is amplified by the amplifier 205, and thereafter presented to the listener 200 by the vibration presenting apparatus 206. It is noted that the present apparatus may have means for displaying the electroencephalogram signal and the brain activity state or have a function capable of directly controlling the feedback contents by the operator via the input part of the controller 204a.

It is noted that a feedback system can be formed by using a physiological index other than the above. For example, there can be considered a cerebral blood flow, a magnetoencephalogram, a skin resistance value, a mental sweating rate, a stress index in saliva, a physiologically active substance concentration in blood, a blood pressure, a heart beat, a skin temperature and the like by MRI (magnetic resonance imaging system) and NIRS (near-infrared ray spectroscopy).

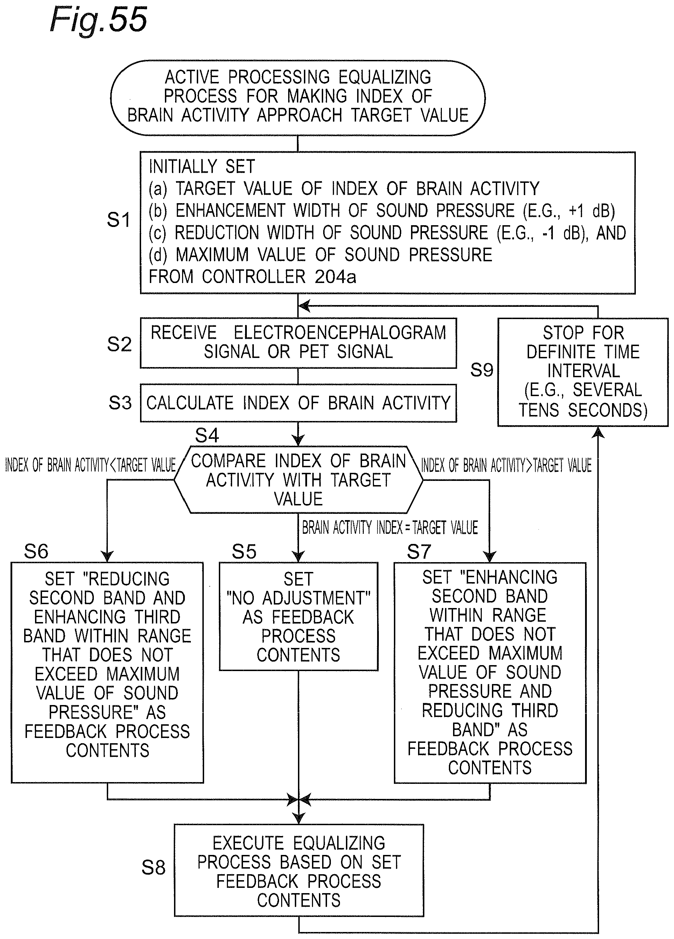

Figure 55 is a flow chart showing an active processing equalizing process to put the index of brain activity approach a target value executed by the active processing equalizer 204 of Figure 53 or the active processing equalizer 204 of Figure 54.

Referring to Figure 55, first of all, in step S1, (a) a target value of the index of brain activity, (b) an enhancement width of sound pressure of, for example, +1 dB, (v) a sound pressure reduction width of, for example, -1 dB, and (d) a sound pressure maximum value (a sound pressure maximum value set in the equalizing process, and so forth hereinafter) are inputted for initial setting from a controller 204a that has an input part (not shown) of, for example, a keyboard. Next, an electroencephalogram signal (PET signal of Figure 54) is received in step S2, and the index of brain activity is calculated in step S3 by the aforementioned method on the basis of the received electroencephalogram signal. In step S4, the calculated index of brain activity is compared with the target value set as above. The program flow proceeds to step S5 when the index of brain activity = target value, proceeds to step S6 when the index of brain activity < target value or proceeds to step S7 when the index of brain activity > target value. In step S5, "no adjustment" is set as feedback process contents, and the program flow proceeds to step S8. In step S6, "reducing the second band by the aforementioned set reduction width and enhancing the third band by the aforementioned set enhancement width within a range that does not exceed the sound pressure maximum value" is set as feedback process contents, and the program flow proceeds to step S8. Further, in step S7, "enhancing the second band within a range that does not exceed the sound pressure maximum value and reducing the third band" is set as feedback process contents, and the program flow proceeds to step S8. The equalizing process is executed in step S8 on the basis of the set feedback process contents, it stops for a definite time interval of, for example, several tens of seconds in step S9, and the program flow returns to step S2.

In the active processing equalizing process of Figure 55, when the index of brain activity measured in comparison with the target value of the preset index of brain activity is high, the second band components of the aerial vibration applied to a human being are enhanced or the third band components are simultaneously attenuated. By this operation, it becomes possible to decrease the brain activity. Conversely when the measured index of brain activity is lower than the target value of the preset index of brain activity, the third band components of the aerial vibration applied to the human being are enhanced or the second band components are simultaneously attenuated. By this operation, it becomes possible to enhance the brain activity. Therefore, it becomes possible to adjust the components of the applied aerial vibration so that they are controlled to the preset brain activity by applying the aerial vibration while measuring the index of brain activity by using the apparatus of the present embodiment.

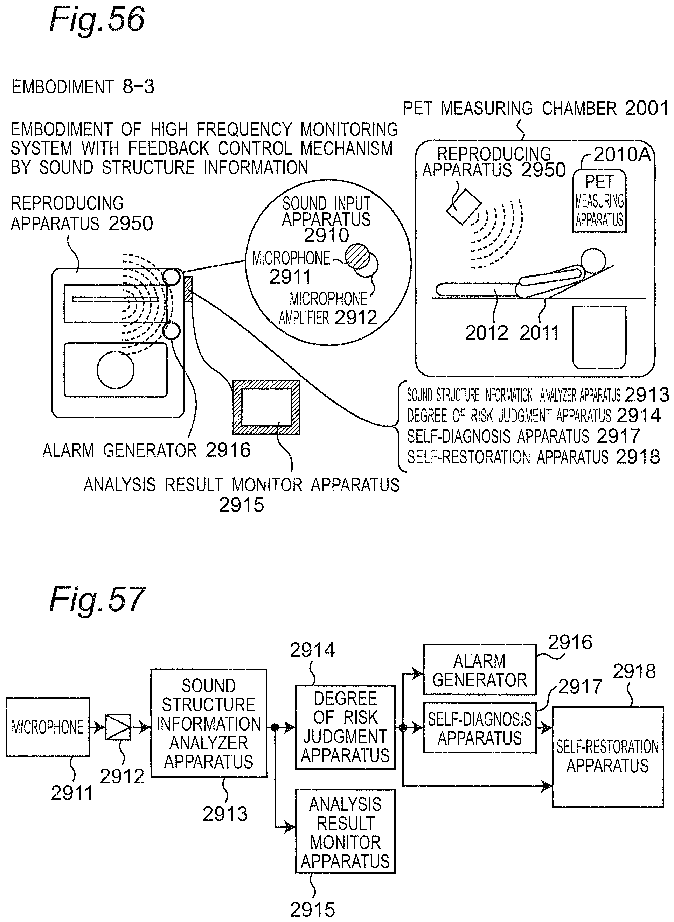

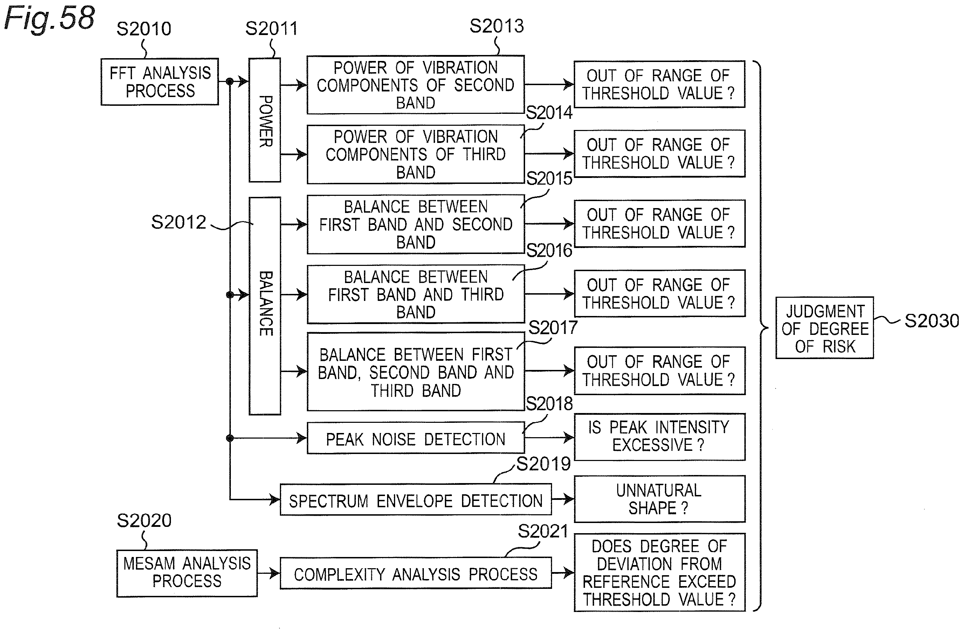

Figure 56 is a block diagram showing an implemental example of a high-frequency monitoring system having a feedback control mechanism by sound structure information according to an embodiment 8-3, and Figure 57 is a block diagram showing a detailed configuration of the high-frequency monitoring system of Figure 56. Figures 58 to 60 are flow charts showing a detailed processing of the high-frequency monitoring system of Figure 56.

The present embodiment is related to sound a structure information monitoring and feedback system, which is intended to confirm the occurrence situation of a super-perception vibration and to adjust the vibration reproduction level of the audible range and the super-perceptual region by feeding back the analysis result of the acoustic structure to a super-perception vibration reproducing apparatus 2950. It is constituted of a microphone 2911 that is installed in the vicinity of the super-perception vibration generator apparatus 2950 or the like of a PET measuring chamber 2001 where a test human subject 2012 is subjected to PET measurement by a PET measuring apparatus 2010A and collects peripheral environment sounds, an analyzer apparatus 2913 or the like to analyze the acoustic structure of the collected data, and a monitor apparatus 2915 that indicates the analysis result. The monitor apparatus 2915 displays, for example, an FFT spectrum to observe the average of the frequency structure, a maximum entropy spectrum array to visually indicate the temporal change of the frequency structure, an ME spectrum first-order differentiation cumulative change amount, a first-order differentiation cumulative change amount and the like, which serve as the indexes of the complicatedness of the sound structure. With this arrangement, the listener and user become able to confirm the structure of the super-perception vibration that cannot be perceived. This becomes help in preventing the occurrence of a negative influence such as a decrease in the cerebral blood flow when a trouble occurs in generating the super-perception vibration. Moreover, it becomes a help in stably enjoying the hypersonic effects by virtue of the existence of musics in the audible range, environmental sounds, broadcasting sounds, voices and the like concurrently with the super-perception vibration.

Referring to Figure 56, the reproducing apparatus 2950 has a sound signal input apparatus 2910 configured to include a microphone 2911 and a microphone amplifier 2912, a sound structure information analyzer apparatus 2913, a degree of risk judgment apparatus 2914, a self-diagnosis apparatus 2917, a self-restoration apparatus 2918, an alarm generator 2916, and an analysis result monitor apparatus 2915. Referring to Figure 57, a sound signal is converted into an electrical signal by the microphone 2911 and thereafter inputted to the sound structure information analyzer apparatus 2913 via the microphone amplifier 2912. The sound structure information analyzer apparatus 2913 analyzes the sound structure information of the inputted sound, calculates, for example, the powers of the second band and the third band, and outputs the analysis results to the degree of risk judgment apparatus 2914 and the analysis result monitor apparatus 2915. The degree of risk judgment apparatus 2914 judges the degree of risk on the basis of the analysis results of the inputted sound information, and outputs the judgment result to the alarm generator 2916, the self-diagnosis apparatus 2917 and the self-restoration apparatus 2918. These concrete processes are described below with reference to Figures 58 to 60.

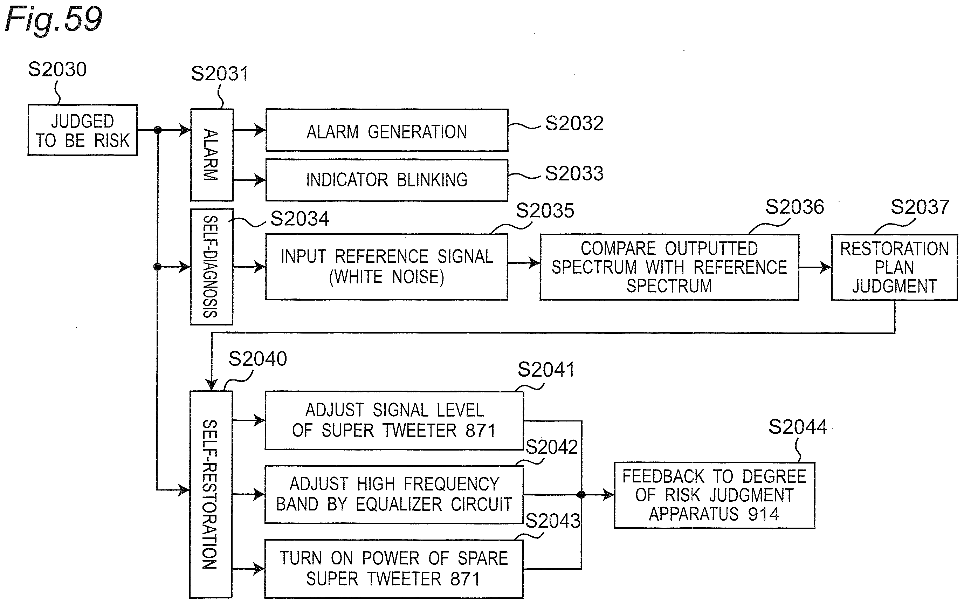

The processes of Figure 58 are executed by the sound structure information analyzer apparatus 2913 and the degree of risk judgment apparatus 2914. Referring to Figure 58, an FFT (Fast Fourier Transform) analysis process (S2010) is executed, so that power detection (S2011), component power balance detection (S2012), peak noise detection (S2018), and spectrum envelope detection (S2019) are executed. In the power detection (S2011), it is judged whether or not the power of the second band (S2013) is out of the range of a predetermined threshold value to judge the degree of risk (S2030), and it is judged whether or not the power of the third band (S2014) is out of the range of a predetermined threshold value to judge the degree of risk (S2030). Moreover, in the component power balance detection (S2012), it is judged whether or not the balance (component ratio) between the first band and the second band is out of the range of a predetermined threshold value to judge the degree of risk (S2030). It is judged whether or not the balance (component ratio) between the first band and the third band is out of the range of a predetermined threshold value to judge the degree of risk (S2030), and it is judged whether or not the balance (component ratio) between the first band, the second band and the third band is out of the range of a predetermined threshold value to judge the degree of risk (S2030). Further, in the peak noise detection (S2018), it is judged whether or not the intensity of the peak is so excessive as to exceed a predetermined level to judge the degree of risk (S2030). Furthermore, in the spectrum envelope detection (S2019), it is judged whether or not it has an unnatural shape different from the preparatorily stored natural shape to judge the degree of risk (S2030). Furthermore, the MESAM analysis process (S2020) is executed, the complexity analysis process (S2021) is executed, and it is judged whether or not the degree of deviation from a predetermined reference exceeds a predetermined threshold value to judge the degree of risk (S2030). In the processes of Figure 59, the degree of risk is judged on the basis of a plurality of judgments.

Referring to Figure 59, when a judgment of risk is made (S2030), an alarm is issued (S2032) in the alarm process (S2031), and an index blinks (S2033). Moreover, in the self-diagnosis process (S2034), a reference signal that is a white noise is inputted via a microphone 2911 (S2035), and the spectrum of the outputted sound signal is compared with a predetermined reference spectrum to make a judgment of a repair policy on this basis (S2037) and execute the following self-restoration process (S2040). Moreover, it is acceptable to execute the following self-restoration process (S2040) when a judgment of risk is made (S2030). In the self-restoration process, the signal level of a super-tweeter 871 that presents the second band is lowered by a predetermined level or the signal level of a super-tweeter 871 that presents the third band is raised by a predetermined level (S2041). The second band is attenuated by a predetermined level by the equalizer circuit or the third band is enhanced by a predetermined level (S2042), and the power of an auxiliary super-tweeter 871 that presents the third band is turned on (S2043). After such a self-restoration process is executed, a feedback is sent to the degree of risk judgment apparatus 2914 to perform again the judgment of risk.

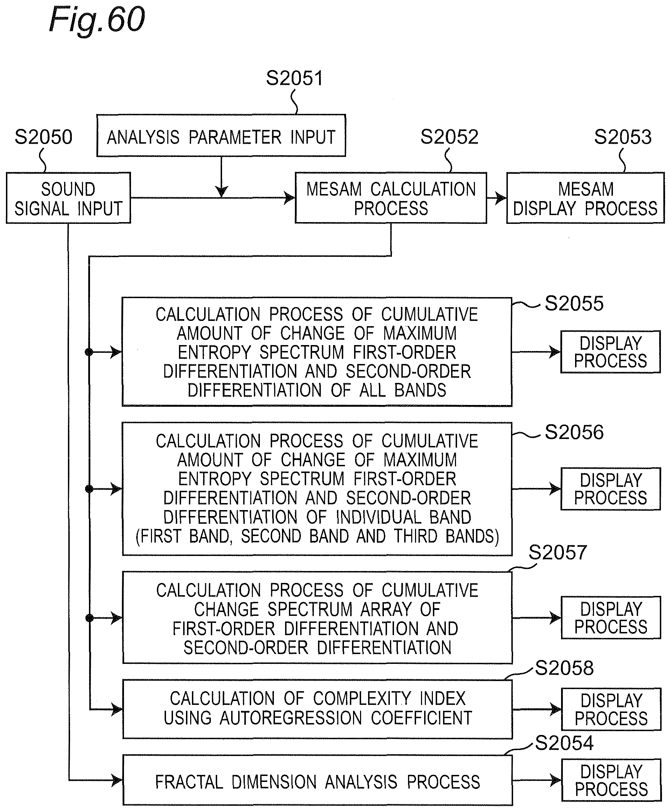

Figure 60 shows items of processes related to the input of sound signals, and items related to calculations and display processes therefor. A sound signal is inputted (S2050), predetermined analysis parameters are inputted (S2051), and the MESAM calculation process (S2052) and the display processes thereof (S2053) are executed. Moreover, a fractal dimension analysis process (S2054) is executed, and the display process thereof is performed. Further, in the MESAM calculation process (S2052), the following various calculation processes are executed.
(1) A calculation process (S2055) of a cumulative amount of change of the maximum entropy spectrum first-order differentiation and second-order differentiation of all bands is executed, and the display process thereof is performed.
(2) A calculation process (S2056) of a cumulative amount of change of the maximum entropy spectrum first-order differentiation and second-order differentiation of individual band (first band, second band, and third band) is executed, and the display process thereof is performed.
(3) A calculation process (S2057) of the cumulative change spectrum array of first-order differentiation and second-order differentiation is executed, and the display process thereof is performed.
(4) A calculation process (S2058) of a complexity index is executed by applying an autoregression coefficient, and the display process thereof is performed.

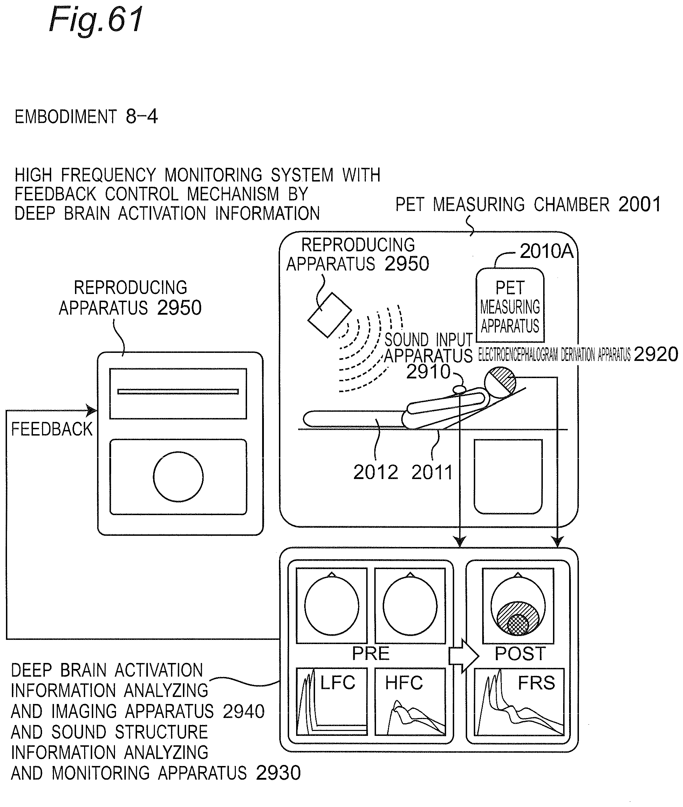

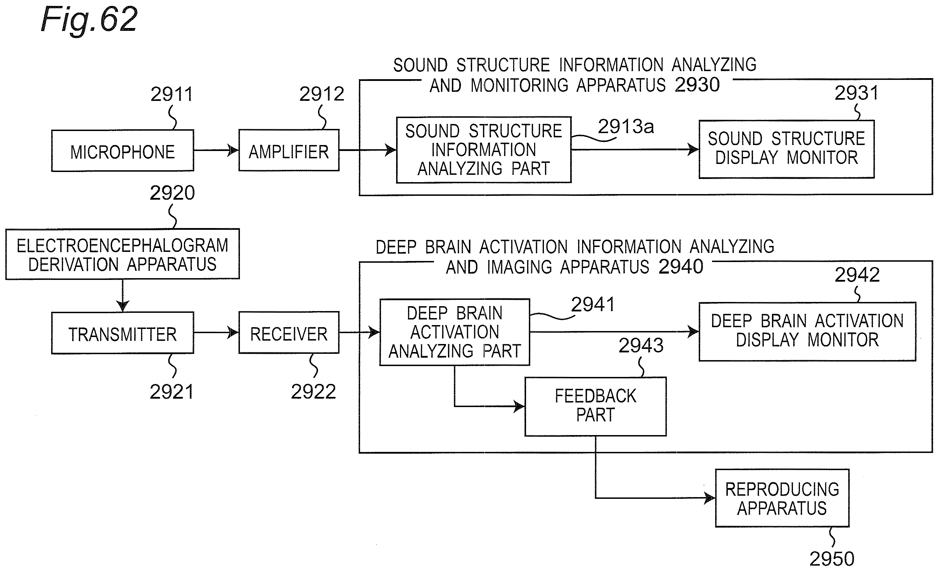

Figure 61 is a block diagram showing an implemental example of a high-frequency monitoring system having a feedback control mechanism by deep brain activation information according to an embodiment 8-4, and Figure 62 is a block diagram showing a detailed configuration of the high-frequency monitoring system of Figure 61. Figures 61 and 62 show an embodiment of a deep brain activation information monitoring and feedback system. In the present embodiment, the situation of the deep brain activation is confirmed, and the result thereof is fed back to the vibration reproducer apparatus 2950 aiming at adjusting the vibration reproduction levels of the first band of the audible range and the second and third bands. It is installed in the vicinity of a test human subject 2012 in a PET measuring chamber 2001 or the like, and is constituted of a sound input apparatus 2910 including a microphone 2911 to collect peripheral environment sounds, an electroencephalogram derivation apparatus 2920 to derive a deep index of brain activity, a deep brain activity information analyzing and imaging apparatus 2940 to analyze the deep index of brain activity, a sound structure information analyzing and monitoring apparatus 2930 to indicate the analysis result, and an apparatus to feed it back to the reproducer apparatus 2950. This becomes help in preventing the occurrence of a hypersonic negative effect of the adverse effect such as a decrease in the cerebral blood flow. Otherwise, it becomes help in stably enjoying the hypersonic positive effect.

Referring to Figure 62, a sound signal reproduced by the reproducer apparatus 2950 is converted into an electrical signal by the microphone 2911 and thereafter inputted to a sound structure information analyzing part 2913a of the sound structure information analyzing and monitoring apparatus 2930 via the amplifier 2912. The sound structure information analyzing part 2913a analyzes the sound structure information of the inputted reproduction sound signal and thereafter displays the sound structure on a sound structure monitor apparatus 2931. Moreover, the information of the deep brain activation index derived by the electroencephalogram derivation apparatus 2920 is transmitted by a transmitter 2921, thereafter received by a receiver 2922, and inputted to a deep brain activation analyzing part 2941 of the deep brain activity information analyzing and imaging apparatus 2940. The deep brain activation analyzing part 2941 analyzes the information of the inputted deep brain activation index, displays the analysis result on the deep brain activation display monitor 2942, and feeds the information back to the reproducer apparatus 2950 via a feedback part 2943. By thus controlling the reproduction parameters of the reproducer apparatus 2950 on the basis of the analysis result of the information of the deep brain activation index, the occurrence of the hypersonic negative effect that is the adverse effect such as a decrease in the cerebral blood flow can be prevented.

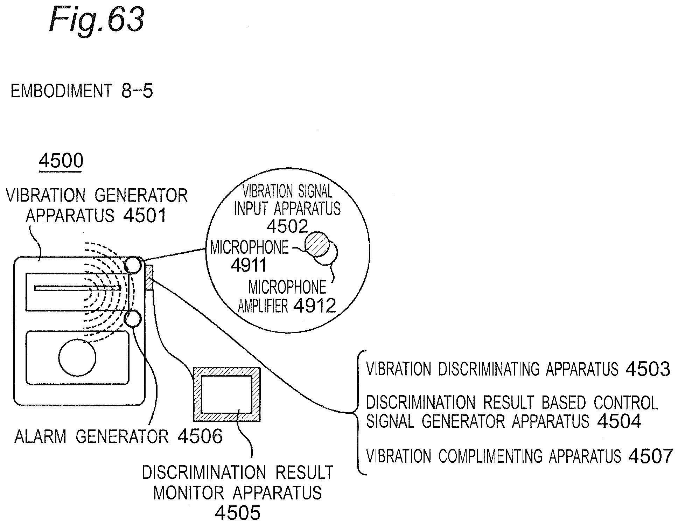

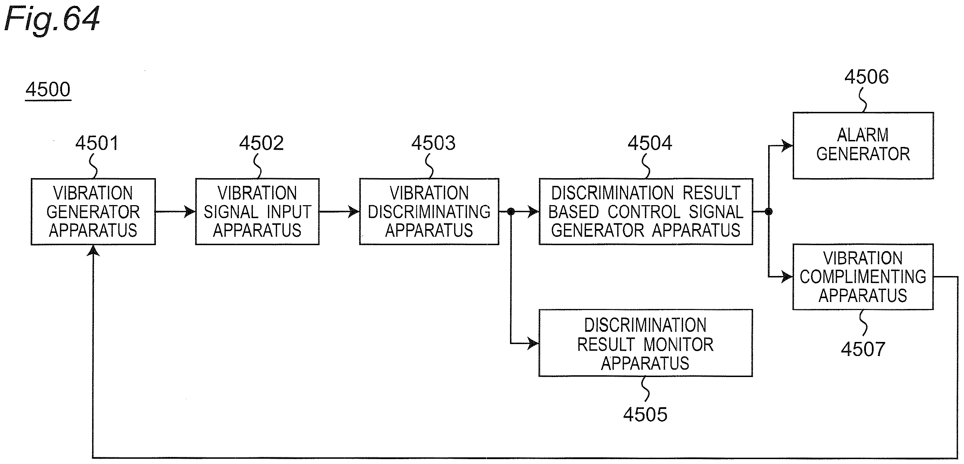

Figure 63 is a perspective view showing an implemental example of a vibration monitoring system 4500 that performs adjustment of vibration generation setting by feedback to a vibration generator apparatus by using the discrimination result of the frequency characteristic of a vibration according to an embodiment 8-5, and Figure 64 is a block diagram showing a detailed configuration of the vibration monitoring system 4500 of Figure 63.

The vibration monitoring system 4500 shown in Figure 63 is configured to include a vibration generator apparatus 4501, a vibration signal input apparatus 4502 configured to include a microphone 4911 and a microphone amplifier 4912, a vibration discriminating apparatus 4503, a discrimination result based control signal generator apparatus 4504, an alarm generator 4506, a vibration complementing apparatus 4507, and a discrimination result monitor apparatus 4505. Referring to Figure 63, the actual vibration generated from the vibration generator apparatus 4501 is converted into an electrical signal by the vibration signal input apparatus 4502 and thereafter inputted to the vibration discriminating apparatus 4503. As disclosed in the Patent Document 9, the vibration discriminating apparatus 4503 discriminates whether or not the inputted vibration signal has the conditions of the vibration that can induce the fundamental brain activation effect, and outputs the discrimination result to the discrimination result based control signal generator apparatus 4504 and the discrimination result monitor apparatus 4505. When it is discriminated that "the inputted vibration signal does not have the conditions of the vibration that can induce the fundamental brain activation effect, i.e., the inputted vibration signal cannot induce the fundamental brain activation effect", the discrimination result based control signal generator apparatus 4504 outputs a control signal to the alarm generator 4506 to issue an alarm, and/or outputs a control signal to the vibration complementing apparatus 4507 to generate a vibration signal including the third band, and adds the generated signal to the signal of the vibration generator apparatus 4501 to generate an addition signal. The discrimination result monitor apparatus 4505 displays the discrimination result.

With such a vibration monitoring system 4500, the listener becomes able to confirm whether the currently listening vibration has the conditions of the vibration that can induce the fundamental brain activation effect or becomes able to receive the vibration that can induce the fundamental brain activation effect even if the conditions are not provided, so that safety can be secured by preventing the hypersonic negative effect due to a decrease in the fundamental brain activation and the hypersonic positive effect that is the positive effect of improving the psychophysical conditions through the activation of the fundamental brain network system can be obtained.

Next, the operational effects and applications of the apparatus, method and space of the present embodiment are described below.

In the embodiments 1 to 7, the level of each band components of the applied vibration is decided in accordance with a predetermined protocol, and therefore, it cannot be optimized depending on a personal difference in the brain activity and the situation when the vibration is applied. In contrast to this, the level of each band of the vibration can be adjusted and optimized in the present embodiment by monitoring the brain activity of the human being to whom the vibration is applied or by monitoring the physiological index of the autonomic nerve system that receives influence from the brain activity. Moreover, various applications by applying this are considered. For example, medical applications of, for example, custom-made therapy and a vibration treatment matched to each individual patient are considered. An arousal level control apparatus to control the arousal level by monitoring the brain activity of the driver of a vehicle is also useful.

Next, (B) a feedback system based on a quantitative measurement index is described below.

The reward system neural circuit, which controls actions on the basis of pleasant and unpleasant emotional reactions, is concentrated in the fundamental brain. That is, when the activity of the fundamental brain is raised by some stimulation (e.g., an aerial vibration in this case), the reward system neural circuit is activated, and a pleasant sensation is generated. Animals receive such stimulations a lot, and therefore, they take actions approaching such stimulations. Conversely, when the activity of the fundamental brain is lowered by some stimulation including the aerial vibration, the activity of the reward system neural circuit is lowered, and the pleasant sensation is impaired. Animals take actions to run away from the stimulation and escape from it so as not to receive such stimulation as far as possible.

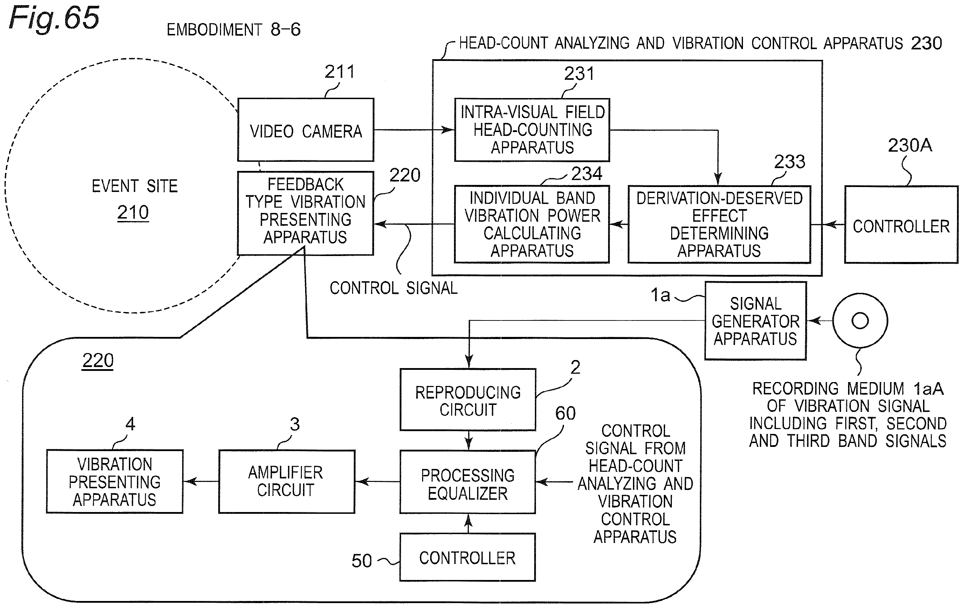

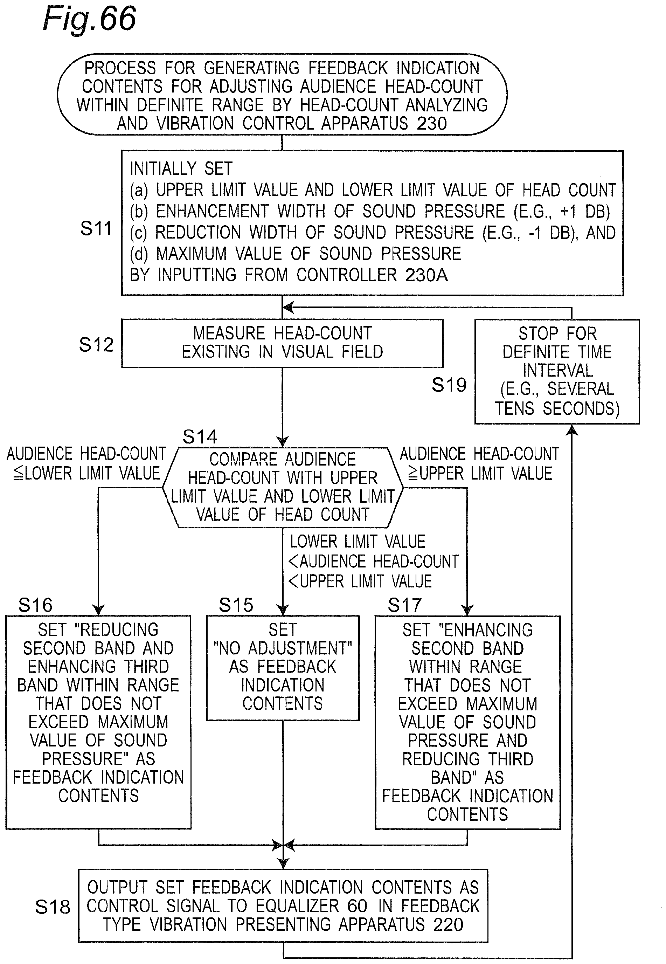

In the present embodiment, Figure 65 shows a feedback system that measures the head-count of audience or customers in a definite region of an event site or a commercial facility and guides the audience or customers using it as an index as an application utilizing the behavioral principle of animals as described above. Figure 65 is a block diagram showing a configuration of a vibration presenting apparatus according to an embodiment 8-6, and Figure 66 is a flow chart showing processes to generate feedback indication contents executed by the head-count analyzing and vibration control apparatus 230 of Figure 65 for gathering audience in a definite region and adjusting the audience head-count within a definite range.

Referring to Figure 65, the actions of audience are monitored by a video camera 211 installed in an event site 210, and the audience head-count in the region in charge in the event site 210 is analyzed by the head-count analyzing and vibration control apparatus 230. In the head-count analyzing and vibration control apparatus 230, the audience head-count existing in the visual field of the video camera 211 is counted by the Intra-visual field head-counting apparatus 231, the count value is inputted to a derivation-deserved effect determining apparatus 233, and the derivation-deserved effect determining apparatus 233 determines the effect to be derived on the basis of the count number. The determination result is inputted to an individual band vibration power calculating apparatus 234, the individual band vibration power is calculated on the basis of the determination result, and a control signal representing the calculation result is inputted to an equalizer in the feedback type vibration presenting apparatus 220, so that the frequency characteristic of the vibration to be presented to the event site 210 is controlled. In this case, the determination condition and the like of the head-count analyzing and vibration control apparatus 230 is inputted by an input part of the controller 230A, and the feedback contents can also be controlled directly from the controller 230A. Moreover, the feedback type vibration presenting apparatus 220 is configured to include a signal generator apparatus 1a including the drive of the recording medium 1aA of the vibration signal, a reproducer circuit 2, a processing equalizer 60, a controller 50, an amplifier circuit 3, and a vibration presenter 4 in a manner similar to that of the embodiment of Figure 31. The controller 50 is used when adjusting the feedback contents and directly controlling the processing equalizer 60.

In the processes of Figure 66, first of all, in step S11, initial setting is performed by inputting (a) the upper limit value and the lower limit value of the head-count, (b) an enhancement width of sound pressure of, for example, +1 dB, (v) a sound pressure reduction width of, for example, -1 dB, and (d) a sound pressure maximum value from the controller 230A that has, for example, a keyboard. Next, the head-count of audience existing in the visual field is measured in step S12. Then, in step S14, the audience head-count is compared with the head-count upper limit value and the head-count lower limit value. The program flow proceeds to step S15 when the lower limit value < audience head-count < upper limit value, proceeds to step S16 when the audience head-count ≤ lower limit value or proceeds to step S17 when the audience head-count ≥ upper limit. In step S15, "no adjustment" is set as feedback process contents, and the program flow proceeds to step S18. In step S16, "reducing the second band by the aforementioned set reduction width, and enhancing the third band by the aforementioned set enhancement width within a range that does not exceed the sound pressure maximum value" is set as feedback indication contents, and the program flow proceeds to step S18. Further, in step S17, "enhancing the second band within a range that does not exceed the sound pressure maximum value, and reducing the third band" is set as feedback indication contents, and the program flow proceeds to step S18. In step S18, the set feedback indication contents are outputted as a control signal to the processing equalizer 60 in the feedback type vibration presenting apparatus 220, it stops for a definite time interval of, for example, several tens of seconds in step S19, and the program flow returns to step S12.

In the feedback system configured as above, when, for example, the audience head-count in a definite region falls below a predetermined lower limit value (flow shift to step S16 from step S14 of Figure 66), the adjustment contents of the vibration power of the individual band are set so that the audience does not go out of the region and the audience is guided from the peripheral regions, i.e., the third band having the positive effect becomes relatively strong. By this operation, the activity of the fundamental brain is exalted, the pleasant sensation is raised, and the approach action to get closer to the stimulation is induced, therefore making it possible to guide the audience so as to voluntarily gather into the region. Conversely, when the audience head-count exceeds a predetermined upper limit value and it is judged that the audience density is increased to a hazardous level (flow shift to step S17 in step S14 of Figure 66), the adjustment contents of the vibration power of the individual band are set so that the audience is guided out of the region, i.e., the second band having the negative effect becomes relatively strong. By this operation, the activity of the fundamental brain is lowered, the pleasant sensation is reduced, and an evasive action to get apart from the stimulation is induced, making it possible to guide the audience so as to voluntarily go out of the region.

In response to this, the processing equalizer 60 built in the feedback type vibration presenting apparatus 220 of Figure 65 installed in each region performs the equalizing process, and the obtained vibration signal is converted into a vibration by the vibration presenter 4 to present it to the event site. In order that the intensity of vibration presentation by feedback is neither delicately changed nor oscillated, the next analysis and feedback indication contents generation are performed after an interval of, for example, several tens of seconds. This makes it possible to adjust a bias in the head-count distribution in an event site within a definite range by the feedback type vibration presenting apparatus 220.

Although the human head-count is used as the subject of analysis in the aforementioned embodiment, the present invention is not limited to this and allowed to be configured to analyze the head-count of living bodies including other animals.

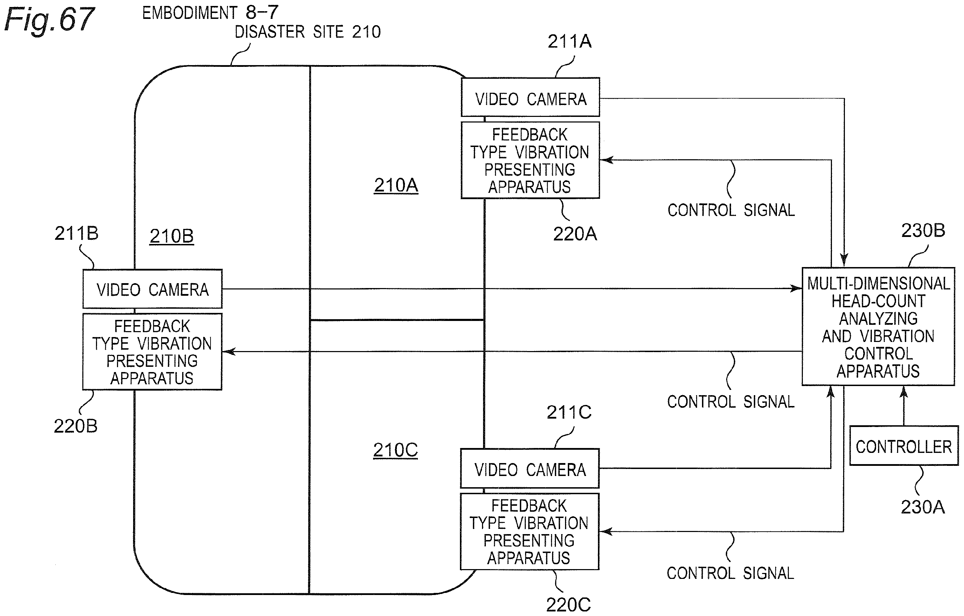

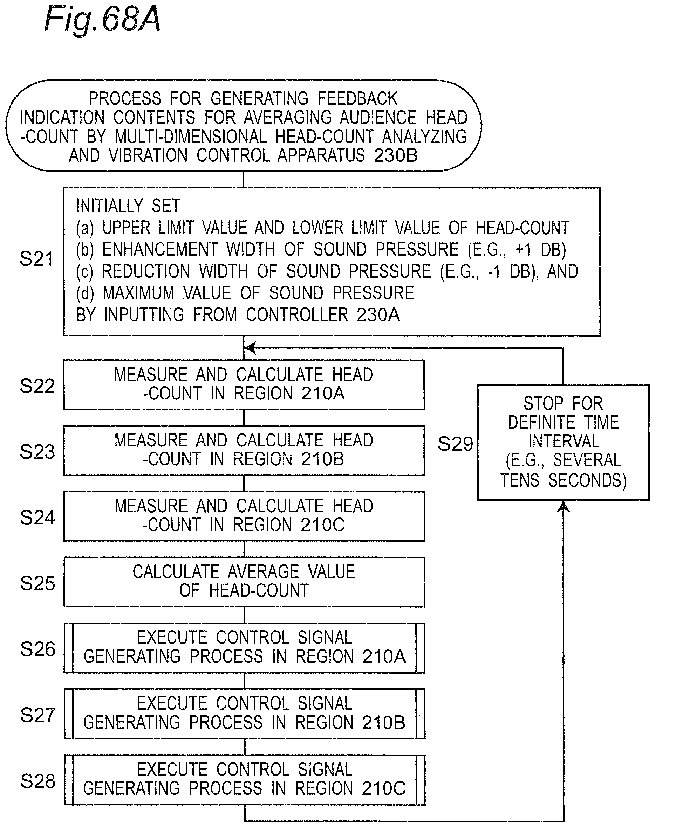

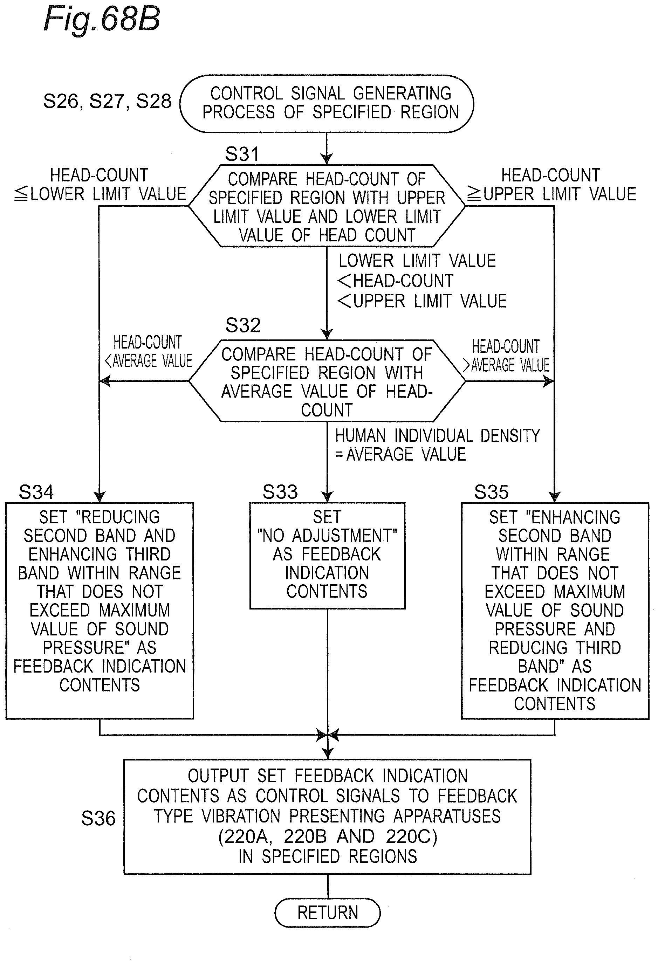

Next, in an embodiment 8-7, a vibration presenting apparatus, method and space to control the audience head-count in a plurality of regions by linking a plurality of vibration presenting apparatuses according to the embodiment 8-6 are described. Figure 67 is a block diagram showing a configuration of a vibration presenting apparatus according to the embodiment 8-7. The vibration presenting apparatus of the embodiment 8-7 is characterized by having a feedback system that measures the audience head-count in each of regions 210A, 210B and 210C by dividing the event site 210 into the plurality of regions 210A, 210B and 210C, and guides the audience using it as an index. That is, although the apparatus of Figure 65 is an apparatus example in which human head-count analysis and vibration control are performed independently in one region of the event site 210, the apparatus of Figure 67 is an example in which control is performed multidimensionally over the plurality of regions 210A, 210B and 210C. Moreover, Figure 68A is a flow chart showing processes to generate feedback indication contents to be executed by the multidimensional head-count analyzing and vibration control apparatus 230B of Figure 67 when the audience head-count distribution is averaged, and Figure 68B is a flow chart showing a control information generating processes (S26, S27, S28) of Figure 68A.

Referring to Figure 67, a video camera 211A and a feedback type vibration presenting apparatus 220A are provided in the region 210A, and they are connected to the multidimensional head-count analyzing and vibration control apparatus 230B that controls the whole. Moreover, a video camera 211B and a feedback type vibration presenting apparatus 220B are provided in the region 210B, and they are connected to the multidimensional head-count analyzing and vibration control apparatus 230B that controls the whole. Further, a video camera 211C and a feedback type vibration presenting apparatus 220C are provided in the region 210C, and they are connected to the multidimensional head-count analyzing and vibration control apparatus 230B that controls the whole. It is noted that the video cameras 211A, 211B and 211C, and the feedback type vibration presenting apparatuses 220A, 220B and 220C are each configured in a manner similar to that of the embodiment 8-6. Moreover, the multidimensional head-count analyzing and vibration control apparatus 230B executes the processes of Figures 68A and 68B.

In the processes of Figure 68A, first of all, in step S21, initial setting is performed by inputting (a) the upper limit value and the lower limit value of the head-count, (b) an enhancement width of sound pressure of, for example, +1 dB, (v) a sound pressure reduction width of, for example, -1 dB, and (d) a sound pressure maximum value. Next, the head-count of audience existing in the region 210A is measured in step S22, and the head-count of audience existing in the region 210B is measured in step S23. The head-count of audience existing in the region 210C is measured in step S24, and an average value of the audience head-count is calculated in step S25. Further, a control signal generating process (Figure 68B) regarding the region 210A is executed in step S26, a control signal generating process (Figure 68B) regarding the region 210B is executed in step S27, and a control signal generating process (Figure 68B) regarding the region 210C is executed in step S28. Then, it stops for a definite time of, for example, several tens of seconds in step S29, and the program flow returns to step S22.

In the subroutine process of Figure 68B, first of all, the audience head-count in a designated region is compared with the upper limit value and the lower limit value of the head-count in step S31, and the program flow proceeds to step S32 when the lower limit value < audience head-count < upper limit value to compare the audience head-count in the designated region with the average head-count. The program flow proceeds to step S34 when the audience head-count ≤ lower limit value in step S31 or proceeds to step S35 when the audience head-count ≥ upper limit value. Moreover, the program flow proceeds to step S33 when the audience head-count = average head-count in step S32, proceeds to step S34 when the audience head-count < average head-count or proceeds to step S35 when the audience head-count > average head-count. In step S33, "no adjustment" is set as feedback indication contents, and the program flow proceeds to step S36. Moreover, in step S34, "reducing the second band by the aforementioned set reduction width, and enhancing the third band by the aforementioned set enhancement width within a range that does not exceed the sound pressure maximum value" is set as feedback indication contents, and the program flow proceeds to step S36. Further, in step S35, "enhancing the second band within a range that does not exceed the sound pressure maximum value, and reducing the third band" is set as feedback indication contents, and the program flow proceeds to step S36. In step S36, the set feedback indication contents are outputted as a control signal to the processing equalizer 60 in the feedback type vibration presenting apparatus (one of 220A, 220B and 220C) of the designated region, and the program flow returns to the original main routine.

In the present embodiment configured as above, the actions of the audience are monitored by the video cameras 211A, 211B and 211C installed in the respective regions 210A, 210B and 210C of the event site 210, and the multidimensional head-count analyzing and vibration control apparatus 230B counts the head-count of the audience in the visual fields of the video cameras 211A, 211B and 211C to calculate the average head-count (steps S22 to S25 of Figure 68A) and thereafter performs discrimination by comparing the audience head-count of each of the regions 210A, 210B and 210C with the predetermined upper limit value and lower limit value (step S31 of Figure 68B). When the audience head-count is lower than the lower limit value (flow shift from step S31 to step S34), the adjustment contents of the vibration power of the individual band are set so that the audience does not go out of the region and the audience is guided from the peripheral regions, i.e., the third band having the positive effect to enhance the fundamental brain activity becomes relatively strong (step S34). Moreover, when the audience head-count is higher than the upper limit value (flow shift from step S31 to step S35), the adjustment contents of the vibration power of the individual band are set so that the audience is guided out of the region, i.e., the second band having the negative effect to lower the fundamental brain activity becomes relatively strong (step S35). When the audience head-count is intermediate between the lower limit value and the upper limit value (flow shift from step S32 to step S33), it is compared with the inter-regional average value of the audience head-count. When it is lower than the value (flow shift from step S32 to step S34), the adjustment contents of the vibration power of the individual band are set so that the audience does not go out of the region and the audience is guided from the peripheral regions, i.e., the third band having the positive effect becomes relatively strong (step S34). When it is higher than the inter-regional average value of the audience head-count (flow shift from step S32 to step S35), the adjustment contents of the vibration power of the individual band are set so that the audience is guided out of the region, i.e., the second band having the negative effect becomes relatively strong (step S35). When the audience head-count in the region is equal to the inter-regional average value of the audience head-count (flow shift from step S32 to step S33), no adjustment of the vibration power of individual band is performed (step S33).

In response to this, the built-in processing equalizers 60 built in the feedback type vibration presenting apparatuses 220A, 220B and 220C installed in the respective regions 210A, 210B and 210C perform the equalizing process, and the vibration presenter 4 converts the obtained vibration signal into a vibration and presents it to the event site 210. By this operation, it becomes possible to adjust the audience distribution in the event site 210 in the averaging direction by the feedback type vibration presenting apparatuses 220A, 220B and 220C.

Although the method for controlling the audience head-count in the averaging direction between regions of the event site has been described here, it is also possible to control the audience to be concentrated on a certain region. Further, it is also possible to control the audience to successively go over and move between regions by successively switching the subject regions of concentration to the adjacent regions.

Although the head-count of human beings is analyzed in the above embodiments, the present invention is not limited to this and allowed to analyze the head-count of living bodies including animals other than human beings.

Next, the operational effects and applications of the apparatus, method and space of the present embodiment are described below.

In a broad space like an event site, each person of the audience cannot look down on the whole space, and there occurs crowding and concentration of audience in a certain region or a small number of audience in a certain region on the other hand. Moreover, it might be a case where the organizer hopes to intensively gather the audience in a certain region. However, it is difficult for a guidance system using a person, broadcasting, notice or the like to look down on the whole broad space and control and guide a great number of audience in an orderly manner. Moreover, there are not a few cases where the audience is concentrating on his or her own interest and not conscious about the announcement contents. Accordingly, the present embodiment, which performs remote feedback taking advantage of the effects that the vibration of the positive band invites the people while looking down on the whole space and the vibration of the negative band drives the people away, makes it possible to smoothly guide the actions of many people. The subjects to which the present embodiment is applied can be variously supposed besides the event site. For example, it can be applied to alleviating congestion and making the people safely move in an orderly manner in the spaces of commercial facilities, station yards, shrines and temples at the time of festivals and so on.

Applications utilizing this can be variously considered otherwise. For example, a system that guides an action by strongly presenting the vibration of the band having the negative effect when a person enters a hazardous territory in order to ensure safety in a configuration site or the like is considered. Moreover, a system that guides actions of customers by strongly presenting the vibration of the band having the negative effect to a customer who is staying for a time longer than a definite time using the stay time of each customer as an index in order to guide the customer who stays long in a convenience store, a coffee shop or the like so as to leave the place is considered. On the other hand, in a pachinko parlor, a game center or the like, a feedback apparatus that makes a customer excited by increasing the brain activity of the customer or conversely cooled down while monitoring the state of ball feed is considered.

Moreover, it can be applied to behavior control of not only human beings but also various animals other than human beings. That is, when an animal approaches an undesirable place or the like, the activity of the reward system neural circuit is lowered by applying the components of the second band having the negative effect with the sound of the first band, guiding the animal so as to escape from the place. Conversely, it becomes possible to make an animal approach the target place by increasing the activity of the reward system neural circuit by applying the components of the third band having the positive effect with the components of the first band in order to guide the animal to the desirable place.

For example, a system such that, when an animal (bear, wild boar, wild monkey or the like) that might give harm to human beings steps into a house or a farm, an escape action of the animal is induced by strongly presenting the vibration of the band having the negative effect is considered. With this arrangement, it is expected to expel the animal without paying much labor of gunfire, capture, or the like. Moreover, it can be applied also to guiding and capturing a stray dog and a homeless cat.

Further, it can be utilized also for behavior control of pastured animals (cow, horse, sheep, duck and the like). Pasturing the animals in a natural environment while limiting the behaviors of these animals within a definite range is effective in raising animals that have temperately solid meat. Therefore, a feedback system that controls animals while making them behave freely within a definite range by inducing an approaching action with the vibration of the band having the positive effect given within the definite range and inducing an escape action with the vibration of the band having the negative effect given when the animal tries to go out of the definite range is considered. With this arrangement, it can be expected to efficiently pasture a number of animals even if there are few watchers. Moreover, it can be applied also to action inducement for swift movement from a definite region to another region such as the inside of a stable block in an orderly manner. This example is not limited to the pastured animals but generally applicable to animals of a livestock, a zoo or the like.

Since it has been known that the audible range upper limit of animals other than human beings differ depending on the species as described above, it is considered that the lower limit and upper limit frequencies of the first band including the audible range, the lower limit frequency and the upper limit frequency of the second band having the negative effect, and the lower limit frequency and the upper limit frequency of the third band having the positive effect possibly assume values different from the frequencies identified by the listening conditions of the present experiment intended for human beings.

Next, application examples of concrete apparatus, method, space and the like by applying the above embodiments and implemental examples are described.

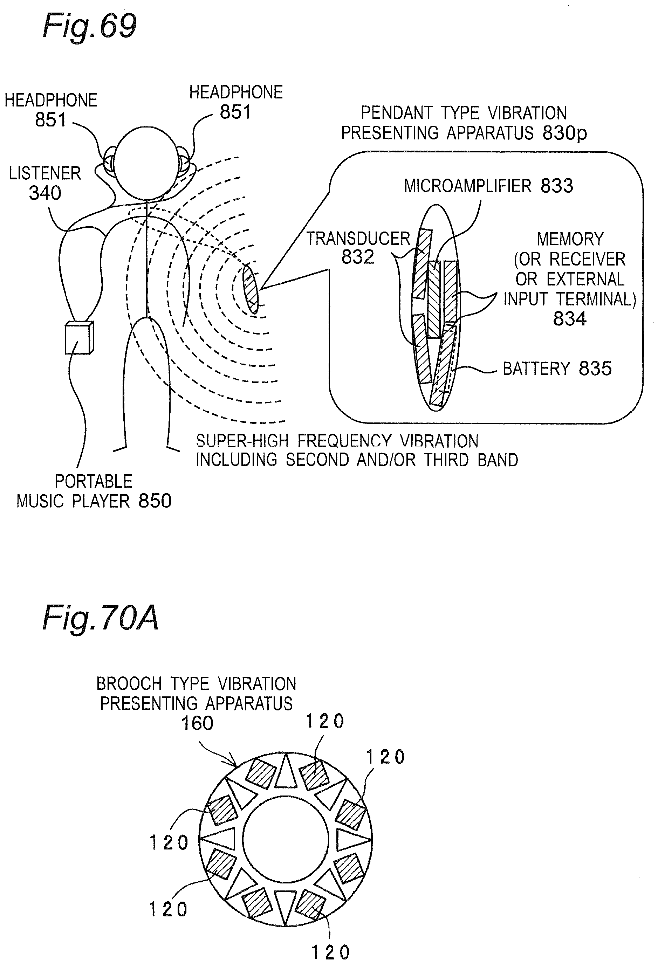

First of all, an application example of a vibration presenting apparatus that effectively applies a super-high frequency vibration including the second and/or the third band to a body surface by utilizing an accessory such as a pendant and a brooch worn extremely adjacent to the body is described. Figure 69 is a perspective view and a sectional view of a pendant type vibration presenting apparatus 830p (the vibration presenting apparatus may be provided for a portable terminal apparatus) according to an application example 1. Figure 69 shows a use example of the pendant type vibration presenting apparatus 830p that utilizes an accessory such as a pendant. Super-high frequency components capable of decreasing or increasing the brain activity because of the containment of the second and/or the third band and are inputted from a memory (or a receiver or an external input terminal) 834 in the vibration presenting apparatus 830p are presented by the vibration presenting apparatus 830p through a microamplifier 833 and a transducer 832, making it possible to apply the super-high frequency components of the vibration capable of decreasing or increasing the brain activity to the body surface of a listener 340 who wears the accessory. On the other hand, the audible range components including the first band are applied from a portable music player 850 to the auditory system of the listener 340 via a headphone 851. By thus applying the audible range components including the first band and the super-high frequency vibration including the second and/or the third band, the brain activity of the listener can be decreased or increased.

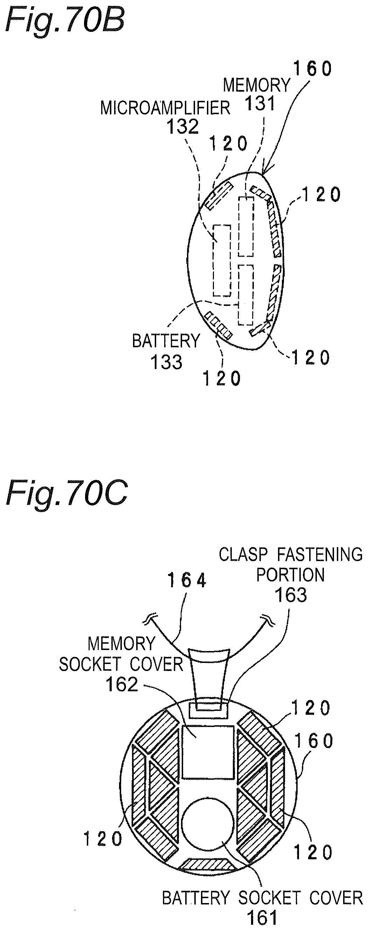

Next, an application example of a vibration generating mechanism that effectively applies a super-high frequency vibration including the second and/or the third band to the body surface by utilizing an accessory such as a brooch is described. Figure 70A is a front view of a brooch (accessory) type vibration presenting apparatus 160 according to an application example 2, Figure 70B is the right side view thereof, and Figure 70C is the backside view thereof. Referring to Figures 70A to 70C, a plurality of super-high frequency vibration generating devices 120 for generating the super-high frequency components of the second and/or the third band among the vibration signals capable of decreasing or increasing the brain activity are provided embedded in the front surface and the back surface of the brooch type vibration presenting apparatus 160. Moreover, a signal reproducing apparatus is provided embedded in the brooch type vibration presenting apparatus 160. It is noted that a battery socket cover 161 into which a battery 133 is to be inserted and a memory socket cover 162 are provided on the back side of the brooch type vibration presenting apparatus 160, and a clasp 164 to hang the brooch is connected to a clasp fastening portion 163 in the upper part of the brooch type vibration presenting apparatus 160. A super-high frequency vibration generating device may be mounted on this clasp. In the brooch type vibration presenting apparatus 160, signal data of the vibration capable of decreasing or increasing the brain activity is preparatorily stored in a nonvolatile fixed memory 131 of, for example, a flash memory. The signal data of the vibration capable of decreasing or increasing the brain activity read from the solid memory 131 is subjected to DA conversion and power amplification in the microamplifier 132 at the time of reproduction, and thereafter outputted to the super-high frequency vibration generating devices 120 to generate and emit a super-high frequency vibration.

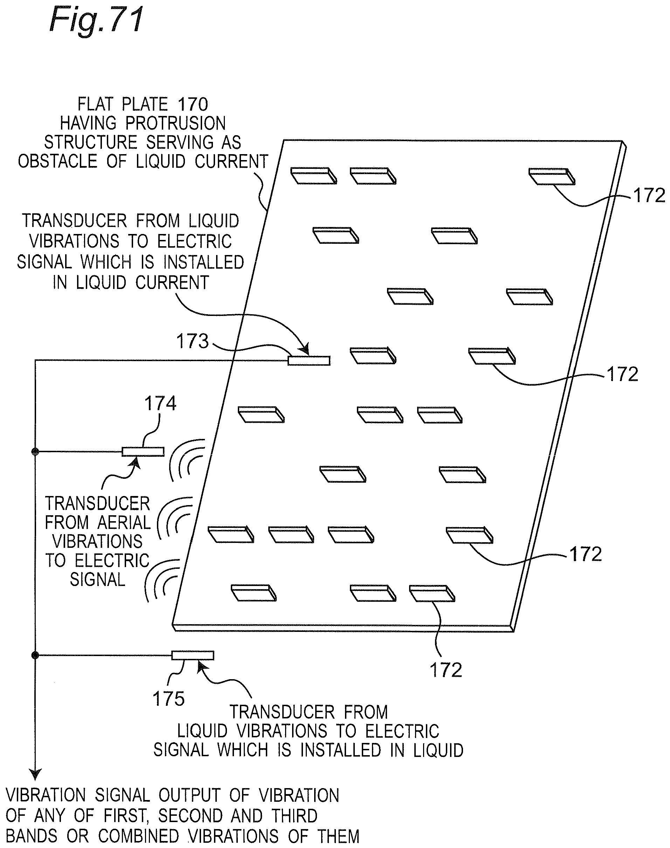

Figure 71 is a structural example of a vibration generator apparatus according to an application example 3, and a perspective view showing an example in which a vibration including any of the first, second and third bands or a vibration of a complex of them is generated by flowing a liquid by making it collide with obstacles. Referring to Figure 71, the vibration generator apparatus is constituted of a structure of a flat plate 170 that has one or more obstacle structures such as projections 172 of which the positions and projection dimensions are variable, and the obstacles can be set in positions at an angle that exceeds zero degrees and is not larger than 90 degrees with respect to the horizontal plane, a liquid stream generator apparatus that flows downstream liquid such as water from an upper portion to the surface of the flat plate 170, and transducers 173, 174 and 175 that convert a vibration generated when the liquid is flowed downstream to this system into an electrical signal. For example, when the projections 172 are regularly placed in an orderly manner, the flow passage of the liquid stream becomes orderly, and the flow rate of liquid flowing through each flow passage are equalized in correspondence, and the vibration is generated comparatively homogeneously from the entire plate surface. On the other hand, referring to Figure 71, by irregularly arranging the projections 172, the flow passage comes to be distributed in heterogeneity, and a variation occurs in the flow rate of each flow passage. As a result, deviation occurs in the space distribution and intra-liquid distribution of the vibrations, and this therefore makes it possible to perform effective multichannel collecting in converting the vibration signal into the electrical signal taking advantage of this. Moreover, by moving the transducer to the electrical signal from immediately near a certain flow passage to immediately near another flow passage at this time, a dynamic variation of a kind that cannot be generated in the flow rate change of the identical flow passages can be added to the variation signal during the moving process. Although the liquid flow is taken as an example here, a vibration generated when a liquid droplet, a particle or the like is dropped and made to collide with the liquid surface may be used. Moreover, it is acceptable to generate airflow in a structure by using a gas instead of a liquid and generate a vibration that includes any of the first, second and third bands or a vibration of a complex of them by an airflow that passes through a gap of an internal structure. Moreover, it is acceptable to generate a vibration that includes any of the first, second and third bands or a vibration of a complex of them by using a solid and playing, rubbing, beating or performing another action of a solid piece of metal or the like or a string. The material of the solid is not limited to metal but allowed to be stone, ceramic, plastic or the like or a material originating from living things such as tree, skin or bone. Further, there may be provided an apparatus such as a resonance box or a resonance tube, which has a function to generate the aerial vibration by amplification by resonation with an aerial vibration generated from these vibration generator apparatuses.

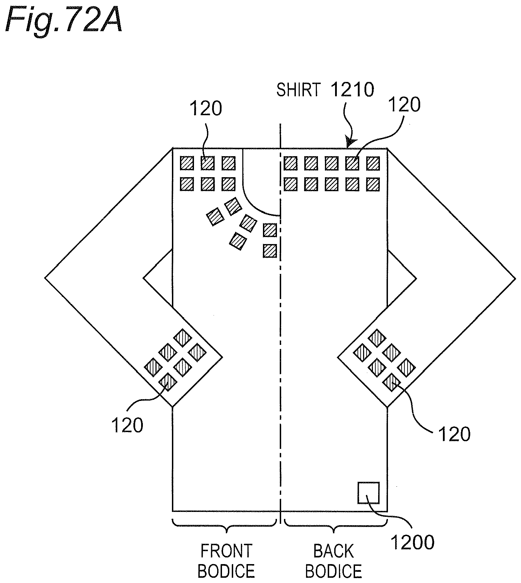

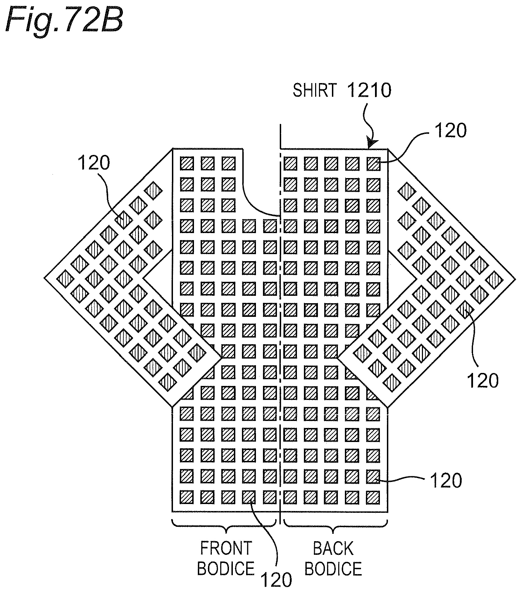

Next, an application example of a vibration generating mechanism that effectively applies the super-high frequency vibration including the second and/or the third band to a body surface by utilizing clothes or the like that covers the body is described. Figure 72A is an external view of a clothes embedded type vibration presenting apparatus according to an application example 4, and Figure 72B is an internal view of Figure 72A. Referring to Figures 72A and 72B, a number of super-high frequency vibration generating devices 120 for generating the super-high frequency components of the vibration that includes the second and/or the third band and is able to decrease or increase the brain activity are provided substantially on the entire surface inside a shirt 1210 and in sleeve portions, collar portions, and the like on the outside. Moreover, a signal reproducing apparatus 1200 is provided in the vicinity of a hem portion of the shirt 1210. In the shirt 1210, a conductive plastic fiber coated with a non-conductive plastic is woven into the cloth in concrete, and part of the conductive plastic fiber is used as wiring between the signal reproducing apparatus 1200 and each of the super-high frequency vibration generating devices 120. Moreover, a piezo fiber is woven, and this may be used as a super-high frequency vibration generating device. According to the shirt 1210 configured as above, a number of super-high frequency vibration generating devices 120 are embedded in the shirt 1210, and the super-high frequency vibration is generated in the entire body, so that the super-high frequency vibration can be applied to the listener conveniently and effectively without using a loudspeaker system. In this case, the audible range components are applied to the listener by a loudspeaker, a headphone or the like.

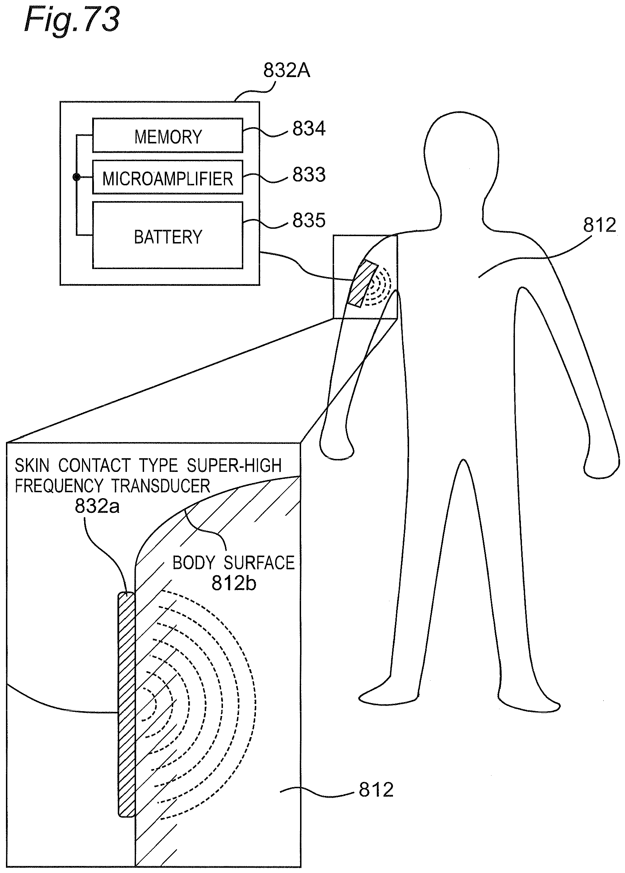

Next, an application example of a vibration presenting apparatus that effectively applies the super-high frequency vibration including the second and/or the third band to the body surface by putting it close to the skin is described. Figure 73 is a sectional view and a block diagram of a body surface attachment type vibration presenting apparatus according to an application example 5. In other words, it is the structure of a vibration presenting apparatus 832A using a skin contact type super-high frequency transducer 832a, showing an apparatus for transmitting to the skin the super-high frequency vibration that includes the second and/or the third band and is able to decrease or increase the brain activity not via air by attaching the vibration presenting apparatus 832A close to the skin of a listener 812. In the vibration presenting apparatus 832A, the apparatus is implemented by wiredly or wirelessly transmitting through a microamplifier 833 that amplifies and transmits the super-high frequency components of the vibration signal capable of decreasing or increasing the brain activity the super-high frequency components of the vibration signal capable of decreasing or increasing the brain activity, which are stored in a memory 834, wiredly or wirelessly received or externally inputted, and directly fixing the skin contact type super-high frequency transducer 832a that is a film-shaped vibration presenting apparatus such as a compact actuator or a piezoelectric device to the body surface 812b of the skin by a plaster, a supporter or the like, so that the super-high frequency components of the vibration capable of decreasing or increasing the brain activity are directly transmitted to the skin. At this time, the audible range components are applied to the listener by a loudspeaker, a headphone or the like.

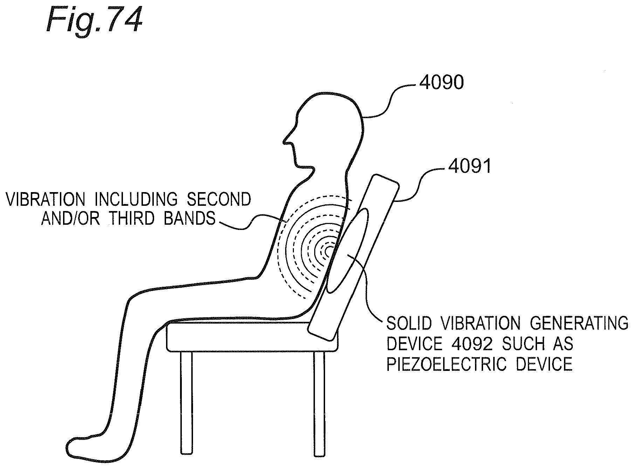

Figure 74 is a side view showing an example of an apparatus that applies the vibration including the second and/or the third band via the body surface and the auditory system by using a solid vibration generating mechanism according to an application example 6. In the application example 6 of Figure 74, an example in which the vibration including the second and/or the third band is generated by transmitting a vibration signal via a solid vibration generating device 4092 such as a piezoelectric device embedded in a chair 4091 or the like on which a human being 4090 to whom it is applied sits is described. Referring to Figure 74, the vibration including the second and/or the third band among the vibrations is received from the body surface, and the vibration of the first band is received from the auditory system to induce an increase or a decrease in the brain activity.

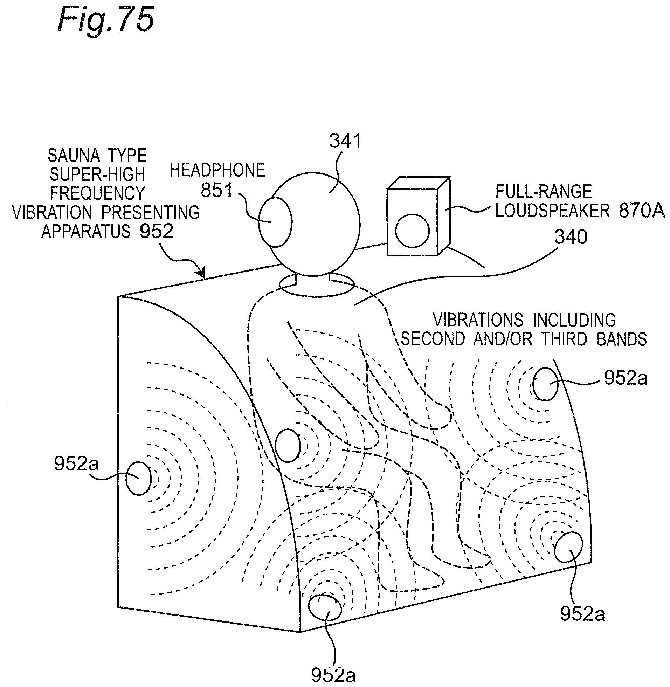

Figure 75 is a perspective view showing an example of a sauna type vibration presenting apparatus according to an application example 7. Referring to Figure 75, it is an example of a space that presents a vibration capable of inducing an increase or a decrease in the brain activity in a sauna type space for personal use by applying the first band as an aerial vibration by a headphone or from a loudspeaker in the space where the head portion is projected, applying the vibration of the second and/or the third band from a loudspeaker installed in the space where the body except for the head portion exists, and adding together the operational effects of both of them. In this case, by entering the sauna type super-high frequency vibration presenting apparatus 952 in which a number of super-high frequency transducers 952a are internally placed, the super-high frequency vibration including the second and/or the third band can be extremely effectively applied to the body surface. The plurality of super-high frequency transducers 952a inside the sauna are similar to those of the aforementioned embodiment. In this case, a listener 340 who enters the sauna is listening to the sound of the first band of the audible range frequency by using a headphone 851 or the like or listening to the sound of the audible range by the airway auditory system including the head portion by using a full-range loudspeaker 870A or the like. At this time, by virtue of the simultaneously existing vibration including the second and/or the third band applied to the body surface, an increase or a decrease in the brain activity can be effectively achieved.

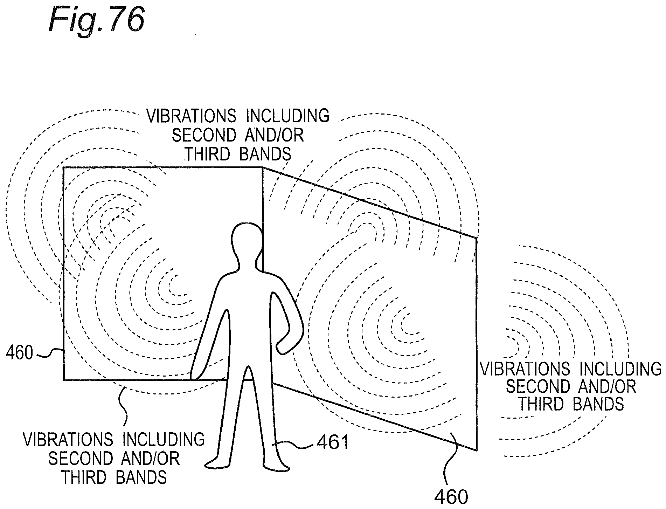

Figure 76 is a perspective view showing an example of a space in which the walls themselves constituting the space vibrate to generate a vibration including the second and/or the third band according to an application example 8. Referring to Figure 76, there is shown the example of the vibration generating space for generating vibrations including the first band of the audible range components and the second and/or the third band in the space by the vibration of the walls 460 that constitute the space and applying the variations to a listener 461. The walls 460 may vibrate by being driven by an electrical signal or may secondarily vibrate by propagating a vibration generated by a vibrating body of a solid, a liquid or a gas installed inside the space or outside the space. For example, in a concert hall or the like, a vibration generating space capable of inducing an increase or a decrease in the brain activity is provided by generating a vibration having the aforementioned specified conditions when instrument sounds, voices, singing voices performed inside the hall space or the sounds generated by a PA apparatus or the like propagate to the walls, and applying it to the audience.

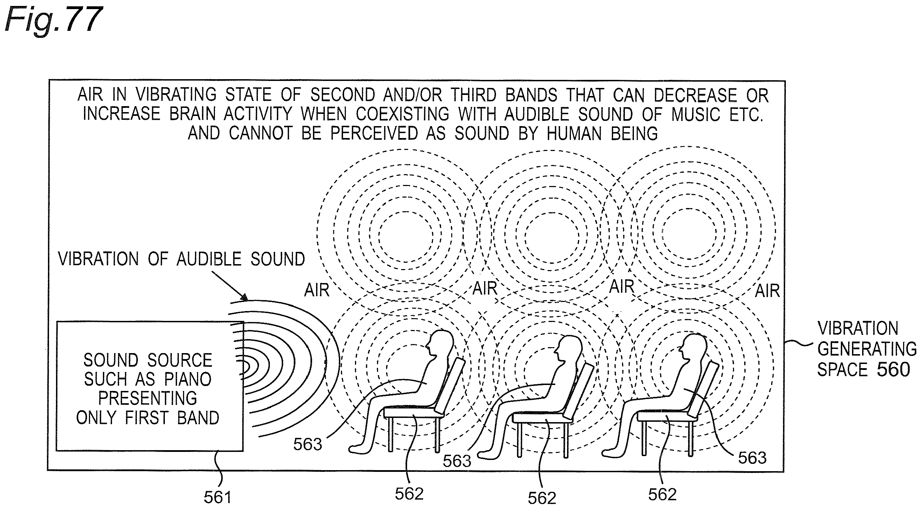

Next, an application example of a vibrating body characterized by having a vibration state that vibrates in the super-high frequency band including the second and/or the third band induced by vibrating a gas, a liquid, a solid or the like is described below. Figure 77 is a side view showing an example of a vibrating body characterized by having a vibration state including the second and/or the third band induced by vibrating air that is the object surrounding human beings in the super-high frequency band according to an application example 9. Referring to Figure 77, when a vibration that scarcely includes the super-high frequency components exceeding the audible range, such as the sound of a sound source 561 such as a piano or the like presenting, for example, only the first band is generated in a vibration generating space 560 formed of a vibration generating space apparatus that surrounds a listener 563 who sits on a chair 562, it is natural that a decrease or an increase in the brain activity is not induced. However, due to the existence of air in the state vibrating in the super-high frequency band (inaudible) serving as a decisive factor, a decrease or an increase in the brain activity can be induced. In this case, the vibrating body may be a gas, a liquid or a solid other than air.

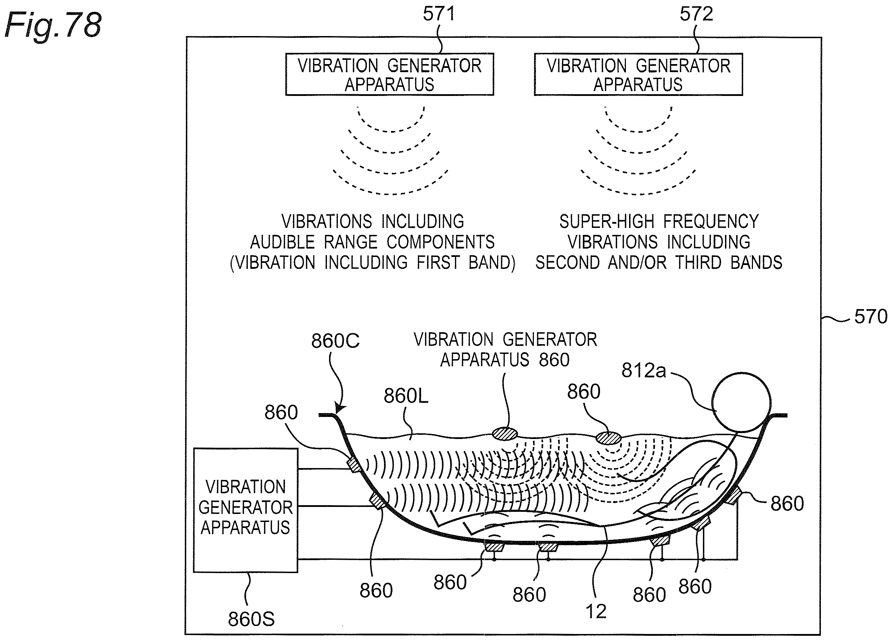

Figure 78 is a block diagram showing an example of a vibrating body in a bathtub according to an application example 10. Referring to Figure 78, an example induced to a vibrating body, which is characterized by having a vibration state in which the air surrounding the head portion 812a of a listener 812 and water or hot water surrounding the human trunk and limbs vibrate in the super-high frequency band including the second and/or the third band in addition to the first band, by applying vibrations from vibration generator apparatuses 571, 572 and 860 installed in a bathroom and the bathtub, is described. In this case, although the listener 812 exists simultaneously in two different vibrating bodies, the listener 812 may exist in a vibration state in which either a liquid or a gas has a specified feature.

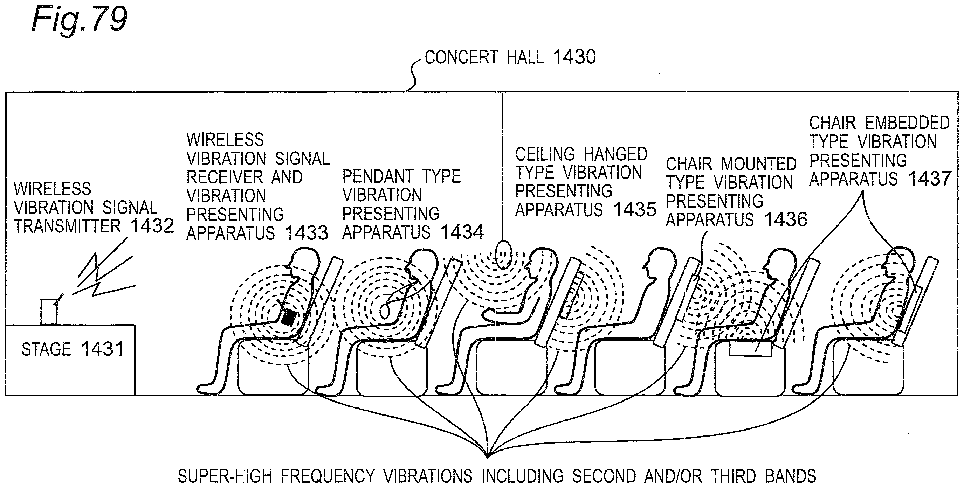

Next, an application example in which a vibration presentation space is achieved is described below. Figure 79 is a perspective view of a vibration presenting apparatus for generating a vibration capable of inducing the effects of increasing or decreasing the brain activity because of the inclusion of the second and/or the third band and the containment of the super-high frequency components at a distance close to the audience in a space of a theater, a concert hall 1430, a hall or the like according to an application example 11. Referring to Figure 79 are shown a stage 1431, a wireless vibration signal transmitter 1432, a wireless signal receiver and vibration presenting apparatus 1433, a pendant type vibration presenting apparatus 1434, a ceiling hanged type vibration presenting apparatus 1435, a chair mounted type vibration presenting apparatus 1436, and a chair embedded type vibration presenting apparatus 1437. That is, a super-high frequency vibration presenting apparatus according to the present application is embedded in the back surface of the seat in front of the audience or his or her own seat. Otherwise, it may be arranged hung from the ceiling or arranged extended from a wall surface or a pillar. Moreover, it may be arranged attached to an accessory and clothes such as a pendant that the audience wears, a portable telephone, a portable music player or the like. Otherwise, the audience may sit down while wearing a super-high frequency vibration presenting apparatus integrated with a wireless vibration signal receiver. The signal of the super-high frequency vibration including the second and/or the third band may be sent off wiredly or wirelessly (electromagnetic waves, infrared rays, LAN, Bluetooth (registered trademark) or the like) from on a stage 1431 or may be recorded in a memory or the like and built in each vibration presenting apparatus. By the methods as described above, all of the audience can receive the super-high frequency vibration including the second and/or the third band.

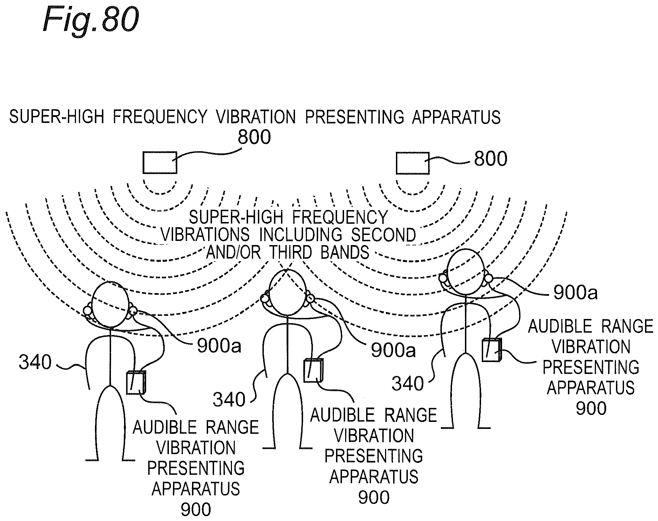

Figure 80 is a side view showing an application example 12 of a space obtained by combining a portable player that reproduces audible range vibration components with a vibration presenting apparatus to simultaneously apply the super-high frequency components that includes the second and/or the third band and are able to decrease or increase the brain activity to a plurality of human beings. The super-high frequency components of the vibration capable of decreasing or increasing the brain activity is reproduced from a super-high frequency vibration presenting apparatus 800 installed in a public space of, for example, a public road, a plaza, an office or a waiting area, and applied to the body surfaces of listeners 340. At this time, the reproduced super-high frequency vibration that includes the second and/or the third band and is inaudible as a sound is common to all the listeners 340. Under this condition, an audible range vibration is reproduced by an audible range vibration reproducing apparatus 900 such as a portable music player, and each listener listens to the sound by, for example, a headphone 900a. At this time, each listener 340 may listen to their mutually different favorite musics and the like.

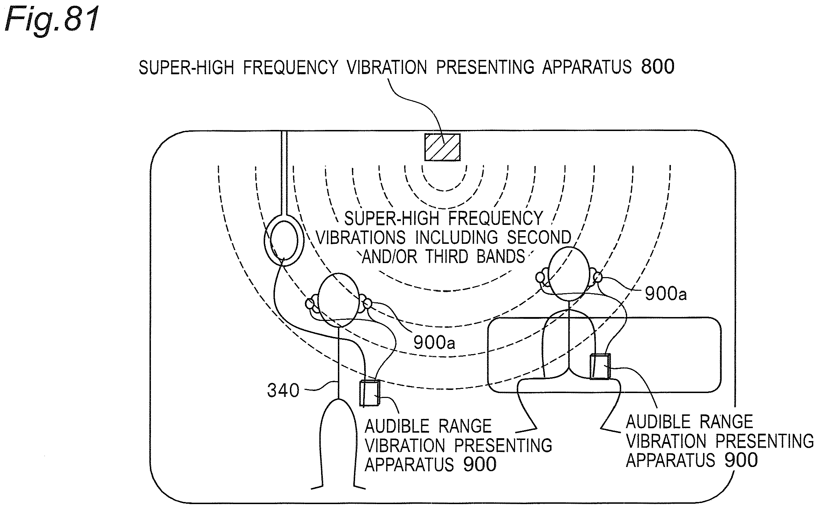

Figure 81 is a side view showing an application example 13 that shows a modified embodiment of the apparatus of Figure 80. Referring to Figure 81, there is shown a configuration in which a vibration capable of decreasing or increasing the brain activity is applied to a plurality of listeners 340 by using a vibration reproducing apparatus 800 in the guest room or the like of a train, a bus and a passenger plane. In this case, a super-high frequency vibration (including the second and/or the third band) is reproduced from the super-high frequency vibration generator apparatus 800 installed in the guest room or the like, and applied to the body surfaces of the plurality of listeners 340 in the train. At this time, the reproduced super-high frequency vibration is common to all the listeners 340. At this time, the plurality of listeners 340 in the train can enjoy the effects of increasing or decreasing the brain activity while listening to their mutually different favorite audible frequency vibrations by using audible range vibration reproducing apparatuses 900 such as portable music players and headphones for music service provided for this vehicle. Moreover, it is acceptable to listen to an environmental sound, a conversation sound, a performance sound or the like existing in the place without the portable player or the like.

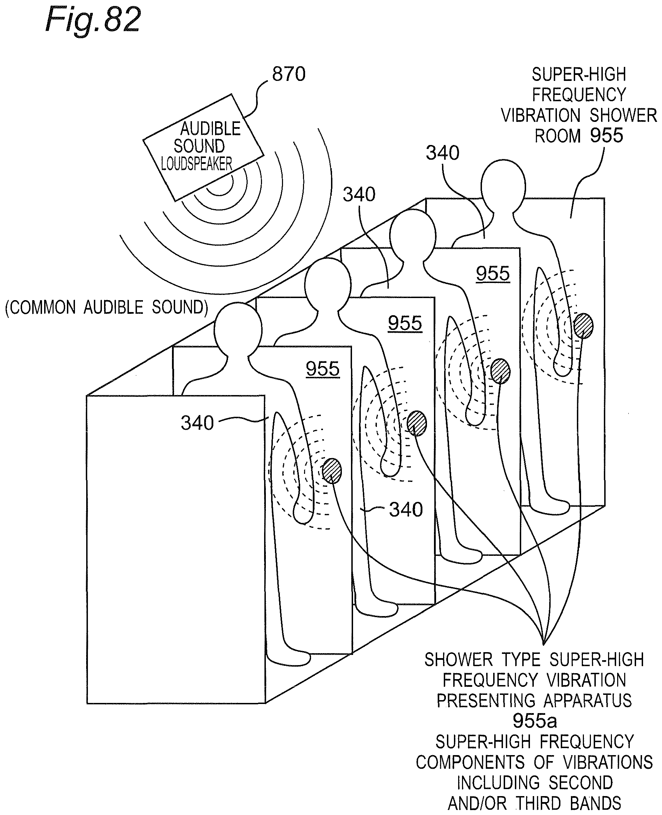

Next, an example of a vibration presentation space to apply vibrations including individual second and/or third band to a plurality of human beings who are listening to a common audible sound in a public space or the like is described. Figure 82 is a perspective view of a shower type vibration presenting apparatus that generates vibrations including the vibration of the first band (audible range components) and the second and/or the third band (super-high frequency components) according to an application example 14. Referring to Figure 82, there is shown a plurality of shower type vibration presenting apparatuses. In this case, a plurality of persons can bath their favorite super-high frequency vibrations in respective high-frequency vibration shower rooms 955 in a shower room type facility that the plurality of persons utilize. Referring to Figure 82, the vibration presenting apparatus 955a that is placed in each super-high frequency vibration shower room 955 and includes the second and/or the third band is able to select the super-high frequency vibration signal chosen by the user from among a number of kinds of super-high frequency vibration signals stored in a memory and to make the users effectively bath the same to the body surfaces. At this time, the users listen to the common audible range music, a broadcasting sound, a voice or the like from a general audible sound loudspeaker 870. They can effectively enjoy an increase or a decrease in the brain activity by virtue of the simultaneous existence of those audible sounds and the super-high frequency components. It is noted that the user may not listen to the common audible sound or may listen to individual favorite audible sounds by bringing in a portable player or the like.

In the application example 14 of Figure 82, an example of a vibration presentation space in which a vibration that increases or decreases the brain activity is generated from a vibration presenting apparatus installed in a public space or the like and applied to a plurality of human beings is shown. In this case, it is acceptable that only the components of equal to or higher than 20 kHz of the upper limit of the human audible range among the vibrations that have the aforementioned characteristic conditions are presented from the vibration presenting apparatus, and the plurality of human beings to whom the vibrations are applied may listen to mutually different their favorite musics or the like by using, for example, an audible range vibration generator apparatus such as portable players. In this case, a vibration that increases or decreases the brain activity is generated as a consequence of an addition of the super-high frequency components that include the second and/or the third band inaudible as a sound for human beings and are emitted in the air to the audible range components generated from the audible range vibration presenting apparatuses to which the individuals are listening. Such a vibration presentation space can be set in (1) intra-building spaces such as play facilities, stations and airport facilities of indoor rooms, gateways, lobbies, passages, stairs, escalator and elevator halls, halls, gyms, stadiums, warehouses, factories, stores, game centers, pachinko parlors, and the like, (2) vehicle spaces such as vehicles, trains, ocean vessels, submarines, aircrafts, rockets, and playing tools, (3) outdoor spaces such as gardens, schoolyards, plazas, parks, amusement parks, grounds, stadiums, building rooftops, roads, bridges, farms, forests, beaches, lakes, marshes and rivers, seas, deserts, and meadows, (4) underground spaces such as caves, tunnels, mineshafts, and underground shopping centers, and (5) semi-open spaces existing at indoor and outdoor boundaries such as shopping street arcades, station platforms, station concourses, stadiums, and seats of racetracks. With this vibration generating space, the plurality of human beings in the space becomes able to induce the fundamental brain activation effect while listening to their freely selected favorite musics or the like.

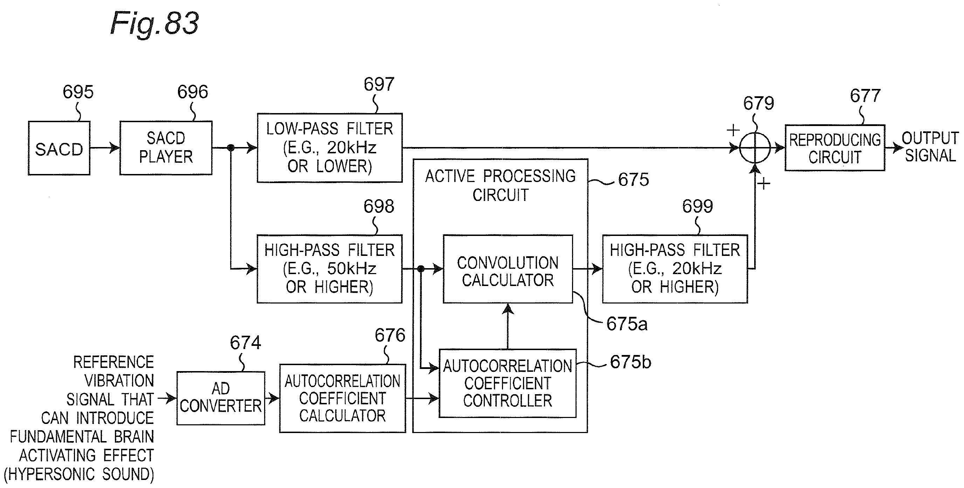

Figure 83 is a block diagram showing an example of an apparatus to generate a vibration that can induce the fundamental brain activation effect by processing a 1-bit quantization noise owned by a high-speed sampling 1-bit quantization system according to an application example 15. Figure 83 generates a vibration signal capable of inducing the fundamental brain activation effect by giving a first property and a second property concerning the autocorrelation order to the 1-bit quantization noise owned by a vibration signal that has been digitalized by the high-speed sampling 1-bit quantization system and recorded in the current SACD (Super Audio CD), a hard disk, a solid memory or the like.

Referring to Figure 83, an SACD 695 is inserted in the drive of an SACD player 696, and its output signal is outputted to an adder 679 via a low-pass filter 697 of a cutoff frequency of, for example, 20 kHz, and outputted to the adder 679 via a high-pass filter 698 of a cutoff frequency of, for example, 50 kHz, an active processing circuit 675 and a high-pass filter 699 of a cutoff frequency of, for example, 20 kHz. The adder 679 adds together the inputted two digital signals, and outputs a digital signal of the addition result to a reproducer circuit 677. Then, the reproducer circuit 677 subjects the inputted digital signal to DA conversion and outputs the resulting signal. It is noted that an A/D converter 674 to process a reference vibration signal and an autocorrelation coefficient calculator 676 are connected to the active processing circuit 675.

When the digital signal recorded by using the high-speed sampling 1-bit quantization system is reproduced, the 1-bit quantization noise theoretically accompanies with certain diffusion around a specific frequency that depends on the sampling frequency and the ΔΣ calculation order. The frequency domain is generated remarkably at and around 50 kHz included in the third band in the current SACD contents and the like adopting the sampling frequency of 2.8 Mbps, and does not induce the fundamental brain activation effect since it does not have an appropriate autocorrelation order. Accordingly, in order to remove the noise at present, a low-pass filter is mounted in the SACD player to remove the high-frequency components of equal to or higher than about 50 kHz.

In the present application example, this 1-bit quantization noise is utilized as a super-high frequency signal material of the third band. The 1-bit quantization noise extracted from an analog signal converted from the digital signal recorded in the SACD 695 by the high-pass filter 698 not via the low-pass filter stated in the preceding paragraph is inputted to the active processing circuit 675, and an autocorrelation coefficient set obtained from the reference vibration signal is inputted to the active processing circuit 675 to perform high-speed convolution calculation between both of them, and a signal of the calculation result is outputted. By adding this signal to the reproduction signal of the SACD contents that do not induce the fundamental brain activation effect or to the audible range components of the first band filtered by the low-pass filter 697, it becomes possible to generate a vibration signal capable of inducing the fundamental brain activation effect. By using this apparatus, a vibration signal capable of inducing the fundamental brain activation effect can be reproduced when the contents recorded by the high-speed sampling 1-bit quantization system including the conventional SACD contents are reproduced. By thus generating the vibration capable of inducing the fundamental brain activation effect because of the containment of the super-high frequency components that have the predetermined autocorrelation order, there can be obtained the effects of inducing the activation of the fundamental brain network (fundamental brain network system) including the reward system responsible for the reactions of pleasure, beauty and emotion in human beings, and the centers of the autonomic nerve system, the internal secretion system and the immune system, which are responsible for the homeostasis and biophylaxis of the whole body, exalting the aesthetic sensitivity and ameliorating and improving the body state. It is acceptable to adjust the timing of the two vibration signals added together by using a delay circuit in the addition stage to adjust the time delay required for the convolution calculation. Although the reproduction signal from the SACD 695 is described as the object in the figure, this may be a media reproduction signal of a hard disk, a solid memory or the like, a signal transmitted and distributed by a network, or the like.

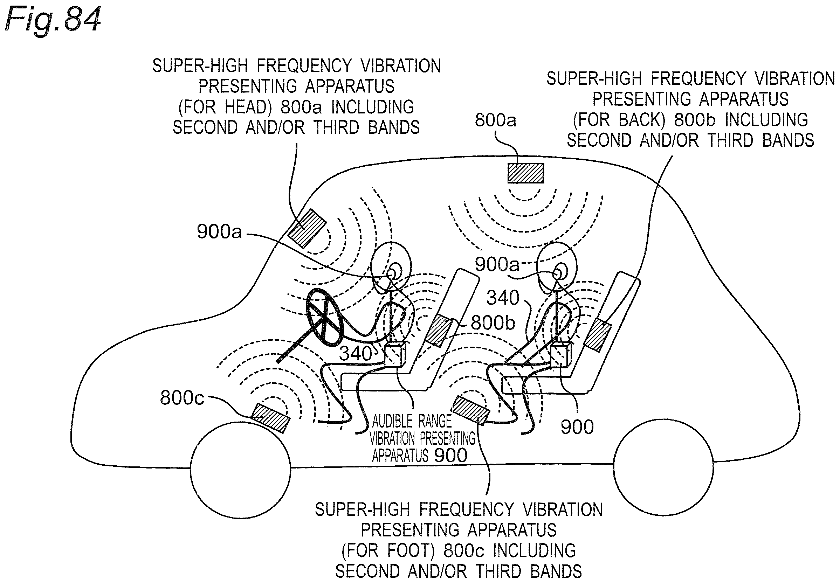

Figure 84 is a side view showing a vibration presenting apparatus in a vehicle according to an application example 16. Referring to Figure 84, there is shown an example of a space in which the audible range components of a vibration that contains the super-high frequency vibration of the second and/or the third band and is able to decrease or increase the brain activity is applied from an audible range vibration reproducing apparatus such as a portable player together with the audible range components in the vehicle, and the super-high frequency components are additionally applied from a loudspeaker or a vibration presenting apparatus embedded in a sheet into the space, presenting a vibration capable of decreasing or increasing the brain activity by virtue of the simultaneous existence of them. In this case, the super-high frequency vibration is presented from super-high frequency vibration presenting apparatuses 800a, 800b and 800c (in this case, 800a is a super-high frequency vibration presenting apparatus for the head portion, 800b is a super-high frequency vibration presenting apparatus for the back, and 800c is a super-high frequency vibration presenting apparatus for the feet) installed in places in a car, and applied to the portions of the faces, bodies, backs and so on of the persons in the car. These presenting apparatuses may present an identical vibration source or concurrently use different vibration sources. At this time, different listeners 340 in the identical car can enjoy the effects of increasing or decreasing the brain activity while listening to their mutually different favorite audible sounds by using an audible range vibration reproducing apparatus 900 such as a portable player, a headphone 900a or the like.

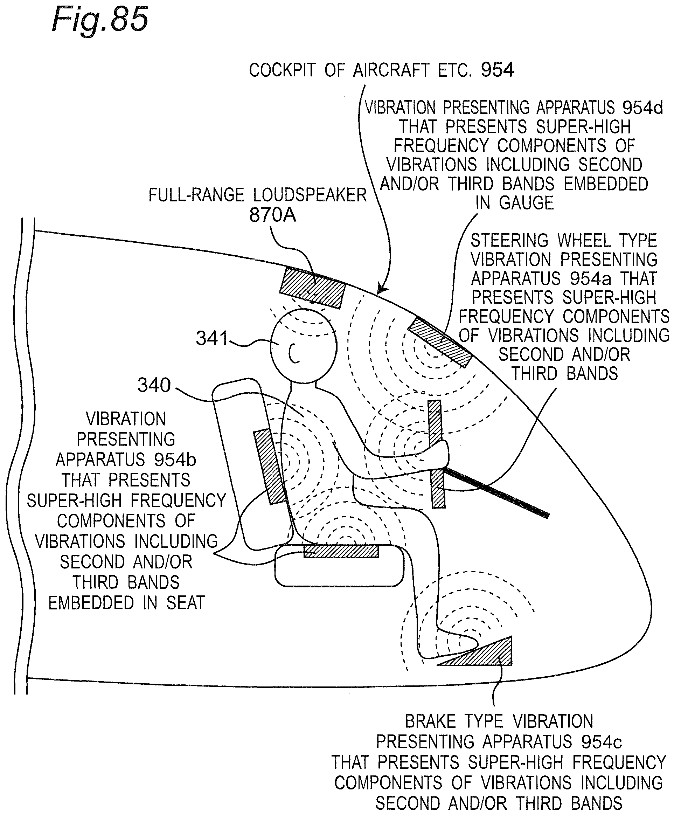

Figure 85 is a side view showing an example of a vibration presentation space of the driver's seat or the cockpit of a public transportation according to an application example 17. Referring to Figure 85, there is shown a vibration presentation space in the driver's seat or the cockpit of a public transportation or the like, which can induce an increase or a decrease in the brain activity in the listener 340 who is the driver by applying a vibration including the second and/or the third band in addition to the vibration of the first band to the listener 340 who is the driver from a full-range loudspeaker installed in the space and additionally effectively applying the super-high frequency components containing the second and/or the third band components of the vibration from vibration generator apparatuses embedded in places such as the seat and a brake type driving apparatus or the like, so that they concurrently exist. A partially see-through external view of the cockpit of an aircraft 954 that has a plurality of super-high frequency vibration presenting apparatuses 954a to 954d is described here. By steering in a state in which a number of super-high frequency vibration presenting apparatuses 954a to 954d are arranged in the cockpit or the cockpit seat of an aircraft 954 (this may be a vehicle such as a locomotive, a train, an ocean vessel, an automobile, and a manned rocket besides the aircraft), the super-high frequency vibration can be effectively presented to the body surface. The super-high frequency vibration presenting apparatuses 954a to 954d effectively present the super-high frequency vibration to the body surface by generating super-high frequency vibrations by the vibration generator apparatuses in a manner similar to that of the aforementioned embodiment. At this time, the listener 340 who is the driver can effectively achieve an increase or a decrease in the brain activity by interactions with the super-high frequency vibrations even when he or she is listening to a music, a broadcasted sound, a voice or the like limited to the audible range frequency by using a general loudspeaker, a headphone or the like. With this arrangement, it can be expected to exalt the safety of steering by promoting the psychosomatic health of the pilot, maintaining the arousal level and preventing human errors. It is noted that this apparatus may be installed in a crew's room, a trainman seat, a guest room, and a seat, not limitative to the cockpit and the cockpit seat.

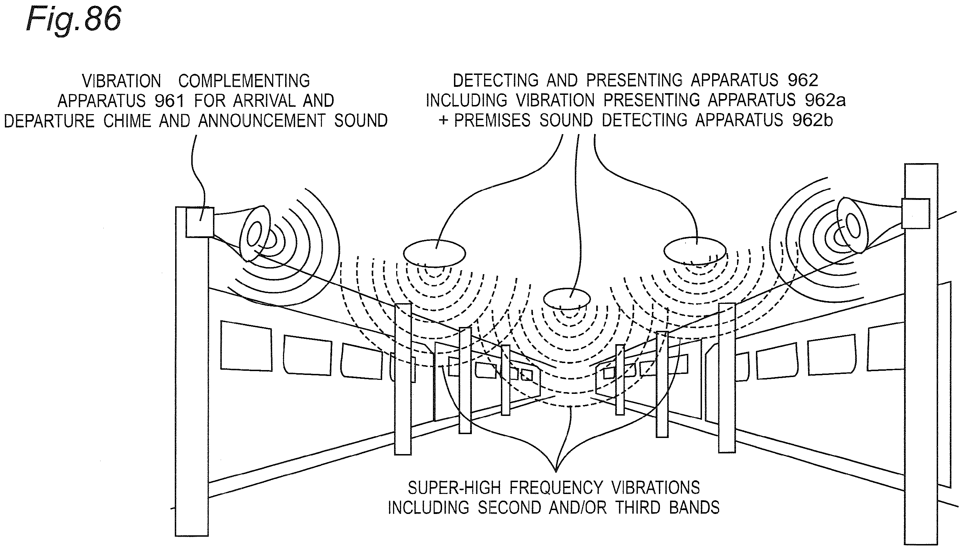

Figure 86 is an external view showing a mounting example of a vibration presenting apparatus 962a at a vehicle passenger platform in a station yard or the like according to an application example 18. For example, with a built-in vibration complementing apparatus 961 of a public-address system installed in the premises provided for recorded arrival and departure chimes, recorded announcement or the like, a vibration capable of inducing the effects of increasing or decreasing the brain activity can be generated by complementation. Moreover, for vibrations such as arrival sounds and starting sounds of trains, announcement issued by a station employee, operating sounds of vending machines, and other environmental noises, which are generated on the scene with the sound level changing intensely and do not induce the effects of increasing or decreasing the brain activity, the vibrations can be effectively complemented by using a detecting and presenting apparatus 962 with built-in premises sound detecting apparatus 962b and gate circuit or voltage-controlled amplifier (VCA). The gate circuit has the operation that a vibration capable of inducing the fundamental brain activation effect is complemented by opening the switch of the gate circuit when the level of the vibration detected by the premises sound detecting apparatus 962b exceeds a definite level and no complementation is effected by closing the switch when the level does not exceed the definite level. The voltage-controlled amplifier (VCA) has the operation that the vibration capable of inducing an increase or a decrease in the brain activity is complemented at a level strongly correlated to the level of the vibration detected by the premises sound detector. As a result, by effecting complementation with the vibration of, for example, the third band at a level appropriately adjusted to the state of existence of the vibration in the premises, the effects of effectively suppressing a decrease in the fundamental brain activity and alleviating the unpleasant sensation can be produced. Moreover, for the troubles such as quarrels and violence between passengers or between a passenger and a station employee in a station yard, which have been serious problems in recent years, the effects of avoiding the troubles can be produced by changing from the brain activity decreased state toward the direction of an increased state to alleviate the irritating feeling and anger sensation by attenuating the level of, for example, the vibration of the second band and effecting complementation with the vibration of the third band. This effect can be applied to trouble avoidance in various public spaces not limitative to the station yard.

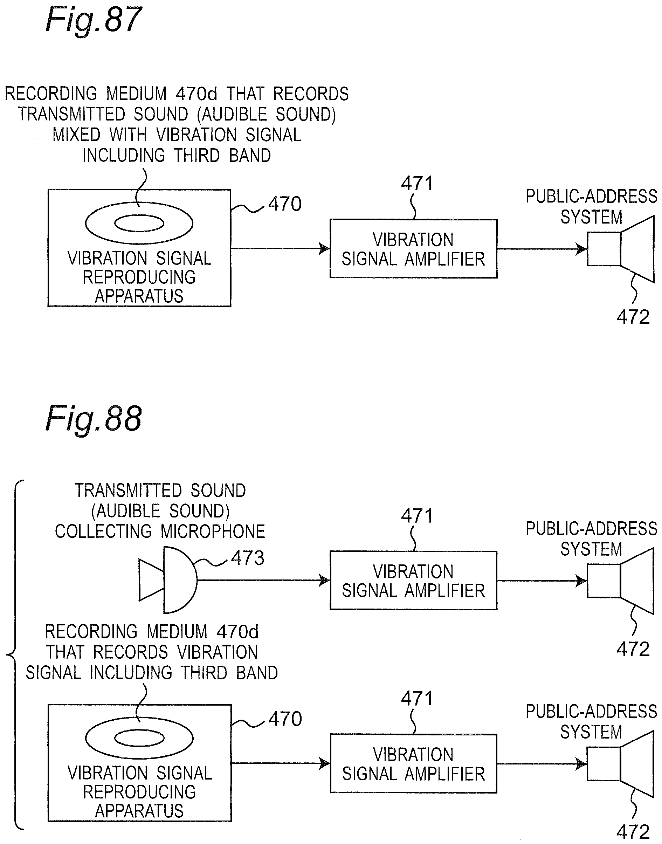

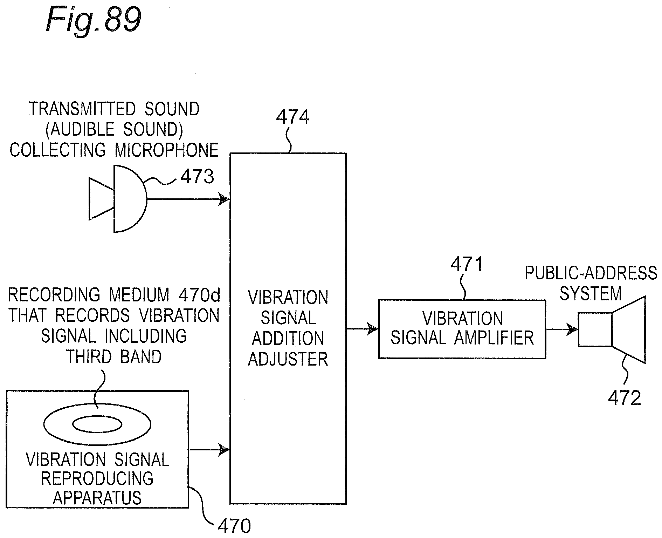

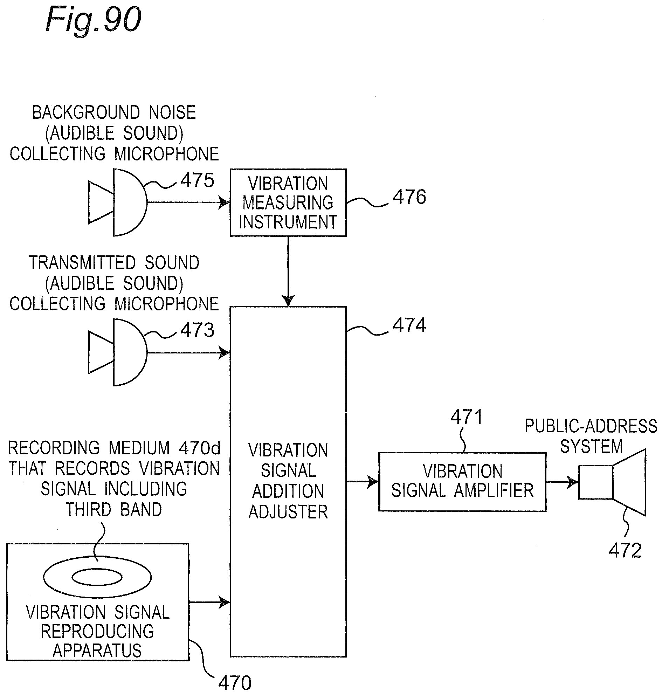

Next, concrete examples of the vibration complementing apparatus and method for complementation with the vibration including the third band as a modified embodiment of the application example 18 are described.
(1) Referring to Figure 87, a transmitted sound (audible sound) and a vibration including the third band are preliminarily recorded in mixture with a balance originally determined, and the signal is reproduced by using a public-address system 472 that has a faithful response performance. In this case, the vibration complementing apparatus is configured to include a vibration signal reproducing apparatus 470 that reproduces a vibration signal by using a recording medium 470d in which the transmitted sound (audible sound) and the vibration including the third band are recorded in mixture, a vibration signal amplifier 471, and a public-address system 472.
(2) Referring to Figure 88, a transmitted sound (audible sound) and a vibration including the third band are generated by using different public-address systems 472 and 472 depending on different sound sources. In this case, level control can be independently performed respectively for the transmitted sound (audible sound) and the vibration including the third band. In this case, the vibration complementing apparatus is configured to include:
   (a) a first apparatus including a microphone 473 to collect the transmitted sound (audible sound), a vibration signal amplifier 471, and a public-address system 472; and
   (b) a second apparatus including a vibration signal reproducing apparatus 470 for reproducing a vibration signal by using a recording medium 470d in which the vibration signal including the third band is recorded, a vibration signal amplifier 471, and a public-address system 472.
(3) Referring to Figure 89, this shows a modified embodiment of the above item (2), which synthesizes a transmitted sound (audible sound) signal and a vibration signal including the third band on the scene, and generated from one public-address system 472. The vibration complementing apparatus is configured to include a microphone 473 to collect the transmitted sound (audible sound), a vibration signal reproducing apparatus 470 to reproduce a vibration signal by using a recording medium 470d in which the vibration including the third band is recorded, a vibration signal addition adjuster 474, a vibration signal amplifier 471, and a public-address system 472. The vibration signal addition adjuster 474 adjusts the levels of inputted two signals and adds together these two signals, and outputs the resulting signal to the public-address system 472 via the vibration signal amplifier 471.
(4) This is an example obtained by further adding an adjustment function to the above item (3). Referring to Figure 90, a background noise (audible sound) is collected by a microphone 475, the feature of the background noise (audible sound) is measured by a vibration measuring instrument 476 on the basis of the collected vibration signal, and the measured data is inputted to the vibration signal addition adjuster 474. Other configurations include a configuration of the above item (3). The vibration signal addition adjuster 474 has the function of adjusting a transmitted sound (audible sound) signal and a vibration signal including the third band in accordance with the feature of the background noise (audible sound). As examples of the adjustment function, there are the function of turning on the vibration including the third band when the noise level of the background noise (audible sound) exceeds a definite level or the function of amplifying the level of the vibration including the transmitted sound (audible sound) and the third band by an amplification factor correlated to the noise level of the background noise (audible sound) or the function of analyzing the frequency characteristic of the background noise (audible sound) and adjusting the intensity of adding the vibration including the third band on the basis of the intensity of the second band and the intensity of the third band.

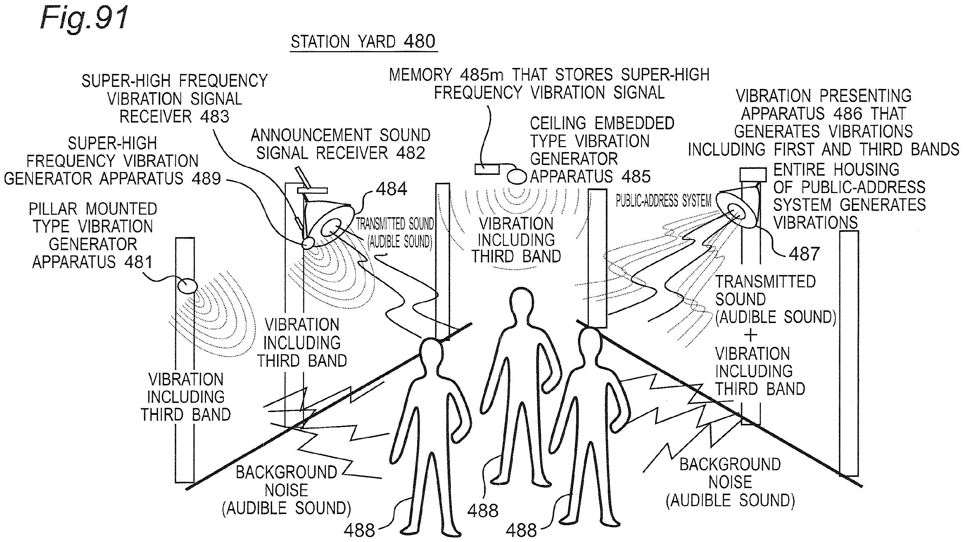

Figure 91 shows an example in which the aforementioned vibration generator apparatus is installed in a station yard 480 by various installation methods. Referring to Figure 91 are shown a pillar installation type vibration generator apparatus 481, a signal receiver 482 of a transmitted sound (audible sound) such as an announcement sound, a super-high frequency vibration signal receiver 483, a loudspeaker (public-address system) 484 to generate a transmitted sound (audible sound), and a super-high frequency vibration generator apparatus 489. There are shown a ceiling embedded type vibration generator apparatus 485 with a built-in memory 485m in which a super-high frequency vibration signal is stored, a vibration generator apparatus 486 to generate a vibration including the third band, a loudspeaker (public-address system) 487 to generate a vibration including the third band with the transmitted sound (audible sound), and human beings 488. This vibration generator apparatus may be newly established or additionally incorporated into the existing premises public address system. Moreover, it is acceptable to externally wiredly input the vibration signal or receive a signal transmitted from the outside wirelessly (by electromagnetic waves, infrared rays, LAN, Bluetooth (registered trademark) etc.) Otherwise, it may be recorded in a memory or the like and built in each vibration generator apparatus. Moreover, it may be artificially generated in the vibration generator apparatus. Moreover, it is acceptable to generate a vibration from the entire casing of the public-address system or generate a vibration from a cable or its sheath, peripheral ceiling and walls, pillars, building materials or the like.

Similar examples of these applications are described below. There may also be applications such that public address broadcasting and the like intended for information transmission in spaces having exceptional background noises such as various announcements of arrival and departure guide, boarding, accident information, schedule change and the like in airports and vehicles, guidance broadcasting at streets, underground shopping centers, event sites, amusement parks, stadiums and the like, and intra-building broadcasting in public institutions, factories and the like are performed.

Moreover, it is important to appropriately guide the sufferers by broadcasting sounds and public address sounds at the disaster sites of fire, earthquake, accident and the like, whereas there is a risk that the sounds cannot be heard by being drown out under megavolume background noises, posing a problem that they tend to cause group panic in that case. As a countermeasure, by effecting complementation with a vibration capable of inducing the fundamental brain activation effect in the space, the sensitivity to the sufferer's sensory information input is sharpened, vocal information to guide the sufferers is made easy to hear, and the reward system neural circuit is activated to alleviate insecurity. As a result, it is useful for appropriately guiding the sufferers without causing the group panic.

Further, there is a problem that the driver and fellow passengers in a car become irritated or sleepy due to congestion on an expressway or ordinary road. As a countermeasure, a vibration signal including the third band is transmitted with broadcasting of traffic information transmitted to the car or independently. A vehicle driven on a road generates the vibration including the third band by receiving this vibration signal with the traffic information or independently or converting the received signal into an aerial vibration by using the vibration signal generator installed in the car. By this operation, the driver and fellow passengers have their arousal level exalted, leading to a cognition power improving effect to the traffic information and an accident preventing effect by raised cognition power and judgment power to the visual information input, and it is expected to alleviate the irritating feeling due to congestion.

Likewise, it is sometimes a case where the broadcasting sounds intended to perform information transmission such as guiding of users or the like can be hardly heard by being drown out under background noises or the like in airports, outdoor and indoor event sites, hospitals, schools, public institutions such as libraries, concert halls, department stores, facilities of amusement parks and the like, shopping streets, station squares, parks and other public spaces having exceptional background noises. As a countermeasure, by effecting complementation with a vibration including the third band capable of inducing the fundamental brain activation effect in the space, the user's sensitivity to the sensory information input is sharpened to make the broadcasting sound easy to hear, and the reward system neural circuit is activated to allow the alleviation of the unpleasant sensation and irritating feeling of "noisy" and the like as well as the improvement of comfort.

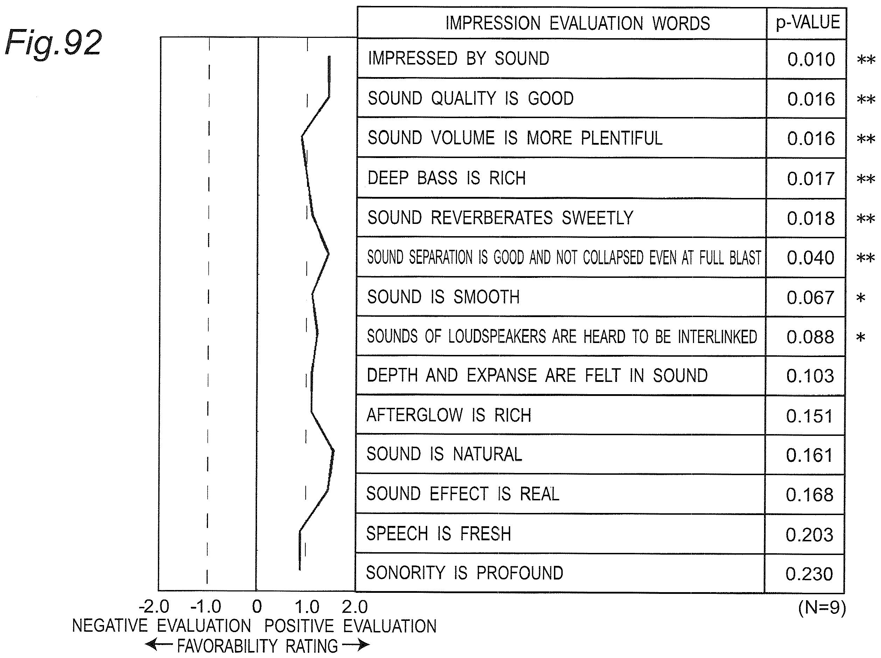

Figure 92 is a graph and table showing impression evaluations of sounds evaluated by a listener under a high-cut sound condition and a full-range sound condition generated by using the "Blu-ray (registered trademark) Disc version AKIRA sound track" according to an application example 24. On the questionnaire used to answer, 14 evaluation words to express the impression of sounds were presented to make evaluations by five-stage evaluation. Analysis was made by using the answers of a total of nine test human subjects.

The left chart of Figure 92 is one in which a difference is obtained by subtracting the impression grade of sound in the high-cut sound condition from the impression grade of the sound in the full-range sound condition regarding each test human subject, and averages are obtained regarding all the test human subjects and plotted. This numerical value indicates that the sound in the full-range sound condition is more positively evaluated as it is greater and defined as "favorability rating". The right chart of Figure 92 shows results of examination by the signed rank test of Wilcoxon (Wilcoxon) as to whether significance exists in the bias of the "favorability rating".

As a result, the favorability of the sound impression was higher in the full-range sound condition than in the high-cut sound condition concerning all the evaluation words. In particular, the evaluation words of the six items: "impressed by sound", "sound quality is good", "sound volume is more plentiful", "deep bass is rich", "sound reverberates sweetly" and "sound separation is good and not collapsed even at full blast" are evaluated statistical significantly positively with p < 0.05 (mark ** in the right figure of Figure 92). Moreover, regarding the evaluation words of the two items: "sound is smooth" and "loudspeaker sounds are heard to be interlinked" are positively evaluated with a high tendency of p < 0.10 (mark * in the right figure of Figure 92). This indicates that the full-range sound induces the activation of the fundamental brain and the fundamental brain network (fundamental brain network system) including the reward system neural circuit of the brain responsible for the generation of reactions of pleasure, beauty and emotion in a human being, consequently enhancing the aesthetic sensibility to sounds and further intensifying the impressions of pleasure, beauty and so on.

In using the vibration generator apparatuses as described above in various facilities, it is acceptable to control on/off and the level of the vibration signal including the third band in accordance with the existence and the number of human beings. That is, for example, when even one person enters a room, the event is automatically sensed by a sensor of infrared rays or the like to turn on the vibration signal or turn off the vibration signal when all persons leave the room. Otherwise, a method for turning on/off also the vibration signal in accordance with turning on/off the illumination interlockedly with an illumination power is considered. Moreover, a method for interlocking it with a room entering/leaving control system of a card key or the like is possible. Further, a system for automatically counting the number of human beings who have entered a room and increasing or decreasing the level of the vibration signal in accordance with an increase and a decrease in the head-count is also considered.

Moreover, as an application of the vibration generator apparatus of the present embodiment, by complementing the original vibration that does not induce the fundamental brain activation effect and accompanies an unpleasant sensation because it does not contain the super-high frequency components or includes the second band in the super-high frequency components with a vibration that includes the third band and induces the fundamental brain activation effect, the effects of suppressing the hypersonic negative effect including a decrease in the fundamental brain activation and alleviating the unpleasant sensation can be produced.

In this case, it is acceptable to use an apparatus that absorbs and removes the vibration accompanied by unpleasant sensation in combination with the vibration complementing apparatus. For example, in combination with a vibration absorbing apparatus that selectively absorbs the vibration in the audible range, a vibration removing apparatus that uses the existing active servo technology or the like, the unpleasant sensation can be effectively alleviated.

Moreover, in this case, it is acceptable to use a vibration detector apparatus and a gate apparatus and (and/or) the circuit of a voltage-controlled amplifier (VCA) together with the vibration complementing apparatus. With this arrangement, it becomes possible to effect complementation with a vibration capable of inducing the fundamental brain activation effect adjusted to an appropriate level in accordance with the state of existence and the level of the unpleasant sounds existing in the environment.

For example, by installing in a station a vibration complementing apparatus for vibrations accompanied by unpleasant sensation such as arrival sounds and departure sounds of trains in the station, premises announcement sounds, vending machine manipulation sounds and the like to add the vibration that contains super-high frequency components including the third band and is able to induce the fundamental brain activation effect, the effects of suppressing the decrease in the fundamental brain activity and alleviating the unpleasant sensation can be produced.

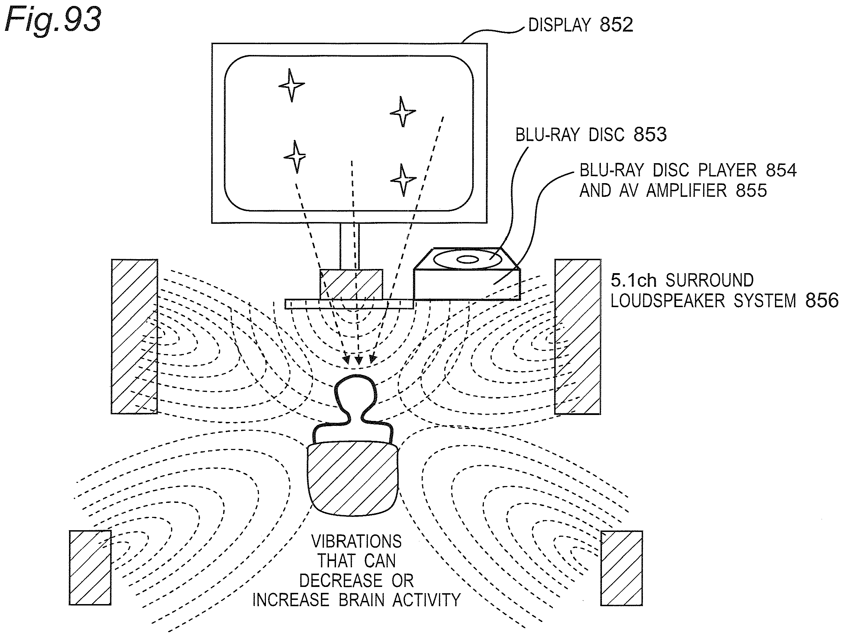

Figure 93 shows an example in which, an increase in the moving impression of image expression and an improvement in the image quality are induced by forming a sound to be put in a soundtrack in video-and-audio complex package media such as a Blu-ray Disc into a vibration that contains the audible range components (first band) and the super-high frequency components (third band) capable of inducing the fundamental brain activation effect. The aesthetic sensibility to video images of a viewer who is watching the video images of a Blu-ray Disc is enhanced, and his or her pleasure, beauty and emotion can be heightened. The video and audio system of Figure 93 is configured to include a display 852, a Blu-ray Disc player 854 in which a Blu-ray Disc 853 is loaded, an AV amplifier 855, and a 5.1-channel surround loudspeaker system 856. Moreover, when the vibration signal recorded in the soundtrack of the video-and-audio complex package media of the Blu-ray Disc or the like is a vibration signal that does not contain super-high frequency components or is unable to induce the fundamental brain activation effect, it is complemented with a vibration capable of inducing the fundamental brain activation effect at terminal equipment by the various kinds of apparatuses and methods described in the embodiments 2, 3 4 or 1A and then reproduced.

An example of an experiment to evaluate a difference in the impression of the video image caused by a difference as to whether the sound has the fundamental brain activation effect by using the "Blu-ray (registered trademark) Disc version AKIRA" as an example of the complex sensory information that comprehensively works on the plurality of sensory systems is described below.

The video image of the "Blu-ray (registered trademark) Disc version AKIRA" used for the experiment is obtained by recording the video image of an animation movie presented in a theater into the image track of the Blu-ray Disc. The sound is edited for the "Blu-ray (registered trademark) Disc version AKIRA soundtrack". The past AKIRA soundtrack, of which the vibration signal has been recorded in DVDs by the digital format of a sampling frequency of 48 kHz and a quantization bit count of 16 bits, can neither record nor reproduce the band components of equal to or higher than the Nyquist frequency of 24 kHz, or a half of the sampling frequency and is therefore unable to induce the fundamental brain activation effect. Accordingly, the audio signal for the DVD version AKIRA soundtrack" was used as an original vibration, and the signal was subjected to band expansion. In addition, by synthesizing an output signal by adding a tropical rain forest environmental sound that is a typical vibration that could induce the fundamental brain activation effect, and the super-high frequency components exceeding the audible range upper limit extracted from it and so on to the sound by using the vibration signal generator apparatus including the vibration complementing apparatus and recording the resulting signal by the digital format of a sampling frequency of 192 kHz and a quantization bit count of 24 bits into a Blu-ray Disc to and this leads to form the "Blu-ray (registered trademark) Disc version AKIRA soundtrack" and record it into the Blu-ray Disc. This sound is a sound that sufficiently contains the super-high frequency components of the third band and is able to induce the fundamental brain activation effect.

In the experiment, an identical video image was consistently presented, while the sound was presented under the blindfold by switchover between two conditions. That is, one of them presented the vibration signal of the "Blu-ray (registered trademark) Disc version AKIRA soundtrack" unchanged as the original reproduced in the state of a vibration signal (full-range sound) that contained super-high frequency components of the third band and was able to induce the fundamental brain activation effect, and the other presented a sound reproduced in the state of a vibration signal (high-cut signal) that was not able to induce the fundamental brain activation effect obtained by removing the frequency components of equal to or higher than 24 kHz from it by a low-pass filter and leaving the first band and the second band remaining. The experiment was conducted under the blindfold by presenting and comparing two kinds of sounds with a video image reproduced from an utterly identical image data.

The test human subjects were made to answer respective impressions by a questionnaire about the video images under the full-range sound condition capable of inducing the fundamental brain activation effect and the high-cut sound condition incapable of inducing the fundamental brain activation effect. Twelve evaluation words expressing the impressions about the video image were shown on the questionnaire, and the subjects were made to perform evaluations by five-rank evaluation. The answers of ten test human subjects in total were analyzed.

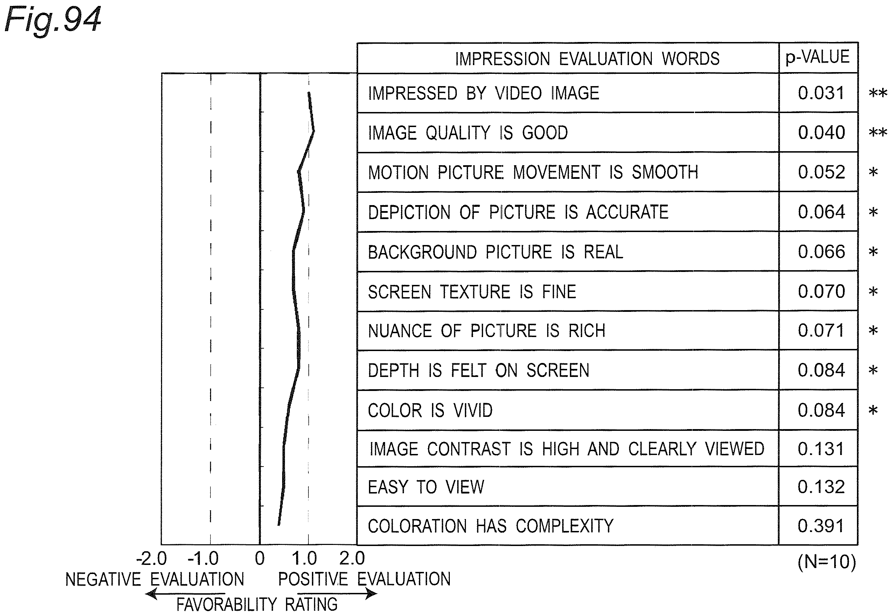

Figure 94 shows experiment results. The left chart is one in which a difference is obtained by subtracting the impression grade of the video image in the high-cut sound condition from the impression grade of the video image in the full-range sound condition regarding each test human subject, and averages are obtained regarding all the test human subjects and plotted. This numerical value indicates that the video image in the full-range sound condition is more positively evaluated as it is greater and defined hereinbefore as "favorability rating". The right chart of Figure 94 shows results of examination by the signed rank test of Wilcoxon (Wilcoxon) as to whether significance exists in the bias of the "favorability rating".

As a result, it was clarified that the video image viewed with the received full-range sound having the fundamental brain activation effect had a higher favorability index and was received more beautifully and movingly than the video image viewed with the received high-cut sound having no such an effect regardless of the fact that the identical video image reproduced from utterly identical image data under the completely identical condition was consistently reproduced in the experiment. In particular, the evaluation words: "impressed by video image" and "image quality is good" are evaluated statistically significantly positively with p < 0.05 (mark ** in the right figure of Figure 94). Moreover, regarding the evaluation words of the seven items of "motion picture movement is smooth", "depiction of picture is accurate", "background picture is real", "screen texture is fine", "nuance of picture is rich", "depth is felt on screen" and "color is vivid" are positively evaluated with a high tendency of p < 0.10 (mark * in the right figure of Figure 94). Besides them, the evaluation words: "image contrast is high and clearly viewed", "easy to view" and "coloration has complexity" are positively evaluated.

As described above, regarding the video-and-audio complex package media of "Blu-ray (registered trademark) Disc version AKIRA", the video image, which was reproduced from the identical image data under the identical conditions and had no difference as the image itself, was received as those having mutually different image qualities depending on whether the sound of the soundtrack concurrently reproduced and presented to the viewer induced the fundamental brain activation effect, and it was statistically significantly indicated that the viewer received the video image as one having higher image quality and more moving when the vibration capable of inducing the fundamental brain activation effect was presented than when the contrary sound was presented.

As described above, the effectiveness of the aforementioned idea of the inventor and others was proven. That is, the inventor and others paid attention to the actual state in which the generation of all reactions of pleasure, beauty and emotion in a human being were unitarily comprehensively governed by the reward system neural circuit of the brain, the fact that the reward system neural circuit was included in the fundamental brain and the fundamental brain network and further the phenomenon that the fundamental brain and the fundamental brain network were activated by applying the vibration including the first and third band, and obtained an idea that, the aesthetic sensibility to a variety of kinds of sensory information inputs other than the auditory sensation was also enhanced in parallel with the enhancement of the aesthetic sensibility to sounds, developing the effects of exalting the sensations of pleasure, beauty and emotion when the fundamental brain and the fundamental brain network (fundamental brain network system) including the reward system neural circuit of the brain unitarily comprehensively responsible for the generation of reactions of pleasure, beauty and emotion of the human being were activated, and obtained an idea to apply it. The above experiment results proved that the idea hit the mark.

The evaluation words: "depiction of picture is accurate", "screen texture is fine" and "background picture is real" of which the favorability ratings are raised by the experiment results surprisingly coincide with the evaluations that indicate an improvement in the image quality characteristically appearing when the information capacity consumed for the image data is increased to densify the video image. The evaluation results indicate the amazing fact that an effect equivalent to increasing the information capacity distributed to the image data can be induced by containing the vibration of the third band that induces the fundamental brain activation effect in the sound information presented simultaneously with the image. That is, the contents, which comprehensively work on the plurality of sensory systems as described above, have the serious problem that, due to restrictions on the recordable and transmittable information capacity and the information transmission rate, a trade-off relation results between different sorts of sensory information such as image quality and sound quality to give a strain to partial sensory information, consequently causing a deterioration in the expressive effect owned by the sensory information and double ruin as a result of trying to make use of each other. Regarding this fateful problem owned by the complex sensory information generating means, it is indicated that superb solution means can be provided by using the apparatus of the present invention. In general, it is necessary for improvements in the image quality to achieve technological developments requiring a huge cost and system as exemplified by, first an increase in the recordable data capacity, further developments in the data compression technology and data transmission technology, and development of hardware for reproduction. However, by using the apparatus of the present invention, it becomes possible to solve the problems by inducing the image quality improvement effect by an extremely realistic acoustic technology and method without depending on the developments in advanced information processing related technologies as described above.

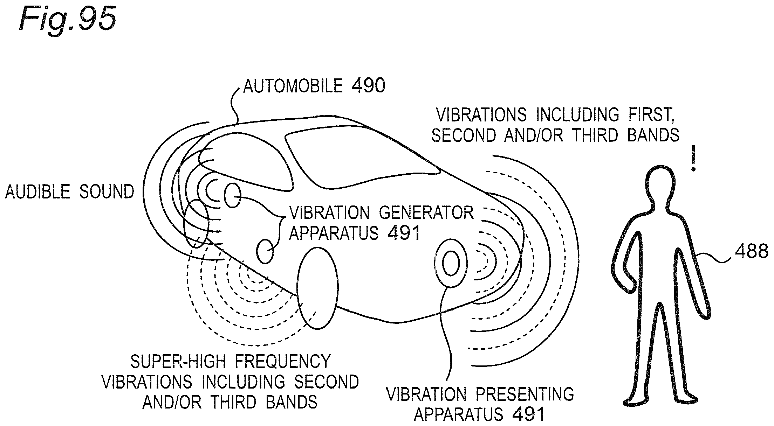

Figure 95 is a perspective view showing an application example 27 in which a vibration presenting apparatus is mounted on the exterior of a vehicle such as an automobile. Recently, in place of the gasoline powered vehicles, development of electric vehicles (including hybrid cars, fuel cell powered cars, solar powered cars, etc.) has been rapidly promoted, and they have many advantages that the exhaust is clean and environment friendly, they have no engine noise and so on. However, there is an emerging serious problem that the electric vehicles, which use motors producing small noises instead of using internal combustion engines that generate blast sounds, have quiet driving noises, and therefore, the pedestrians, bicycle riders, car drivers and the like on the road tend to fail in perceiving approaching electric vehicles, increasing the risk of traffic accidents, and urgent countermeasures need to be devised. Moreover, the noise levels of the cars have generally been decreased as the result of technological innovations. Accordingly, by complementing a car 490 such as an electric vehicle with super-high frequency components including the second and/or the third band, the level of the sound generated from the car 490 can be raised up to a height sufficient for making the human being 488 of a pedestrian or the like recognize the approach of the car and secure safety. Referring to Figure 95, a vibration presenter 491 is provided for the car 490. The vibration presenting apparatuses described in the embodiments of the present invention can be applied to the vibration presenting apparatus and the sound source in order to achieve this. Moreover, this vibration presenting apparatus may be preliminarily incorporated into the car body, a tire, a window pane or the like or externally attached. Moreover, it is acceptable to generate a vibration including the audible sound and the super-high frequency vibration from a single vibration presenting apparatus or to generate the audible sound and the super-high frequency vibration from separate vibration presenting apparatuses and make the levels and balance freely adjustable. Further, the sound source may be one that is recorded in a recording medium or transmitted by broadcasting and a communication system. Moreover, the vibration presenting apparatus may be mounted on another object such as a vehicle that has a risk of colliding with a human being 488, other than the automobile.

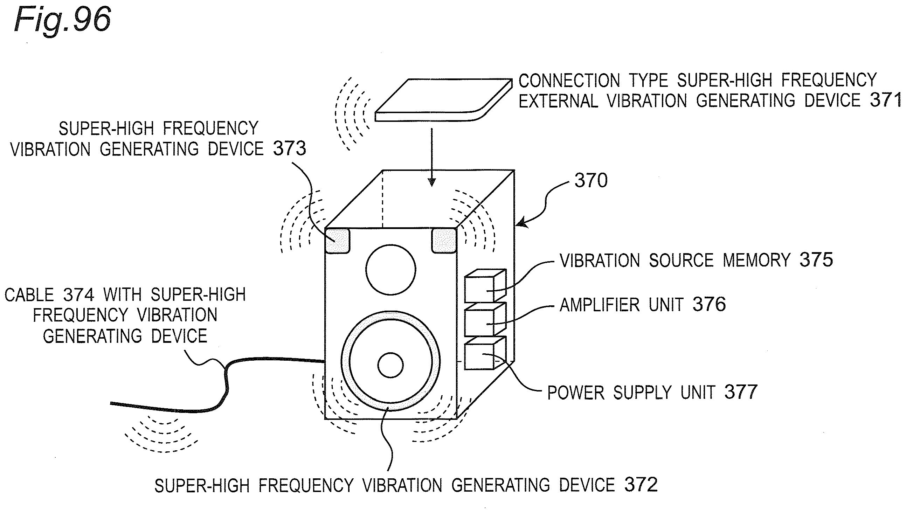

Figure 96 is a perspective view showing one example of a vibration presenting apparatus 370 of a loudspeaker system according to an application example 28. As a vibration source, a memory 375 in which a signal containing super-high frequency components including the second and/or the third band is stored, and auxiliary apparatuses of an amplifier unit 376, a power supply unit 377 and so on to drive a loudspeaker or a super-high frequency vibration generating device by the signal are equipped for the loudspeaker system itself. Moreover, an apparatus that generates the super-high frequency components including the second and/or the third band inside the loudspeaker system may be built in. As a vibration generating mechanism, there are the following means besides generating the super-high frequency vibration including the second and/or the third band by using the vibration generating mechanism of the loudspeaker itself. A vibration generating function is built in the loudspeaker system to vibrate the casing or the like itself. Otherwise, the casing or the like is equipped (embedded, stuck, wrapped) with super-high frequency vibration generating devices 372 and 373. Moreover, the outside of the casing or the like is provided with a sheath material of piezoplastic or the like. Further, the super-high frequency vibration generating devices 372 and 373 are connected to generate a vibration. Moreover, a cable 374 for connecting the equipment with the loudspeaker system can be equipped with a super-high frequency vibration generating device. In this case, a power supply method may be implemented by feeding electric power from an external power source or by a built-in power unit 377, a battery (primary cell (dry battery), secondary cell (storage battery), built-in fuel cell etc.) or the like. Moreover, as a method for feeding power from the connected equipment, there are the phantom system, i.e., a method for feeding the audio cable with electric power with a dc power source superimposed, a method for making audio signal transmission and feeding electric power coexist by a USB cable or the like, and so on. Besides them, a wireless electric power feeding mechanism may be equipped.

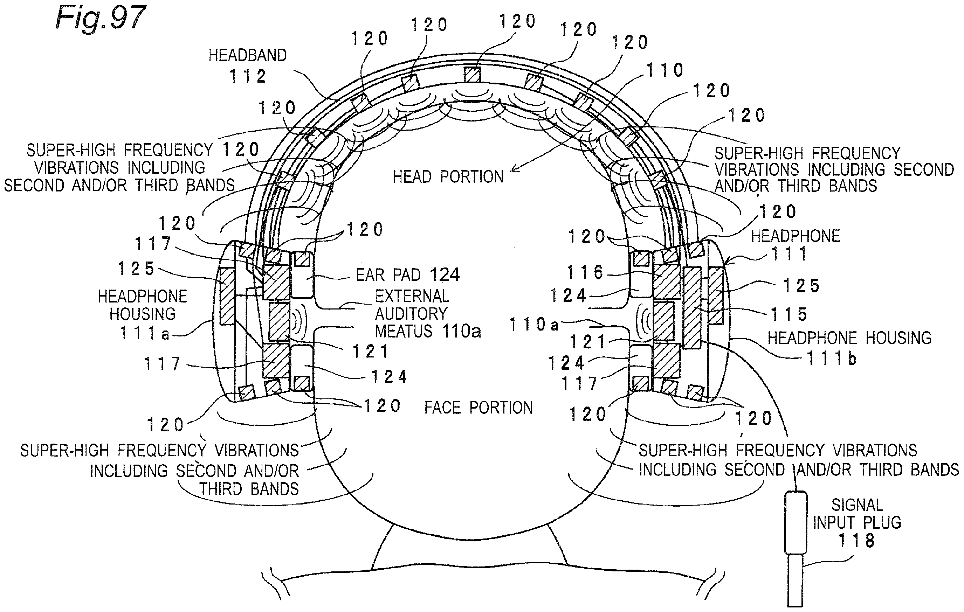

Figure 97 is a front view of a headphone type vibration presenting apparatus according to an application example 29. Referring to Figure 97, a headphone 111 is constituted of one pair of approximately cylindrical headphone housings 111a and 111b arranged oppositely to cover listener's both ears, and a headband 112 for mechanically connecting the headphone housings 111a and 111b and placing them on the listener's head portion 110. Ring-shaped ear pads 124 are provided for the headphone housings 111a and 111b in close contact with the peripheries of the entrances of the external auditory meatus 110a on the side surface located on the listener side of the headphone housings 111a and 111b, and super-high frequency component vibration generating devices 120 for generating the super-high frequency components of the vibration capable of decreasing or increasing the brain activity are provided in the peripheral portions of the ear pads 124. Moreover, a number of super-high frequency vibration generating devices 120 are provided at regular intervals on the surfaces located on the listener's head 110 side of the headband 112. Further, a plurality of super-high frequency component vibration generating devices 120 are provided in the peripheral portions of the headphone housings 111a and 111b, a headphone cable and so on, and audible range loudspeakers 121 that generate the audible range components of the vibration capable of decreasing or increasing the brain activity are provided in places corresponding to the external auditory meatus 110a on the inner side surfaces of the headphone housings 111a and 111b. The headphone cable and so on are allowed to be made of a piezoplastic material to generate a super-high frequency vibration from there or to devise a design to vibrate the cable itself. Circuits and devices 115, 115, 117, 120 and 121 of the signal reproducing apparatus and a small battery 125 that supplies electric power necessary for generating the super-high frequency vibration are arranged in each of the headphone housings 111a and 111b, a signal input plug 118 is connected to the input terminal of a signal band dividing circuit 115 of the signal reproducing apparatus, and the signal input plug 118 is connected to a predetermined signal reproducing apparatus. With these devices, the present vibration presenting apparatus makes it possible to apply the super-high frequency vibration including the second and/or the third band to the listener's body surface while applying the vibration containing the audible sound to the listener's airway auditory system. It is also possible to generate the audible range components and the super-high frequency components from separate vibration generating devices by using separate vibration signals.

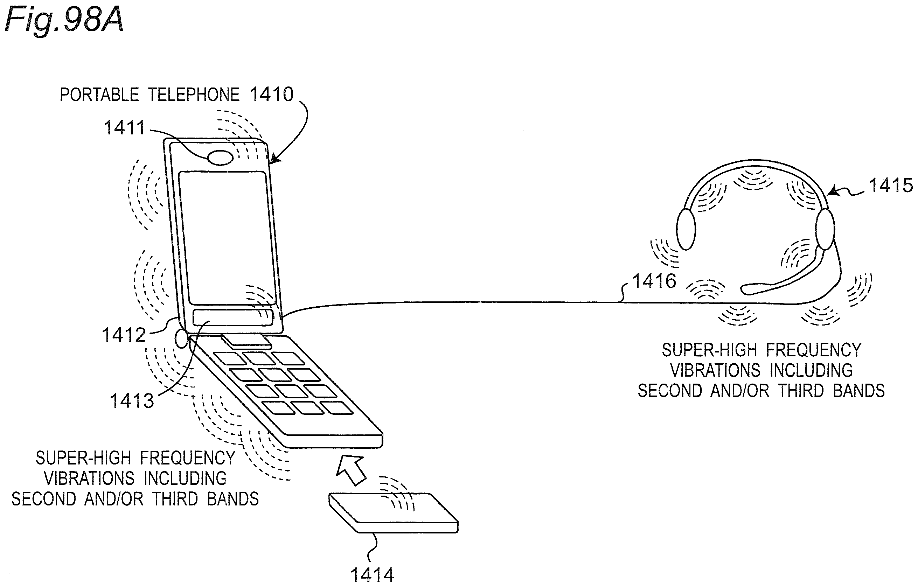

Figures 98A to 98F are perspective views showing examples of a vibration generating mechanism utilizing a portable telephone 1410 according to an application example 30.

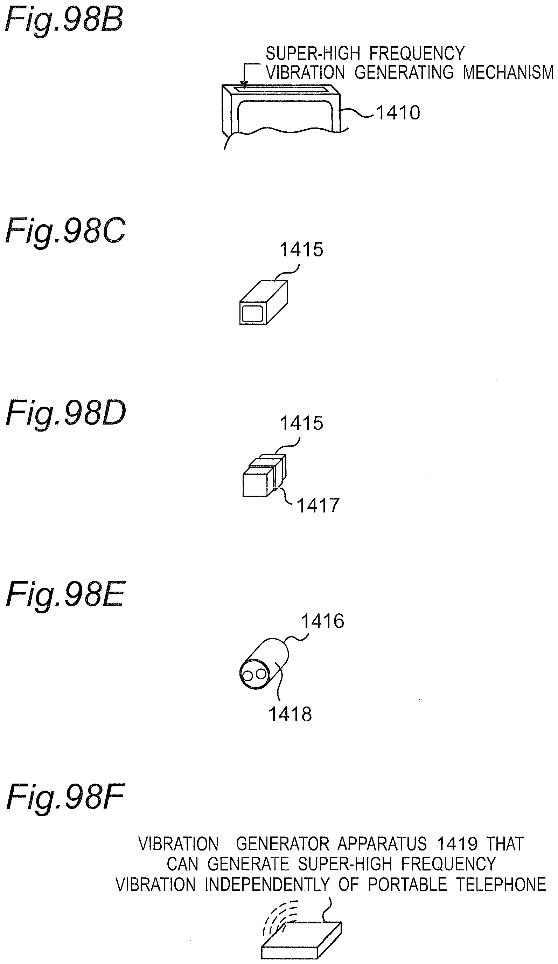

A. Figures 98A and 98B show examples of vibration generating mechanisms utilizing the main body of the portable telephone 1410.
   A-(1) A vibration generating function (super-high frequency vibration generating mechanism) is built in the portable telephone 1410, and a super-high frequency vibration including the second and/or the third band is generated by vibrating the portable telephone 1410 (casing 1412, LCD screen, operating buttons, etc.), and applied to a human being directly or via an aerial vibration.
   A-(2) A super-high frequency vibration including the second and/or the third band is generated by making sound generation means such as a loudspeaker 1411 originally provided for the portable telephone 1410 have a function to faithfully reproduce the super-high frequency vibration, and applied to a human being.
   A-(3) A casing 1412 or the like of the portable telephone 1410 is newly equipped with a super-high frequency vibration generating device 1414 (this may have a sheet shape 1413 to cover the surface), and a super-high frequency vibration including the second and/or the third band is generated, and applied to a human being.
   A-(4) A super-high frequency vibration generating device 1414 is connected to the portable telephone 1410, and a super-high frequency vibration including the second and/or the third band is generated, and applied to a human being.
B. Examples of vibration generating mechanisms utilizing a headset 1415 to be used by being connected to the portable telephone 1410 are described.
   B-(1) A vibration generating function is built in the headset 1415, and a super-high frequency vibration including the second and/or the third band is generated by vibrating a head strap, a microphone arm, an ear pad or the like, and applied to a human being directly or via an aerial vibration (Figure 98C).
   B-(2) The headset 1415 is newly equipped (embedded, stuck or wrapped) with a super-high frequency vibration generating device 1417, and a super-high frequency vibration including the second and/or the third band is generated, and applied to a human being (Figure 98D).
   B-(3) The outside of the headset is a sheath 1418 with a material of piezoplastic or the like, and a super-high frequency vibration including the second and/or the third band is generated from the sheath 1418, and applied to a human being.
C. Examples of vibration generating mechanisms utilizing a cable 1416 that connects the portable telephone 1410 with the headset 1415 or the like are described (Figure 98A).
   C-(1) A super-high frequency vibration including the second and/or the third band is generated by vibrating the electrical signal wire in the cable 1416, and applied to a human being.
   C-(2) A material of piezoplastic or the like is used for the cable sheath 1418, and a super-high frequency vibration including the second and/or the third band is generated from the sheath, and applied to a human being (Figure 98E).
   C-(3) A super-high frequency vibration generating device 1417 is embedded in the cable sheath, and a super-high frequency vibration including the second and/or the third band is generated from there, and applied to a human being.
   C-(4) The outside of the cable is equipped (embedded, stuck or wrapped) with a super-high frequency vibration generating device sheath 1418, and a super-high frequency vibration including the second and/or the third band is generated, and applied to a human being.
D. Otherwise, auxiliaries of an earphone microphone, a strap, an accessory, a soft casing or the like attached to the portable telephone are equipped with a super-high frequency vibration generating device, and a super-high frequency vibration including the second and/or the third band is generated, and applied to a human being.
E. Referring to Figure 98F, a vibration presenting apparatus 1419 capable of generating a super-high frequency vibration including the second and/or the third band may be separately prepared in the vicinity of, for example, the portable telephone 1410 of Figure 98A independently of the portable telephone 1410.

It is noted that the vibration generating mechanisms of the above items A to E may be combined together. Although the examples in which the portable telephone 1410 and the headset 1415 or the like are wiredly connected together are described here, they may be connected together wirelessly (Bluetooth (registered trademark) communications, infra-red communications, human body communications, etc.) It is noted that the aforementioned vibration generating mechanism can be provided for other portable communication equipment (information terminal, transceiver, intercom or the like using wireless IP communications, infra-red communications, etc.), portable broadcasting receiver equipment (one-segment broadcasting receiver etc.) or the like.

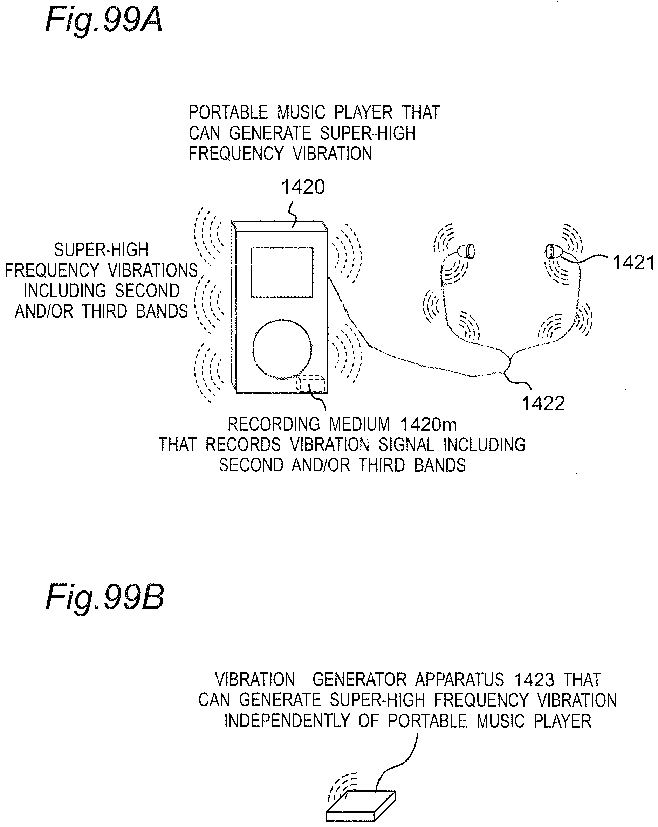

Next, examples in which portable type equipment such as a portable music player (iPod (registered trademark), Walkman (registered trademark), a portable video player, a portable game machine is provided with an appropriate vibration generating mechanism are described. As a concrete example, Figures 99A and 99B are perspective views showing examples of vibration generating mechanisms utilizing a portable music player 1420 such as iPod (registered trademark) according to an application example 31. Referring to Figures 99A and 99B, as a concrete vibration generating mechanism to generate a super-high frequency vibration, in a manner similar to those of Figures 98A to 98F, there are the means utilizing A. a vibration generating mechanism utilizing the main body of the portable music player 1420 (including a memory 1420m in which a vibration signal of a vibration including the third band is recorded), B. a vibration generating mechanism utilizing an earphone 1421 or the like, C. a vibration generating mechanism utilizing a cable 1422, D. other vibration generating mechanisms utilizing an auxiliary of a strap or the like, E. a vibration presenting apparatus 1423 (Figure 99B) that is independent of the portable music player 1420 but placed in the vicinity of it, and so on. Moreover, the vibration generating mechanisms of the items A to E according to the application example 30 may be combined together. It is noted that the aforementioned vibration generating mechanism can be provided likewise for a portable video player, a portable game machine or the like.

Recently, the functions of portable equipment are made complex such as fusion of a portable telephone and a portable player as observed in smart phones and tablet equipment, and therefore, and therefore, the examples described with reference to Figures 98A to 98F, 99A and 99B may be partially or totally implemented in a complex manner. Further, as simpler method having a high feasibility, the earphone 1421, the headset 1415 or the like, which has conventionally been considered as the auxiliaries of portable equipment, is provided with an independent vibration generating function and provided with a function to generate a vibration of super-high frequency components including the second and/or the third band singly or by being connected to some equipment. With this arrangement, the listener becomes able to receive the vibration including the second and/or the third band merely by connecting the earphone 1421, the headset 1415 or the like described in this application example even if various kinds of equipment that can generate only the audible sound inclusive of the portable telephone and the portable music player 1420 are connected. As a vibration source, a memory 1425 to store a vibration signal including the second and/or the third band is mounted inside the casing of an earphone 1421, partway in a cable 1422 or the like. Moreover, it is acceptable to provide a built-in apparatus that artificially generates the super-high frequency components including the second and/or the third band in the earphone 1421. Further, it is acceptable to provide a built-in microamplifier (not shown) for amplifying the vibration signal if the occasion demands. There are following means as a vibration generating mechanism for generating the super-high frequency vibration. The earphone 1421 is provided with a built-in vibration generating function, and the casing, the ear pad or the like itself is vibrated. Otherwise, the earphone 1421 casing, ear pad or the like is equipped (embedded, stuck, wrapped etc.) with a super-high frequency vibration generating device. Moreover, the outside of the earphone 1421 casing, ear pad or the like is provided with a sheath material of piezoplastic or the like. Moreover, means for utilizing the cable 1422 include vibrating the electrical signal wire in the cable 1422, using a material such as piezoplastic for the sheath of the cable 1422, embedding a super-high frequency vibration generating device in the cable sheath, equipping (stacking, wrapping etc.) the cable 1422 externally with a super-high frequency vibration generating device. It is noted that these vibration generating mechanisms may be combined together. Moreover, the headset 1415, the headphone or the like can be provided with a similar function.

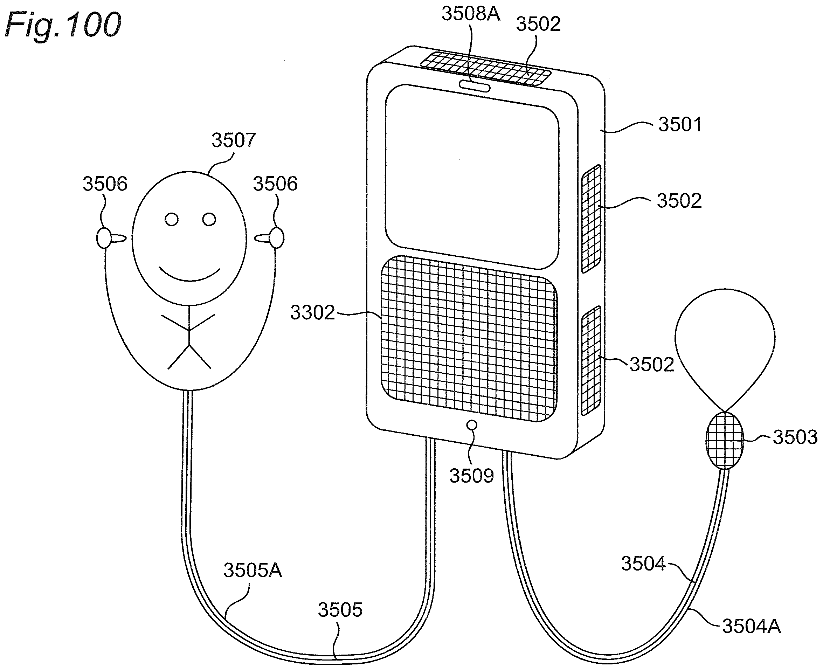

Figure 100 is a perspective view showing an appearance of a portable player 3501 according to an application example 32. In the present embodiment, a signal that contains at least the super-high frequency vibration signal of the third band and is able to induce the hypersonic positive effect is referred to as an HS signal, and a high resolution (high-resolution) containing frequency components exceeding the audible range or a high-definition (high-definition) signal is referred to as an HD signal. Moreover, the HS signal, HD signal and the audible range sound signal include, for example, music signals, environmental sound signals, audio signals and so on. In this case, the HS signal and the HD signal sometimes include or do not include the audible range sound signal depending on cases, and have maximum frequencies determined by the respective sampling frequencies. That is, the HS signal is a signal that is an HD signal and includes at least the vibration signal of the third band, and the HD signal is a signal that does not include the vibration signal including the third band.

Referring to Figure 100, a liquid crystal display 3552 is provided on the front surface of a portable player 3501, and built-in actuators 3502 that generate a signal or a vibration including the HS signal are provided on each surface. Moreover, a built-in loudspeaker 3508A to generate an audible range sound signal is provided on the upper side of the front face of the portable player 3501, and a microphone 3509 for monitoring is provided on the lower side of the front face. Moreover, two connection jacks (not shown) are provided on the lower face of the portable player 3501, and, for example, a neck hung pendant type external actuator 3503 is connected via a plug and cable 3504 that is engaged and connected with one connection jack. In this case, an external actuator 3504A that is connected to the cable 3504 and generates a vibration is formed on the external sheath of the cable 3504. Moreover, an earphone 3506 that is worn by a user 3507 of a human being and reproduces an audible range sound signal is connected via a plug and cable 3505 that is engaged and connected with the other one connection jack. In this case, an external actuator 3505A that is connected to the other core wire of the cable 3505 and generates a vibration is formed on the external sheath of the cable 3505. In this case, the actuators 3502 to 3505 generate the vibration of the HS signal or the HD signal and emit it to the body surface of the user 3507.

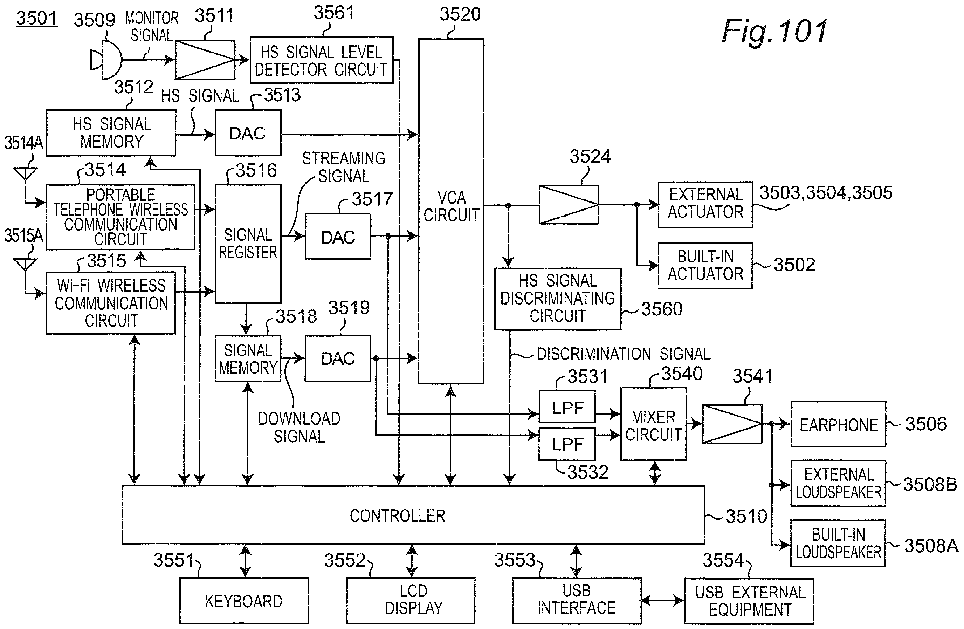

Figure 101 is a block diagram showing a configuration of the portable player 3501 of Figure 100.

Referring to Figure 101, an HS signal memory 3512 preparatorily stores the data of the HS signal including at least the super-high frequency vibration signal of the third band, reproduces a predetermined HS signal on the basis of an instruction signal from a controller 3510, and outputs it to a D/A converter 3513. The D/A converter 3513 subjects the inputted HS signal to DA conversion and outputs the resulting signal to a VCA circuit 3520.

A portable telephone wireless communication circuit 3514 receives the audible range sound signal and the HS signal or the HD signal, which have been distributed from a predetermined server apparatus via a portable telephone network and thereafter received by using an antenna 3514A, demodulates the signals, and thereafter performs predetermined signal processing. Thereafter, the processed signal is outputted as a streaming signal via a signal register 3516 to the VCA circuit 3520 via the D/A converter 3517, and only the audible range sound signal is outputted to a mixer circuit 3540 via a low-pass filter 3531. Moreover, in a case where the received signal is not the streaming signal but a download signal that can be stored in a memory, the portable telephone wireless communication circuit 3514 outputs and stores the processed signal into a signal memory 3518, reads the signal from the signal memory 3518 and outputs it as a download signal to the VCA circuit 3520 via a D/A converter 3519 in response to an instruction signal from the controller 3510 at need, and outputs only the audible range sound signal to the mixer circuit 3540 via the low-pass filter 3531.

A Wi-Fi wireless communication circuit 3515 receives an audible range sound signal and an HS signal or an HD signal, which have been distributed from a predetermined server apparatus via a predetermined network such as the Internet and received by using an antenna 3515A, demodulates the signals, and executes predetermined signal processing. Thereafter, the processed signal is outputted as a streaming signal via the signal register 3516 to the VCA circuit 3520 via the D/A converter 3517, and only the audible range sound signal is outputted to a mixer circuit 3540 via a low-pass filter 3532. Moreover, in a case where the received signal is not the streaming signal but a download signal that can be stored in a memory, the Wi-Fi wireless communication circuit 3515 outputs and stores the processed signal into the signal memory 3518, reads the signal from the signal memory 3518 and outputs it as a download signal to the VCA circuit 3520 via the D/A converter 3519 in response to an instruction signal from the controller 3510 at need, and only the audible range sound signal is outputted to the mixer circuit 3540 via the low-pass filter 3532. It is noted that the low-pass filters 3531 and 3532 low-pass filter only the audible range sound signal of the input signal and outputs the resulting signal.

It is noted that the D/A converters 3513, 3517 and 3519 are, for example:
(1) a D/A converter for DA conversion of a PCM signal at a sampling frequency of 192 kHz;
(2) a D/A converter for DA conversion of a DSD signal at a sampling frequency of 2.8 MHz; or
(3) a D/A converter for DA conversion of a DSD at a sampling frequency of 5.6 MHz,
   capable of performing DA conversion of not only the audible range but also the super-high frequency signal.

Moreover, the maximum frequencies of the HD signal and the HS signal are each one frequency within the ranges of, for example, 88.2 kHz, 96 kHz, 100 kHz, 176.4 kHz, 192 kHz, 200 kHz, 300 kHz, 352.8 kHz, 384 kHz, 500 kHz, 705.6 kHz, 768 kHz, 1 MHz, 1.4 MHz or 2.8 MHz (maximum frequencies, each of which is provided in the case of a PCM signal, and the sampling frequencies thereof are the twofold frequencies). That is, the maximum frequencies of the HD signal and the HS signal are each one frequency ranging from, for example, 88.2 kHz to 2.8 MHz.

Further, the HS signal memory 3512 and the signal memory 3518 have a large memory capacity of, for example, not smaller than 64 GBytes, and are able to store the data of the HS signal that does not contain, for example, the data of the audible range sound source. Further, the signal memory 3518 can store the data listed below.
(1) Data of the audible range sound source
(2) Data of the HD signal that does not contain the HS signal
(3) Data of the HD signal including the HS signal

A microphone 3509 monitors and detects the environmental sound around the portable player 3501, and outputs it as a monitor signal to an HS signal level detector circuit 3561 via a signal amplifier 3511. The HS signal level detector circuit 3561 extracts components including the HS signal in the inputted monitor signal, detects the level of the signal, and outputs it to a controller 3510. In concrete, for example, the signal level of the frequency peculiar to the HS signal is detected.

The VCA circuit 3520 mixes up the inputted signals, performs level adjustment and amplification according to an instruction signal from the controller 3510, outputs the resulting signal to an HS signal discriminating circuit 3560, and outputs it to a built-in actuator 3502 and external actuators 3503, 3504 and 3505 via a signal amplifier 3524. The HS signal discriminating circuit 3560 discriminates whether or not the HS signal exists at a level of equal to or higher than a predetermined threshold level in the inputted signal, and outputs a discrimination signal representing the discriminate result to the controller 3510. It is noted that the VCA circuit 3520 detects the signal level of each inputted signal, and outputs it to the controller 3510.

The mixer circuit 3540 mixes up the inputted audible range sound signals, and outputs the resulting signal to an earphone 3506, a built-in loudspeaker 3508A and an external loudspeaker 3508B via a signal amplifier 3541. By this operation, the audible range sound signal is emitted to the ear of the auditory system of a user 3507 of a human being, allowing the user 3507 to hear it.

To the controller 3510 are connected a keyboard 3551 with which the user 3507 inputs manipulation instructions and operation instructions, a liquid crystal display 3552 that displays indication data of each instruction result, HS signal discrimination result, HS signal level detection result and the like, and a USB interface 3553. In this case, USB external equipment 3554 can be connected to the USB interface 3553, and it is also possible to connect, for example, USB external equipment 3554 provided with a nonvolatile memory that stores the data of the HS signal, the HD signal, the audible range sound signal and the like, and to store the data of the HS signal, the HD signal, the audible range sound signal and the like from the USB external equipment 3554 into an HS signal memory 3512 or a signal memory 3518 via a USB interface 3553 and the controller 3510.

The controller 3510 controls the operation of each of the circuits 3514, 3515, 3512, 3518, 3520 and 3540 in the portable player 3501. In this case, when the discrimination signal from the HS signal discriminating circuit 3560 indicates that the HS signal does not exist, the controller 3510
(1) reads the data of the HS signal stored in the HS signal memory 3512, generates the HS signal, and outputs it to the VCA circuit 3520 via a D/A converter 3513, or
(2) reads the data of the HS signal stored in the HS signal memory 3518, generates the HS signal, and outputs it to the VCA circuit 3520 via a D/A converter 3519 when the data of the HS signal is stored as a downloaded signal in the signal memory 3518.

By this operation, a vibration of the HS signal can be generated in each of the actuators 3502 to 3505 with the HS signal consistently included in the signal outputted from the VCA circuit 3520 while the audible range sound signal is emitted to the ears of the auditory system of the user 3507 by using, for example, the earphone 3506. Therefore, the effects of comprehensively exalting the mental activity and the physical activity by activating the brain, i.e., the hypersonic positive effect can be induced by presenting the vibration to the user 3507.

Moreover, while the audible range sound signal is emitted to the ears of the auditory system of the user 3507 by using, for example, the earphone 3506, when the detection level is smaller than a predetermined threshold value on the basis of the detection level from the HS signal level detector circuit 3561 (when the hypersonic positive effect cannot be induced), the controller 3510 controls the VCA circuit 3520 so that the aforementioned detection level of the HS signal inputted to the VCA circuit 3520 increases in excess of the predetermined threshold value. By this operation, the HS signal that is not smaller than the predetermined threshold value is consistently included in the signal outputted from the VCA circuit 3520, and the vibration of the HS signal can be generated in each of the actuators 3502 to 3505. Therefore, the effects of comprehensively improving the mental activity and the physical activity by activating the brain, i.e., the hypersonic positive effect can be induced by presenting the vibration to the user 3507.

Further, the controller 3510 controls the VCA circuit 3520 so that the levels of the signals inputted to the VCA circuit 3520 and mixed become, for example, substantially identical or come to have a specified level relation.

As described above, the following operational effects are provided according to the present embodiment.
(1) A portable player that reproduces a vibration capable of inducing the hypersonic positive effect and presents it to the body surface of the user 3507 while emitting the audible range sound signal to the ears of the auditory system of the user 3507 by using, for example, the earphone 3506 can be provided.
(2) It is discriminated whether or not the HS signal exists in the vibration generated by the actuators 3502 to 3505 by the HS signal discriminating circuit 560, and the vibration of the HS signal is consistently generated by using the data of the HS signal preparatorily stored in the HS signal memory 3512 or the HS signal that has preparatorily been distributed, downloaded and stored when the signal does not exist. Therefore, the vibration capable of inducing the hypersonic positive effect can be effectively and reliably presented to the user 3507.
(3) It is determined whether or not the signal level of the HS signal is not smaller than the aforementioned threshold value in the vibration of the environmental sound actually generated by the actuators 3502 to 3505 by the HS signal level detector circuit 3561, and the VCA circuit 3520 is controlled so as to increase the signal level of the HS signal when not. Therefore, the vibration capable of inducing the hypersonic positive effect can be effectively and reliably presented to the user 3507.

Although the portable telephone has been described in the above application example 32, the present invention is not limited to this but allowed to constitute portable electronic equipment such as a portable personal computer, and a portable telephone of a smart phone or the like having a similar function.

Although the portable player that generates the audible range sound signal, the HS signal, and the HD signal is described in the application example 32, the present invention is not limited to this but allowed to generate at least the audible range sound signal and the HS signal.

As described in detail above, according to the application example 32, portable electronic equipment that can generate the vibration capable of inducing the hypersonic positive effect and presenting it to the body surface of the user while presenting the audible range sound to the ears of the auditory system of the user can be provided. Moreover, by providing the aforementioned discrimination means or detection means, portable electronic equipment that can reproduce the vibration capable of inducing the hypersonic positive effect and present it to the body surface of the user effectively and reliably while presenting the audible range sound to the ears of the auditory system of the user can be provided.

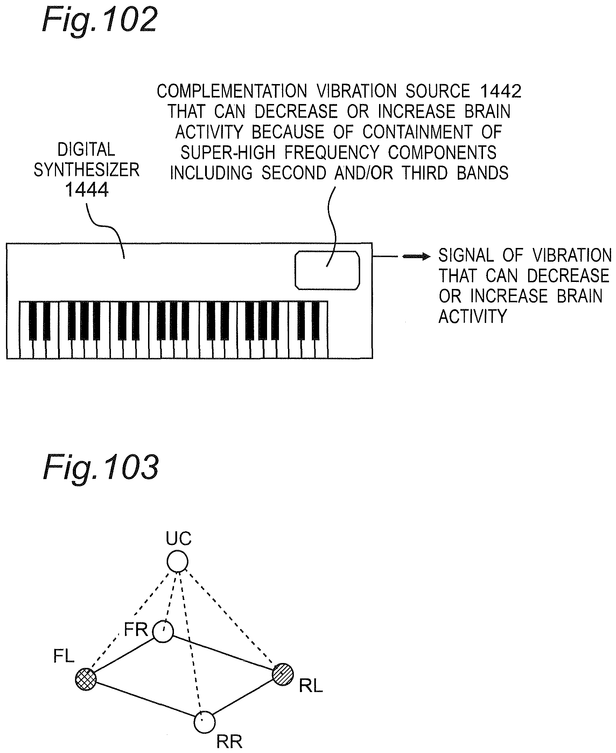

Figure 102 is a perspective view showing an example of an electronic musical instrument apparatus including a vibration complementing apparatus that adds a super-high frequency vibration signal including the second and/or the third band to an original vibration that does not induce the effect of increasing or decreasing the brain activity generated by playing the electronic musical instrument according to an application example 33. The electronic musical instrument such as the current digital synthesizer 1444 uses a digital format that can neither record nor reproduce super-high frequency components, and therefore, no super-high frequency components are included in the vibration of the performance sound, so that the effect of increasing or decreasing the brain activity cannot be induced. Accordingly, referring to Figure 102, the vibration complementing apparatus uses the vibration signal of the performance sound of the electronic musical instrument as an original vibration signal, adds a vibration signal capable of decreasing or increasing the brain activity read from a complementary vibration source 1442 to it by an adder (not shown), and outputs the signal of the vibration that induces the effect of increasing or decreasing the brain activity because of the containment of the super-high frequency components including the second and/or the third band. Although the vibration complementing apparatus is built in the digital synthesizer 1444 that is the electronic musical instrument of Figure 102, it may be externally attached or existing independently of the electronic musical instrument without being limited to this. Moreover, the complementary vibration source 1442 may have various kinds of built-in storage apparatuses such as a solid memory in which the vibration signal capable of decreasing or increasing the brain activity is recorded or be supplied by communications of a vibration signal capable of decreasing or increasing the brain activity synthesized by an analog synthesizer or the like. Although the digital synthesizer 1444 is taken as an example above, vibration signals of the performance sounds of other electronic musical instruments, karaoke systems and the like besides this can be similarly complemented with the vibration signal capable of decreasing or increasing the brain activity. Otherwise, it is also possible to make an electrical signal of the vibration of the performance sound of an acoustic musical instrument by a microphone or the like and complement it with a vibration signal capable of decreasing or increasing the brain activity. Further, in the so-called PA (public address) that once signalizes the performance of a musical instrument group, singing or the like in a concert hall or the like and reproduces it, complementation with a vibration signal capable of decreasing or increasing the brain activity can be similarly effected by the vibration complementing apparatus.

A front left loudspeaker FL and a rear left loudspeaker RL are located on the same left-hand side in the normal 4-channel surround loudspeaker arrangement according to the prior art, it becomes like the application example 34 of Figure 103 in the case of a double helical matrix arrangement. Referring to Figure 103 of the application example 34, in contrast to the front left loudspeaker FL located on the left-hand side, the rear left loudspeaker RL is located on the right-hand side. With this arrangement, a person who is in this space is to face the sound on the left-hand side and the sound on the right-hand side whichever direction of the four directions the person faces. Moreover, the person is to listen to the sounds of all the five channels. It is also the feature of the double helical matrix that stereophonic effects and continuity are achieved by adding an upper middle loudspeaker UC.

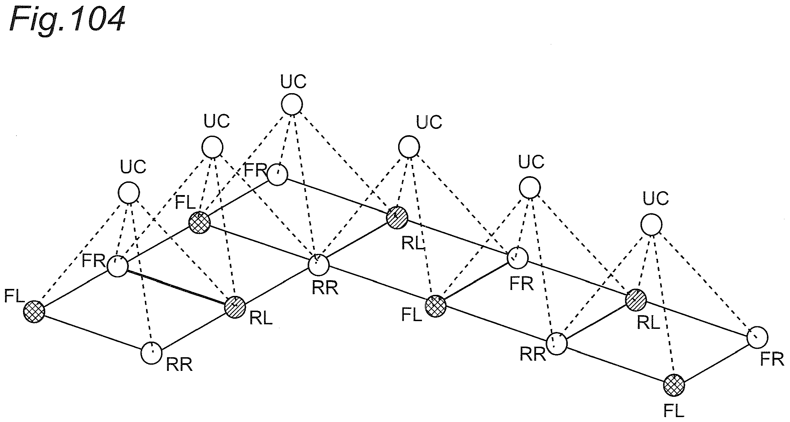

Figure 104 shows a case where the double helical matrixes are consecutively iteratively arranged in two directions. In the loudspeaker arrangement of Figure 104, a person in this space is to consistently face the sound on the left-hand side and the sound on the right-hand side and listen to the sounds of all the five channels. Further, referring to Figure 104, the sound on the left-hand side and the sound on the right-hand side intertwine with each other, and the row of the left-hand loudspeakers and the row of the right-hand loudspeakers are each helicoidal iterating the front side, the rear side, the front side, the rear side, ...

Next, a loudspeaker arrangement using the six-dimension consecutive matrix coordination method is described.

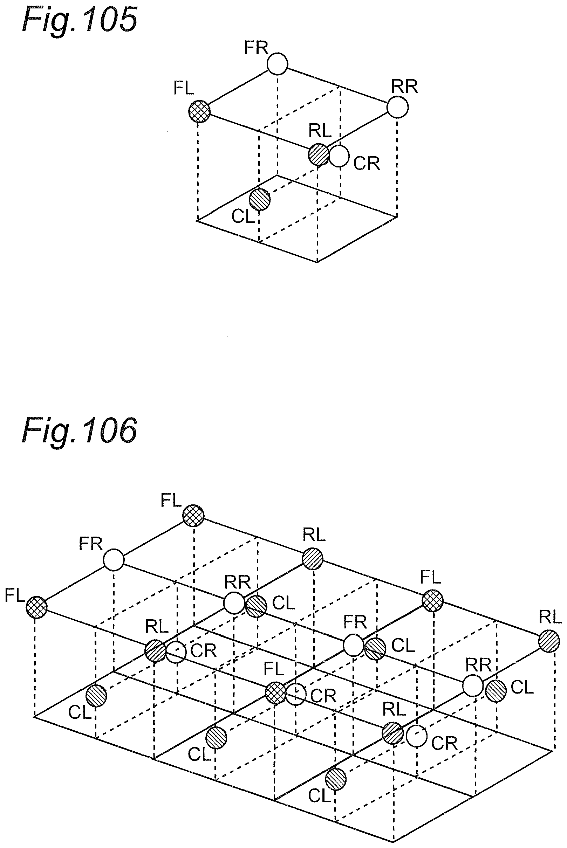

The prior art 4-channel surround loudspeaker arrangement is lifted up to a predetermined height as shown in Figure 105 of an application example 36. Then, channels of sounds located between the front side and the rear side are added, and the loudspeakers are served as a center left loudspeaker CL and a center right loudspeaker CR. These center left loudspeaker CL and the center right loudspeaker CR are placed on the ground or at a height slightly higher than the ground. The loudspeaker arrangement of Figure 105 is referred to as the matrix in the present implemental example. In this case, it is acceptable to use a modified arrangement such that FL, FR, RL, and RR are placed at a height slightly higher than the ground and CL and CR are placed upside.

When the matrixes of Figure 105 are arranged consecutively, iteratively, and bidirectionally in the vertical and horizontal directions, the resulting arrangement becomes as shown in Figure 106 according to an application example 37. Referring to Figure 106, since there are the left sound column and the right sound column in any of the matrixes, the sound field is formed so as to be felt normal. Moreover, the front sound and the rear sound appear alternately. Further, since there is a center sound that interlinks the front sound with the rear sound, a continuous space can be felt.

It is also acceptable to reproduce only the audible range components of a vibration by using the double helical matrix coordination method, the six-dimension consecutive matrix coordination method or the like and reproduce the super-high frequency components exceeding the human audible frequency upper limit in a stereophonic reproduction or monaural reproduction manner.

Next, an implemental example according to a network application to transmit and distribute a vibration signal including the second and/or the third band is described. An apparatus that generates a vibration capable of inducing a hypersonic positive effect by using portable communication equipment such as a portable telephone and portable broadcasting receiver equipment, a portable music player such as iPod (registered trademark) and a portable video player, a portable game machine, a personal computer and a television set connectable to a network such as the Internet or LAN, AV equipment, electronic equipment or the like including a video deck connected to it by utilizing the network is described below.

In the modern society, portable communication equipment (portable telephone, information terminal using wireless IP communication, infra-red communication, etc., transceiver, intercom etc.), portable broadcasting receiver equipment (one-segment broadcasting receiver etc.), portable music players (iPod (registered trademark), Walkman (registered trademark), etc.), portable video players, portable game machines and the like are explosively popularized. In addition, the frequency of use is increased, and a style of long-time watching is increased. Many of the equipment are carried and used for a long time under a condition very close to the human body, and therefore, an influence that they exert on the body and spirit of the carrier person cannot be ignored. What is problematic in this case is that an acoustic vibration generated by the portable equipment does not contain the super-high frequency components of the third band and is constituted of only the audible range components, and therefore, it is highly possible that the activity of the fundamental brain is lowered by comparison to the normal background noise state. Using the portable equipment has the problem that not only the health of the modern people is threatened but also unpleasant and irritating feelings are induced, inducing to stress reactions such as an increase in adrenaline concentration and an increase in the risk of triggering violence and abnormal behaviors.

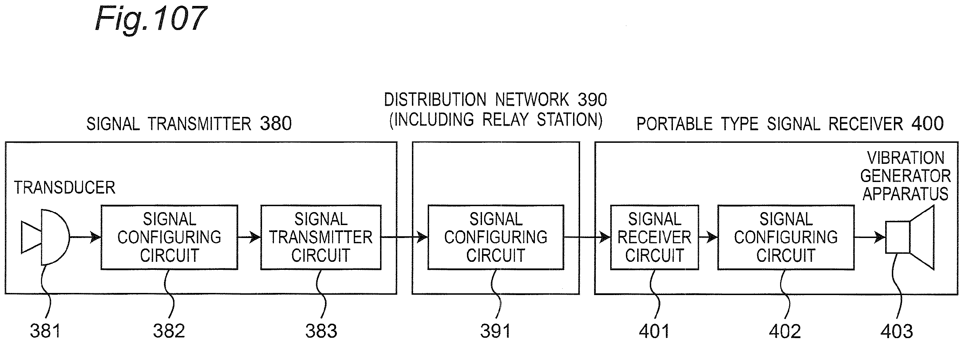

A drastic method for solving this problem is to provide all the relevant equipment with the functions to appropriately generate, transmit, transfer and receive a vibration signal capable of inducing the fundamental brain activation effect because of the inclusion of the third band and to make it possible to generate them as an actual vibration in the portable equipment in the system as shown in Figure 107 of an application example 38.

In concrete, first of all, a signal transmitter 380 is made to have a function to reconfigure a signal of a vibration capable of inducing the fundamental brain activation effect because of the containment of the super-high frequency components because of the third band by performing appropriate signal processing by a signal reconfiguring circuit 382, and to transmit the signal by a signal transmitter circuit 383 for a case where the vibration signal does not contain the super-high frequency components of the third band or a case where the second band vibration components are included together with a function to transmit an acoustic vibration by converting it to a vibration signal faithfully to the super-high frequency components.

Next, an apparatus or a network to transmit a vibration signal is made to have a function to reconfigure a transmission signal into a vibration signal including the third band in, for example, the signal reconfiguring circuit 391 by performing appropriate signal processing by a relay station such as a telephone exchange office and a broadcasting station or by a server on a net for a case where the vibration signal that becomes a transmission material does not contain the super-high frequency components of the third band in addition to the function to perform transmission faithfully to the super-high frequency components. In this case, the network for transmission may be not only communications and broadcasting intended for a large area but also a LAN, a ubiquitous network, inter-equipment communications or the like in a specified space or region of a home, a company, a premise or the like.

Finally, a portable signal receiver 400 includes a signal receiver circuit 401, a signal reconfiguring circuit 402, and a vibration generator apparatus 403. The portable signal receiver 400 is made to have a function to reconfigure a vibration signal including the third band by performing appropriate signal processing in the receiver itself and thereafter convert the signal into an actual vibration generated for a case where the received vibration signal does not contain the super-high frequency components of the third band in addition to a function to faithfully receive the transmitted vibration signal and convert it into an actual vibration.

With these arrangements, the person who carries and uses portable equipment becomes able to receive the vibration including the first and third bands. In addition to the fact that the hypersonic negative effect including a decrease in the fundamental brain activity can be prevented securing safety, the hypersonic positive effect that is the affirmative effect of ameliorating and improving the psychophysical conditions through the activation of the fundamental brain and the fundamental brain network (fundamental brain network system) is obtained.

However, it is difficult in the equipment and the function relevant to the transmission and transfer of the vibration signal to immediately achieve a drastic problem solving method as described above. Accordingly, only the portable signal receiver is made to have an appropriate signal processing function and a vibration generating function as a problem solving method having an immediate effect and high feasibility. With this arrangement, it becomes possible to generate a vibration including the third band in the portable signal receiver even though there are limitations on the equipment and functions related to the transmission and transfer and merely the vibration signal constituted of only the audible range components that lack super-high frequency components can be received.

A concrete method for making the portable signal receiver have an appropriate signal processing function is described below.

A vibration signal including the third band is preparatorily stored in a memory or the like provided inside, for example, a portable signal receiver, and the received signal of the audible sound is complemented with the signal of a vibration including the third band. The signal to be complemented may be provided not only by using the one stored in the memory but also inputted or received from the outside of the portable signal receiver or generated inside the portable signal receiver. Moreover, the level of the vibration signal to be complemented can be automatically changed in correlation with the level of the received audible sound or arbitrarily adjusted by the user of the portable equipment.

It is noted that the received signal of the audible sound and the signal of the vibration including the third band may either be provided by generating an actual vibration by an identical vibration generating mechanism or by generating an actual vibration by independent separate vibration generating mechanisms.

Although the portable equipment has been described here as an example of the equipment connected to the network, the present implemental example can be applied to all the AV equipment and electronic equipment including personal computers and television sets connectable to a network and video decks connected to them. Moreover, although the example for presenting the vibration including the third band has been described here, the same thing can be said for the vibration including the second band.

### REFERENCE IMPLEMENTAL EXAMPLE 1.

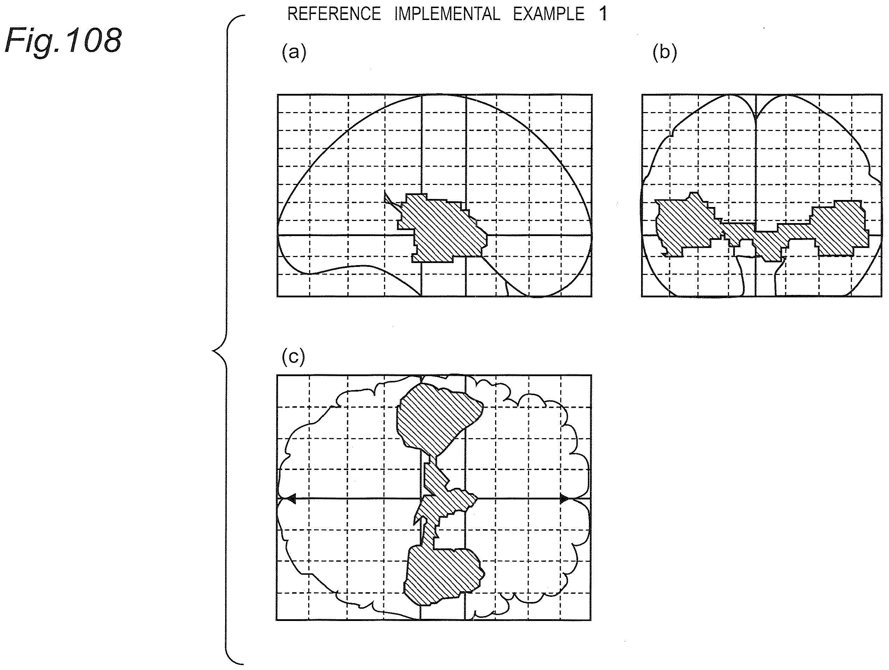

Figure 108 is a projection view of the experiment results (reference implemental example 1) of the head portion of a test human subject of a PET apparatus when the second band and part of the third band are combined with the first band and simultaneously applied measured in the embodiment 3. Figure 108(a) is a sagittal projection view, Figure 108(b) is a coronal projection view, and Figure 108(c) is a horizontal plane projection view. Reference experiment data to ensure the operational effects of the band combination according to the embodiment 3 is shown described in a reference implemental example 1.

In the reference implemental example 1, the gamelan music was used as a vibration source, and the vibration of the first band (equal to or lower than 16 kHz in the present experiment), the vibration of the second band (16 kHz to 32 kHz in the present experiment) and the vibration of the third band (equal to or higher than 32 kHz in the present experiment) were simultaneously presented to the test human subject. As a result, the cerebral blood flow of the fundamental brain of the brain stem, thalamus and the like and the auditory area increased by comparison to when the vibration was not in particular presented. The "negative effect" inducing a decrease in the brain activity and the "positive effect" inducing an increase in the brain activity coexisted in the vibration presented by this experiment, and it was considered that the ratio of the third band components inducing the "positive effect" was relatively large, and the brain activity increased for the reason.

### REFERENCE IMPLEMENTAL EXAMPLE 2.

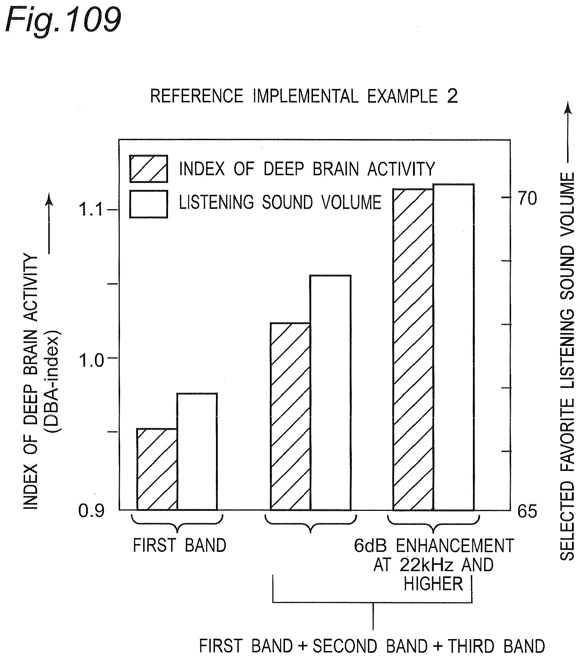

Figure 109 is the results of the behavioral experiment and the electroencephalogram experiment (reference implemental example 2) measured in the embodiment 4 showing a deep index of brain activity (DBA-index) with respect to each band. Reference experiment data to ensure negative and positive intensity changes according to the embodiment 4 is described here in the reference implemental example 2.

In the reference implemental example 2, the gamelan music was used as a vibration source, and the following three kinds of vibrations were presented to the test human subject.
(Vibration A): A vibration including only the first band (equal to or lower than 16 kHz in the present experiment) and part of the second band (16 kHz to 22 kHz in the present experiment).
(Vibration B): A vibration including the first band (equal to or lower than 16 kHz in the present experiment), the second band (16 kHz to 22 kHz in the present experiment) and the third band (equal to or higher than 32 kHz in the present experiment).
(Vibration C): A vibration obtained by enhancing the signal intensity of a partial band though the band width is the same as that of the (vibration B). In other words, it is a vibration presented by enhancing the signals of part of the second band (22 kHz to 32 kHz in the present experiment) and the third band (equal to or higher than 32 kHz in the present experiment) by 6 dB.

In the present reference implemental example 2, the test human subject adjusted the level to his or her favorite level of each vibration, and the final adjustment result was assumed to be the "comfortable listening level". Moreover, the electroencephalogram of the test human subject was measured, and the deep index of brain activity (DBA-index) was obtained. As a result, the "comfortable listening level" considered to reflect the activation of the emotion system nerve circuit and the deep index of brain activity (DBA-index) were proportional to each other, exhibiting high values in the order of (vibration A) < (vibration B) < (vibration C). It was considered that the result: (vibration A) < (vibration B) was ascribed to the fact that the ratio of the signal of the third band having the "positive effect" was relatively large compared to the second band having the "negative effect". Moreover, it was considered that the result: (vibration B) < (vibration C) was ascribed to the fact that the "positive effect" was induced relatively strongly since the entire band of the third band having the "positive effect" was enhanced in contrast to the fact that the second band having the "negative effect" is turned only partially enhanced.

### REFERENCE IMPLEMENTAL EXAMPLE 3.

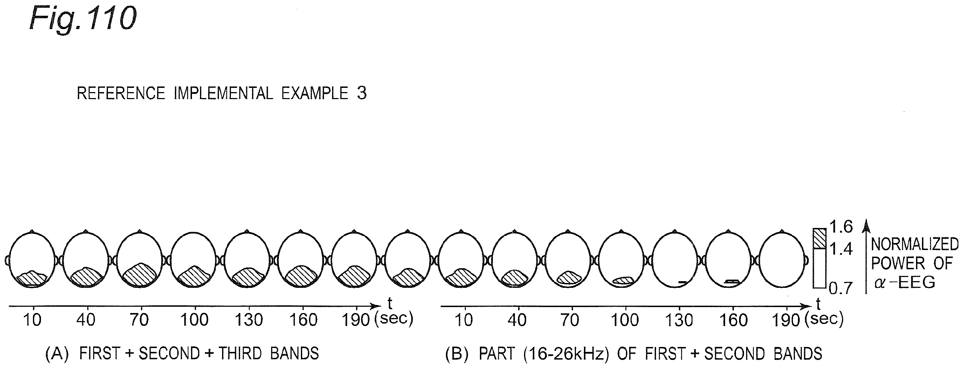

Figure 110 is a graph showing an electroencephalogram experiment results (reference implemental example 3) measured in the embodiment 6, or a time series variation of a head portion horizontal plane projection view of the normalized power of α-EEG of not smaller than a predetermined value. That is, reference experiment data that ensures the changeover presentation is shown in a reference implemental example 3. In the present experiment, the gamelan music that contained abundant high-frequency components and had a predetermined autocorrelation order structure (See the Patent Documents 7 and 9) was used as a vibration source. First of all, a vibration including (A) the first band (equal to or lower than 16 kHz in the present experiment), the second band (16 kHz to 32 kHz in the present experiment) and the third band (equal to or higher than 32 kHz in the present experiment) was first presented for 200 seconds. Next, (B) a vibration including the first band (equal to or lower than 16 kHz in the present experiment) and part of the second band (16 kHz to 26 kHz in the present experiment) was presented for 200 seconds. Then, as shown in Figure 52, the components of the brain wave α wave reflecting the activity of the brain was displayed in a topography every 30 seconds. As a result, as apparent from Figure 52, the brain activity increased in (A), and subsequently the brain activity relatively decreased in (B). It is noted that the Z score is a relative value of the α2 band component intensity of the head portion of the test human subject.

### MODIFIED EMBODIMENTS

Although the vibration is presented to a human being or the like in the above embodiments and implemental examples, the vibration may be presented to a living body such as an animal other than the human being.

Regarding the above embodiments, implemental examples and application examples, they may be configured partially or totally combined together.

In the above embodiments, the first band, the second band and the third band have the respective specified bandwidths. Although the first band is used here, the present invention is not limited to this, and the first band may be at least part of the band of equal to or lower than 20 kHz. Moreover, although the second band is used, the present invention is not limited to this, and the second band may be a band that includes at least the band of 16 kHz to 32 kHz and at least part of the band from 16 to 32 kHz, 40 kHz or 48 kHz. Further, although the third band is used, the present invention is not limited to this, and the third band may be at least part of a band of equal to or higher than the second band (the maximum frequency of the band may be one of the frequencies of 96 kHz, 112 kHz to 192 kHz and so on).

### INDUSTRIAL APPLICABILITY

As described in detail above, the present inventor and others discovered the principle that the brain activity inside and outside the fundamental brain was increased or decreased and controlled at various levels by applying with various temporal organizations the super-high frequency vibration having various frequency bands and intensities that are not perceived as a sound by human beings while applying an aerial vibration including the frequency band perceived as a sound by human beings from the auditory system. In particular, it was discovered that the positive effect of increasing the brain activity was produced when the vibration having the third band was applied to a living body while the negative effect of decreasing the brain activity was produced when the vibration having the second band was applied to the living body.

The apparatus, method, space and so on to remove or attenuate the vibration structure of the second frequency band that induces a decrease in the brain activity have the effects of removing the negative influence including the causes of the modern diseases, securing safety, and increasing the pleasant sensation and aesthetic sensitivity by removing or attenuating the specific vibration structure that decreases the brain activity from various environmental sounds including traffic noises and artificial sounds generated by electronic equipment, signals recorded in media, and voices, music signals, their reproduced sounds and so on distributed or transmitted by broadcasting or communications, and this makes it possible to develop a variety of applications.

In particular, the majority of the audio digital media such as CDs and DVDs that are currently widely used have the recordable and reproducible frequency band limited to the first band of the audible range components and part of the second band having the negative effect. Therefore, exposure to the sounds reproduced by such audio digital media for a long time has the risk of decreasing the brain activity and causing a serious pathological state. Accordingly, by band converting the second band having the negative effect into the third band components having the positive effect by the present invention, it becomes possible to increase the brain activity while utilizing the audio digital media that are currently widely used and to produce the effects of removing the negative influence including the causes of the modern diseases and increasing the pleasant sensation and aesthetic sensitivity.

Furthermore, the majority of the signal transfer means such as digital broadcasting that are currently widely popularized have the transmittable frequency band limited to the first band of the audible range components and part of the second band having the negative effect, and unable to transmit the aforementioned third band components having the positive effect. Accordingly, by encoding the third band components having the positive effect into the frequency band transmittable by the transmission means on the signal transmission side, and decoding the above-mentioned components into the third band components having the positive effect on the receiving side of the signal by the present invention, it becomes possible to increase the brain activity while utilizing the signal transmission means that are currently widely put to practical use.

As described above, it is possible to inexpensively provide a broadcasting or communication system that can secure the safety of the listener without drastically reviewing the hardware regarding the musics and voices of the digital broadcasting and communications in the current state in which the listener's brain activity might be decreased as an utilization for the broadcasting industry or the communication industry. Further, contents that effectively improve the artistic effects can be produced as an utilization for the contents industry, and contributions to the society can be achieved in the aspect of safety and health by settling and popularizing the next generation format capable of recording and reproducing sufficiently to the first, second and third bands.

Moreover, the vibration in the frequency band that induces the effect of raising the brain activity and the apparatus, method, space and so on to present it through enhancement can be applied to the medical industry without modification as remedial and preventive means of safety and high comfort free of side effects without using medicines for the pathology induced by a decrease in the brain activity.

In addition, the vibration in the frequency band that induces the effect of raising the activity of the emotion system nerve circuit of the brain and the apparatus, method, space and so on to present it can be applied as effective means for exalting the reactions of pleasure, beauty and emotion to the artistic productions including musics and increasing the expressive effects. Furthermore, the effects of making the environments of living spaces, duty spaces, amusement spaces, public spaces, vehicles and so on comfortable are induced, and therefore, they can be developed as applications of environment setting techniques in various industrial fields.

The vibration applied in the present invention, which is processed as information inputted to the brain, therefore has no irreversible invasiveness to the structure and function of the nerve circuit of the brain. Moreover, by comparison to the technique of controlling the brain activity by medication or the like, the risk of inducing side effects can be suppressed extremely low. By using this invention, it becomes possible to control a decrease and an increase in the brain activity promptly, safely, arbitrarily and reversibly without using any craniotomy procedure, medicine administration, electromagnetic stimulations, operant conditioning or the like, which have the risk of exerting unrecoverable infringement on the structure and functions of the brain.

By applying this, a model that has the pathological state of the brain safely and effectively and a remedial and preventive model therefor can be produced. For example, by presenting the vibration of a specific frequency band that induces the effect of decreasing the brain activity to a healthy test human subject, it becomes possible to decrease the activity of a specific brain region within several minutes and to form a pathological state model. Moreover, it is possible to reverse the activity and turn it to an increase within several minutes if the vibration of another frequency band that induces the effect of increasing the brain activity is subsequently presented. Therefore, it is also possible to induce both the pathological state in which the brain activity is decreased and the healthy state in which the brain activity is increased and the mental and physical conditions are improved in a short time in an identical test human subject.

Furthermore, by utilizing this method, even in a case where an experiment that might cause a decrease in the brain activity of the test human subject is performed, if the vibration of the frequency band having the effect of increasing the brain activity is presented in advance, and experiment is enforced in a state in which the baseline of the brain activity of the test human subject is increased, it becomes possible to eventually maintain the level of brain activity to a definite level at which no adverse effect is exerted on the spirit and body even if a relative decrease in the brain activity is induced by the experiment. Accordingly, there is the advantage that the experiment can be performed with a safety secured without inducing actual harms on the aspect of health. In addition, by utilizing these methods, the effects of preventing various mental and physical diseases attributed to a decrease in the brain activity can be expected.

This method, which also has the important advantage that it is safe and effective for human beings, is able to make a pathological model and its remedial and preventive model promptly, reversibly and efficiently even when an experiment using animals other than human beings is performed, and is therefore extremely effective.

As described above, according to the present invention, it becomes possible to make a pathological model and a remedial and preventive model therefor intended for human beings and animals other than human beings extremely safely and efficiently, and to produce high effectiveness for a lot of clinical studies aimed at developing new remedial methods and the curative medicines.

Furthermore, by variously decreasing or increasing the activity of the emotion system nerve circuit that strongly controls human beings and their actions, it becomes possible to induce an action to approach a specific object and stimulation, and an action to conversely avoid a specific object and stimulation, and to guide human beings and animals other than human beings so that they can choose safe and appropriate actions according to situations. These effects can be variously applied as techniques to secure safety and making the environment comfortable by appropriately guiding the residents in terms of disaster prevention, configuration, and city planning industries.

Further, by variously decreasing or increasing the activity of the emotion system nerve circuit that strongly controls the actions of animals other than human beings in the stock raising industry or the like, it becomes possible to induce an action to approach a specific object and stimulation and an action to conversely avoid a specific object and stimulation in the objective animals. These effects can be variously applied in controlling the behaviors of the objective animals in the stock raising industry.

### REFERENCE NUMERALS

1a, 1b, 1c, 1d, 1e, 1f, 1g, 1h: signal generator apparatus
1A, 1aA, 1bA, 1cA, 1dA, 1eA, 1fA, 1gA, 1hA, 11A, 11B, 21A: recording medium
1C: vibration signal synthesizing apparatus
1D: vibration generator apparatus
2, 2a, 2b, 2c, 2d, 2e, 2f, 2g, 2h: reproducer circuit
2A: signal transducer apparatus
3: amplifier circuit
4: vibration presenter
5: adder
6: band-eliminate filter (BEF)
11: vibration generator apparatus
12: reproducer circuit
13: low-pass filter (LPF)
13A: band-eliminate filter (BEF)
15: signal transducer apparatus
16: phase inverter circuit
17: band-pass filter (BPF)
18: equalizer
21: vibration generator apparatus
22: reproducer circuit
23, 33: band-pass filter (BPF)
50: controller
60, 60A: processing equalizer
61: low-pass filter (LPF)
62: band-pass filter (BPF)
63: high-pass filter (HPF)
64: attenuator
65: amplifier circuit
66: adder
80, 80A: encoder apparatus
81: low-pass filter (LPF)
82, 82A: high-pass filter (HPF)
83, 83A: band shift circuit
84: adder
85, 85A: FFT circuit
86, 86A: inverse FFT circuit
86B: adder
87, 88: down-sampling circuit
89: signal transmitter apparatus
90, 90A: decoder apparatus
91: low-pass filter (LPF)
92: high-pass filter (HPF)
93: band shift circuit
94, 95: D/A converter (DAC)
96: adder
97: FFT circuit
98: inverse FFT circuit
101, 103: up-sampling circuit
102: down-sampling circuit
104, 105: D/A converter (DAC)
111: headphone
111a, 111b : headphone housing
112: head strap
115: signal band divider circuit
116, 117: signal amplifier
118: signal input plug
120: super-high frequency vibration generating device
121: audible range loudspeaker
124: ear pad
125: small battery
131: memory
132: microamplifier
133: battery
160: brooch type vibration presenting apparatus
161: battery socket cover
162: memory socket cover
163: clasp fastening portion
170: flat plate
171, 171a: liquid current generator
172, 172a-172i: projection
173, 174, 175: transducer
200: listener
200a: head portion
200b: cap
200c: fundamental brain
201: electroencephalogram signal wireless transmitter apparatus
201A: cap type PET apparatus
201a: antenna
202: electroencephalogram signal wireless receiver apparatus
204: active processing equalizer
204a: controller
205: amplifier circuit
206: vibration presenting apparatus
207: PET signal analyzer apparatus
208: vibration source storage apparatus
210: event site
210A, 210B, 210C: region
211, 211A, 211B, 211C: video camera
220, 220A, 220B, 220C: feedback type vibration presenting apparatus
230: head-count analyzing and vibration control apparatus
230A: controller
230B: multidimensional head-count analyzing and vibration control apparatus
231: intra-visual field head-counting apparatus
233: derivation-deserved effect determining apparatus
234: individual band vibration power calculating apparatus
300: signal source disc
301: player
302: preamplifier
310: left channel circuit
311: high-pass filter (HPF)
312: low-pass filter (LPF)
313, 313a, 313b: earphone amplifier
314, 314a, 314b: power amplifier
320: right channel circuit
330: loudspeaker system
331: tweeter
332: full-range loudspeaker
333: woofer
334, 334a, 334b: earphone
335: power distribution network
340: listener
341: listener's head portion
370: vibration generator apparatus
371, 372, 373: super-high frequency vibration generating device
374: cable with super-high frequency vibration generating device
375: memory of vibration source
376: amplifier unit
377: power supply unit
401: vibration presenting apparatus
402: super-high frequency vibration monitor apparatus
403: super-high frequency vibration sensor
404: super-high frequency vibration presentation state display apparatus
410: frame
411, 411a: vibration presenting apparatus
412: super-high frequency vibration sensor
413: bolt and nut
414a, 414b: network circuit
415: super-high frequency vibration presentation state display apparatus
416: rectifier
417: low-voltage module
418: accumulator battery
420: frame
421: capacitor
422: amplifier circuit
423: high-pass filter
424: band-pass filter
431, 433: light-emitting diode
430: module
441: vibration presenting apparatus operation state judgment apparatus
442: super-high frequency vibration presentation state recording apparatus
443: power source
451: super-high frequency vibration sensor
452: super-high frequency vibration presenting apparatus
453: middle range vibration presenting apparatus
454: low range vibration presenting apparatus
460: vibrating wall
461: listener
470: vibration signal reproducing apparatus
470d: recording medium
471: vibration signal amplifier
472: public-address system
473: microphone
474: vibration signal addition adjuster
475: microphone
476: vibration measuring instrument
480: station yard
481: pillar mounted type vibration generator apparatus
482: signal receiver
483: super-high frequency vibration signal receiver
484: loudspeaker (public-address system)
485: vibration generator apparatus
485m: memory
486: vibration presenting apparatus to generate vibration including first and third bands
487: loudspeaker (public-address system)
488: human being
490: car
491: vibration presenting apparatus
501: gamelan
502: microphone
503: preamplifier
504: A/D converter
505: D/A converter
506: reproduction amplifier
507a: high-pass filter (HPF)
507b: low-pass filter (LPF)
508a, 508b: power amplifier
509aa, 509ba: right loudspeaker
509ab, 509bb: left loudspeaker
509ca: right earphone
509cb: left earphone
509caa: high frequency signal sound generating part of right earphone 9ca
509cba: low frequency signal sound generating part of right earphone 9ca
509cab: high frequency signal sound generating part of left earphone 9cb
509cbb: low frequency signal sound generating part of left earphone 9cb
510: magnetic recording and reproducing apparatus
511: magnetic recording part
512: magnetic recording head
513: magnetic tape
514: magnetic reproducing head
515: magnetic reproducing part
520, 520a: room
530: human being
531: electroencephalogram data recording apparatus
532: electroencephalogram transmitter apparatus
533, 34: antenna
541: tomograph apparatus
542: tomographic detector apparatus
560: vibration generating space
561: sound source
562: chair
563: listener
570: vibration generating space
571, 572: vibration generator apparatus
581, 582, 584: amplifier circuit
583: adder
610: CD player
611: signal complementing apparatus
612: amplifier
613: loudspeaker
614: AV apparatus
615: network
616: server apparatus
620: portable player
621: signal complementing apparatus
622: earphone
623: super-high frequency vibrating body
624: listener
630: television receiver
631: signal complementing apparatus
632: loudspeaker
641, 643: reproducer circuit
642: band extending circuit
644: adder
645: high-pass filter
674: A/D converter
675: active processing circuit
675a: convolution calculator
675b: autocorrelation coefficient controller
676: autocorrelation coefficient calculator
677: reproducer circuit
695: Super Audio CD (SACD)
696: SACD player
697: low-pass filter
698, 699: high-pass filter
800, 800a, 800b, 800c: super-high frequency vibration presenting apparatus
812: listener
812a: body surface
830p: pendant type vibration presenting apparatus
832A: vibration generator apparatus
832a: skin contact type super-high frequency transducer
833: microamplifier
834: memory
835: battery
850: portable music player
851: headphone
852: display
853: Blu-ray Disc
854: Blu-ray Disc player
855: AV amplifier
856: 5.1-channnel surround loudspeaker system
860: super-high frequency vibration presenting apparatus
860S: signal generator apparatus
860C: bathtub
860L: liquid
870: audible sound loudspeaker
870A: full-range loudspeaker
871: super-tweeter
900: audible range vibration presenting apparatus
900a: headphone
952: sauna type super-high frequency vibration presenting apparatus
952a: super-high frequency transducer
954: cockpit of aircraft or the like
954a-954d: super-high frequency vibration presenting apparatus
955: super-high frequency vibration shower room
955a: shower type super-high frequency vibration presenting apparatus
961: vibration complementing apparatus
962: detecting and presenting apparatus
962a: vibration presenting apparatus
962b: premises sound detecting apparatus
1200: signal reproducing apparatus
1210: shirt
1410: portable telephone
1411: loudspeaker
1412: casing
1413: sheet
1414: super-high frequency vibration generating device
1415: headset
1416: cable
1417: super-high frequency vibration generating device
1418: piezoplastic sheath
1419: vibration generator apparatus
1420: portable music player
1420m: memory
1421: earphone
1422: cable
1423: vibration generator apparatus
1430: concert hall
1431: stage
1432: wireless vibration signal transmitter
1433: wireless vibration signal receiver and vibration presenting apparatus
1434: pendant type vibration presenting apparatus
1435: ceiling hanged type vibration presenting apparatus
1436: chair mounted type vibration presenting apparatus
1437: chair embedded type vibration presenting apparatus
1442: interpolation vibration source
1444: digital synthesizer
2001: PET measuring chamber
2010A: PET measuring apparatus
2011: bed
2012: test human subject
2911: microphone
2912: microamplifier
2913: sound structure information analyzer apparatus
2913a: sound structure information analyzing part
2914: degree of risk judgment apparatus
2915: analysis result monitor apparatus
2916: alarm generator
2917: self-diagnosis apparatus
2918: self-restoration apparatus
2920: electroencephalogram derivation apparatus
2921: transmitter
2922: receiver
2930: sound structure information analyzing and monitoring apparatus
2931: sound structure display monitor
2940: deep brain activity information analyzing and imaging apparatus
2941: deep brain activation analyzing part
2942: deep brain activation display monitor
2943: feedback part
2950: reproducing apparatus
3501: portable player
3502: built-in actuator
3503, 504, 505: external actuator
3506: earphone
3507: user
3508A: built-in loudspeaker
3508B: external loudspeaker
3509: microphone
3510: controller
3511: signal amplifier
3512: HS signal
3513: D/A converter
3514: portable telephone wireless communication circuit
3514A: antenna
3515: Wi-Fi wireless communication circuit
3515A: antenna
3516: signal register
3517: D/A converter
3518: signal memory
3519: D/A converter
3520: VCA circuit
3531, 3532: low-pass filter
3540: mixer circuit
3541: signal amplifier
3551: keyboard
3552: liquid crystal display
3553: USB interface
3554: USB external equipment
3560: HS signal discriminating circuit
3561: HS signal level detector circuit
4090: human being (listener)
4091: chair
4092: vibration generating device
4500: vibration monitoring system
4501: vibration generator apparatus
4502: vibration signal input apparatus
4503: vibration discriminating apparatus
4504: discrimination result based control signal generator apparatus
4505: discrimination result monitor apparatus
4506: alarm generator
4507: vibration complementing apparatus
SW1, SW2, SW11, SW12, SW13, SW14: switch

## Claims

1. A vibration processing apparatus comprising generating means (1, 11, 21) configured to generate a vibration or an oscillation signal, that includes
a first frequency band having a frequency range of lower than 16 kHz and including vibration components perceived as sound by an auditory system of a living body,
a second frequency band having a frequency range of 16 kHz to 32 kHz, including vibration components exhibiting an effect of lowering brain activity, and
a third frequency band having a frequency range of higher than 32 kHz, wherein
the vibration processing apparatus further comprises vibration attenuator apparatuses configured to receive the vibration or the oscillation signal from the generating means (1, 11, 21), **characterized in that**
the vibration attenuator apparatuses are configured to remove or attenuate only the second band from the vibration or the oscillation signal by band-filtering only the second band from the vibration or the oscillation signal, such that the negative effect of decreasing the brain activity of a living body is reduced, and.
further comprising vibration presenting means (4) configured to apply the vibration obtained by the generation means (1, 11, 21) to the living body.

2. The vibration processing apparatus as claimed in claim 1, wherein the vibration presenting means (4) are configured to apply at least one of a vibration of the second frequency band and a vibration of the third frequency band to the living body together with a vibration of the first frequency band, to present the vibrations for setting degree of decrease or degree of increase of the deep index of brain activity level of the living body to a predetermined value.

3. The vibration processing apparatus as claimed in claim 1, wherein the vibration presenting means (4) are configured to change an intensity of at least one of the vibration of the second frequency band applied to the living body and the vibration of the third frequency band applied to the living body, to present the vibrations for setting degree of decrease or degree of increase of the deep index of brain activity level of the living body to a predetermined value.

4. The vibration processing apparatus as claimed in claim 1, wherein the vibration presenting means (4) are configured to apply the vibration of the second frequency band to the living body together with the vibration of the first frequency band, and thereafter, apply the vibration of the third frequency band to the living body together with the vibration of the first frequency band.

5. The vibration processing apparatus as claimed in claim 1, wherein the vibration presenting means (4) are configured to apply the vibration of the third frequency band to the living body together with the vibration of the first frequency band, and thereafter, apply the vibration of the second frequency band to the living body together with the vibration of the first frequency band.

6. The vibration processing apparatus as claimed in any one of claims 1 to 5, further comprising a signal processing apparatus (642) configured to band-convert or band-expand a signal representing the vibration of the second frequency band into a signal representing the vibration of the third frequency band.

7. The vibration processing apparatus as claimed in any one of claims 1 to 5, further comprising: an encoder apparatus (80) configured to band-convert or band compress a signal representing the vibration of the third frequency band into a signal representing the vibration of the second frequency band, and thereafter, add a signal representing the vibration of the first frequency band to a processed signal to output a signal of addition result as a transmission signal; and a decoder apparatus configured to receive the transmission signal, (a) band-convert a signal having the second frequency band into a signal having the third frequency band from among the transmission signal when the transmission signal is band-converted, and (b) band-expand a signal having the second frequency band into a signal having the third frequency band from among the transmission signal when the transmission signal is band-compressed, and thereafter, add the signal having the first fre-quency band from among the transmission signal to a processed signal to output a signal of addition result.

8. The vibration processing apparatus as claimed in any one of 1 to 5, further comprising means configured to detect a vibration level of at least one of the vibration of the second frequency band and the vibration of the third frequency band.

9. The vibration processing apparatus as claimed in any one of claims 1 to 5 wherein the vibration presenting means (4) is configured to present the vibration for setting a degree of decrease or a degree of increase of the deep index of brain activity level of the living body to a predetermined value, based on a control signal from an external apparatus.

10. The vibration processing apparatus as claimed in claim 9, wherein the control signal from the external apparatus is a control signal of a physiological index representing the degree of the deep index of brain activity measured from the living body, and wherein the vibration presenting means (4) is configured to set the deep index of brain activity of the living body within a predetermined range.

11. The vibration processing apparatus as claimed in claim 9, wherein the control signal from the external apparatus is a control signal representing a number of living bodies in a predetermined area, and wherein the vibration presenting means (4) is configured to present the vibration for setting a degree of decrease or a degree of increase of the deep index of brain activity of the living body to a predetermined value so as to adjust a number of living bodies in the area within a predetermined range.

12. The vibration processing apparatus as claimed in claim 9, wherein the control signal from the external apparatus is a control signal representing a number of living bodies in a plurality of predetermined areas, and wherein the vibration presenting means (4) is configured to present the vibration for setting a degree of decrease or a degree of increase of the deep index of brain activity level of the living body to a predetermined value such that respective numbers of living bodies in the areas have a predetermined ratio of them.

## Patentansprüche

1. Vorrichtung zum Verarbeiten von Schwingungen, eine Erzeugungseinrichtung (1, 11, 21) umfassend, die konfiguriert ist zum Erzeugen eines Schwingungs- oder Oszillationssignals, Folgendes einschließend:
ein erstes Frequenzband, das einen Frequenzbereich von weniger als 16 kHz aufweist und Schwingungskomponenten einschließt, die von einem Hörsystem eines lebenden Körpers als Schall wahrgenommen werden,
ein zweites Frequenzband, das einen Frequenzbereich von 16 kHz bis 32 kHz aufweist und Schwingungskomponenten einschließt, die eine Wirkung zur Verringerung der Gehirnaktivität aufweisen, und
ein drittes Frequenzband, das einen Frequenzbereich von mehr als 32 kHz aufweist, wobei
die Vorrichtung zum Verarbeiten von Schwingungen ferner Schwingungsdämpfungsvorrichtungen umfasst, die konfiguriert sind, um das Schwingungs- oder Oszillationssignal von der Erzeugungseinrichtung (1, 11, 21) zu empfangen, **dadurch gekennzeichnet, dass**
die Schwingungsdämpfungsvorrichtungen konfiguriert sind, um nur das zweite Band von dem Schwingungs- oder Oszillationssignal zu entfernen oder zu dämpfen, durch Bandfilterung von nur dem zweiten Band von dem Schwingungs- oder Oszillationssignal, so dass die negative Auswirkung der Verringerung der Gehirnaktivität eines lebenden Körpers vermindert wird, und
ferner umfassend Schwingungsdarstellungsmittel (4), die konfiguriert sind, um die durch die Erzeugungseinrichtung (1, 11, 21) erzielte Schwingungen auf den lebenden Körper anzuwenden.

2. Vorrichtung zum Verarbeiten von Schwingungen nach Anspruch 1, wobei die Schwingungsdarstellungsmittel (4) konfiguriert sind, um eine Schwingung des zweiten Frequenzbandes und / oder eine Schwingung des dritten Frequenzbands auf den lebenden Körper anzuwenden, zusammen mit einer Schwingung des ersten Frequenzbandes, um die Schwingungen zum Einstellen eines Umfangs der Senkung oder eines Umfangs der Steigerung des Tiefenindex des Gehirnaktivitätspegels des lebenden Körpers auf einen vorbestimmten Wert darzustellen.

3. Vorrichtung zum Verarbeiten von Schwingungen nach Anspruch 1, wobei die Schwingungsdarstellungsmittel (4) konfiguriert sind, um eine Intensität von den Schwingungen des zweiten Frequenzbandes, die auf den lebenden Körper angewendet werden, und / oder den Schwingungen des dritten Frequenzbandes, die auf den lebenden Körper angewendet werden, zu ändern, um die Schwingungen zum Einstellen des Umfangs der Senkung oder des Umfangs der Steigerung des Tiefenindex des Gehirnaktivitätspegels des lebenden Körpers auf einen vorbestimmten Wert darzustellen.

4. Vorrichtung zum Verarbeiten von Schwingungen nach Anspruch 1, wobei die Schwingungsdarstellungsmittel (4) konfiguriert sind, um die Schwingungen des zweiten Frequenzbandes zusammen mit den Schwingungen des ersten Frequenzbandes auf den lebenden Körper anzuwenden, und danach die Schwingungen des dritten Frequenzbandes zusammen mit den Schwingungen des ersten Frequenzbandes auf den lebenden Körper anzuwenden.

5. Vorrichtung zum Verarbeiten von Schwingungen nach Anspruch 1, wobei die Schwingungsdarstellungsmittel (4) konfiguriert sind, um die Schwingungen des dritten Frequenzbandes zusammen mit den Schwingungen des ersten Frequenzbandes auf den lebenden Körper anzuwenden, und danach die Schwingungen des zweiten Frequenzbandes zusammen mit den Schwingungen des ersten Frequenzbandes auf den lebenden Körper anzuwenden.

6. Vorrichtung zum Verarbeiten von Schwingungen nach einem der Ansprüche 1 bis 5, ferner umfassend eine Signalverarbeitungsvorrichtung (642), die konfiguriert ist, um ein Signal, das die Schwingungen des zweiten Frequenzbands darstellt, in ein Signal, das die Schwingungen des dritten Frequenzbandes darstellt, bezüglich der Bandbreite umzuwandeln oder zu erweitern.

7. Vorrichtung zum Verarbeiten von Schwingungen nach einem der Ansprüche 1 bis 5, ferner umfassend: eine Codiervorrichtung (80), die konfiguriert ist, um ein Signal, das die Schwingungen des dritten Frequenzbandes darstellt, in ein die Schwingungen des zweiten Frequenzbandes darstellendes Signal bezüglich der Bandbreite umzuwandeln oder zu komprimieren, und danach ein Signal, das die Schwingungen des ersten Frequenzbandes repräsentiert, zu einem verarbeiteten Signal hinzuzufügen, um ein Signal des Additionsergebnisses als ein Sendesignal auszugeben; und eine Decodervorrichtung, die konfiguriert ist, um das Sendesignal zu empfangen, (a) ein Signal mit dem zweiten Frequenzband in ein Signal mit dem dritten Frequenzband aus dem Sendesignal bezüglich der Bandbreite umzuwandeln, wenn das Sendesignal bezüglich der Bandbreite umgewandelt wird, und (b) ein Signal mit dem zweiten Frequenzband in ein Signal mit dem dritten Frequenzband aus dem Sendesignal bezüglich der Bandbreite zu erweitern, wenn das Sendesignal bezüglich der Bandbreite komprimiert wird, und danach Addieren des Signals mit dem ersten Frequenzband aus dem Sendesignal mit einem verarbeiteten Signal, um ein Signal des Additionsergebnisses auszugeben.

8. Vorrichtung zum Verarbeiten von Schwingungen nach einem der Ansprüche 1 bis 5, ferner Mittel umfassend, die konfiguriert sind, um einen Schwingungspegel von den Schwingungen des zweiten Frequenzbandes und / oder den Schwingungen des dritten Frequenzbandes zu ermitteln.

9. Vorrichtung zum Verarbeiten von Schwingungen nach einem der Ansprüche 1 bis 5, wobei das Schwingungsdarstellungsmittel (4) konfiguriert ist, um die Schwingungen darzustellen, zum Einstellen eines Umfangs der Senkung oder eines Umfangs der Steigerung des Tiefenindex des Gehirnaktivitätspegels des lebenden Körpers auf einen vorbestimmten Wert, basierend auf einem Steuersignal von einer externen Vorrichtung.

10. Vorrichtung zum Verarbeiten von Schwingungen nach Anspruch 9, wobei das Steuersignal von der externen Vorrichtung ein Steuersignal eines physiologischen Indexes ist, der den Grad des Tiefenindex der Gehirnaktivität darstellt, der am lebenden Körper gemessen wird, und wobei das Schwingungsdarstellungsmittel (4) konfiguriert ist, um den Tiefenindex der Gehirnaktivität des lebenden Körpers innerhalb eines vorbestimmten Bereichs einzustellen.

11. Vorrichtung zum Verarbeiten von Schwingungen nach Anspruch 9, wobei das Steuersignal von der externen Vorrichtung ein Steuersignal ist, das eine Anzahl von lebenden Körpern in einem vorbestimmten Bereich darstellt, und wobei das Schwingungsdarstellungsmittel (4) konfiguriert ist, um die Schwingungen darzustellen, zum Einstellen eines Umfangs der Senkung oder eines Umfangs der Steigerung des Tiefenindex der Gehirnaktivität des lebenden Körpers auf einen vorbestimmten Wert, um eine Anzahl von lebenden Körpern in dem Bereich innerhalb eines vorbestimmten Umfangs einzustellen.

12. Vorrichtung zum Verarbeiten von Schwingungen nach Anspruch 9, wobei das Steuersignal von der externen Vorrichtung ein Steuersignal ist, das eine Anzahl von lebenden Körpern in einer Mehrzahl von vorbestimmten Bereichen darstellt, und wobei das Schwingungsdarstellungsmittel (4) konfiguriert ist, um die Schwingungen darzustellen, zum Einstellen eines Umfangs der Senkung oder eines Umfangs der Steigerung des Tiefenindex der Gehirnaktivität des lebenden Körpers auf einen vorbestimmten Wert, so dass die jeweilige Anzahl von lebenden Körpern in den Bereichen ein vorbestimmtes Verhältnis von ihnen aufweist.

## Revendications

1. Appareil de traitement de vibration comprenant des moyens de génération (1, 11, 21) configurés pour générer une vibration ou un signal d'oscillation, qui comporte
une première bande de fréquence ayant une plage de fréquences inférieure à 16 kHz et comportant des composantes de vibration perçues sous forme de son par un système auditif d'un corps vivant,
une deuxième bande de fréquence ayant une plage de fréquences de 16 kHz à 32 kHz, comportant des composantes de vibration présentant un effet d'abaissement d'activité cérébrale, et
une troisième bande de fréquence ayant une plage de fréquences supérieure à 32 kHz, dans lequel
l'appareil de traitement de vibration comprend en outre des appareils atténuateurs de vibration configurés pour recevoir la vibration ou le signal d'oscillation en provenance des moyens de génération (1, 11, 21), **caractérisé en ce que**
les appareils atténuateurs de vibration sont configurés pour éliminer ou atténuer uniquement la deuxième bande de la vibration ou du signal d'oscillation par filtrage de bande uniquement de la deuxième bande de la vibration ou du signal d'oscillation, de sorte que l'effet négatif de diminution de l'activité cérébrale d'un corps vivant soit réduit, et
comprenant en outre des moyens de présentation de vibration (4) configurés pour appliquer la vibration obtenue par les moyens de génération (1, 11, 21) au corps vivant.

2. Appareil de traitement de vibration selon la revendication 1, dans lequel les moyens de présentation de vibration (4) sont configurés pour appliquer au moins l'une d'une vibration de la deuxième bande de fréquence et d'une vibration de la troisième bande de fréquence au corps vivant conjointement à une vibration de la première bande de fréquence, pour présenter les vibrations afin de régler un degré de diminution ou un degré d'augmentation de l'indice de profondeur de niveau d'activité cérébrale du corps vivant à une valeur prédéterminée.

3. Appareil de traitement de vibration selon la revendication 1, dans lequel les moyens de présentation de vibration (4) sont configurés pour changer une intensité d'au moins l'une de la vibration de la deuxième bande de fréquence appliquée au corps vivant et de la vibration de la troisième bande de fréquence appliquée au corps vivant, pour présenter les vibrations afin de fixer un degré de diminution ou un degré d'augmentation de l'indice de profondeur de niveau d'activité cérébrale du corps vivant à une valeur prédéterminée.

4. Appareil de traitement de vibration selon la revendication 1, dans lequel les moyens de présentation de vibration (4) sont configurés pour appliquer la vibration de la deuxième bande de fréquence au corps vivant conjointement à la vibration de la première bande de fréquence, et ensuite, appliquer la vibration de la troisième bande de fréquence au corps vivant conjointement à la vibration de la première bande de fréquence.

5. Appareil de traitement de vibration selon la revendication 1, dans lequel les moyens de présentation de vibration (4) sont configurés pour appliquer la vibration de la troisième bande de fréquence au corps vivant conjointement à la vibration de la première bande de fréquence, et ensuite, appliquer la vibration de la deuxième bande de fréquence au corps vivant conjointement à la vibration de la première bande de fréquence.

6. Appareil de traitement de vibration selon l'une quelconque des revendications 1 à 5, comprenant en outre un appareil de traitement de signal (642) configuré pour convertir en bande ou agrandir en bande un signal représentant la vibration de la deuxième bande de fréquence en un signal représentant la vibration de la troisième bande de fréquence.

7. Appareil de traitement de vibration selon l'une quelconque des revendications 1 à 5, comprenant en outre : un appareil encodeur (80) configuré pour convertir en bande ou comprimer en bande un signal représentant la vibration de la troisième bande de fréquence en un signal représentant la vibration de la deuxième bande de fréquence, et ensuite, additionner un signal représentant la vibration de la première bande de fréquence à un signal traité pour fournir en sortie un signal de résultat d'addition en tant que signal de transmission ; et un appareil décodeur configuré pour recevoir le signal de transmission, (a) convertir en bande un signal ayant la deuxième bande de fréquence en un signal ayant la troisième bande de fréquence parmi le signal de transmission lorsque le signal de transmission a subi une conversion de bande, et (b) agrandir en bande un signal ayant la deuxième bande de fréquence en un signal ayant la troisième bande de fréquence parmi le signal de transmission lorsque le signal de transmission a subi une compression de bande, et ensuite, additionner le signal ayant la première bande de fréquence parmi le signal de transmission à un signal traité pour fournir en sortie un signal de résultat d'addition.

8. Appareil de traitement de vibration selon l'une quelconque des revendications 1 à 5, comprenant en outre des moyens configurés pour détecter un niveau de vibration d'au moins l'une de la vibration de la deuxième bande de fréquence et de la vibration de la troisième bande de fréquence.

9. Appareil de traitement de vibration selon l'une quelconque des revendications 1 à 5, dans lequel le moyen de présentation de vibration (4) est configuré pour présenter la vibration afin de fixer un degré de diminution ou un degré d'augmentation de l'indice de profondeur de niveau d'activité cérébrale du corps vivant à une valeur prédéterminée, d'après un signal de commande provenant d'un appareil externe.

10. Appareil de traitement de vibration selon la revendication 9, dans lequel le signal de commande provenant de l'appareil externe est un signal de commande d'un indice physiologique représentant le degré de l'indice de profondeur d'activité cérébrale mesuré à partir du corps vivant, et dans lequel le moyen de présentation de vibration (4) est configuré pour fixer l'indice de profondeur d'activité cérébrale du corps vivant dans une plage prédéterminée.

11. Appareil de traitement de vibration selon la revendication 9, dans lequel le signal de commande provenant de l'appareil externe est un signal de commande représentant un nombre de corps vivants dans une zone prédéterminée, et dans lequel le moyen de présentation de vibration (4) est configuré pour présenter la vibration afin de fixer un degré de diminution ou un degré d'augmentation de l'indice de profondeur d'activité cérébrale du corps vivant à une valeur prédéterminée de façon à ajuster un nombre de corps vivants dans la zone dans une plage prédéterminée.

12. Appareil de traitement de vibration selon la revendication 9, dans lequel le signal de commande provenant de l'appareil externe est un signal de commande représentant un nombre de corps vivants dans une pluralité de zones prédéterminées, et dans lequel le moyen de présentation de vibration (4) est configuré pour présenter la vibration afin de fixer un degré de diminution ou un degré d'augmentation de l'indice de profondeur de niveau d'activité cérébrale du corps vivant à une valeur prédéterminée de sorte que des nombres respectifs de corps vivants dans les zones aient un rapport prédéterminé entre eux.
